# EUROPEAN PATENT APPLICATION

(11) **EP 1 275 717 A1**
(43) Date of publication of application: **15.01.2003**
(21) Application number: 01917591.8
(22) Date of filing: 29.03.2001
(51) Int. Cl.: C12N 15/12, C12Q 1/68, C07K 14/47

(54) **PROLIFERATIVE GLOMERULAR NEPHRITIS-ASSOCIATED GENE**

(30) Priority: 29.03.2000 JP 2000090137
(71) Applicant: KYOWA HAKKO KOGYO CO., LTD., Chiyoda-ku, Tokyo 100-8185 (JP)
(72) Inventor: TAKEUCHI, Kyoko Tokyo Research Laboratories, Machida-shi Tokyo 194-8533 (JP); SEKINE, Susumu Tokyo Research Laboratories, Machida-shi Tokyo 194-8533 (JP); KIKUCHI, Y. Head Office Kyowa Hakko Kogyo Co.,Ltd., Tokyo 100-8185 (JP); SAKURADA, Kazuhiro Tokyo Research Laboratories, Machida-shi Tokyo 194-8533 (JP)
(74) Representative: VOSSIUS & PARTNER
(86) International application number: JP0102623
(87) International publication number: WO01073022

(57) **Abstract**

A gene which is useful in searching for a therapeutic agent which restores tissue that suffered damage in a renal disease, a polypeptide encoded by the gene and an antibody which recognizes the polypeptide, are provided.

A gene whose expression level changes depending on progression and recovery of the pathology in a proliferative glomerulonephritis model animal is obtained, and a polypeptide encoded by the gene and an antibody which recognizes the polypeptide are produced. These gene, polypeptide and antibody can be used in the search for an agent for restoring kidney tissue that suffered damage and an agent for diagnosing kidney disorder.

## Description

### TECHNICAL FIELD

The present invention relates to complementary DNA (cDNA) for mRNA obtained by using a subtraction method and differential hybridization method based on mRNA whose expression level increases at a recovery period of proliferative glomerulonephritis, and a polypeptide encoded by the cDNA. The present invention further relates to an antibody against the polypeptide, a method of detecting the polypeptide and the DNA, and a diagnostic agent and therapeutic agent for renal disease which comprise the DNA, polypeptide, antibody or the like.

### BACKGROUND ART

The kidney has a high reserve function, and in many cases even when the remaining functions are half the normal functions, symptoms due to functional disorder are not observed. Damage of nephron composed of highly differentiated cell groups is irreversible, and degeneration of tissue structure beginning in glomerulosclerosis is accompanied by tubular disorder and stromal fibrosis and ultimately results in a serious condition of renal failure which requires kidney dialysis. It is generally thought that this process is not related to the type of the primary disease, and is roughly common among the diseases. In clinical practice, administration of steroid agents, oral absorbents, antihypertensive agents, ACE inhibitors and the like, low protein diet treatment method and the like are employed for the main purpose of lightening the burden on remaining nephron and extending the period until the introduction of dialysis. However, there are many unknown points regarding the mechanism of onset and progress of renal failure, and a method for basic remedy has not been established.

In proliferative glomerulonephritis occurring in children and some animal models, it is known that natural healing occurs without progressing towards continuous decreasing of renal functions after the glomerulus or renal tubule is damaged, however, the mechanism of this natural healing is also not clarified. Analysis at the molecular level of the pathologic progression of proliferative glomerulonephritis or of the mechanism of natural healing is considered to be important for the diagnosis of renal disease and the development of therapeutic agents. An effective means for this purpose is, for example, comprehensively obtaining and analyzing a group of genes whose expression level changes in accordance with the progress of a renal disease and the recovery therefrom. It is not practical to conduct such an analysis by using tissue of an actual renal disease patient in respect of the obtainment of tissue and the non-uniformity of symptoms among patients. It is considered that by using a suitable model animal of proliferative glomerulonephritis, obtainment of comprehensive group of genes and analysis at the molecular level can be relatively easily conducted. It is considered that, in principle, genes obtained in this manner include factors which are markers of progression or recovery of pathology, as well as factors which actively promote recovery.

Several experimental models are known as a model animal of nephritis, which are mainly rats. Among these model, as for Thy-1 nephritis model [Laboratory Investigation, 55, 680 (1986)] obtained by intravenous injection into rat of an antibody (anti-Thy-1 antibody) against Thy-1.1 antigen which is present as a membrane protein of a mesangial cell (hereinafter, this nephritis model rat is referred to as "Thy-1 nephritis rat"), a considerable amount of analysis in the progression of pathology and the pathological findings has been conducted. In the Thy-1 nephritis rat, after mesangiolysis, renal tubular damage accompanying inflammatory cell infiltration to the stromata is observed, and then proliferation of mesangial cells and production of extracellular matrix occurs. It is known that reconstitution of damaged tissue takes place thereafter, and spontaneous recovery is got in approximately days beyond 2 weeks. Therefore, the Thy-1 nephritis rat is considered to be suitable as a model of progression of symptoms of proliferative glomerulonephritis and spontaneous recovery therefrom.

There have already been several reports of analysis at the molecular level of the kidney condition of the Thy-1 nephritis rat, for example, analyses regarding genes whose expression level changes depending on the kidney condition. Examples include reports regarding genes of growth factors such as TGF- β, which is considered to be involved in proliferation of mesangial cells [J. Clin. Invest, 86 453 (1990)), heparin binding EGF-like growth factor [Experimental Nephrology, 4, 271 (1996)], PDGF [Proc. Natl. Acad. Sci. USA, 88, 6560 (1991)], and FGF [J. Clin. Invest., 90, 2362 (1992)], or regarding genes such as polypeptides relating to extracellular matrix, such as type IV collagen [Kidney International, 86, 453 (1990)], laminin [Kidney International, 86, 453 (1990)], tenascin [Experimental Nephrology, 5, 423 (1997)], profilin, and CD44 [J. American Society of Nephrology, 7, 1006 (1996)].

Since growth factors such as TGF-β and PDGF in glomerulonephritis show high level of expression even in human glomerulonephritis such as lupus nephritis or IgA nephropathy as in experimental animals, these factors are considered to work as mediators of kidney failure progression through proliferation response of mesangial cells and the like, extracellular matrix production stimulation and the like [Pediatric Nephrology, 9, 495 (1995)]. It is reported that administration of PDGF neutralizing antibody [J. Exp. Med., 175, 1413 (1992)] or an inhibitor of TGF-β, decholin [Nat. Med., 2, 418 (1996)] is effective in Thy-1 nephritis rat, but, the effect of these factors has not been verified at the clinical level.

Development of a kidney protective agent by using factors involved in kidney development has been studied by Creative BioMolecules Inc. (U.S.A.), and OP-1 (BMP7) polypeptide has been provided. This is a factor belonging to BMP subfamily within TGF-β superfamily, which factor was first discovered as a factor which induces ectopic ossification [EMBO J., 9, 2085 (1990)]. This factor is expressed in mesenchymal cells surrounding the ureteric bud at a time of nephrogenesis in the fetal period, and is an important factor for interaction between the epithelium and mesenchyme. Further, it has recently been reported that this factor enables proliferation and differentiation of nephrogenic tissue by suppressing apoptosis in mesenchymal cells [Genes & Dev., 13, 1601 (1999)].

Results of a test in which the recombinant OP-1 protein of Creative BioMolecules Inc. was administered to model animals of chronic renal failure was reported at the annual convention of the American Society of Nephrology [Nikkei Biotech, November 10, 1999].

In that preclinical test which was conducted by the Washington University School of Medicine (U.S.), chronic renal failure model rats in which unilateral ureter occlusion was induced were used. This model shows progressive fibrillation and renal damage which are very similar to the cicatrisation as observed in chronic renal failure patients. The results of the test indicate that OP-1 suppresses the formation of cicatricial tissue in the kidney and decreases the tubular damage. Further, in the group administered with OP-1, approximately 30% of the filtration function of a normal kidney and 65% of the blood flow amount of a kidney at normal times were maintained, showing that OP-1 also has a kidney function protection effect. In the groups administered with placebo or ACE inhibitor, filtration function and blood flow could not be determined. Tubulointerstitial lesion is more closely correlated with a decrease in kidney function than glomerular lesion, and is also a representative tissue lesion from which prognosis of a disease is predictable. Preservation of renal tubular structure was observed in only the OP-1-administered group. While the mechanism of tissue protection in the kidney is currently still not clear, the foregoing results suggest that cell groups that are the target of OP-1 are present even in the adult kidney, as in the nascent mesenchymal cells. However, various sites of action other than the kidney also exist for OP-1, and in particular, chondrogenic activity is thought to be one of the serious side effects of OP-1, and thus its clinical application is difficult to achieve. Accordingly, there is a need to find a factor, which is expressed specifically in the kidney, and capable of inducing OP-1 or controling the regenerative function of the kidney downstream of the OP-1.

Autotaxin is known as a molecule related to cell differentiation and migration, which is induced in mesenchymal cells by factors belonging to BMP or TGF-β families such as OP-1. Autotaxin has been separated and cloned from culture supernatant of human melanoma cell strain as a cytokine having cancer cell migration activity [J. Biol. Chem., 267, 683 (1996)].

Thereafter, it has been showed that autotaxin is, in the nascent period, expressed in mesenchymal cells which are being under differentiation to bone cells and cartilage cells [Mechanisms of Dev., 84, 121 (1999)], and also that the expression is increased by stimulating the precursor cells of the bone and cartilage with BMP2 [Dev. Dynam., 213, 398 (1998)]. Autotaxin is classified as PC-1 family because of the structural homology. PC-1 family is composed of 3 kinds of type II membrane proteins; PC-1, PD-1α (autotaxin) and PD-1β (B10), each having activity of phosphodiesterase I; EC 3.1.4.1 / nucleotide pyrophosphatase; EC 3.6.1.9 extracellularly, and also having autophosphorylation ability. Such findings suggest that PC-1 family is expressed by stimulation of a factor belonging to BMP family, and plays an important role in cell migration, differentiation or intercellular interaction.

### DISCLOSURE OF THE INVENTION

The object of the present invention is to obtain a gene (proliferative glomerulonephritis-related gene) whose expression level changes depending on the pathologic recovery in proliferative glomerulonephritis animals, and to provide a polypeptide which is useful in searching for a therapeutic agent which restores tissue that suffered damage in a renal disease, DNA encoding the polypeptide, and an antibody which recognizes the polypeptide, as well as a method for using the same.

### (A means for solving the object)

As a result of thorough studies to address the above-mentioned problems, the present inventors have completed the present invention.

Specifically, the present invention provides the following items (1) to (50).
(1) DNA encoding a polypeptide having an amino acid sequence selected from the group consisting of the amino acid sequences shown by SEQ ID NOs: 2,4 and 6.
(2) DNA having a nucleotide sequence selected from the group consisting of the nucleotide sequences shown by SEQ ID NOs: 1, 3 and 5.
(3) DNA which hybridizes under stringent conditions with DNA having a nucleotide sequence selected from the group consisting of the nucleotide sequences shown by SEQ ID NOs: 1, 3 and 5, and is capable of detecting a gene whose expression level increases in tissue affected by onset of proliferative glomerulonephritis
(4) DNA having a sequence which is the same as consecutive 5 to 60 nucleotides within a nucleotide sequence selected from the group consisting of the nucleotide sequences shown by SEQ ID NOs: 1, 3 and 5.
(5) DNA having a sequence complementary with the DNA having a nucleotide sequence selected from the group consisting of the nucleotide sequences shown by SEQ ID NOs: 1, 3 and 5.
(6) A method of detecting mRNA of a gene whose expression level increases in tissue affected by onset of proliferative glomerulonephritis by using the DNA according to any one of (1) to (5) above
(7) A diagnostic agent for renal disease, which comprises the DNA according to any one of (1) to (5) above.
(8) A method of detecting a causative gene of renal disease by using the DNA according to any one of (1) to (5) above.
(9) A method of screening a substance which suppresses or promotes transcription or translation of a gene whose expression level increases in tissue affected by onset of proliferative glomerulonephritis, by using the DNA according to any one of (1) to (5) above.
(10) A method of screening a therapeutic agent for renal disease by using the DNA according to any one of (1) to (5) above.
(11) A therapeutic agent for renal disease, which comprises the DNA according to any one of (1) to (5) above.
(12) A recombinant vector which comprises the DNA according to any one of (1) to (5) above.
(13) A recombinant vector which comprises RNA of a sequence which is homologous to a sense strand of the DNA according to any one of (1) to (5) above.
(14) The vector according to (12) or (13) above, wherein the recombinant vector is a virus vector.
(15) A therapeutic agent for renal disease, which comprises the recombinant vectors according to any one of (12) to (14) above.
(16) A method of detecting mRNA of a gene whose expression level increases in tissue affected by onset of proliferative glomerulonephritis by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(17) A diagnostic agent for renal disease, which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(18) A method of detecting a causative gene of renal disease by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(19) A method of screening a substance which suppresses or promotes transcription or translation of a gene whose expression level increases in tissue affected by onset of proliferative glomerulonephritis, by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(20) A method of screening a therapeutic agent for renal disease by using DNA having a nucleotide sequence selected from the group consisting of the nucleotide sequences shown by SEQ ID NO: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(21) A therapeutic agent for renal disease, which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(22) A recombinant vector which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(23) A recombinant vector which comprises RNA having a sequence which is homologous to a sense strand of DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.
(24) The vector according to (22) or (23) above, wherein the recombinant vector is a virus vector.
(25) A therapeutic agent for renal disease which comprises the recombinant vector according to any one of (22) to (24) above.
(26) A polypeptide encoded by the DNA according to (1) or (2) above.
(27) A polypeptide having an amino acid sequence selected from the group consisting of the amino acid sequences shown by SEQ ID NOs: 2, 4, and 6.
(28) A polypeptide having an amino acid sequence wherein one or more amino acids are deleted, substituted or added in the amino acid sequence of the polypeptide according to (26) or (27) above, and having activity involved in restoration of a kidney which suffered damage.
(29) A recombinant DNA obtained by incorporating DNA encoding the polypeptides according to any of (26) to (28) above into a vector.
(30) A transformant which is obtained by introducing the recombinant DNA according to (29) above into a host cell.
(31) A method of preparing a polypeptide wherein the transformant according to (30) above is cultured in a medium, the polypeptide according to any one of (26) to (28) above is produced and accumulated in the culture, and the polypeptide is collected from the culture.
(32) A method of screening a therapeutic agent for renal disease by using the culture which is obtained by culturing the transformant according to (30) above in a medium.
(33) A method of screening a therapeutic agent for renal disease by using the polypeptide according to any one of (26) to (28) above.
(34) A therapeutic agent for renal disease, which comprises the polypeptide according to any one of (26) to (28) above.
(35) An antibody which recognizes the polypeptide according to any one of (26) to (28) above.
(36) A method of immunologically detecting the polypeptides according to any one of (26) to (28) above by using the antibody according to (35) above.
(37) A method of screening a therapeutic agent for renal disease by using the antibody according to (35) above.
(38) A method of screening a substance which suppresses or promotes transcription or translation of a gene whose expression level increases in tissue affected by onset of proliferative glomerulonephritis by using the antibody according to (35) above.
(39) A diagnostic agent for renal disease, which comprises the antibody according to (35) above.
(40) A therapeutic agent for renal disease, which comprises the antibody according to (35) above.
(41) A method of drug delivery wherein a fusion antibody which is obtained by binding the antibody according to (35) above and an agent selected from a radioisotope, a polypeptide and a low molecular weight compound is led to a site of kidney damage.
(42) A recombinant DNA which is obtained by incorporating into a vector, DNA encoding a polypeptide having an amino acid sequence selected from the group consisting of the amino acid sequences shown by SEQ ID NOs: 8, 10, 12, 14, 16, 158 and 160.
(43) A method of screening a therapeutic agent for renal disease by using a culture which is obtained by culturing in a medium a transformant obtained by introducing the recombinant DNA according to (42) above into a host cell.
(44) A method of screening a therapeutic agent for renal disease by using a polypeptide having an amino acid sequence selected from the group consisting of the amino acid sequences shown by SEQ ID NOs: 8, 10, 12, 14, 16, 158 and 160.
(45) A therapeutic agent for renal disease, which comprises a polypeptide having an amino acid sequence selected from the group consisting of the amino acid sequences shown by SEQ ID NOs: 8, 10, 12, 14, 16, 158 and 160.
(46) A method of screening a therapeutic agent for renal disease by using an antibody which recognizes a polypeptide having an amino acid sequence selected from the group consisting of the amino acid sequences shown by SEQ ID NOs: 8, 10, 12, 14, 16, 158 and 160.
(47) A method for screening a substance which suppresses or promotes transcription or translation of a gene whose expression level increases in tissue affected by onset of proliferative glomerulonephritis, by using an antibody which recognizes a polypeptide having an amino acid sequence selected from the group consisting of the amino acid sequences shown by SEQ ID NOs: 8, 10, 12, 14, 16, 158 and 160.
(48) A diagnostic agent for renal disease, which comprises an antibody which recognizes a polypeptide having an amino acid sequence selected from the group consisting of the amino acid sequences shown by SEQ ID NOs: 8, 10, 12, 14, 16, 158 and 160.
(49) A therapeutic agent for renal disease, which comprises an antibody which recognizes a polypeptide having an amino acid sequence selected from the group consisting of the amino acid sequences shown by SEQ ID NOs: 8, 10, 12, 14, 16, 158 and 160.
(50) A method of drug delivery wherein a fusion antibody obtained by binding the antibody which recognizes a polypeptide having an amino acid sequence selected from the group consisting of the amino acid sequences shown by SEQ ID NOs: 8, 10, 12, 14, 16, 158 and 160 and an agent selected from a radioisotope, a polypeptide or a low molecular weight compound, is led to a site of kidney damage.

The DNA of the present invention is DNA of a gene whose expression level increases in tissue affected by onset of proliferative glomerulonephritis. Examples thereof include DNA encoding a polypeptide having an amino acid sequence selected from the group consisting of the amino acid sequences shown by SEQ ID NO: 2, 4 and 6, DNA having a nucleotide sequence selected from the group consisting of the nucleotide sequences shown by SEQ ID NO: 1, 3 and 5, and DNA which can hybridize under stringent conditions with said DNA and can detect a gene whose expression level changes in tissue affected by onset of proliferative glomerulonephritis.

The above-described DNA which hybridizes under stringent conditions with a nucleotide sequence selected from the group consisting of the nucleotide sequences shown by SEQ ID NO: 1, 3 and 5 means DNA obtained by using a colony hybridization method, a plaque hybridization method, a southern blotting hybridization method or the like where DNA having the nucleotide sequence shown by SEQ ID NO: 1, 3 or 5 is employed as a probe. Specifically, it means DNA which can be identified by using a filter on which colony-derived or plaque-derived DNA is immobilized, performing hybridization at 65°C in the presence of 0.7 to 1.0 mol/l of sodium chloride and then washing the filter under a condition of 65°C using 0.1-2-fold SSC solution (1-fold SSC solution comprises 150 mmol/l of sodium chloride and 15 mmol/l of sodium citrate). Hybridization can be performed in accordance with methods described in Molecular Cloning, A Laboratory Manual, Second Edition, Cold Spring Harbor Laboratory Press (1989) (hereinafter abbreviated to "Molecular Cloning Second Edition"); Current Protocols in Molecular Biology, John Wiley & Sons (1987-1997) (hereinafter abbreviated to "Current Protocols in Molecular Biology"); DNA Cloning 1: Core Techniques, A Practical Approach, Second Edition, Oxford University (1995); and the like. Examples of DNA which can hybridize include DNA having at least 60% homology with a nucleotide sequence shown by SEQ ID NO: 1, 3 or 5, preferably DNA having at least 80% homology, and more preferably DNA having at least 95% homology, when calculated using BLAST (J. Mol. Biol., 215, 403 (1990)), FASTA (Methods in Enzymology, 183, 63-98 (1990) and the like.

Further, DNA of the present invention also includes an oligonucleotide having partial sequence of the above-described DNA of the present invention, and an anti-sense oligonucleotide. Examples of the oligonucleotide include an oligonucleotide having the same sequence, for example as consecutive 5 to 60 nucleotides, preferably consecutive 10 to 40 nucleotides, within the nucleotide sequence selected from the nucleotide sequences shown by SEQ ID NOs: 1, 3 and 5. Examples of an anti-sense oligonucleotide include an anti-sense oligonucleotide of the above oligonucleotide.

Examples of the polypeptide of the present invention include a polypeptide (renal restoration factor) having an activity related with restoration of a kidney which suffered damage (renal restoration activity). Specific examples thereof include a polypeptide having an amino acid sequence selected from the amino acid sequences shown by SEQ ID NOs: 2, 4 and 6, or a polypeptide having an amino acid sequence derived from the amino acid sequence of the aforementioned polypeptide by deletion, substitution, or addition of one or more amino acids, and having renal restoration activity.

A protein having an amino acid sequence wherein one or more amino acids are deleted, substituted or added in the amino acid sequence shown by SEQ ID NO: 2, 4 or 6, and having activity as a growth factor of the protein, can be obtained, for example, by introducing site-directed mutation into DNA encoding a polypeptide having an amino acid sequence shown by SEQ ID NO: 2, 4 or 6 using a method of site-directed mutagenesis as described in Molecular Cloning Second Edition, Current Protocols in Molecular Biology, Nucleic Acids Research, 10, 6487 (1982), Proc. Natl. Acad. Sci. USA, 79, 6409 (1982), Gene, 34, 315 (1985), Nucleic Acids Research, 13, 4431(1985), Proc. Natl. Acad. Sci. USA, 82, 488 (1985), or the like.

The number of the amino acids which are deleted, substituted or added is not particularly limited, but it may be the number which is deleted, substituted or added according to a known method such as site-directed mutagenesis as described above, and may be from 1 to several dozen amino acids, preferably 1 to 20, more preferably 1 to 10, and further preferably 1 to 5.

Further, in order for the polypeptide of the present invention to have an activity related with restoration of a kidney which suffered damage, the polypeptide preferably has at least 60% or more, normally 80% or more, and particularly preferably 95% or more of homology with an amino acid sequence shown by SEQ ID NO: 2, 4 or 6, when calculated by using BLAST or FASTA or the like.

The present invention is described in detail below.

### 1. preparation of Proliferative Glomerulonephritis-Related Gene

### (1) Production of Thy-1 Nephritis Rat

Thy-1 nephritis rat which is a model of mesangial proliferative glomerulonephritis is produced as follows in accordance with literature [Laboratory Investigation, 55, 680 (1986)]. By intravenously injecting an anti-Thy-1.1 antibody which is present as a membrane protein of mesangial cell of rat, into experimental rats such as Wistar rats at a dose of 1 mg/kg, mesangilysis occurs, and hyperplasia of mesangial stromata and mesangial cell proliferation are induced, thus enabling production of Thy-1 nephritis rat. Mesangiolysis can be detected by means of urinary protein and albumin.

### (2) Preparation of Thy-1 Nephritis Rat Kidney Subtracted cDNA Library and Selection of cDNA from Library by Differential Hybridization

As proliferative glomerulonephritis-related DNA, cDNA of a gene whose expression level increases in Thy-1 nephritis rat kidney as compared with normal rat kidney is prepared as follows. First, a Thy-1 nephritis rat kidney cDNA library which was subjected to subtraction using normal rat kidney mRNA is prepared, and thereby a glomerulonephritis-related cDNA clone is concentrated. This cDNA can be obtained by performing again differential hybridization using Thy-1 nephritis rat kidney RNA and normal rat kidney RNA as probes respectively with regard to the cDNA clone in the obtained subtracted cDNA library, and selecting cDNA clone whose expression level increases in Thy-1 nephritis rat kidney.

### (2)-1 Preparation of Thy-1 Nephritis Rat Kidney Subtracted cDNA library

"Subtraction" means a method of selecting cDNA of a gene whose expression level is increased as compared with a control rat by preparing single strand cDNA from mRNA extracted from tissue or cells of a certain condition and hybridizing it with mRNA of cells of a control rat, and removing only the cDNA which hybridized to the mRNA.

There are several methods of preparing a subtracted cDNA library. The method used in the present invention is one in which subtraction is performed after preparing a Thy-1 nephritis rat kidney cDNA library by a conventional method and converting it into single strand DNA using helper phage [Proc. Natl. Acad. Sci. USA, 88, 825 (1991)]. Subtraction is performed by a method where the cDNA is hybridized with biotinylated mRNA of normal rat kidney, and streptavidin is further bonded to the hybridized biotinylated mRNA-cDNA complex, and then separation is carried out by phenol extraction.

### (2)-1-A Preparation of Thy-1 Nephritis Rat Kidney cDNA Library

It is considered that the nephritic symptoms of Thy-1 nephritis rat differ depending on the number of days after intravenous injection, and that the expressing genes differ depending on the phases from deterioration of nephritis symptoms to natural healing. Therefore, kidney is extirpated from rats on each of days 2, 4, 6, 8, and 10 after injection, and RNA is individually extracted from each kidney. Extraction of RNA can be performed by the guanidine thiocyanate-cesium trifluoroacetate method [Methods in Enzymol., 154, 3 (1987)] or the acid guanidine thiocyanate/phenol/chloroform method [Analytical Biochemistry, 162, 156 (1987)] or by a method using a kit such as Fast Track mRNA Isolation Kit (Invitrogen). Since polyA is generally added to the 3' end of mRNA, mRNA can be purified from RNA by a method using oligo(dT) Sepharose (Molecular Cloning Second Edition).

Preparation of a cDNA library from mRNA can be preformed with reference to the method described in the manual of the ZAP-c DNA preparation kit manufactured by Stratagene by preparing double strand cDNA using oligo(dT) primer and reverse transcriptase and inserting it into a cloning vector.

A cloning vector should have properties for a general cloning vector, i.e., it should be able to be replicated at a high copy number in Escherichia coli, and have a marker gene for transformation such as ampicillin resistance gene or kanamycin resistance gene, and have a multicloning site capable of cDNA insertion, and also should be such that the conversion into single strand DNA can be easily performed. Accordingly, as a cloning vector, there is used a phagemid vector which contains a replication signal IG (intergenic space) of M13 phage, and can be converted into a single strand DNA phage by means of infection with a helper phage, such as pBluescriptSK(-), pBluescriptII KS(+), pBS(-), pBC(+) [all of the foregoing are manufactured by Stratagene] or pUC118 [manufactured by Takara Shuzo]. Alternatively, there is used λ phage vector which can be converted into phagemid by in vivo excision utilizing a helper phage, such as λ ZAPII or ZAP Express (both manufactured by Stratagene). In vivo excision, the method for conversion into a single strand DNA phage and the method for purifying single strand DNA from phages in culture supernatant, can be conducted according to the manuals attached with the respective commercially available vectors.

As Escherichia coli to which a vector incorporating cDNA is introduced, any Escherichia coli which can express the introduced gene can be used. Examples thereof include Escherichia coli XL1-Blue MRF' [manufactured by Stratagene, Strategies, 5, 81 (1992)], Escherichia coli C600 [Genetics, 39, 440 (1954)], Escherichia coli Y1088 [Science, 222, 778 (1983)], Escherichia coli Y1090 [Science, 222, 778 (1983)], Escherichia coli NM522 [J. Mol. Biol., 166, 1 (1983)], Escherichia coli K802 [J. Mol. Biol., 16, 118 (1966)], and Escherichia coli JM105 [Gene, 38, 275 (1985)].

Hybridization of cDNA with mRNA of a normal rat is used in subtraction. When single strand DNA is made from a phagemid, which of the two strands can be made is determined by the type of phagemid. Therefore, in preparing cDNA library, preparation of cDNA and the insertion direction into the vector are arranged in such a way that an anti-sense strand (strand having a complementary nucleotide sequence to the actual mRNA) can be made as single strand DNA from any cDNA clone. For example, as described in the manual attached with Stratagene's ZAPcDNA synthesis kit, cDNA synthesis can be performed by reverse transcriptase using an oligo(dT) primer having an Xho I site at the 5' end and dNTP, as a substrate, which contains 5-methyl dCTP (which makes Xho I cleavage within cDNA after synthesis impossible) instead of dCTP. EcoR I adaptor is added to both ends of the synthesized cDNA, and the cDNA is cleaved at Xho I and inserted into EcoR I/Xho I site of vector λ ZAPII. Thereby, the EcoR I site side is always the 5' side of the cDNA and the Xho I site side is always the 3' side of the cDNA, and thus the insertion direction to the vector is constant. After this cDNA library is converted into a cDNA library using phagemid vector pBluescript SK(-) as a vector by in vivo excision, helper phage is infected to produce single strand DNA in which the cDNA part is an anti-sense strand.

### (2)-1-B Subtraction using Control Group Rat Kidney mRNA

By infecting the cDNA library of the phagemid vector prepared in (2)-1-A with a helper phage, single strand DNA phage is released in the culture, and cDNA which has become single strand DNA is purified and collected from the culture medium. In the case of λ phage vector, the same procedure is performed after the vector is converted into phagemid by in vivo excision (Molecular Cloning Second Edition).

The specific procedure for subtraction, the compositions of the agents and the reaction conditions can be selected according to a method described in Genes to Cells, 3, 459 (1998). After biotinylating control group rat kidney mRNA prepared in (2)-1-A using Photoprobe biotin [manufactured by Vector Laboratories] or the like, the mRNA is hybridized with the above-described single strand Thy-1 nephritis rat kidney cDNA. After the hydrophobicity is increased by binding streptavidin to the cDNA hybridized with biotinylated mRNA by reacting the solution after hybridization with streptavidin which tightly binds with biotin, phenol is added to perform extraction procedure. cDNA which was not hybridized can be fractionated from the aqueous layer. cDNA hybridized with biotinylated mRNA is extracted into the phenol layer.

### (2)-1-C Reverse Subtraction

In the subtraction operation described in (2)-1-B, not only cDNA of a gene whose expression level is high specifically in Thy-1 nephritis rat kidney, but also cDNA whose expression level is extremely low in both Thy-1 nephritis rat kidney and control rat kidney and whose number of clones is also small, as well as clones of only a vector where cDNA is not inserted, tend to be concentrated. However, such types of cDNA are not suitable for the object of the present invention. Accordingly, in order to select cDNA which present in certain level of clone number in Thy-1 nephritis rat kidney, hybridization and separation are performed on cDNA after subtraction and biotinylated mRNA of Thy-1 nephritis rat kidney in the same manner as in subtraction. In a reverse manner to normal subtraction, cDNA which hybridized with biotinylated mRNA is isolated from cDNA which did not hybridize and fractionated as a phenol layer. After heating the fractionated-hybridized biotinylated mRNA-cDNA after fractionation at 95°C, the cDNA and biotinylated mRNA are dissociated by quenching, and then extracted by adding water, to thereby isolate cDNA hybridized with mRNA in the aqueous layer.

### (2)-1-D Preparation of cDNA Library after Subtraction

After the subtraction and reverse subtraction described in (2)-1-B and (2)-1-C, the cDNA is converted into double strand by using a suitable primer having a nucleotide sequence complementary to the sequence of vector region and DNA polymerase such as BcaBEST (manufactured by Takara Shuzo) or Klenow fragment, and then is introduced into Escherichia coli, and thereby cDNA library can be prepared again. As the method for introduction into Escherichia coli, electroporation having high transformation efficiency is preferred.

### (2)-2 Differential Hybridization

In the subtracted cDNA library prepared in (2)-1, cDNA of a proliferative glomerulonephritis related gene whose expression level increases in Thy-1 nephritis rat kidney is concentrated. However, all the cDNA clones in the library are not necessarily proliferative glomerulonephritis-related genes. To select cDNA of proliferative glomerulonephritis-related genes from these cDNA clones, the respective mRNA levels in normal rat kidney and Thy-1 nephritis rat kidney are compared by northern hybridization (Molecular Cloning Second Edition) in which each cDNA clone is used as a probe or RT-PCR [PCR Protocols, Academic Press (1990)] using a primer based on a nucleotide sequence of a cDNA clone. This enables selection of cDNA of a nephritis-related gene whose expression level actually increases in Thy-1 nephritis rat kidney. Further, by performing the differential hybridization described below, it is possible to comprehensively and efficiently select cDNA clones whose expression level increases.

First, the subtracted cDNA library obtained by the method described in (2)-1 is diluted to such a concentration that individual colonies can be separated, and cultured on agar medium. The separated colonies are individually cultured under the same condition in a liquid culture medium. The culture medium is inoculated in the same amount on 2 nylon membranes, and the membranes are placed on agar medium and cultured under the same conditions to thereby grow colonies of roughly equal amounts on 2 membranes. DNA in the colonies of roughly equal amounts is thus blotted in the nylon membranes. After performing denaturation and neutralization of the DNA by a method described in Molecular Cloning Second Edition, DNA is immobilized on the membranes by ultraviolet irradiation. In the above procedure, it is preferable to separate and culture the individual separated colonies on agar medium in 96-well plates, and inoculate them onto the nylon membranes by using an automatic microdispenser suitable for a 96-well plate such as Hydra96 (manufactured by Robbins Scientific), since two membranes in which equal amounts of DNA derived from many colonies are blotted can be prepared rapidly, and the colonies can be identified easily by comparing the membrane with the original plate. Colony hybridization is performed using the entire mRNA of the Thy-1 nephritis rat kidney as a probe for one of the above membranes, and using the entire mRNA of the normal rat kidney as a probe for the other membrane. Then, by comparing the hybridization signal strengths, a clone whose expression level increases in Thy-1 nephritis rat kidney is selected.

As a probe, while it is possible to use labeled cDNA prepared by using a random primer and reverse transcriptase for the entire mRNA in the same manner as for a normal DNA probe, an RNA probe is desirable since it hybridizes more tightly to DNA on the membrane than DNA probe and imparts a strong signal. For example, by performing cDNA synthesis-reaction by the same method as described in (2)-1-A by using reverse transcriptase and an oligo(dT) primer having at its 5' end an RNA polymerase-specific promoter sequence such as T7, T3 or SP6, cDNA having the promoter sequence at its terminus is synthesized from mRNA. By allowing RNA polymerase specific to the promoter sequence to act on this cDNA using a labeling nucleotide as a substrate, RNA probe which is uniform and contains labeled RNA at high ratio can be easily synthesized in a large amount. As a label of the probe, there can be used a radioisotope such as ³²P or ³⁵S, or a nonradioactive substance which can be easily detected, such as digoxigenin (DIG) or biotin.

After hybridizing the respective RNA probes of Thy-1 nephritis rat kidney and control rat kidney with the membranes prepared as described above, probes hybridized with each DNA colony are detected. In the detection of hybridized probes, methods suitable for the respective labeling substances can be used. Methods which can be used for detecting sensitively or quantitatively include, for example, in the case of a radioisotope, a method using autoradiography where X-ray film or an imaging plate is directly exposed; or in the case of DIG, a method where an anti-DIG antibody labeled with alkaline phosphatase is bound in accordance with instructions in the DIG system users guide (manufactured by Roche), a substrate such as CSPD which emits light by reacting with alkaline phosphatase is reacted, and then X-ray film is exposed.

If a gene which expresses at high level in kidney of a Thy-1 nephritis rat as compared with kidney of a control rat is present, the number of mRNA molecule of the gene present in the probe is also large. Therefore, even though equal amounts of DNA are blotted on a membrane, more probes will bind to a spot of cDNA corresponding to that gene. Therefore, by comparing the strengths of hybridization signals on 2 membranes on which DNA of the same cDNA clone is blotted, cDNA of a gene whose expression level increases in Thy-1 nephritis rat kidney as compared with normal rat kidney can be selected.

### (3) Analysis of Nucleotide Sequence of DNA

With regard to cDNA of a gene whose expression level increases in Thy-1 nephritis rat kidney as compared with normal rat kidney obtained in the manner described above, its nucleotide sequence can be determined by using a dideoxy method [Sanger et al., Proc. Natl. Acad. Sci., USA, 74, 5463 (1977)] or a DNA sequencer.

Examples of cDNA obtained in this manner include DNA having the nucleotide sequence shown by SEQ ID NO: 1, 3, 5, 7, 9, 13, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, or 157.

By translating the obtained nucleotide sequence into an amino acid sequence, the amino acid sequence of a polypeptide encoded by the gene can be obtained. Further, by comparing the obtained nucleotide sequence with nucleotide sequences in nucleotide sequence databases such as GenBank or EMBL using a homology analysis program such as BLAST or FASTA, it is possible to confirm whether or not the obtained nucleotide sequence is a novel nucleotide sequence, and to search for a nucleotide sequence having homology with the obtained nucleotide sequence. In addition, by comparing the amino acid sequence obtained from the nucleotide sequence with the amino acid sequence databases such as SwissProt, PIR, or GenPept, it is possible to search for a polypeptide having homology with a polypeptide encoded by the nucleotide sequence, for example, a polypeptide derived from a corresponding gene in an organsms other than rat, or a family polypeptide which is estimated to have similar activity or functions.

### (4) Preparation of Full-Length cDNA

The cDNA obtained in (2) may include incomplete cDNA which does not encode the full length of a polypeptide, because a part of mRNA is degraded or synthesis by reverse transcriptase is stopped on the way from the 3' end of mRNA to the 5' end. In analysis of a nucleotide sequence of such incomplete cDNA, all the amino acid sequence of a polypeptide encoded by the cDNA cannot be clarified. In analysis of a nucleotide sequence, it is sometimes anticipated from results of comparison with a nucleotide sequence or amino acid sequence having homology or of comparison of a length of mRNA obtained by the northern blotting method described in 5. with the length of obtained cDNA that the obtained cDNA is not full length. When the obtained cDNA is incomplete cDNA, full-length cDNA can be obtained in the manner described below.

### (4)-1 Re-Searching of cDNA Library

By employing the obtained nephritis-related cDNA as a probe and performing colony hybridization or plaque hybridization (Molecular Cloning Second Edition) using Thy-1 nephritis rat kidney cDNA library, a hybridizing cDNA clone is obtained. DNA is prepared from the obtained clone by a method described in Molecular Cloning Second Edition, and DNA having the longest insert fragment is selected by cleaving with restriction enzyme. For the Thy-1 nephritis rat kidney cDNA library, a subtracted cDNA library may be prepared again. However, depending on the subtractive operation, there is a tendency for a clone containing a longer cDNA to be lost. Therefore, there is higher possibility of obtaining a full-length cDNA clone when using a pre-differentiation Thy-1 nephritis rat kidney cDNA library.

### (4)-2 Rapid Amplification of cDNA Ends (RACE)

By adding an adaptor oligonucleotide to both ends of Thy-1 nephritis rat kidney cDNA and performing 5'-RACE and 3'-RACE [Proc. Natl. Acad. Sci. USA, 85, 8998 (1988)] where PCR is performed with a primer based on the nucleotide sequence of the adaptor and the nucleotide sequence of the obtained cDNA clone, it is possible to obtain cDNA fragment which corresponds to external regions of the 5'-end and 3'-end of cDNA obtained in (2). The nucleotide sequence of the obtained cDNA is determined in the manner described in (3). By ligating the cDNA obtained by this method and the cDNA obtained in (2), cDNA of full length can be obtained.

Examples of full-length cDNA of a rat proliferative glomerulonephritis-related gene obtained in this manner include cDNA of a rat proliferative glomerulonephritis-related gene having the nucleotide sequence shown by SEQ ID NO: 1, 3, 5, 7, 9, 13, 17 or 157.

### (4)-3 Utilization of Database Information and PCR

When performing homology analysis of a nucleotide sequence of cDNA determined in (3) with the nucleotide sequence database, in some cases, while matching with a nucleotide sequence of an known gene may not be found, matching with an EST (expressed sequence tag) which is a nucleotide sequence of a terminus region of a random cDNA clone may be found. In such case, these ESTs, ESTs having a nucleotide sequence matching with the nucleotide sequence of the ESTs, and ESTs derived from clones identical to such ESTs are collected as ESTs derived from an identical gene. By ligating the nucleotide sequences of these ESTs derived from an identical gene, a nucleotide sequence of a region that extends further to the 5' side or 3' side than the cDNA obtained in (2) may be sometimes found. In this case, by performing PCR employing Thy-1 nephritis rat kidney cDNA or Thy-1 nephritis rat kidney cDNA library as a template, using a forward primer having a nucleotide sequence of the 5' end of the nucleotide sequence obtained by ligating ESTs or a reverse primer having a complementary nucleotide sequence to the nucleotide sequence of the 3' end, there can be obtained a cDNA fragment which corresponds to external region of the 5' end or 3' end of a nucleotide sequence of cDNA obtained in (2). The nucleotide sequence of the obtained cDNA can be determined in the same manner as described in (3), and a cDNA of full length can be obtained by ligating with the cDNA obtained in (2). When many numbers of ESTs of rat derived from a target nephritis-related gene have been obtained from a database, it may be possible to clarify the nucleotide sequence of the full-length cDNA of a nephritis-related gene by ligating the nucleotide sequences of the collected ESTs without performing RT-PCR.

Further, after clarifying the nucleotide sequence of the obtained full-length cDNA as described above, a full-length cDNA can be obtained by preparing a primer based on the nucleotide sequence of the cDNA and performing PCR employing Thy-1 nephritis rat kidney cDNA or cDNA library as a template. Also, based on the determined nucleotide sequence of a nephritis-related gene, it is possible to chemically synthesize nephritis-related gene DNA using a DNA synthesizer. Examples of a DNA synthesizer include DNA synthesizer model 392 manufactured by Perkin-Elmer, which utilizes a phosphoramidite method.

### (5) Obtaining of Human-Corresponding Gene

In order to apply a proliferative glomerulonephritis-related gene to treatment and diagnosis of proliferative glomerulonephritis in humans, a human-derived gene is necessary. In general, even if a polypeptide having the same function is of a different species, there is a high homology in the amino acid sequence, and there is also a tendency for a high homology to exist in the nucleotide sequence of the gene encoding the polypeptide. Therefore, by performing screening by hybridization under somewhat stringent conditions using cDNA library of human kidney, preferably kidney of a proliferative glomerulonephritis patient, employing rat cDNA as a probe, it is possible to obtain human cDNA. The term "somewhat stringent conditions" used herein, while differing depending on the homology of human cDNA and rat cDNA, means that southern blotting is performed under several hybridization conditions of differing degrees by employing rat cDNA as a probe for human chromosome DNA cleaved with restriction endonuclease, and the most stringent conditions of these conditions is used at which band is detected clearly. For example, in the case of a hybridization using a hybridization solution which does not contain formamide, the composition of the hybridization solution is fixed to be a salt concentration of 1 mol/l and hybridization is performed under several conditions in which the hybridization temperature is gradually changed between 68°C and 42°C. Then, the condition is determined by washing with 2-fold SSC containing 0.5% SDS at the same temperature as hybridization. In the case of a hybridization using a hybridization solution containing formamide, the temperature (42°C) and salt concentration (6-fold SSC) is fixed, and hybridization is performed under several conditions where the formamide concentration is gradually changed between 50% and 0%. Then, a condition is decided by washing with 6-fold SSC containing 0.5% SDS at 50°C.

Further, for a nucleotide sequence of rat cDNA obtained in (2) or (4), a search regarding the novelty and homology of the nucleotide sequence is performed in the same manner as described in (3), so as to search whether there is a nucleotide sequence of human cDNA showing high homology (specifically, 80% or more) in particular in a overall region encoding a polypeptide within the nucleotide sequence of rat cDNA. Human cDNA showing high homology is presumed to be cDNA of a human gene corresponding to the rat gene obtained in (2) or (4). Therefore, by performing RT-PCR using a primer corresponding to the nucleotide sequence of the 5' and 3' ends of this human DNA, and using RNA derived from human cell or tissue, preferably from kidney tissue or cell derived from kidney, and more preferably from kidney of a proliferative glomerulonephritis patient as a template, the human cDNA can be amplified and isolated. Further, while there may be cases where human cDNA found in a database is not a full-length cDNA or is merely the nucleotide sequence of an EST, in such cases also, full-length human cDNA can be obtained by the same method as described in (4) with respect to rat cDNA.

Further, analysis of a nucleotide sequence of human cDNA obtained in this manner can be performed in the same manner as described in (3), and thus the amino acid sequence of a human polypeptide encoded by that cDNA can be clarified.

Examples of human cDNA of a proliferative glomerulonephritis-related gene obtained in this manner include cDNA having the nucleotide sequences shown by SEQ ID NO: 11, 15 or 159.

In addition, for other non-human mammalians also, a corresponding gene can be obtained by using the similar method.

### (6) Obtaining of Genome Gene

Genome DNA of rat- or human- gene of the present invention can be obtained by screening a genome DNA library prepared using chromosome DNA isolated from tissue or cells of a rat or human by a method such as plaque hybridization using rat or human cDNA obtained in (2) or (5) as a probe according to a method described in Molecular Cloning Second Edition. By comparing the nucleotide sequence of genome DNA and the nucleotide sequence of cDNA, it is possible to clarify the exon/intron structure of the gene. Further, in particular by employing the 5' end region of the cDNA as a probe, the nucleotide sequence of a genome gene region which regulates transcription such as a promoter for the gene of the present invention can be clarified. This sequence is useful for analysis of the regulatory mechanism of transcription of a gene of the present invention.

Using the similar method, also for other non-human mammalians, a genome gene of the present invention can be obtained and a nucleotide sequence of a promoter region or the like can be clarified.

### (7) Preparation of Oligonucleotide

Using nucleotide sequence information of DNA of the present invention obtained by the above-described method, an oligonucleotide having a partial sequence of DNA of the present invention, such as an anti-sense oligonucleotide or sense oligonucleotide, can be prepared by means of a DNA synthesizer.

Examples of the oligonucleotide include DNA having the same sequence as consecutive 5 to 60 nucleotides within a nucleotide sequence of the above DNA, or DNA having a complementary sequence with the DNA. Specific examples include DNA having the same sequence as consecutive 5 to 60 nucleotides within the nucleotide sequences shown by SEQ ID NO: 1, 3 or 5, or DNA having a complementary sequence with these DNA. In the case of using as a forward primer and reverse primer of PCR, the oligonucleotide is preferred to be an oligonucleotides of which melting temperatures (Tm) and numbers of nucleotides of both are not greatly different and which have 5 to 60 nucleotides.

Further, a derivative of these oligonucleotides (hereinafter referred to as an "oligonucleotide derivative") can also be used as an oligonucleotide of the present invention.

Examples of the oligonucleotide derivative include an oligonucleotide derivative in which phosphodiester bond within an oligonucleotide was converted into phosphorothioate bond, an oligonucleotide derivative in which phosphodiester bond within an oligonucleotide was converted intto N3'-P5' phosphamidate binding, an oligonucleotide derivative in which ribose and phosphodiester bond within an oligonucleotide were converted into peptide nucleic acid bond, an oligonucleotide derivative in which uracil within an oligonucleotide was substituted with C-5 propynyl uracil, an oligonucleotide derivative in which uracil within an oligonucleotide was substituted with C-5 thiazol uracil, an oligonucleotide derivative in which cytosine within an oligonucleotide was substituted with C-5 propynyl cytosine, an oligonucleotide derivative in which cytosine within an oligonucleotide was substituted with phenoxazine-modified cytosine, an oligonucleotide derivative in which ribose within an oligonucleotide was substituted with 2'-O-propylribose, and an oligonucleotide derivative in which ribose within an oligonucleotide was substituted with 2'-methoxyethoxyribose [Cell Technology, 16, 1463 (1997)].

### 2. Production of Proliferative Glomerulonephritis-Related Polypeptide

Hereinafter, a method for producing a proliferative glomerulonephritis-related polypeptide will be described.

Based on a full-length cDNA, a DNA fragment of an adequate length containing a region encoding the polypeptide is prepared as necessary.

By inserting the DNA fragment or full-length cDNA downstream of a promoter in an expression vector, a recombinant vector which expresses the polypeptide is constructed.

The recombinant vector is introduced into a host cell suitable for the vector.

As a host cell, any host cell which is capable of expressing the target DNA can be used as a host cell. Examples include bacteria belonging to the genus Escherichia, Serratia, Corynebacterium, Brevibacterium, Pseudomonas, Bacillus, Microbacterium or the like; yeast belonging to the genus Kluyveromyces, Saccharomyces, Shizosaccharomyces, Trichosporon, Schwanniomyces or the like; animal cells, and insect cells.

As an expression vector, any vector which is capable of autonomous replication in a host cell or integration into a chromosome in a host cell, and contains a promoter in a position that can work in transcribing proliferative glomerulonephritis-related DNA, can be used.

When bacteria is used as a host cell, a proliferative glomerulonephritis-related DNA recombinant vector is preferably a recombinant vector which is capable of autonomous replication in the bacteria and comprises a promoter, a ribosome binding sequence, proliferative glomerulonephritis-related DNA, and a transcription termination sequence. The vector may comprise a gene which controls a promoter.

Examples of an expression vector include pBTrp2, pBTac1, pBTac2 (all commercially available from Boeringer Mannheim), pKK233-2 (manufactured by Amersham Pharmacia Biotech), pSE280 (manufactured by Invitrogen), pGEMEX-1 (manufactured by Promega), pQE-8 (manufactured by QIAGEN), pKYP10 (Japanese Published Unexamined Patent Application No. 110600/83), pKYP200 [Agricultural Biological Chemistry, 48, 669 (1984)], pLSA1 [Agric. Biol. Chem., 53, 277 (1989)], pGEL1 [Proc. Natl. Acad. Sci. USA, 82, 4306 (1985)], pBluescript II SK(-) (manufactured by Stratagene), pGEX (manufactured by Amersham Pharmacia Biotech), pET-3 (manufactured by Novagen), pTerm2 (USP4686191, USP4939094, USP5160735), pSupex, pUB110, pTP5, pC194, and pEG400 [J. Bacteriol., 172, 2392 (1990)].

As an expression vector, an expression vector, in which the distance between a Shine-Dalgarno sequence which is a ribosome binding sequence, and an initiation codon is adjusted at an adequate distance (for example, 6 to 18 nucleotides), is preferably used.

As a promoter, any promoter can be used as long as it can work in a host cell. Examples include promoters derived from Escherichia coli or phage such as trp promoter (Ptrp), lac promoter (Plac), PL promoter, PR promoter and T7 promoter; SPO1 promoter, SPO2 promoter, and penP promoter. Further, an artificially modified promoter such as a promoter having two Ptrp in tandem (Ptrp x 2), tac promoter, letI promoter [Gene, 44, 29 (1986)], or lacT7 promoter can also be used.

By substituting nucleotides of a region encoding a polypeptide of proliferative glomerulonephritis-related DNA of the present invention so as to make an optimal codon for expression of the host, the production efficacy of the target polypeptide can be increased.

While a transcription termination sequence is not necessarily required for expression of proliferative glomerulonephritis-related DNA of the present invention, ideally, a transcription termination sequence is desirably placed directly downstream a structural gene.

Examples of a host cell include a microorganism belonging to the genus Escherichia, Serratia, Corynebacterium, Brevibacterium, Pseudomonas, and Bacillus, such as Escherichia coli XL1-Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No.49, Escherichia coli W3110, Escherichia coli NY49, Bacillus subtilis, Bacillus amyloliquefaciens, Brevibacterium ammoniagenes, Brevibacterium immariphilum ATCC14068, Brevibacterium saccharolyticum ATCC14066, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, and Pseudomonas sp. D-0110.

As a method of introducing a recombinant vector, any method can be used as long as it is a method of introducing DNA to a host cell. Examples thereof include the method using calcium ion [Proc. Natl. Acad. Sci. USA, 69, 2110 (1972)], the protoplast method (Japanese Published Unexamined Patent Application No. 248394/88), and the method described in Gene, 17, 107 (1982) or Molecular & General Genetics, 168, 111 (1979).

When yeast is used as a host cell, examples of an expression vector include YEp13 (ATCC37115), YEp24 (ATCC37051), YCp50 (ATCC37419), pHS19, and pHS15.

As a promoter, any promoter which can work in yeast may be used. Examples thereof include PHO5 promoter, PGK promoter, GAP promoter, ADH promoter, gal 1 promoter, gal 10 promoter, heat shock protein promoter, MFa1 promoter, and CUP 1 promoter.

Examples of a host cell include Saccharomyces cerevisiae, Schizosaccharomyces pombe, Kluyveromyces lactis, Trichosporon pullulans, and Schwanniomyces alluvius.

As a method of introducing a recombinant vector, any method can be used as long as it is a method of introducing DNA to yeast. Examples include the electroporation method [Methods in Enzymol., 194, 182 (1990)], the spheroplast method [Proc. Natl. Acad. Sci. USA, 75, 1929 (1978)], the lithium acetate method [J. Bacteriol., 153, 163 (1983)], and the method described in Proc. Natl. Acad. Sci. USA, 75, 1929 (1978).

When an animal cell is used as a host cell, examples of an expression vector include pcDNAI (manufactured by Invitrogen), pcDM8 (manufactured by Invitrogen), pAGE107 [Japanese Published Unexamined Patent Application No. 22979/91; Cytotechnology, 3, 133 (1990)], pAS3-3 (Japanese Published Unexamined Patent Application No. 227075/90), pCDM8 [Nature, 329, 840 (1987)], pcDNAI/Amp (manufactured by Invitrogen), pREP4 (manufactured by Invitrogen), pAGE103 [J. Biochem., 101, 1307 (1987)], and pAGE210.

As a promoter, any promoter that work in an animal cell may be used, and examples include an immediate early gene promoter of cytomegalovirus (human CMV), early promoter of SV40, promoter of retrovirus, metallothionein promoter, heat shock protein promoter, and SRα promoter. Also, an immediate early gene enhancer of human CMV may be used together with a promoter.

Examples of a host cell include Namalwa cell which is a human cell, COS cell which is a simian cell, CHO cell which is a cell of a Chinese hamster, and HBT 5637 [Japanese Published Unexamined Patent Application No. 299/88].

As a method of introducing a recombinant vector, any method which can introduce DNA to an animal cell can be used. For example, the electroporation method [Cytotechnology, 3, 133 (1990)], the calcium phosphate method (Japanese Published Unexamined Patent Application No.227075/90), the lipofection method [Proc. Natl. Acad. Sci., USA, 84, 7413 (1987); Virology, 52, 456 (1973)] or the like can be used. Obtaining and culturing of a transformant can be carried out in accordance with the method described in Japanese Published Unexamined Patent Application No. 227075/90 or Japanese Published Unexamined Patent Application No. 257891/90.

When an insect cell is used as a host, a polypeptide can be expressed according to the method described in, for example, Baculovirus Expression Vectors, A Laboratory Manual, Oxford University Press (1994); Current Protocols in Molecular Biology; Bio/Technology 6, 47 (1988); or the like.

Specifically, after co-introducing a recombinant gene introduction vector and baculovirus into an insect cell to obtain a recombinant virus in the insect cell culture supernatant, the insect cell is infected with the recombinant virus to thereby express a polypeptide.

Examples of a vector for introduction of gene include pVL1392, pVL1393 and pBlueBacIII (all manufactured by Invitrogen).

As a baculovirus, for example, Autographa californica nuclear polyhedrosis virus which is a virus infecting insects of Barathra, or the like can be used.

As an insect cell, Sf9 and Sf21 which are ovarian cells of Spodoptera frugiperda [Baculovirus Expression Vectors, A Laboratory Manual, W. H. Freeman and Company, New York, (1992)]; High 5 which is an ovarian cell of Trichoplusia ni (manufactured by Invitrogen); or the like, can be used.

Examples of a method of co-introducing the above recombinant gene introduction vector and the above baculovirus into an insect cell to prepare a recombinant virus, include the calcium phosphate method [Japanese Published Unexamined Patent Application No. 227075/90] and the lipofection method [Proc. Natl. Acad. Sci., USA, 84, 7413 (1987)].

As a method of expressing a gene, direct expression may be used, and also secretion production, fusion polypeptide expression or the like can be performed in accordance with a method described in Molecular Cloning Second Edition or the like.

In the case of expression by means of a yeast, an animal cell or an insect cell, a polypeptide to which a sugar or sugar chain is added can be obtained.

A transformant which carries a recombinant vector incorporating proliferative glomerulonephritis-related DNA is cultured in a culture medium to produce and accumulate proliferative glomerulonephritis-related polypeptide in the culture, and then the polypeptide is collected from the culture to prepare proliferative glomerulonephritis-related polypeptide.

The method for culturing a transformant for preparing a proliferative glomerulonephritis-related polypeptide of the present invention in a culture medium can be performed in accordance with a conventional method used in culturing of a host cell.

When a transformant of the present invention employs a prokaryote such as Escherichia coli or a eukaryote such as yeast as a host cell, a medium for culturing the transformant may be either a natural medium or a synthetic medium, as long as it is a medium which contains carbon source that the host cell can assimilate, nitrogen source, minerals and the like and by which culturing of the transformant can be efficiently carried out.

As a carbon source, any carbon source which the respective host cell can assimilate may be used. For example, there can be used a carbohydrate such as glucose, fructose, sucrose, molasses containing these, starch or starch hydrolysate; organic acid such as acetic acid or propionic acid; and alcohol such as ethanol or propanol.

As a nitrogen source, there is used various inorganic acids such as ammonia, ammonium chloride, ammonium sulfate, ammonium acetate, and ammonium phosphate, or organic acid ammonium salts, other nitrogen containing compounds, as well as peptone, meat extract, yeast extract, corn steep liquor, casein hydrolysate, soybean cake and soybean cake hydrolysate, various microorganisms obtained by fermentation and their digest product, and the like.

As a mineral, there can be used potassium dihydrogenphosphate, dipotassium hydrogenphosphate, magnesium phosphate, magnesium sulfate, sodium chloride, ferrous sulfate, manganese sulfate, copper sulfate, calcium carbonate, and the like.

Culturing is performed under aerobic conditions such as shaking culture or submerged spinner culture. Culturing temperature may be from 15 to 40°C, and culturing period is normally between 16 hours to 7 days. pH during culturing is maintained between 3.0 to 9.0. Adjustment of pH is performed by using inorganic or organic acid, alkaline solution, urea, calcium carbonate, ammonia, or the like.

Further, if necessary, antibiotics such as ampicillin or tetracycline may be added to the culture medium during culturing.

When culturing a transformant obtained by using a recombinant vector having an inducible promoter as a promoter, an inducer may be added to the culture medium, if necessary. For example, when culturing a transformant that used a recombinant vector containing lac promoter, isopropyl-β-D-thiogalactopyranoside (IPTG) or the like may be added to the culture medium, and when culturing a transformant that obtained by using a recombinant vector containing trp promoter, indoleacrylic acid (IAA) or the like may be added to the culture medium.

As a medium for culturing a transformant obtained by employing an animal cell as a host cell, the generally used RPMI 1640 [The Journal of the American Medical Association, 199, 519 (1967)], Eagle's MEM medium [Science, 122, 501 (1952)], Dulbecco's modified MEM medium [Virology, 8, 396 (1959)], 199 medium [Proceeding of the Society for the Biological Medicine, 73, 1 (1950)], or a medium in which fetal calf serum or the like was added to these mediums, or the like can be used.

Culturing is normally performed for 1 to 7 days under conditions of a pH of 6 to 8, and a temperature of 30 to 40°C in the presence of 5% CO₂.

Further, if necessary during culturing, antibiotics such as kanamycin or penicillin may be added to the medium.

As a medium for culturing a transformant obtained by employing an insect cell as a host cell, the generally used TNM-FH medium (manufactured by Pharmingen), Sf-900 II SFM medium (manufactured by Life Technologies), ExCell400 and ExCell405 (both manufactured by JRH Biosciences), Grace's Insect Medium [Grace, T.C.C., Nature, 195, 788 (1962)], or the like can be used.

Culturing is normally performed for 1 to 5 days under conditions of a pH of 6 to 7 and a temperature of 25 to 30°C.

Further, if necessary during culturing, an antibiotic such as gentamicin may be added to the medium.

To isolate and purify a proliferative glomerulonephritis-related polypeptide from the culture of a transformant, a conventional method for isolating and purifying a polypeptide may be used.

For example, when a polypeptide is produced in soluble form within cell, after completion of culturing, the cell is recovered by centrifugation and is suspended in an aqueous buffer, and then the cell is disrupted by means of, for example, an ultrasonic homogenizer, a French press, a Manton-Gaurin homogenizer, or Dyno-Mill, to obtain cell-free extract. From supernatant obtained by centrifuging the cell-free extract, purified polypeptide can be obtained using, either alone or in combination, a conventional technique for isolating and purifying a polypeptide, such as solvent extraction, salting-out using ammonium sulfate or the like, desalting, precipitation using an organic solvent, anion exchange chromatography using a resin such as diethylaminoethyl (DEAE)-Sepharose or DIAION HPA-75 (manufactured by Mitsubishi Chemical Corporation), cation exchange chromatography using a resin such as S-Sepharose FF (manufactured by Amersham Pharmacia Biotech), hydrophobic chromatography using a resin such as butyl-Sepharose or phenyl-Sepharose, gel filtration using a molecular sieve, affinity chromatography, chromatofocusing, and an electrophoresis method such as isoelectric focusing.

Further, when a polypeptide is produced as inclusion bodies within a cell, after recovering the cell, the cell is disrupted and is subjected to centrifugation, to thereby recover the inclusion bodies of the polypeptide as a precipitation fraction.

The recovered inclusion bodies of the polypeptide is solubilized with a protein denaturing agent. The structure of the polypeptide is returned to a normal steric structure by lowering the concentration of the protein denaturing agent in the lysate by subjecting the lysate to dilution or dialysis, and then purified polypeptide is obtained by the method of isolation and purification as described above.

When a polypeptide or its glycosylated derivative or the like is secreted to outside the cell, the polypeptide or its glycosylated derivative or the like can be recovered from the culture supernatant. Specifically, by recovering the culture supernatant from the culture by using a technique such as centrifugation, and then using the method of isolation and purification as described above, a purified polypeptide can be obtained from the culture supernatant.

Examples of a polypeptide obtained in this manner include a polypeptide having an amino acid sequence shown by SEQ ID NO: 2, 4, 6, 8, 10, 12, 14, 16, 158 or 160.

Further, the polypeptide of the present invention can also be produced by a chemical synthesis method such as the Fmoc method (fluorenylmethyloxycarbonyl method) or the tBoc method (t-butyloxycarbonyl method). Further, synthesis can also be conducted by using a peptide synthesizer such as a peptide synthesizer manufactured by Advanced ChemTech (USA), Perkin-Elmer, Amersham Pharmacia Biotech, Protein Technology Instrument (USA), Synthecell-Vega (USA), PerSeptive (USA) or Shimadzu Corporation.

### 3. Preparation of Antibody Specifically Recognizing Proliferative Glomerulonephritis-Related Polypeptide

By using, as an antigen, a purified full length or partial fragment of a proliferative glomerulonephritis-related polypeptide or a synthetic peptide having a partial amino acid sequence of KRGF-1 protein, an antibody which recognizes a proliferative glomerulonephritis-related polypeptide, such as a polyclonal antibody or monoclonal antibody, can be produced.

### (1) Production of Polyclonal Antibody

A polyclonal antibody can be produced by subcutaneously, intravenously or intraperitoneally administering an antigen to an animal with using a purified full length or partial fragment of the polypeptide of the present invention or a peptide having a partial amino acid sequence of the protein of the present invention as an antigen, together with an adequate adjuvant [for example, Complete Freund's Adjuvant, aluminum hydroxide gel, or pertussis vaccine].

As an animal to be administered, rabbit, goat, rat, mouse, hamster, or the like can be used.

A dosage of antigen is preferably 50 to 100 µg per animal.

When a peptide is used, it is preferred to use an antigen in which the peptide is covalently bonded to a carrier protein such as keyhole limpet haemocyanin or bovine thyroglobulin. A peptide to be used as an antigen can be synthesized by a peptide synthesizer.

Administration of the antigen is performed 3 to 10 times at 1- to 2-week intervals after the initial administration. On days 3 to 7 after each administration, a blood sample is collected from ocular fundus plexus venosus, and the reaction of the serum with the antigen used for immunity is confirmed by enzyme linked immunoassay [Enzyme Linked Immunoassay (ELISA): Igaku-Shoin Publication (1976), Antibodies-A Laboratory Manual, Cold Spring Harbor Laboratory (1988)].

For the antigen used for immunity, serum is obtained from a non-human mammalian whose serum shows a sufficient antibody titer, and then the serum is separated and purified to obtain a polyclonal antibody.

Examples of a separation and purification method include a method using, either alone or in combination, techniques such as centrifugation, salting-out by 40-50% saturated ammonium sulfate solution, caprylic acid precipitation [Antibodies, A Laboratory Manual, Cold Spring Harbor Laboratory, (1988)], and chromatography using DEAE-Sepharose column, anion exchange column, protein A- or G-column or gel filtration column, or the like.

### (2) Production of Monoclonal Antibody

### (a) Preparation of antibody producing cell

For a partial fragment polypeptide of the polypeptide of the present invention used for immunity, a rat whose serum shows a sufficient antibody titer is provided as a source of antibody producing cells.

On day 3 to 7 after final administration of an antigen to a rat showing such antibody titer, the spleen is extirpated from the rat.

The spleen is macerated in MEM medium (manufactured by Nissui Pharmaceutical Co., Ltd.), loosen with a pin set, subject to centrifugation for 5 min at 1,200 rpm, and then the supernatant is discarded.

After treating spleen cells of the obtained precipitation fraction with tris-ammonium chloride buffer (pH 7.65) for 1-2 minutes to remove erythrocytes, the cells are washed with MEM medium 3 times, and the obtained spleen cells are used as antibody producing cells.

### (b) Preparation of myeloma cells

An established cell line obtained from a mouse or rat is used as myeloma cells. For example, 8-azaguanine resistant mouse (BALB/c derived) myeloma cell line P3-X63Ag8-U1 (hereinafter referred to as "P3-U1") [Curr. Topics. Microbiol. Immunol., 81, 1 (1978), Europ. J. Immunol., 6, 511 (1976)], SP2/0-Ag14(SP-2) [Nature, 276, 269 (1978)], P3-X63-Ag8653(653) [J. Immunol., 123, 1548 (1979)], P3-X63-Ag8(X63) [Nature, 256, 495 (1975)] or the like can be used. These cell lines are subcultured with 8-azaguanine medium [a medium obtained by adding glutamine (1.5 mmol/l), 2-mercaptoethanol (5 x 10⁻⁵ mol/l), gentamicin (10 µg/ml), and fetal calf serum (FCS) (manufactured by CSL, 10%) to RPMI-1640 medium (the obtained medium is hereinafter referred to as "normal medium"), and then adding 8-azaguanine (15 µg/ml)]. The cells are cultured in the normal medium 3-4 days prior to cell fusion. 2 x 10⁷ or more of the cells is used for fusion.

### (c) Preparation of hybridoma

The antibody producing cells obtained in (a) and the myeloma cells obtained in (b) are washed well in MEM medium or PBS (disodium hydrogenphosphate 1.83g, potassium dihydrogenphosphate 0.21g, sodium chloride 7.65g, 1 liter of distilled water, pH 7.2), and the cells are mixed such that the number of cells is of a ratio where antibody producing cells : myeloma cells = 5:1 to 10:1, and centrifuged for 5 minutes at 1,200 rpm, and the supernatant is discarded.

The cell groups in the obtained precipitation fraction are loosened well, and 0.2 to 1 ml of a solution obtained by mixing 2 g of polyethylene glycol-1000 (PEG-1000), 2 ml of MEM and 0.7 ml of dimethyl sulfoxide (DMSO) is added to the cell groups per 10⁸ antibody producing cells while stirring at a temperature of 37°C, and further 1 to 2 ml of MEM medium is added several times at 1-2 minute intervals.

After addition, MEM medium is added so as to make the total volume of 50 ml. The prepared fluid is centrifuged at 900 rpm for 5 minutes, and then the supernatant is discarded. After gently loosening the cells in the obtained precipitation fraction, the cells are gently suspended in 100 ml of HAT medium [medium obtained by adding hypoxanthine (10⁻⁴ mol/l), thymidine (1.5 x 10⁻⁵ mol/l) and aminopterin (4 x 10⁻⁷ mol/l) to normal solution] by pipetting.

The suspension is dispensed onto 96-well culture plates at 100 µl/well, and is cultured in 5% CO₂ incubator at 37°C for 7-14 days.

After culturing, an aliquot of the culture supernatant is taken out, and a hybridoma which specifically reacts with a partial fragment polypeptide of the protein of the present invention is selected by an enzyme immunoassay method described in Antibodies [Antibodies, A Laboratory Manual, Cold Spring Harbor Laboratory, Chapter 14 (1988)] or the like.

Specific examples of an enzyme immunoassay method include the following method.

At the time of immunization, a partial fragment polypeptide of the polypeptide of the present invention used as an antigen is coated on a suitable plate, and is reacted with the hybridoma culture supernatant or purified antibody obtained in (d) below as primary antibody. Then, anti-rat or anti-mouse immunoglobulin antibody labeled with biotin, an enzyme, a chemiluminescent substance or a radioactive compound or the like is reacted as a secondary antibody, and a reaction suitable for the labeling substance is carried out. Then, those which specifically react with the protein of the present invention are selected as hybridomas which produce a monoclonal antibody of the present invention.

Using the hybridomas, cloning is repeated two times by limiting dilution method [using HT medium (medium obtained by excluding aminopterin from HAT medium) at the first time, and normal medium at the second time], and those which show a stable and strong antibody titer are selected as a hybridoma line which produces a monoclonal antibody of the present invention.

### (d) Preparation of monoclonal antibody

5 x 10⁶ to 20 x 10⁶ cells/mouse of hybridoma cells which produce a monoclonal antibody of the present invention, obtained in (c) above are intraperitoneally injected to Pristane-treated (intraperitoneally injecting 0.5 ml of 2,6,10,14-tetramethylpentadecane (Pristane), and growing animals for 2 weeks) 8-10 week-old mice or nude mice. After 10 to 21 days, the hybridoma becomes an ascites tumor.

Ascites fluid is taken out from a mouse in which an ascites tumor developed, and centrifuged at 3,000 rpm for 5 minutes to remove solid content.

A monoclonal antibody can be purified and obtained from the obtained supernatant by using the same method as that used for the polyclonal antibody.

The determination of the subclass of the antibody is carried out by using a mouse monoclonal antibody typing kit or a rat monoclonal antibody typing kit. The amount of the polypeptide is calculated by the Lowry method or by the absorbance at 280 nm.

### 4. Method of Preparing Recombinant Virus Vector Which Produce Proliferative Glomerulonephritis-Related Polypeptide

Hereinafter, a method of preparing a recombinant virus vector for producing the proliferative glomerulonephritis-related polypeptide of the present invention in specific human tissue is described.

Based on the full length cDNA of the proliferative glomerulonephritis-related gene, a DNA fragment of a suitable length which contains a region encoding the polypeptide is prepared if necessary.

By inserting the DNA fragment or the full length cDNA downstream of a promoter within a virus vector, a recombinant virus vector is constructed.

In the case of an RNA virus vector, cRNA homologous with full length cDNA of the proliferative glomerulonephritis-related gene or an RNA fragment homologous with a DNA fragment of a suitable length which contains a region encoding the polypeptide is prepared, and inserted downstream of a promoter within a virus vector to thereby construct a recombinant virus. For the RNA fragment, in addition to double-stranded RNA, either single strand of the sense strand or antisense strand may also be selected depending on the type of virus vector. For example, in the case of a retrovirus vector, RNA homologous to a sense strand is selected, and in the case of Sendai virus, RNA homologous to an antisense strand is selected.

The recombinant virus vector is introduced into a packaging cell suitable for the vector.

As a packaging cell, any cell can be used so long as it can supplement for the deleted polypeptide of a recombinant virus vector in which at least one of the genes which encode a polypeptide necessary for packaging of a virus is deleted. For example, human kidney-derived HEK293 cell, mouse fibroblast NIH3T3 or the like can be used. Examples of a polypeptide supplemented by packaging cell include, in the case of a retrovirus vector, polypeptides such as mouse retrovirus-derived gag, pol, and env; in the case of a lentivirus vector, polypeptides such as HIV virus-derived gag, pol, env, vpr, vpu, vif, tat, rev, and nef; in the case of an adenovirus vector, polypeptides such as adenovirus-derived E1A·E1B; in the case of an adeno associated virus, polypeptides such as Rep (p5, p19, p40) and Vp (Cap); and in the case of Sendai virus, polypeptides such as NP, P/C, L, M, F, and HN.

As a virus vector, a virus vector which can produce a recombinant virus in the above-described packaging cell and contain a promoter in a position that work in transcription of proliferative glomerulonephritis-related DNA in a target cell is used. As a plasmid vector, MFG [Proc. Natl. Acad. Sci. USA, 92, 6733-6737 (1995)], pBabePuro [Nucleic Acids Res., 18, 3587-3596 (1990)], LL-CG, CL-CG, CS-CG, CLG [Journal of Virology, 72, 8150-8157 (1998)], pAdex1 [Nucleic Acids Res., 23, 3816-3821 (1995)], or the like is used. As a promoter, any promoter can be used so long as they can work in human tissue. Examples include IE (immediate early) gene promoter of cytomegalovirus (human CMV), early promoter of SV40, retrovirus promoter, metallothionein promoter, heat shock protein promoter, and SR α promoter. Further, human CMV IE gene enhancer may also be used together with the promoter.

Examples of a method for introducing a recombinant virus vector into a packaging cell include the calcium phosphate method (Japanese Published Unexamined Patent Application No. 227075/90) and the lipofection method [Proc. Natl. Acad. Sci. USA, 84, 7413 (1987)].

### 5 Method for Detecting mRNA of Proliferative Glomerulonephritis-Related Gene

Hereinafter, a method for detecting mRNA of a proliferative glomerulonephritis-related gene by using proliferative glomerulonephritis related DNA of the present invention is described.

Examples of DNA which can be used in this method include DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159.

Examples of a method for detecting an expression level or structural change of proliferative glomerulonephritis-related gene mRNA include (1) northern blotting, (2) in situ hybridization, (3) quantitative PCR, (4) differential hybridization, (5) a DNA chip method, and (6) RNase protection assay.

As a specimen to be subjected to the analysis according to the above method, mRNA or total RNA obtained from a biological specimen such as kidney tissue, blood serum, or saliva obtained from a kidney patient or a healthy subject, or a primary cultured cell specimen obtained by collecting cells from such biological specimen and culturing them in an adequate medium in vitro (hereinafter, the mRNA and total RNA are referred to as "specimen-derived RNA") is used. Further, tissue which was obtained from the biological specimen and isolated as paraffin section or cryostat section can also be used.

In the northern blotting method, by separating the specimen-derived RNA by gel electrophoresis, transcribing it on support such as a nylon filter, and performing hybridization using a labeled probe prepared from DNA of the present invention and washing, the expression level as well as the structural change of mRNA derived from proliferative glomerulonephritis-related gene can be detected by detecting a band specifically bonded to mRNA derived from proliferative glomerulonephritis-related gene. In performing hybridization, incubation is carried out under conditions in which a probe and the mRNA derived from the proliferative glomerulonephritis-related gene in the specimen-derived RNA can form a stable hybrid. To prevent false positive, it is preferable that hybridization and washing process are performed under highly stringent conditions. The conditions are determined according to several factors including temperature, ionic strength, nucleotide composition, probe length, and formamide concentration. These factors are described in, for example, Molecular Cloning Second Edition.

A labeled probe used in northern blotting can be prepared, for example, by incorporating a radioisotope, biotin, a fluorescent group, a chemiluminescent group or the like into DNA of the present invention or an oligonucleotide designed from the nucleotide sequence of the DNA in accordance with a known method (e.g. nick translation, random priming, or kinasing). Since the amount of binding of the labeled probe reflects the expression level of mRNA derived from proliferative glomerulonephritis-related gene, the expression level of mRNA derived from proliferative glomerulonephritis-related gene can be determined by determining the amount of bonded labeled probe. Further, by analyzing the labeled probe binding sites, structural changes of the mRNA derived from the proliferative glomerulonephritis-related gene can be known.

The expression level of mRNA derived from the proliferative glomerulonephritis-related gene can be detected according to in-situ hybridization method where hybridization and washing processes are carried out by using the above labeled probe and tissue obtained from a living organism and isolated as paraffin section or cryostat section. In the in-situ hybridization method, in order to prevent false positive, it is desirable that the hybridization and washing processes are performed under highly stringent conditions. The conditions are determined according to several factors including temperature, ionic strength, nucleotide composition, probe length, and formamide concentration. These factors are described in, for example, Current Protocols in Molecular Biology.

A method for detecting mRNA derived from proliferative glomerulonephritis-related gene by quantitative PCR, differential hybridization or a DNA chip method can be performed according to a method based on synthesizing cDNA using specimen-derived RNA, an oligo(dT) primer or random primer, and reverse transcriptase (hereinafter, the cDNA is referred to as "specimen-derived cDNA"). When the specimen-derived RNA is mRNA, either of the above-mentioned primers can be used, but when the specimen-derived RNA is total RNA, it is necessary to use oligo(dT) primer.

In quantitative PCR, by performing PCR using a primer designed based on a nucleotide sequence of proliferative glomerulonephritis-related DNA of the present invention and employing specimen-derived cDNA as a template, DNA fragments derived from mRNA derived from proliferative glomerulonephritis-related gene are amplified. Since the amount of amplified DNA fragments reflects the expression level of mRNA derived from proliferative glomerulonephritis-related gene, it is possible to determine the level of mRNA derived from proliferative glomerulonephritis-related gene by using DNA encoding actin or G3PDH (glyceraldehyde 3-phosphate dehydrogenase) or the like as an internal control. Further, by separating the amplified DNA fragments by gel electrophoresis, structural changes of the mRNA derived from proliferative glomerulonephritis-related gene can also be known. In this detection method, it is desirable to use an adequate primer that works specifically and efficiently to amplify the target sequence. An adequate primer can be designed on the basis of conditions such as not causing binding between primers or within primers, specifically binding to target cDNA at an annealing temperature, disconnecting from target cDNA under denaturing conditions, and the like. It is necessary that quantitative determination of amplified DNA fragments be performed during PCR reaction in which amplification products are increasing exponentially. Such PCR reaction can be confirmed by recovering the amplified DNA fragments produced at each reaction and performing quantitative analysis by gel electrophoresis.

Using specimen-derived cDNA as a probe, variations in an expression level of mRNA derived from proliferative glomerulonephritis-related gene can be detected by performing hybridization and washing for filter or support such as slide glass or silicon on which DNA of the present invention is immobilized. The methods based on this principle include the differential hybridization method [Trends in Genetics, 7, 314-317 (1991)] and DNA chip method [Genome Research, 6, 639-645 (1996)]. In both methods, by immobilizing an internal control such as actin or G3PDH on a filter or support, differences in the expression of mRNA derived from proliferative glomerulonephritis-related gene between a control specimen and a target specimen can be accurately detected. Further, by synthesizing target cDNA using different NTP which is labeled respectively based on RNA derived from a control specimen and a target specimen, and hybridizing two labeled cDNA probes to 1 filter or 1 support at the same time, expression level of mRNA derived from proliferative glomerulonephritis-related gene can be determined accurately.

In the case of RNase protection assay, a promoter sequence such as T7 promoter or SP6 promoter is bound to the 3' end of DNA of the present invention, and then labeled antisense RNA is synthesized by using rNTP labeled by in-vitro transcription system using RNA polymerase. The labeled antisense RNA is bound with specimen-derived RNA to form an RNA-RNA hybrid, followed by digesting with Rnase. Then, the RNA fragments protected from digestion is subjected to gel electrophoresis to form a band, and is detected. By quantitatively determining the obtained band, the expression level of mRNA derived from proliferative glomerulonephritis-related gene can be determined.

### 6 Method for Detecting Causative Gene of Renal Disease

Hereinafter, a method for detecting a causative gene of renal disease by using proliferative glomerulonephritis-related DNA of the present invention is described.

Examples of DNA which can be used in this method include DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159.

The most clear test for assessing the presence of a mutation which is a cause of renal disease in a proliferative glomerulonephritis-related gene locus is to directly compare a gene from a control group with a gene from a renal disease patient.

Specifically, a human biological specimen such as kidney tissue, blood serum, or saliva or a specimen derived from primary culture cells established from this biological specimen is collected from a renal disease patient and a healthy subject, and DNA is extracted from the biological specimen or the specimen derived from primary culture cells (hereinafter, this DNA is referred to as "specimen-derived DNA"). The specimen-derived DNA or proliferative glomerulonephritis-related DNA which was amplified by using a primer designed on the basis of a nucleotide sequence of DNA of the present invention can be used as specimen DNA. As an alternate method, a DNA fragment containing a proliferative glomerulonephritis-related DNA sequence amplified by performing PCR with primers designed on the basis of a nucleotide sequence of DNA of the present invention with employing the specimen-derived cDNA as a template, can be used as specimen DNA.

To detect whether or not a mutation which is a cause of renal disease is present in proliferative glomerulonephritis-related DNA, a method for detecting a hetero double strands which is formed by hybridization of a DNA strand having a wild-type allele with a DNA strand having a mutant allele can be used.

Examples of methods of detecting a hetero double strands include (1) hetero double strands detection by polyacrylamide gel electrophoresis [Trends Genet., 7, 5 (1991)], (2) single strand conformation polymorphism analysis [Genomics, 16, 325-332 (1993)], (3) chemical cleavage of mismatches (CCM) [Human Molecular Genetics (1996), Tom Strachan and Andrew P. Read (BIOS Scientific Publishers Limited), (4) enzymatic cleavage of mismatches [Nature Genetics, 9, 103-104 (1996)], and (5) denaturant gradient gel electrophoresis [Mutat. Res., 288, 103-112 (1993)].

By employing specimen-derived DNA or specimen-derived cDNA as a template and using primers designed based on a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159, proliferative glomerulonephritis-related DNA is amplified as fragments smaller than 200 bp, and subjected to polyacrylamide gel electrophoresis. When hetero double strands is formed by a mutation of proliferative glomerulonephritis-related DNA, mobility is slower than a homo double strands having no mutation, and they can be detected as an additional band. Separation is improved when using a specially prepared gel (Hydro-link, MDE, or the like). When conducting a search of fragments smaller than 200 bp, insertion, deletion, and most single nucleotide substitutions can be detected. It is preferable to perform hetero double strands analysis on one sheet of gel in combination with single strand conformation polymorphism analysis described below.

In single strand conformation polymorphism analysis (SSCP analysis), proliferative glomerulonephritis-related DNA is amplified as fragments smaller than 200 bp by using specimen-derived DNA or specimen-derived cDNA as a template and using primers designed based on a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159, and the DNA is denatured. Then, the DNA is subjected to electrophoresis in non-denaturing polyacrylamide gel. When performing DNA amplification, the amplified proliferative glomerulonephritis-related DNA can be detected as a band by labeling the primer with a radioisotope or fluorescent dye, or alternatively by silver staining unlabeled amplified products. In order to clarify the differences with a wild-type pattern, the control specimen is subjected to electrophoresis at the same time, and thereby fragments having a mutation can be detected by detecting the difference in mobility.

In chemical cleavage of mismatches (CCM), DNA fragments of proliferative glomerulonephritis-related DNA is amplified by employing specimen-derived DNA or specimen-derived cDNA as a template and using primers designed based on a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159. The amplified DNA fragments is hybridized with labeled DNA obtained by incorporating a radioisotope or fluorescent dye into DNA of the present invention, and treated with osmium tetroxide, and thereby one of the strands of DNA is cleaved at a mismatching place to enable detection of mutation. The CCM method is one of the detection methods with the highest sensitivity, and can also be adapted to a specimen of kilobase length.

Instead of the above osmium tetroxide, by combining an enzyme involved in mismatch repairing in a cell such as T4 phage lyzolbase or endonuclease VII, and RNaseA, a mismatch can be enzymatically cleaved.

In denaturing gradient gel electrophoresis (DGGE), DNA fragments of proliferative glomerulonephritis-related DNA is amplified by employing specimen-derived DNA or specimen-derived cDNA as a template and using primers designed based on a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, *57,* 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159, and is subjected to electrophoresis using a gel having a concentration gradient of a chemical denaturing agent and temperature gradient of a chemical denaturant. The amplified DNA fragments shift in the gel as far as a position at where the DNA is denatured into a single strand, and stop to shift after denaturation. Because the mobility of amplified DNA in the gel differs between the case where a mutation exists in proliferative glomerulonephritis-related DNA and the case where mutation does not exist, the presence of a mutation can be detected. The detection sensitivity may be raised by adding a poly(G:C) terminal to the respective primers.

Another method of detecting a causative gene of renal disease is the protein truncation test (PTT) [Genomics, 20, 1-4 (1994)]. This test enables specific detection of a frameshift mutation, splice site mutation and nonsense mutation which generate deletion of a polypeptide. In the PTT method, specific primers are designed in which T7 promoter sequence and a translation initiation sequence of eukaryote are connected to the 5' end of DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159, and cDNA is prepared from specimen-derived RNA by reverse transcription PCR (RT-PCR) using the primers. When in vitro transcription and translation is performed using the cDNA, a polypeptide is produced. The polypeptide is subjected to gel electrophoresis, and if the migration position of the polypeptide is a position equivalent to that of a full length polypeptide, there is no mutation which generates a deletion. If the polypeptide has a deletion, the polypeptide migrates to a position shorter than that of a full length polypeptide, and from the position the extent of deletion can be detected.

In order to determine a nucleotide sequence of specimen-derived DNA or specimen-derived cDNA, a primer designed on the basis of a nucleotide sequence of DNA of the present invention can be used. By analyzing the determined nucleotide sequence, it can be judged whether or not a mutation that is a cause of renal disease is present in the specimen-derived DNA or specimen-derived cDNA.

A mutation present in the region other than the coding region of the proliferative glomerulonephritis-related gene can be detected by examining a non-coding region such as the vicinity of the gene or an intron and regulating sequence therein. Renal disease caused by mutation in a non-coding region can be confirmed by detecting mRNA of an abnormal size or of an abnormal production amount in a renal disease patient when compared with a control specimen in accordance with the method described above.

The gene where the presence of a mutation in a non-coding region is suggested, can be cloned by using, as hybridization probe, DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159. The mutation in the non-coding region can be searched in accordance with any of the aforementioned methods.

By conducting statistical processing according to a method described in Handbook of Human Genetics Linkage (The John Hopkins University Press, Baltimore (1994)), the discovered mutation can be identified as an SNPs (single nucleotide polymorphism) which is linked to a renal disease. Further, by obtaining DNA from a family having a clinical history of renal disease by the previously shown method and detecting a mutation, a causative gene of renal disease can be identified.

### 7 Method for Diagnosing Onset Probability and Prognosis of Renal Disease Using Proliferative Glomerulonephritis-Related DNA

Examples of DNA that can be used in this method include DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159, as well as DNA fragments obtained from these DNA.

A cause of renal disease can be confirmed by detecting a mutation of a gene in any tissue of a human. For example, when a mutation exists in a germ line, there is a probability that the individual which inherited the mutation is prone to develop renal disease. The mutation can be detected by examining DNA from any tissue of the body of the individual. For example, renal disease can be diagnosed by collecting blood, extracting DNA from cells of the blood, and testing for a gene mutation using the DNA. Further, antenatal diagnosis can be performed by testing for a gene mutation using embryonic cells, placental cells or amniocyte.

Further, by testing DNA obtained from tissue of a lesion site of a patient affected by renal disease, the results can be utilized to, for example, diagnose the type of the renal disease and select a medicament to be administered. To detect a gene mutation in tissue, it is useful to isolate lesion site tissue released from surrounding normal tissue. Kidney of a renal disease patient can be extracted by means of biopsy. The tissue obtained in this manner is treated with trypsin or the like, and the obtained cells are cultured in an adequate medium. Chromosome DNA and mRNA can be extracted from the cultured cells.

Hereinafter, DNA obtained from a human specimen by any of the aforementioned methods for the purpose of diagnosis is referred to as "diagnostic specimen-derived DNA". Further, cDNA synthesized from RNA obtained from a human specimen by any of the aforementioned methods for the purpose of diagnosis is referred to as "diagnostic specimen-derived cDNA".

Diagnosis of a renal disease can be performed by a method in accordance with the above-described method for detecting a causative gene of renal disease using proliferative glomerulonephritis-related DNA and diagnostic specimen-derived DNA or diagnostic specimen-derived cDNA.

Further, in the diagnosis of a renal disease using proliferative glomerulonephritis-related DNA and diagnostic specimen-derived DNA or diagnostic specimen-derived cDNA, there can be used methods such as (1) detecting a restriction enzyme site, (2) a method using an allele specific oligonucleotide probe (ASO: allele specific oligonucleotide hybridization), (3) PCR using an allele specific oligonucleotide (ARMS: amplification refractory mutation system), (4) oligonucleotide ligation assay (OLA), (5) PCR-preferential homoduplex formation assay (PCR-PHFA), and (6) a method using oligo DNA array [Tanpakushitsu Kakusan Koso (Protein Nucleic Acid Enzyme), 43, 2004-2011 (1998)].

When a restriction site is eliminated or generated by a single nucleotide modification, mutation can be simply detected by amplifying diagnostic specimen-derived DNA or diagnostic specimen-derived cDNA with primers designed based on the sequence of DNA of the present invention, digesting it with the restriction enzyme, and then comparing the obtained restriction enzyme-cleaved DNA fragment with that in the case of a normal person. However, since a single nucleotide modification rarely occurs, for diagnostic purposes, an oligonucleotide probe is designed based on a combination of sequence information about DNA of the present invention and separately-identified mutation information, and mutation is detected by reverse dot blotting where the oligonucleotide probe is bound to a filter and hybridization is carried out.

A short synthetic DNA probe hybridizes only with a completely matched sequence. Therefore, utilizing this characteristic, a single nucleotide mutation can be easily detected by using an allele specific oligonucleotide probe (ASO). For diagnostic purposes, there is often used a reverse dot blotting where an oligonucleotide designed based on the sequence of DNA of the present invention and the identified mutation is bound to a filter, and hybridization is carried out by using a probe prepared by PCR using primers designed based on a sequence of DNA of the present invention from diagnostic specimen-derived DNA or diagnostic specimen-derived cDNA and labeled dNTP. The DNA chip method, where a high-density array is prepared by synthesizing an oligonucleotide designed based on the sequence of DNA of the present invention and the mutation directly onto a support such as a slide glass or silicon, is a mutation detection method suitable for large-scale diagnostic purposes, since a variety of mutations can be more easily detected for a small amount of diagnostic specimen-derived DNA or diagnostic specimen-derived cDNA.

A nucleotide mutation can also be detected by oligonucleotide ligation assay (OLA) described below.

Two oligonucleotides having about 20 nucleotides based on the sequence of DNA of the present invention, which hybridize to both sides of a mutation site, are prepared. Using diagnostic specimen-derived DNA or diagnostic specimen-derived cDNA as a template and using primers designed based on the sequence of proliferative glomerulonephritis-related DNA, proliferative glomerulonephritis-related DNA fragments is amplified by PCR. The amplified fragments are hybridized to the aforementioned oligonucleotides. After hybridization, the two oligonucleotides are ligated by DNA ligase. Whether or not ligation has occurred can be quickly detected by, for example, attaching biotin to one of the oligonucleotides and a different label such as digoxigenin to the other oligonucleotide. Since electrophoresis or centrifugation operation is not necessary in OLA, OLA is a mutation detection method suitable for effectively diagnosing a large number of samples in a short time.

Further, small amounts of mutated gene can be quantitatively and simply detected by the PCR-PHFA method described below.

The PCR-PHFA method is a combination of 3 methods: polymerase chain reaction (PCR), hybridization in liquid phase which shows extremely high specificity, and ED-PCR (enzymatic detection of PCR product) which detects PCR product by an operation similar to ELISA. PCR is performed using DNA of the present invention as a template and using a primer set labeled with dinitrophenyl (DNP) and biotin, to thereby prepare amplified products labeled at both ends. To these are mixed 20- to 100-fold excess amount of unlabeled amplified products obtained by performing amplification using a primer set having the same sequence but without labels and employing diagnostic specimen-derived DNA or diagnostic specimen-derived cDNA as a template. Then, after heat denaturation, the mixture is cooled at a moderate temperature gradient of about 1°C/5-10 minutes, to preferentially form a complete complementary strand. The thus reconstituted labeled DNA is captured and adsorbed in streptavidin immobilized wells via biotin, and is then detected by coloring reaction with an enzyme by binding it with an enzyme-labeled anti-DNP antibody via DNP. If gene having the same sequence as labeled DNA is not present in the specimen, the original double-stranded labeled DNA is reconstituted preferentially and exhibits coloring. In contrast, if a gene of the same sequence is present, because the amount of reconstituted labeled DNA decreases since substitution of complementary strand occurs at random, coloring decreases remarkably. Thus, detection and quantitative determination of a known mutation and polymorphic gene become possible.

### 8 Method of Immunological Detection and Quantitative Determination of Proliferative Glomerulonephritis-Related Polypeptide Using Antibody which Specifically Recognizes Proliferative Glomerulonephritis-Related Polypeptide

Examples of a method for immunological detection and quantitative determination of a microorganism, animal cell or insect cell or tissue which expresses a proliferative glomerulonephritis-related polypeptide intracellularly or extracellularly using an antibody (polyclonal antibody or monoclonal antibody) which specifically recognizes a proliferative glomerulonephritis-related polypeptide of the present invention include a fluorescent antibody method, enzyme linked immunosorbent assay (ELISA), a radioimmunoassay (RIA), immunohistochemical staining methods such as immunohistological staining and immunological cell staining (ABC method, CSA method, and the like), western blotting, dot blotting, immunoprecipitation, and sandwich ELISA[Experimental Manual for Monoclonal Antibodies (in Japanese) (Kodansha Scientific) (1987), Biochemical Experimental Lectures (Second Series) 5, Methods of Immunobiochemical Investigation (In Japanese) (Tokyo Kagaku Dojin (1986)).

In the fluorescent antibody method, an antibody of the present invention is reacted with a microorganism, animal cell or insect cell or tissue which expresses a proliferative glomerulonephritis-related polypeptide intracellularly or extracellularly, and an anti-mouse IgG antibody labeled with a fluorescent substance such as fluorescein isothiocyanate (FITC) or a fragment thereof is further reacted, and then fluorochrome is measured by a flow cytometer.

In the enzyme linked immunosorbent assay (ELISA), an antibody of the present invention is reacted with a microorganism, animal cell or insect cell or tissue which expresses a proliferative glomerulonephritis-related polypeptide intracellularly or extracellularly, and an anti-mouse IgG antibody or bonded fragment labeled with an enzyme such as peroxidase, or biotin is further reacted, and then coloring dye is measured by an absorptiometer.

In the radioimmuno assay (RIA), an antibody of the present invention is reacted with a microorganism, animal cell or insect cell or tissue which expresses a proliferative glomerulonephritis-related polypeptide intracellularly or extracellularly, and an anti-mouse IgG antibody labeled with a radioactive label or a fragment thereof is further reacted, and then measurement is conducted using a scintillation counter or the like.

In the immunological cell staining and the immunohistological staining, an antibody which specifically recognizes a proliferative glomerulonephritis-related polypeptide is reacted with a microorganism, animal cell or insect cell or tissue which expresses a proliferative glomerulonephritis-related polypeptide intracellularly or extracellularly, and an anti-mouse IgG antibody or a fragment thereof labeled with a fluorescent substance such as FITC or an enzyme such as peroxidase, or biotin is further reacted, and then observation is carried out using a microscope.

In the western blotting method, extract of a microorganism, animal cell or insect cell or tissue which expresses a proliferative glomerulonephritis-related polypeptide intracellularly or extracellularly is fractionated by SDS-polyacrylamide gel electrophoresis [Antibodies-A Laboratory Manual, Cold Spring Harbor Laboratory, (1988)], and the gel is blotted on a PVDF membrane or nitrocellulose membrane. Then, an antibody which specifically recognizes a proliferative glomerulonephritis-related polypeptide of the present invention is reacted with this membrane, and further an anti-mouse IgG antibody or a flagment thereof labeled with a fluorescent substance such as FITC or an enzyme such as peroxidase, or biotin is reacted, and the detection is carried out.

In the dot blotting method, extract of a microorganism, animal cell or insect cell or tissue which expresses a proliferative glomerulonephritis-related polypeptide intracellularly or extracellularly is blotted on a nitrocellulose membrane, and an antibody of the present invention is reacted with the membrane, and then an anti-mouse IgG antibody or bonded fragment labeled with a fluorescent substance such as FITC or an enzyme such as peroxidase, or biotin is further reacted, and the detection is carried out.

In the immunoprecipitation, extract of a microorganism, animal cell or insect cell or tissue which expresses a polypeptide of the present invention intracellularly or extracellularly is reacted with an antibody which specifically recognizes a proliferative glomerulonephritis-related polypeptide of the present invention, and then a support having a specific binding ability to immunoglobulin, such as protein G-Sepharose, is added, and thereby an antigen-antibody complex is precipitated.

In the sandwich ELISA method, two types of antibodies having different antigen recognition site, which specifically recognize a proliferative glomerulonephritis-related polypeptide of the present invention, are used. One antibody is adsorbed on a plate beforehand, and the other antibody is labeled with a fluorescent substance such as FTTC or an enzyme such as peroxidase, or biotin. Then, extract of a microorganism, animal cell or insect cell or tissue which expresses a proliferative glomerulonephritis-related polypeptide intracellularly or extracellularly is reacted with the antibody adsorbed plate, and the labeled antibody is reacted, and the reaction suitable for the label is carried out.

### 9 Method of Diagnosing Renal Disease Using Antibody which Specfically Recognizes Proliferative Glomerulonephritis Related Polypeptide

Identification of changes in an expression level of a proliferative glomerulonephritis related polypeptide and structural changes of an expressing polypeptide in a human biological specimen and human primary culture cells is useful from the viewpoint of understanding the risk of onset of renal disease in the future or a cause of a renal disease that has already developed.

Methods of detecting and diagnosing expression level and structural changes of a proliferative glomerulonephritis related polypeptide include the fluorescent antibody method, enzyme linked immuno sorbent assay (ELISA), radioimmuno assay method (RIA), immunohistochemical staining such as immunohistological staining and immunological cell staining (ABC method, CSA method, and the like), western blotting, dot blotting, immunoprecipitation, and sandwich ELISA method, as mentioned above.

As a specimen to be subjected to the diagnosis according to the above methods, a biological specimen itself obtained from the patient such as tissue of the lesion site of the renal disease, blood, blood serum, urine, stool or saliva , or cells or cell extract obtained from the biological specimen is used. Further, tissue obtained from a biological specimen is isolated as a paraffin or cryostat section, can be used.

### 10 Method for Screening a Therapeutic Agent for Renal Disease Using Proliferative Glomerulonephritis-Related Polypeptide, DNA Encoding the Polypeptide, or Antibody Recognizing the Polypeptide

Examples of DNA which can be used in this screening method include DNA having a nucleotide sequence shown by SEQ ID NO: 1, 3, 5, 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 or 159. Examples of a polypeptide which can be used include a polypeptide having an amino acid sequence selected from the amino acid sequences shown by SEQ ID NO: 2, 4, 6, 8, 10, 12, 14, 16, 158 and 160, or a polypeptide derived from the amino acid sequence of said polypeptide by deletion, substitution or addition of one or more amino acids and having an activity involved in formation and repair of renal lesion. Examples of an antibody include an antibody which recognizes the polypeptide.

A microorganism, animal cell or insect cell, transformed by introduction of proliferative glomerulonephritis-related DNA of the present invention so as to produce a proliferative glomerulonephritis-related polypeptide or a polypeptide which constitutes a part of a proliferative glomerulonephritis-related polypeptide of the present invention, as well as a purified proliferative glomerulonephritis-related polypeptide or proliferative glomerulonephritis-related polypeptide, are useful for screening an agent which specifically acts on a proliferative glomerulonephritis-related polypeptide. An agent obtained by the screening is useful in treatment of renal disease.

One method of the above-described screening is to select a target compound which specifically binds to a microorganism, animal cell or insect cell transformed so as to produce a proliferative glomerulonephritis-related polypeptide or a polypeptide which constitutes a part of a proliferative glomerulonephritis-related polypeptide of the present invention (hereinafter referred to as an "exploratory transformant"). By performing comparison with a control group of microorganisms, animal cells or insect cells which have not been transformed, the specific target compound can be detected. Further, competitive screening of a target compound can be performed by using, as an index, inhibition of binding of the compound or polypeptide which binds specifically to the exploratory transformant.

A purified proliferative glomerulonephritis-related polypeptide of the present invention or a polypeptide which constitutes a part of the proliferative glomerulonephritis-related polypeptide, can be used to select a target compound which specifically binds to a proliferative glomerulonephritis-related polypeptide. Quantitative determination of a target compound can be performed by an immunological method described above using an antibody which specifically recognizes a proliferative glomerulonephritis-related polypeptide of the present invention. Further, competitive screening of a target compound can be performed by using, as an index, inhibition of binding of a proliferative glomerulonephritis-related polypeptide or a compound which binds to the proliferative glomerulonephritis-related polypeptide.

Another example of a method for the above-described screening is a method in which many peptides constituting a part of a proliferative glomerulonephritis-related polypeptide are synthesized at high density on a plastic pin or a certain type of solid support, and a polypeptide or compound which selectively binds to the peptides is efficiently screened (WO84/03564).

In a kidney-derived cell strain, an agent for regulating expression which promotes expression of mRNA derived from proliferative glomerulonephritis-related gene or proliferative glomerulonephritis-related polypeptide is also useful in the treatment of renal disease.

By adding various test compounds to a kidney-derived cell line and assaying an increase or decrease in expression of mRNA derived from proliferative glomerulonephritis related gene using proliferative glomerulonephritis-related DNA of the present invention, a compound which suppresses or promotes transcription or translation of the proliferative glomerulonephritis related gene can be screened. The increase or decrease in expression of mRNA derived from proliferative glomerulonephritis-related gene can be detected by the above-described PCR, northern blotting, or RAase protection assay methods.

By adding various test compounds to a kidney-derived cell line and assaying an increase or decrease in expression of a proliferative glomerulonephritis-related polypeptide using an antibody which specifically recognizes a proliferative glomerulonephritis-related polypeptide of the present invention, a compound which promotes transcription or translation of a proliferative glomerulonephritis-related gene can be screened. The increase or decrease in expression of a proliferative glomerulonephritis-related polypeptide can be detected by radioimmuno assay (RIA), immunohistochemical staining such as immunohistological staining and immunological cell staining (ABC method, CSA method, and the like), western blotting, dot blotting, immunoprecipitation, and sandwich ELISA, as mentioned above.

By administering a compound obtained by the above method as an agent to a renal disease model animal such as Thy-1 nephritis rat, anti-GBM nephritis, serum sickness type nephritis, PAN nephrosis, daunomycin nephrosis, 5/6 nephrectomized rat, or spontaneous lupus nephritis, and measuring urinary polypeptides or albumin of the animal, the therapeutic effect of the compound in a renal disease can be evaluated.

### 11 Method for Delivering Drug Specifically to Kidney Using Antibody which Specifically Recognizes Proliferative Glomerulonephritis-Related Polypeptide (Drug Delivery Method)

An antibody which can be used in this drug delivery method may be any antibody which recognizes a proliferative glomerulonephritis-related polypeptide of the present invention, but in particular, it is desirable to use a humanized antibody.

Examples of a humanized antibody include human-type chimeric antibody, and human-type CDR (Complementary Determining Region, hereinafter referred to as "CDR") transplantation antibody.

Human-type chimeric antibody means an antibody comprising an antibody heavy chain variable domain (hereinafter also referred to as "HV" or VH," with "heavy chain" referred to as "H chain" and "variable region" referred to as "V region") and antibody light chain variable region (hereinafter also referred to as "LV" or VL," with "light chain" referred to as "L chain") of a non-human animal, and heavy chain constant region (hereinafter also referred to as "CH," with "constant region" referred to as "C region") of a human antibody and light chain constant region (hereinafter also referred to as "CL") of a human antibody. Any animal such as mouse, rat, hamster or rabbit can be used as a non-human animal, as long as it is possible to prepare a monoclonal antibody producing hybridoma.

A human-type chimeric antibody of the present invention can be produced by obtaining cDNA encoding VH and VL from a hybridoma which produces a monoclonal antibody which binds to a proliferative glomerulonephritis related polypeptide and neutralizes the action of the polypeptide of the present invention, inserting each cDNA into expression vectors for animal cell having a gene encoding human antibody CH and human antibody CL to construct a human-type chimeric antibody recombinant vector, and introducing and expressing the vector in an animal cell.

A CH of human-type chimeric antibody may be any of those belonging to human immunoglobulin (hereinafter referred to as "hIg"), and those of hIgG class are preferable, and any of subclasses hIgG1, hIgG2, hIgG3 and hIgG4 belonging to hIgG class can be used. Further, a CL of human-type chimeric antibody may be any of those belonging to hIg, and those of κ class or λ class can be used.

"Human-type CDR-grafted antibody" means an antibody in which an amino acid sequence of CDR of VH and VL of an antibody of a non-human animal was transplanted into a suitable position of VH and VL of a human antibody.

The human-type CDR-grafted antibody of the present invention can be produced by; constructing cDNA encoding V regions in which CDR sequences of VH and VL of any human antibody have been respectively substituted with CDR sequences of VH and VL of an antibody of a non-human animal which reacts with the proliferative glomerulonephritis-related polypeptide of the present invention, binds to the proliferative glomerulonephritis-related polypeptide of the present invention, and neutralizes an action of the proliferative glomerulonephritis-related polypeptide of the present invention; inserting the respective cDNA into an expression vector for animal cell having a gene encoding CH of a human antibody and CL of a human antibody to construct a human-type CDR-grafted antibody recombinant vector; and introducing and expressing the vector in an animal cell.

A CH of human-type CDR-grafted antibody may be any of those belonging to hIg, and those of hIgG class are preferable, and any of subclasses hIgG1, hIgG2, hIgG3 and hIgG4 belonging to hIgG class can be used. Further, a CL of human-type CDR-grafted antibody may be any of those belonging to hIg, and those of κ class or λ class can be used.

"Human antibody" originally means an antibody naturally present in the human body, but also includes an antibody obtained from a human antibody phage library or human antibody-producing transgenic animal produced based on recent advances in genetic engineering, cellular engineering and developmental engineering techniques.

An antibody present in the human body can be obtained, for example, by the following method.

Human peripheral blood lymphocytes are isolated, and are immortalized by infecting them with EB virus or the like, and then are cloned. The obtained lymphocytes producing the antibody of interest are cultured to obtain the antibody from the culture product.

A human antibody phage library is a library in which antibody fragments such as Fab or single chain antibody are expressed on a phage surface by inserting an antibody gene prepared from human B cell into a phage gene. From this library, by using binding activity to a substrate on which antigen is immobilized as an index, the phage which expresses an antibody fragment having a antigen binding activity of interest can be recovered. The antibody fragment can be further converted into a complete type human antibody by a genetic engineering technique.

"Human antibody-producing transgenic animal" means an animal in which a human antibody gene has been intracellularly incorporated. Specifically, a human antibody-producing transgenic animal can be produced by introducing a human antibody gene into a mouse ES cell, transplanting the ES cell to an early embryo of another mouse, and then developing it. Examples of a method of producing human antibody from a human antibody-producing transgenic animal include a method of producing and accumulating human antibody in culture product by obtaining and culturing a human antibody-producing hybridoma by a hybridoma production method which is conventionally performed in a non-human mammalian.

Examples of an antibody fragment include Fab, Fab', F(ab') 2, single strand antibody, disulfide stabilized V region fragment (hereinafter also referred to as "dsFv"), and peptide including CDR.

Fab is an antibody fragment having a molecular weight of approximately 50,000 and antigen binding activity wherein, among the fragments obtained by treatment of IgG with protease papain (cleaved at 224th amino acid residue of H chain), approximately half of the N-terminus side of H chain and all of L chain are bonded by disulfide bond.

Fab of the present invention can be obtained by treating an antibody which specifically reacts with a polypeptide of the present invention with protease papain. Further, Fab can be obtained by inserting DNA encoding Fab of the antibody into an expression vector for a prokaryote or an expression vector for a eukaryote, introducing the vector into a prokaryote or eukaryote, and expressing the DNA.

F(ab') 2 is an antibody fragment having a molecular weight of approximately 100,000 and antigen binding activity, which is slightly larger fragment than those obtained by binding Fab via disulfide binding of hinge region among the fragments obtained by treatment of IgG with protease pepsin (cleaved at 234th amino acid residue of H chain).

F(ab') 2 of the present invention can be obtained by treating an antibody which specifically reacts with the polypeptide of the present invention with protease pepsin. Further, Fab' can be acquired by inserting DNA encoding F(ab') 2 of the antibody into an expression vector for a prokaryote or an expression vector for a eukaryote, introducing the vector into a prokaryote or eukaryote, and expressing the DNA.

Fab' is an antibody fragment having a molecular weight of approximately 50,000 and antigen binding activity which is obtained by cleaving disulfide binding of hinge region of the above-described F(ab') 2.

Fab' of the present invention can be obtained by treating an antibody which specifically reacts with a polypeptide of the present invention with a reducing agent, dithiothreitol. Further, Fab' can be obtained by inserting DNA encoding Fab' fragment of the antibody into an expression vector for a prokaryote or an expression vector for a eukaryote, introducing the vector into a prokaryote or eukaryote, and expressing the DNA.

"Single chain antibody" (hereinafter also referred to as "scFv") means a VH-P-VL or VL-P-VH polypeptide obtained by connecting a single chain VH and single chain VL using an adequate peptide linker (herein referred to as "P"). For VH and VL contained in scFv used in the present invention, an antibody which specifically reacts with a polypeptide of the present invention, for example, an antibody derived from humanized antibody or human antibody, can be used.

Single chain antibody of the present invention can be obtained by the following method.

cDNA encoding VH and VL of an antibody which specifically reacts with a polypeptide of the present invention is obtained, and then DNA encoding single chain antibody is constructed. The DNA is inserted into an expression vector for a prokaryote or an expression vector for a eukaryote, and the recombinant vector is introduced into a prokaryote or eukaryote to express the DNA. Thus, single chain antibody can be obtained.

"Disulfide-stabilized V region fragment" (dsFv) means a fragment obtained by binding the polypeptides wherein 1 amino acid residue in VH and VL is substituted with a cysteine residue via disulfide binding between the cysteine residues. An amino acid residue to be substituted with a cysteine residue can be selected based on the prediction of the steric structure of an antibody in accordance with a method shown by Reiter et al. [Protein Engineering, 7, 697 (1994)]. For VH and VL contained in dsFv used in the present invention, an antibody which specifically reacts with the polypeptide of the present invention, for example, an antibody derived from humanized antibody or human antibody, can be used.

Disulfide-stabilized V region fragment (dsFv) of the present invention can be obtained by the following method.

cDNA encoding VH and VL of an antibody which specifically reacts with the polypeptide of the present invention is obtained, and then DNA encoding dsFv is constructed. The DNA is inserted into an expression vector for a prokaryote or an expression vector for a eukaryote. Then, the recombinant vector is introduced into a prokaryote or eukaryote; and the DNA is expressed to obtain dsFv.

A peptide containing CDR can be prepared by a chemical synthesis method such as an Fmoc method or tBoc method.

A fusion antibody described below which is prepared by using an antibody of the present invention can be used in drug delivery for transporting an agent or protein to a renal lesion.

"Fusion antibody" means an antibody in which an agent such as a radioisotope, polypeptide or low molecular weight compound or the like is bonded chemically or by genetic engineering to an antibody which specifically reacts with a polypeptide of the present invention, for example, a humanized antibody, a human antibody or an antibody fragment thereof.

The fusion antibody of the present invention can be produced by binding chemically or by genetic engineering, an agent such as a radioisotope, polypeptide or low molecular weight compound or the like to the N terminus side or C terminus side of an H chain or L chain of an antibody which specifically reacts with the polypeptide of the present invention or an antibody fragment thereof, to an adequate substituent or side chain in the antibody or antibody fragment, or to a sugar chain in the antibody or antibody fragment.

Examples of a radioisotope include ¹³¹I and ¹²⁵I, which can be bound to an antibody or antibody fragment by a chloramine-T method or the like.

Examples of a low molecular weight compound include alkylating agents such as nitrogen mustard and cyclophosphamide; antimetabolites such as 5-fluorouracil and methotrexate; antibiotics such as daunomycin, bleomycin, mitomycin C, daunorubicin and doxorubicin; anticancer agents including plant alkaloids such as vincristine, vinblastine and vindesine, and hormone agents such as tamoxifen and dexamethasone [Clinical Oncology (in Japanese) (Japanese Clinical Tumor Society Edition, 1996, Cancer and Chemotherapy Co.)]; as well as steroid drugs such as hydrocortisone and prednisone; nonsteroid drugs such as aspirin and indomethacin; immunomodulators such as gold thiomalate and penicillamine; immunosuppressors such as cyclophosphamide and azathioprine; and anti-inflammatory agents such as antihistamines like chlorpheniramine maleate and clemastine [Inflammation and Antiinflammation Therapy (in Japanese) (1982) Ishiyaku Shuppan Kabushiki Kaisha].

A low molecular weight compound can be bonded to the above-described antibody by a standard method. For example, methods for binding daunomycin with an antibody include a method of binding daunomycin with an amino group of an antibody via glutaraldehyde, and a method of binding an amino group of daunomycin and a carboxyl group of an antibody via soluble carbodiimide.

As a polypeptide, cytokines which activates immunocompetent cells or growth controlling factors of vascular endothelium, vascular smooth muscle and the like are preferable, and examples include human interleukin 2, human granulocyte-macrophage colony-stimulating factor, human macrophage colony-stimulating factor, human interleukin 12, fibroblast growth factor-2 (FGF-2), and platelet-derived growth factor (PDGF).

A fusion antibody with a polypeptide can be obtained by the following method.

cDNA encoding a polypeptide is ligated to cDNA encoding an antibody or antibody fragment to construct DNA encoding a fusion antibody. The fusion antibody can be obtained by inserting the DNA into an expression vector for a prokaryote or an expression vector for a eukaryote, introducing the recombinant vector into a prokaryote or eukaryote, and expressing the DNA.

### 12 Therapeutic Agent for Renal Disease which Comprises Proliferative Glomerulonephritis-Related Polypeptide

The proliferative glomerulonephritis-related polypeptide of the present invention can be used for the reconstruction of a structure and function of a kidney in renal disease typically represented by nephritis.

A therapeutic agent for renal disease which comprises a proliferative glomerulonephritis-related polypeptide of the present invention may contain only the polypeptide as an active ingredient, however, it is preferable that the polypeptide is normally mixed together with one or more pharmacologically acceptable carriers and provided as a medical preparation prepared by any method well-known in the technical field of pharmaceutics.

As the route of administration, it is preferred to use the most effective one at the time of treatment, and examples include oral administration, or parenteral administration such as intraoral, tracheobronchial, endorectal, subcutaneous, intramuscular and intravenous administration. Examples of dosage form include a nebula, a capsule, a tablet, a granule, syrup, an emulsion, a suppository, an injection, an ointment, and a tape.

Examples of a suitable preparation for oral administration include an emulsion, syrup, a capsule, a tablet, a powder, and a granule. For example, liquid preparations such as an emulsion or syrup can be produced by using, as an additive, water; sugars such as sucrose, sorbitol and fructose; glycols such as polyethylene glycol and propylene glycol; oils such as benne oil, olive oil and soybean oil; an antiseptic such as p-hydroxybenzoic acid ester; flavors such as strawberry flavor and peppermint; and the like. A capsule, a tablet, a powder, a granule, and the like can be produced by using, as an additive, an excipient such as lactose, glucose, sucrose or mannitol; a disintegrating agent such as starch or sodium alginate; a lubricant such as magnesium stearate or talc; a binding agent such as polyvinyl alcohol, hydroxypropylcellulose or gelatin; a surfactant such as fatty acid ester; a plasticizer such as glycerine; and the like.

Examples of a suitable preparation for parenteral administration include an injection, a suppository and a nebula. For example, an injection is prepared by using a carrier or the like comprising a salt solution, a glucose solution or a mixture thereof. A suppository is prepared by using a carrier comprising theobroma oil, hydrogenated fat, carboxylic acid, or the like. Further, a nebula is prepared by using the polypeptide alone or together with a carrier or the like which can disperse the polypeptide as fine particles and makes the absorption easy without stimulating the oral cavity and mucosa of respiratory tract of a recipient. Specific examples of a carrier include lactose and glycerine. Depending on the properties of the polypeptide and carrier to be used, a formulation such as an aerosol or a dry powder is also possible. Further, the ingredients exemplified above as additives for an oral agent can be added in these parenteral agents.

An administration dose and administration frequency differs depending on the type of the disease, the administration method, the treatment period, the age, the body weight and the like. Normally, a dose for an adult is 10 µg/kg to 8mg/kg per day.

### 13 Gene Therapy Agent which Comprises Proliferative Glomerulonephritis-Related DNA

A gene therapy agent using a virus vector containing proliferative glomerulonephritis-related DNA of the present invention can be produced by combining the recombinant virus vector prepared in Item 4. above and a base for use in a gene therapy agent [Nature Genet., 8, 42(1994)].

As a base for use in a gene therapy agent, any base which is normally used in an injection may be used, and examples include distilled water; salt solution such as sodium chloride or a mixture of sodium chloride and an inorganic salt; a sugar solution such as mannitol, lactose, dextran or glucose; an amino acid solution such as glycine or arginine; and a mixture of organic acid solution or salt solution and glucose solution. Further, by using an auxiliary agent such as an osmoregulation agent, a pH adjuster, vegetable oil such as benne oil or soybean oil, or a surfactant such as lecithin or a nonionic surfactant together with the aforementioned bases in accordance with the usual manner, an injection may be prepared as a solution, a suspension, or a dispersion. These injections can also be prepared as formulations to be dissolved before use by an operation such as powderization or lyophillization. The gene therapy agent of the present invention can, in the case of a liquid, be used for treatment as it is, and in the case of a solid, be used after dissolving immediately before gene therapy in the above-described base which has been sterilized as necessary. Examples of an administration method of the gene therapy agent of the present invention include a method of administering locally so as to be absorbed in treatment area of the kidney of the patient.

As a system for transporting a virus vector to a renal lesion more specifically, there is a method of using a fusion protein of a single chain antibody which specifically recognizes BMP-7 receptor and Env protein of a retrovirus vector [Proc. Natl. Acad. Sci. USA, 92, 7570-7574 (1995)]. This system is not limited to a retrovirus vector, and may also be applied to a lentivirus vector and the like.

A virus vector can be prepared by preparing a complex by combining a proliferative glomerulonephritis-related DNA of the present invention of a suitable size with polylysine-conjugate antibody which is specific to adenovirus hexon polypeptide, and then binding the obtained complex to an adenovirus vector. The virus vector stably reaches a target cell, is incorporated into the cell by an endosome, and is decomposed within the cell to efficiently express the gene.

A virus vector based on Sendai virus which is (-)strand RNA virus has also been developed (WO97/165 WO97/16539), and it is also possible to prepare a Sendai virus vector incorporating KRGF-1 gene for the purpose of gene therapy.

Proliferative glomerulonephritis-related DNA can also be transported to a renal lesion by a non-viral gene transfer technique.

Examples of non-viral gene transfer techniques known in the art include a calcium phosphate coprecipitation [Virology, 52, 456-467 (1973); Science, 209, 1414-1422 (1980)], a microinjection [Proc. Natl. Acad. Sci. USA, 77, 5399-5403 (1980); Proc. Natl. Acad. Sci. USA, 77, 7380-7384 (1980); Cell, 27, 223-231 (1981); Nature, 294, 92-94 (1981)], membrane fusion mediated by liposome [Proc. Natl. Acad. Sci. USA, 84, 7413-7417 (1987); Biochemistry, 28, 9508-9514 (1989); J. Biol. Chem., 264, 12126-12129 (1989); Hum. Gene Ther., 3, 267-275, (1992); Science, 249, 1285-1288 (1990); Circulation, 83, 2007-2011 (1992)], and a direct DNA incorporation and receptor-mediation DNA transfer [Science, 247, 1465-1468 (1990); J. Biol. Chem., 266, 14338-14342 (1991); Proc. Natl. Acad. Sci. USA, 87, 3655-3659 (1991); J. Biol. Chem., 264, 16985-16987 (1989); BioTechniques, 11, 474-485 (1991); Proc. Natl. Acad. Sci. USA, 87, 3410-3414 (1990); Proc. Natl. Acad. Sci. USA, 88, 4255-4259 (1991); Proc. Natl. Acad. Sci. USA, 87, 4033-4037 (1990); Proc. Natl. Acad. Sci. USA, 88, 8850-8854 (1991); Hum. Gene Ther., 3, 147-154 (1991)].

It has been reported in tumor-related research that in the method of transferring gene using membrane fusion mediated by liposome by directly administering a liposome preparation to target tissue, localized incorporation and expression of the gene in the tissue becomes possible [Hum. Gene Ther. 3, 399-410 (1992)]. Therefore, a similar effect can also be expected in a renal lesion. For direct targeting of DNA to a renal lesion, a direct DNA-incorporation technique is preferred. Transferring DNA mediated by receptor is performed, for example, by conjugating DNA (normally, a supercoiled plasmid which is covalently cyclized) to a protein ligand via polylysine. The ligand is selected based on the presence of a corresponding ligand receptor on the cell surface of a target cell or tissue. Examples of a receptor and ligand combination include a combination of BMP-7 receptor and BMP-7. The ligand-DNA conjugate can, as desired, be directly injected into a blood vessel, and can be directed to target tissue in which binding of receptor and internalization of the DNA-polypeptide complex occurs. To prevent intracellular destruction of DNA, it is possible to simultaneously infect with adenovirus and destroy the endosome functions.

### 14 Therapeutic Agent for Renal Disease which Comprises Antibody which Specifically Recognizes Proliferative Glomerulonephritis-Related Polypeptide

An antibody which specifically recognizes a proliferative glomerulonephritis-related polypeptide of the present invention can be directly utilized in the treatment of diseases such as renal cancer in which cell neogenesis in the kidney is progressing.

A therapeutic agent which comprises an antibody which specifically recognizes a proliferative glomerulonephritis-related polypeptide of the present invention may contain only the antibody as an active ingredient. Normally, it is preferable that the polypeptide is mixed together with one or more pharmacologically acceptable carriers and provided as a medical preparation prepared by any method well-known in the technical field of pharmaceutics. Preparation and administration of the therapeutic agent can be carried out in accordance with the description for the agent containing a proliferative glomerulonephritis-related polypeptide described in 13. above.

Hereinafter, examples of the present invention are described.

### BEST MODE FOR CARRYING OUT THE INVENTION

### Example 1 Preparation of Thy-1 nephritis rat kidney cDNA library

Anti-rat Thy-1 monoclonal antibody OX-7 (manufactured by Cedarlane) was administered by tail vein injection to 20 Wistar rats (males) (manufactured by Japan SLC Co., body weight; approximately 200 g) at a dose of 1mg/kg, thereby inducing nephritis. Physiological saline was administered to a control group. For these rats, the polypeptide concentration in urine was measured using urine analysis stick pretest 6B (manufactured by Wako Pure Chemical Industries), and was used as an index of nephritis condition.

On each of days 2, 4, 6, 8, 10, 13 and 16 after administration of OX-7, kidney was extracted from 3 rats (on day 16 only, from 2 rats), as well as from rats of the control group, and total RNA was extracted from each individual by the guanidine thiocyanate-trifluoroacetic acid cesium technique [Methods in Enzymology, 154, 3 (1987)]. For the control group, total RNA was not extracted from each individual, and instead total RNA was extracted from a mixture comprising equal amounts of renal tissue lysate prepared by treating the kidneys of 3 individuals on day 2 after administration of physiological saline and renal tissue lysate prepared by treating the kidneys of 3 individuals on day 10 after administration. Of these total RNAs, the total RNA of each individual rat kidney of days 2, 4, 6, 8 and 10 after OX-7 administration were mixed together in equal amounts, and poly (A) RNA was prepared by using oligo(dT) cellulose. Then, using ZAP-cDNA Synthesis Kit (manufactured by Stratagene), a cDNA library (total independent plaques of 1.0 x 10⁶) was prepared (Thy-1 nephritis rat kidney cDNA library). Details of the method for preparing the cDNA are as described in the kit manual. This cDNA library is inserted between the Xho I/EcoR I sites of the vector in such a way that the 5' end of cDNA was on the EcoR I site side, with using λ phage vector λ ZAP II (manufactured by Stratagene) as a vector.

### Example 2 Preparation of subtracted library

### (1) Preparation of single strand DNA

By infecting host cell Escherichia coli XL1-Blue MRF' (manufactured by Stratagene) with the Thy-1 nephritis rat kidney cDNA library prepared in Example 1 together with helper phage ExAssist (manufactured by Stratagene) and performing in vivo excision, a phagemid pBluescript SK(-) region containing cDNA was excised from the vector as a single strand DNA phage, and was released into a culture supernatant. The method of in vivo excision was performed in accordance with Strategene's manual 200 µl of this culture supernatant (titer: 8.5 x 10⁵ cfu/µl) was added to 7ml of 10 mmol/l MgSO₄ containing 1.8 x 10¹⁰ Escherichia coli SORL (manufactured by Stratagene) as a host cell which cannot be infected with ExAssist, and incubated at 37°C for 15 minutes. The total volume was added to 200 ml of 2-fold YT culture medium (1.6% Bacto-tryptone, 1% yeast extract), and the mixture was cultured with shaking for 1 hour at 37°C, and was infected with single strand DNA phage containing cDNA. Ampicillin was added thereto at a concentration of 50 µg/ml, the mixture was again subjected to shaking culture for 1 hour at 37°C to culture only phage-infected Escherichia coli. The number of the cells was measured by an absorbance of 600 nm, and the result was 4 x 10¹⁰ cells. Helper phage R408 (manufactured by Stratagene) was added at multiplicity of infection (moi) = 13 (5.3 x 10¹¹ pfu), and the mixture was cultured with shaking for 7 hours at 37°C to release single strand DNA again in the supernatant. The culture medium was transferred to a sterile tube, centrifuged for 10 minutes at 10,000 rpm at 4°C, and only supernatant containing phage was transferred and recovered in a new sterile tube. After centrifugation of this supernatant under the same conditions, the cells were completely removed by passing through a sterile filter of 0.22 mm pore size (manufactured by Millipore). 20 ml of 10-fold buffer [100 mmol/l Tris-HC1 (pH 7.5), 100 mmol/l MgCl₂] and 140 units of deoxyribonuclease I (manufactured by Nippon Gene) were added, and the mixture was reacted at 37°C for 30 minutes. 2.5 mol/l NaCl solution (1/4 volume of the reacted mixture ) of 20% polyethyleneglycol (molecular weight 6,000) was added thereto, and the mixture was mixed well at room temperature and allowed to stand for 20 minutes, and then centrifuged for 10 minutes at 10,000 rpm at 4°C to precipitate phage. The supernatant was completely removed, and the obtained phage precipitate was dissolved in 400 µl of TE [10 mmol/l Tris-HC1 (pH 8.0), 1 mmol/l EDTA (pH 8.0)]. Then, 4 µl of 10% SDS and 625 µg (25 µl) of proteinase K were added thereto, and the mixture was allowed to react for 1 hour at 42°C. After phenol extraction, phenol-chloroform extraction and chloroform extraction, the aqueous layer was fractionated, then ethanol precipitation was performed to obtain 77.6 µg of single strand DNA [vector pBluescript SK(-)] of Thy-1 nephritis rat kidney cDNA library.

### (2) Biotinylation of RNA

From 1.2 mg of total RNA prepared from kidney of control group rat of Example 1, 20 µg of poly(A) RNA was prepared by using oligo(dT) cellulose. To 10 µg of it was added distilled water to bring to 20 µl in a test tube, and 30 µg (30 µl) of 1 mg/ml PHOTOPROBE biotin (manufactured by Vector Laboratories) was added thereto in a dark place. The cap of the test tube was opened and the tube was placed on ice, and RNA was biotinylated by light irradiation for 20 minutes using a mercury lamp from a height of about 10 cm. After addition of 50 µl of a solution of 100 mmol/l 1 Tris-HC1 (pH 9.5) and 1 mmol/l EDTA (pH 8.0) to the reaction solution, extraction with water saturated butanol was performed 3 times, and chloroform extraction was performed twice, and then the aqueous layer was precipitated with ethanol. The recovered precipitate of RNA was dissolved in 20 µl of distilled water, the above-described biotinylation reaction operation (operation from addition of PHOTOPROBE biotin to precipitation with ethanol) was performed once more to obtain biotinylated RNA.

### (3) Subtraction

To 0.5 µg (1 µl) of single strand DNA of the Thy-1 nephritis rat kidney cDNA library prepared in (1) was added 12.5 µl of 2x hybridization buffer [80% formamide, 100 mmol/l HEPES (pH 7.5), 2 mmol/l EDTA (pH 8.0), 0.2% SDS], 2.5 µ l of 2.5 mol/l NaCl, and 1 µg (1 µl) of poly(A) (manufactured by Amersham Pharmacia Biotech), and then 8 µl of biotinylated RNA (RNA 10 µg) prepared in (2) was dissolved in distilled water and added. After the mixture was heated at 65°C for 10 minutes, hybridization was performed at 42°C for 63 hours.

To the solution after hybridization reaction was added 400 µl of buffer [500 mmol/l NaCl, 50 mmol/l HEPES (pH 7.5), 2 mmol/l EDTA (pH 8.0)], and 10 µg (5 µl) of streptavidin (manufactured by Life Technologies) was then added thereto, and the mixture was allowed to react at room temperature for 5 minutes. Phenol-chloroform extraction was performed and a complex of streptavidin-biotinylated RNA-hybridized cDNA was removed from the aqueous layer. 10 µg of streptavidin was again added to the aqueous layer and allowed to react at room temperature for 5 minutes, and phenol-chloroform extraction was performed twice, after which chloroform extraction was performed and the aqueous layer recovered. After passing the aqueous layer through Unit Filter Ultra Free C3 Plus TK (manufactured by Millipore) to allow cDNA be adsorbed in the filter, and the filter was washed. Then, concentrated and desalted cDNA was recovered in 30 µl of 1/10 TE [1 mmol/l Tris-HC1 (pH8.0), 0.1 mmol/l EDTA (pH 8.0)]. Operations using this filter were performed in accordance with Millipore's manual. By this subtraction operation, cDNA of a gene whose expression level was high in both Thy-1 nephritis rat kidney and control group rat kidney was removed from the cDNA library, and cDNA of a gene which was expressed in Thy-1 nephritis rat kidney but almost not expressed in control group rat kidney was concentrated. However, when using only the above subtraction operation, cDNA of a gene which was expressed at a very low level in Thy-1 nephritis rat kidney but almost not expressed in control group rat kidney would also be concentrated. Therefore, reverse differentiation as described in (5) below was performed, and a library was prepared that did not include cDNA of a gene expressing at a very low level in Thy-1 nephritis rat kidney.

### (4) Amplification of cDNA after subtraction

Since it was considered that the amount of the cDNA had decreased considerably following the subtraction operation of (3), in order to perform the reverse subtraction described in (5) below, the amount was increased in the following manner. 14 µl of distilled water and 2 µg (1 µl) of 5'-AP primer was added to a half amount (15 µl) of the cDNA (single strand DNA) after subtraction. After heating for 10 minutes at 65°C, the mixture was left to stand at room temperature for 5 minutes to anneal the primer to the single strand DNA. 5 µl of 10x Bca BEST reaction buffer [which is attached with BcaBEST Dideoxy Sequencing Kit (manufactured by Takara Shuzo)], 10 µl of 1 mmol/l dNTP (mixture of 1 mmol/l each of dATP, dGTP, dCTP, and TTP), 1.5 µg (0.5 µl) of single strand DNA-binding polypeptide (manufactured by USB), 4 units (2 µl) of BcaBEST DNA polymerase (manufactured by Takara Shuzo) and 2.5 µl of distilled water were added thereto, and the solution was allowed to react for 1 hour at 65°C to synthesize double strand DNA. 50 µl of distilled water was added to the reaction solution, and phenol-chloroform extraction and chloroform extraction were performed. Then, double stranded DNA was finally recovered in 20 µl of TE by using a Unit Filter Ultra Free C3 Plus TK in the same manner as described in (3).

The total amount of the recovered double strand DNA (4 µl) was introduced into Escherichia coli DH12S (manufactured by Life Technologies) by electroporation. To the Escherichia coli DH12S after the electroporation operation was added 1.5 ml of SOC culture medium, and this was then inoculated into 42.5 ml of LB-Ap culture medium (1% Bacto-tryptone 0.5% yeast extract, 1% NaCl, 50 µg/ml ampicillin). The titer at this stage was 4.3 x 10⁶ cfu. After culturing at 37°C for 4 hours, the number of the cells were measured by measuring the absorbance of 600nm, and the result was 1x 10⁸ to 1.5 x 10⁸ cells/ml. Dimethyl sulfoxide was added to the half amount of the culture at a concentration of 7%, and this was stored at -80°C. The remaining half amount of the culture was infected with helper phage R408 of moi = 14-20 (5 x 10⁸ pfu). After culturing at 37°C for 15 minutes, each 5 ml of the mixture was inoculated into 5 x 45 ml of 2-fold YT culture medium, and then cultured at 37°C. 2 hours and 30 minutes after the start of culturing, ampicillin was added at a concentration of 100 µg/ml, and this was then cultured for a further 5 hours and 30 minutes to release single strand DNA phage to the culture solution. In the same manner as (1), 30.8 µg of single strand DNA was purified from this culture solution.

### (5) Reverse subtraction

2.5 µg of poly(A) RNA of Thy-1 nephritis rat kidney prepared in Example 1 was biotinylated in the same manner as in (2). This biotinylated RNA was added to 2.5 µg of the single strand DNA prepared in (4) after subtraction, and distilled water was added thereto to bring to 9 µl. Added thereto was 12.5 µl of 2-fold hibridization buffer which was the same as that used in the subtraction of (3), 2.5 µl of 2.5 mol/l NaCl, and 1 µg (1 µl) of poly(A). After heating the mixture at 65°C for 10 minutes, hybridization was performed at 42°C for 59 hours.

Streptavidin was reacted with the solution after hybridization reaction in the same manner as in the differentiation of (3). Phenol-chloroform extraction was performed to remove the aqueous layer, and a phenol-chloroform layer containing a complex of biotinylated RNA derived from Thy-1 nephritis rat kidney and hybridized cDNA was recovered. After repeating 3 times the operation of addition of TE and extraction, TE was again added to the phenol-chloroform layer, and the mixture was heated at 95°C for 5 minutes, to thereby dissociate the biotinylated RNA and cDNA. After the reaction layer was quenched by immersion in ice water, the solution was stirred vigorously and dissociated cDNA was extracted into an aqueous layer. After heating this solution once more for 5 minutes at 95°C, the quenching and extraction operation was repeated, and an aqueous layer containing dissociated cDNA was recovered by centrifugation. After performing phenol-chloroform extraction and chloroform extraction for the aqueous layer, the aqueous layer was passed through Unit Filter Ultra Free C3 Plus TK in the same manner as in (3). After cDNA was adsorbed in the filter, the filter was washed. Concentration and desalting of cDNA was performed to recover cDNA in 30 µl of 1/10 TE.

### (6) Preparation of cDNA library

For the single strand cDNA obtained in (5) after reverse subtraction, half amount of the cDNA was made into double stranded DNA in the same manner as in (4). 1/8 amount of the double strand DNA was introduced into Escherichia coli DH12S by electroporation to prepare a reverse-subtracted cDNA library. From the analysis using one portion of the library, it was estimated that the number of independent colonies of the library was 2.5 x 10⁴ cfu and that the ratio of cDNA insertion was 98%.

### Example 3 Differential Hybridization

### (1) Preparation of array filter

Using the reverse-subtracted cDNA library prepared in (6) of Example 3, colonies were formed on LB-Ap agar medium, and 9,600 colonies among them were inoculated onto 100 96-well plates in which 100 µl of LB-Ap culture medium had been added, at 1 colony/well. After each colony was cultured in the 96-well plates at 37°C, 50 µl of 50% glycerol was added thereto and the colonies were then stored at -80°C (this storage culture solution is hereinafter referred to as "glycerol stock").

Onto 96-well plates, each well of which contains 100 µl of LB-Ap culture medium, glycerol stock was again inoculated using 96 pin replicators, and the plates were left to stand for culturing overnight at 37°C. Using an automatic microdispenser, Hydra 96, the culture solution containing Escherichia coli was spotted in spots of 0.5 µl each on nylon membranes in the same lattice formation as the 96-well plate (8 vertically x 12 horizontally). On one nylon membrane were spotted 384 colonies in a lattice formation (16 vertically x 24 horizontally), which corresponded to the total amount of 4 plates of 96-well plates, and one colony was spotted in the same position on 2 membranes so that 2 of the same membranes could be prepared. Membranes spotted with the culture were placed on LB-Ap agar medium, with the spotted surface upward, and were cultured overnight at 37°C.

After the membranes on which colonies of Escherichia coli had grown sufficiently were stripped from the culture medium, the membranes were placed on paper soaked with denaturing solution for DNA (0.5 mol/l NaOH, 1.5 mol/l NaCl), and left at room temperature for 10 minutes to denature DNA. The membranes were then transferred to paper soaked with neutralizing solution [1.0 mol/l Tris-HC1 (pH 7.5), 1.5 mol/l NaCl] and left at room temperature for 10 minutes. After abrasively washing the cell clusters on the membrane in a sufficient amount of 2-fold SSC (0.3 mol/l sodium chloride, 30 mmol/l sodium citrate) containing 0.5% SDS which was prepared in a bath, washing was performed by replacing the same buffer two times. Membranes were transferred to polyethylene bags, a reaction buffer [50 mol/l tris-hydrochloric acid (pH8.5), 50 mol/l EDTA, 100 mol/l sodium chloride, 1% sodium lauroyl sarcosinate] in which proteinase K was dissolved at a concentration of 250 µg/ml was added thereto, and the bags were sealed, and reaction was carried out for 2 hours at 37°C. After the membranes were removed from the bags and washed with 2-fold SSC, the membranes were once again put into polyethylene bags, 2-fold SSC containing proteinase inhibitor Pefabloc (manufactured by Roche) at a concentration of 400 µg/ml was added thereto, and the bags were sealed and treated at room temperature for 1 hour. The membranes were removed from the bags and washed with 2-fold SSC. Finally, DNA was immobilized on the membranes by ultraviolet irradiation using crosslinker optimal link (manufactured by Funakoshi). The thus obtained membranes are referred to as "array filters."

### (2) Preparation of riboprobe

Using poly(A) RNA derived from Thy-1 nephritis rat kidney and control group rat kidney prepared in Example 1, digoxigenin (DIG) labeled riboprobes were prepared in the following manner. Since the number of membranes is large and a large amount of probes is required, 150 µg of probes is necessary for performing hybridization for 50 membranes of 100 cm². Firstly, double stranded cDNA was prepared from poly(A) RNA, and T7 RNA polymerase reaction was carried out with employing this cDNA as a template, to thereby obtain riboprobes incorporated with DIG.

### (2)-1 Preparation of double stranded cDNA

5 µg of each poly(A) RNA was mixed with 8 µg of T7(dT) primer (SEQ ID NO: 161; having T7 promoter sequence at 5' end), and distilled water (pure water that was distilled a further 2 times, the same applies hereinafter) was added to bring the mixture to 7.8 µl. The mixture was heated at 70°C for 10 minutes and was quenched on ice. 4 µl of 5-fold hybridization buffer (a buffer attached with commercially available enzyme), 2 µl of 100 mmol/l DTT and 1.2 µl of 10 mmol/l dNTP was added thereto, and the mixture was mixed well by pipetting. After incubating at 37°C for 2 minutes to perform annealing, 5 µl of Superscript II reverse transcriptase (manufactured by Life Technologies) was added, and the reaction was carried out at 42°C for 1 hour to synthesize single strand cDNA, and then the mixture was cooled with ice.

To the solution after reaction were added 92.3 µl of distilled water, 32 µl of 5-fold hybridization buffer [94 mmol/l tris-hydrochloric acid (pH 6.9), 453 mmol/l potassium chloride, 23 mmol/l magnesium chloride, 750 µmol/l β -NAD, 50 mmol/l ammonium sulfate], 3 µl of 10 mmol/l dNTP, 6 µl of 100 mmol/l DTT, 15 units (2.5 µl) of Escherichia coli DNA ligase (manufactured by Takara Shuzo), 40 units (11.5 µl) of Escherichia coli DNA polymerase I (manufactured by Takara Shuzo), and 1.2 units (2 µl) of Escherichia coli ribonuclease H (manufactured by Takara Shuzo), in that order. After mixing well by pipetting on ice, the mixture was allowed to react at 16°C for 2 hours and 30 minutes to synthesize double stranded cDNA. The Escherichia coli DNA ligase and Escherichia coli ribonuclease H were used after diluting with a 1-fold reaction buffer immediately before the reaction to dilute the stock solutions to 6 units/µl and 0.6 units/µl, respectively. After reaction, 2 µl of 0.5 mol/l EDTA and 2 µl of 10% SDS were added to stop the reaction, and an aqueous layer was recovered by phenol-chloroform extraction. Then, 70 µl of TE was further added to the phenol-chloroform layer excluding the aqueous layer, and extraction was carried out, and the aqueous layer was combined with the aqueous layer recovered earlier. To this aqueous layer was added 12 µl of proteinase K (2mg/ml), and this was allowed to react at 42°C for 1 hour. After recovering an aqueous layer by phenol-chloroform extraction in the solution after reaction, 70 µl of TE was added to the phenol-chloroform layer excluding the aqueous layer and extracted, and the aqueous layer was combined with the aqueous layer recovered earlier.

Using Unit Filter Ultra Free C3LTK (manufactured by Millipore), the aqueous layer was subjected to concentration and desalting. Specifically, the aqueous layer was placed in a filter cup and centrifuged at 8,000 rpm for 5 minutes, thereby adsorbing DNA in the filter. Solution which moved to the lower part was removed, and 300 µl of distilled water was placed in the filter cup, which was again centrifuged at 8,000 rpm for 5 minutes, thereby washing the filter. After repeating this washing operation once more, 25 µl of distilled water was placed in the filter cup, and suspended by pipetting to extract DNA. The filter cup was taken out and inserted upside-down into a tube for centrifugation (Falcon 2059), which was then centrifuged to collect the suspension in the bottom of the tube. 25 µl of distilled water was placed in the filter cup once more, and the suspension was recovered in the same manner (total of 50 µl).

### (2)-2 Synthesis and labeling of RNA

From double stranded cDNA obtained in the above-described manner, DIG labeled riboprobes were prepared using DIG RNA Labeling Kit (manufactured by Roche). The method was in accordance with Roche's DIG System Users Guide. Specifically, 20 µl of a mixture of 1 µg of cDNA (prepared to 14 µl with distilled water), 2 µl of 10x reaction buffer (buffer included in kit), 2 µl of NTP labeling mix (included in kit, and containing DIG-11-UTP) and 2 µl of T7 RNA polymerase (manufactured by Roche) was mixed by pipetting, and then allowed to react at 37°C for 2 hours. After stopping the reaction by adding 0.8 µl of 0.5 mol/l EDTA, 2.3 µl 4mol/l lithium chloride (1/9 volume of reaction solution) and 65 µl ethano (2.5-3 times volume of reaction solution) were added, and the mixture was left at -80°C for 30 minutes (alternatively, at -20°C overnight) to precipitate RNA. After centrifugation at 4°C, the supernatant was removed, and the precipitate was washed with 70% ethanol, and air-dried in a clean bench, and was then dissolved in 100 µl distilled water. The yield of synthesized riboprobe was assayed in accordance with Roche's DIG System Users Guide.

### (3) Hybridization

The method of hybridization and detection of hybridized spots as well as the reagents were adopted in accordance with Roche's DIG System Users Guide.

To 20 ml of hybridization buffer [5-fold SSC, 0.1% sodium lauroyl sarcosinate, 0.02% SDS, 2% blocking agent (manufactured by Roche), 50% formamide] heated to 50°C, was added 1 mg (final concentration 50 µg/ml) of Poly(U) (manufactured by Amersham Pharmacia Biotech) which had been quenched after heating at 95°C for 5 minutes. This was sealed together with a membrane in hybridization bag, and pre-hybridization was performed at 50°C for 2 hours. The hybridization buffer was transferred to a tube, and 5-6 µg of riboprobe (final concentration 0.25-0.3 µg/ml) which was quenched after heating for 5 minutes at 95°C was added to the hybridization buffer and mixed. Then, the mixture was returned to a polyethylene bag, and the bag was sealed again. Hybridization was performed at 50°C for 1 night to 3 days, while shaking in such way that the filter moved within the bag (approximately 12 rpm). Since 2 membranes spotted with the same DNA in (1) were prepared, 1 membrane was hybridized with a riboprobe of Thy-1 nephritis rat kidney and 1 membrane was hybridized with a riboprobe of control group rat kidney.

### (4) Detection of spots

The membranes were removed from the hybridization bags and washed with 2-fold SSC containing 0.1% SDS at 68°C for 10 minutes. Then, the membranes were washed again in the same condition with using a fresh washing solution. Then, the washing at 68°C for 15 minutes with 2-fold SSC containing 0.1% SDS was repeated two times.

After equilibrating the membranes by soaking them for 1 minutes in a small amount of buffer 1 [0.15 mol/l NaCl, 0.1 mol/l maleic acid, (pH 7.5)], the membranes were sealed in a polyethylene bag together with buffer 2 [buffer prepared by dissolving the blocking agent (manufactured by Roche) in buffer 1 at a final concentration of 1%] of an amount such that the membranes could move, and gently shaken at room temperature for 1 hour or more to perform blocking. After transferring buffer 2 in the polyethylene bags into a tube and adding alkaline phosphatase-labeled anti-DIG [antibody Anti-DIG-AP (manufactured by Roche)] in a volume of 1/10,000 and mixing, the mixture was returned to the bag and the bag was resealed. Then, the reaction was carried out while shaking gently at room temperature for 30 minutes to 1 hour. The membranes were removed from the bag, and was then subjected to 2 repetitions of washing while shaking for 15 minutes using buffer 1 added with 0.3% Tween 20. After equilibrating the membranes on opened bag by soaking them for 2 minutes in a small amount of buffer 3 [0.1 mol/l Tris (pH9.5), 0.1 mol/l NaCl, 50 mmol/l MgCl₂], CSPD which is a luminous alkaline phosphatase substrate (manufactured by Roche) which was diluted to 100 times with buffer 3 was applied on the membrane surface at 0.5-1.0 ml per 100 cm². After the membrane was covered with a bag, and the substrates were spread uniformly over the membrane surface, and reaction was allowed for 5 minutes. After excess moisture was removed, the bags were sealed, and reaction was allowed at 37°C for 15 minutes. Then, X-ray film, Hyperfilm ECL (manufactured by Amersham Pharmacia Biotech), was exposed, and the film was developed. Exposure time was adjusted in such a way that the background concentration was the same level for those hybridized with riboprobe of Thy-1 nephritis rat kidney or riboprobe of control group rat kidney.

454 clones which had strongly hybridized with riboprobe of Thy-1 nephritis rat kidney in comparison with riboprobe of control group rat kidney were selected. Clones were identified from their array position, and each clone was cultured from the respective glycerol stock prepared in Example 3(1), and plasmid DNA was prepared.

### Example 4 Nucleotide Sequence and Expression Analysis

### (1) Nucleotide sequence determination

The nucleotide sequences of cDNA of the 454 clones selected by differential hybridization of Example 3 were determined by using a DNA sequencer, beginning with determining the nucleotide sequences from the ends. Regarding these nucleotide sequences, the homology to the sequences in nucleotide sequence databases GenBank, EMBL, and Gene Seq [manufactured by Derwent] was examined using the analysis program BLAST. As a results of this analysis, it was found that 148 clones among the 454 clones were cDNA of osteopontin, suggesting that osteopontin gene is expressed in a large amount in Thy-1 nephritis rat kidney. For clones which were considered to be novel nucleotide sequences from the result of homology analysis, the nucleotide sequence of the complete cDNA was determined. The thus obtained nucleotide sequences of the genes whose expression are increased in Thy-1 nephritis rat are shown in SEQ ID NOs: 1, 3, 5, 7, 9, 13, and 17-142. From the obtained nucleotide sequence of cDNA, the amino acid sequences of a polypeptide encoded by the gene were deduced, and with regard to these amino acid sequences also, the homology to the sequences in amino acid sequence databases SwissProt, PIR, GenPept, TREMBL and Gene Seq was examined using the analysis program BLAST.

### (2) Expression analysis

From the 454 clones selected in (1), clones of interest, mainly those of a novel nucleotide sequence, were selected, and the variations of the expression level in Thy-1 nephritis rat kidney of each gene was examined with time course by comparison with control group rat kidney by RT-PCR method. As a template, single strand cDNA was synthesized using SUPERSCRIPT Preamplification System for First Strand cDNA Synthesis Kit (manufactured by Life Technologies) in accordance with the kit's manual, from 5 µg each of the followings prepared in Example 1: total RNAs of kidney of rats on each of days 2, 4, 6, 8, 10, 13 and 16 after administration of anti-Thy-1 antibody OX-7; a mixture of equivalent amounts of the aforementioned total RNAs (called "Thy-1 mix"): and total RNA of kidney of rats of the control group administered with physiological saline. The single strand cDNA was finally dissolved in 250 µl of distilled water. As to the primers, based on the partial nucleotide sequence of cDNA of a clone to be examined, one set of forward primers and reverse primers for PCR having nucleotide sequences specific to that cDNA was designed and synthesized . (Primers of a chain length of 18-22 nucleotides were designed. The forward primer has the same nucleotide sequence as the partial nucleotide sequence of 5' side of the cDNA, and the reverse primer has a complementary nucleotide sequence with the partial nucleotide sequence of 3' side of the cDNA). The PCR conditions were as follows. For 1 µl of each single strand cDNA (equivalent to 20 ng of total RNA) to be employed as a template, there were added 2 µl of 10-fold reaction buffer (buffer attached to rTaq), 2 µl of 2.5 mmol/l dNTP, 1 µl of 10 µmol/l forward primer, 1 µl of 10 µmol/l reverse primer, 12.8 µl of distilled water, and Taq DNA polymerase rTaq (manufactured by Takara Shuzo). Then, the mixture was heated at 94°C for 5 minutes, and subjected to 20 cycles of a reaction cycle of 1 minutes at 94°C (denaturation)/1 minute at X°C (annealing)/1 minute at 72°C (elongation reaction) using an apparatus for PCR, and the mixture was then stored at 4°C. As the anneal temperature (X), an optimum temperature was selected depending on the primer. An aliquot of solution after reaction was subjected to electrophoresis, amplified DNA fragments were stained with fluorescent dye Cybergreen (manufactured by FMC BioProducts), and the amount of the amplified fragment was determined by Fluoroimager (manufactured by Molecular Dynamics). As a control, glycerardehyde-3-phosphate dehydrogenase (hereinafter referred to as "G3PDH") gene, a housekeeping gene which is thought to show an almost fixed expression level in Thy-1 nephritis rat kidney of each stage and control group rat kidney, was subjected to RT-PCR (anneal temperature of 58°C) in a similar manner with each template, using primers having the nucleotide sequences shown by SEQ ID NOs: 162 and 163, and the amount of the amplified fragments was determined. The comparison of control group rats with Thy-1 nephritis rats were then carried out based on the amount that was adjusted by dividing the amount of amplified fragments of each gene by the amount of amplified fragments of G3PDH gene for which amplification was performed with the same templates.

As a result, it was confirmed also by RT-PCR that 7 genes of TRDH-110, TRDH-122, TRDH-292, TRDH-344, TRDH-271, TRDH-284 and TRDH-363 show increased expression in Thy-1 nephritis rat kidney. These genes are described below.

### (3) TRDH-271 gene

A nucleotide sequence of cDNA clone of TRDH-271 gene was determined (shown by SEQ ID NO:1), and its homology with sequences in databases was searched. As a result, there was no completely matching sequence and it was a novel nucleotide sequence. However, sequences showing extremely high homology existed in mouse EST (accession number AA981464 and the like).

In the nucleotide sequence of SEQ ID NO:1, ORF of 694 amino acids shown by SEQ ID NO:2 exists, and it is considered that TRDH-271 gene encodes a novel polypeptide having this amino acid sequence.

The result of RT-PCR (anneal temperature of 60°C) using PCR primers having the nucleotide sequences shown by SEQ ID NOs: 145 and 146 showed that, over the entire period from day 2 to day 16 after administration of antibody, TRDH-271 gene showed a somewhat high expression level of 1.2 to 1.6 times more than the control group. RT-PCR for this gene was conducted with the number of reaction cycles being 23.

### (4) TRDH-284 gene

A nucleotide sequence of cDNA clone of TRDH-284 gene was determined (shown by SEQ ID NO:3), and its homology with sequences in databases was searched. As a result, there was no completely matching sequence and it was a novel nucleotide sequence. However, sequences showing extremely high homology existed in mouse EST (GenBank accession number AA050211 and the like).

In the nucleotide sequence of SEQ ID NO:3, ORF of 350 amino acids shown by SEQ ID NO:4 exists, and it is considered that TRDH-284 gene encodes a novel polypeptide having this amino acid sequence.

The result of RT-PCR (anneal temperature of 60°C) using PCR primers having the nucleotide sequences shown by SEQ ID NOs: 147 and 148 showed that, over the period from day 4 to day 16 after administration of antibody, TRDH-284 gene showed a somewhat high expression level of 1.2 to 1.9 times more than the control group.

### (5) TRDH-363 gene

A nucleotide sequence of cDNA clone of TRDH-336 gene was determined (shown by SEQ ID NO:5), and its homology with sequences in databases was searched. As a result, there was no completely matching sequence and it was a novel nucleotide sequence. However, sequences showing extremely high homology existed in mouse EST and human EST (GenBank accession numbers AA117617, AA315924 and the like).

The nucleotide sequence of SEQ ID NO:5 encodes the amino acid sequence shown by SEQ ID NO:6, and it is considered that TRDH-336 encodes a novel polypeptide having this amino acid sequence.

The result of RT-PCR (anneal temperature of 64°C) using PCR primers having the nucleotide sequences shown by SEQ ID NOs: 149 and 150 showed that, over the period from day 2 to day 16 after administration of antibody, TRDH-363 gene showed a high expression level of 1.5 to 2.8 times more than the control group.

### (6) TRDH-292 gene (secreted polypeptide gene 2)

A nucleotide sequence of cDNA clone of TRDH-292 gene was determined (shown by SEQ ID NO:13), and its homology with sequences in databases was searched. The results showed that it had a high homology of 86% with deduced human secretory polypeptide gene 2 (WO98/39446; SEQ ID NO:15) having a homology with stromal cell derived factor-2. SEQ ID NO:16 shows an amino acid sequence encoded by human secretory polypeptide gene 2. Therefore, it was deduced that TRDH-292 gene was secretory polypeptide gene 2 in rat. SEQ ID NO:14 shows an amino acid sequence of a polypeptide of 220 amino acids encoded by the nucleotide sequence of SEQ ID NO:13.

The result of RT-PCR (anneal temperature of 60°C) using PCR primers having the nucleotide sequences shown by SEQ ID NOs: 151 and 152 showed that, over the period from day 2 to day 16 after administration of OX-7, TRDH-292 gene showed a high expression level of 1.3 to 2 times more than the control group.

### (7) TRDH-344 gene (TSC-22 analogous protein-2)

A nucleotide sequence of cDNA clone of TRDH-344 gene was determined (shown by SEQ ID NO:7), and its homology with sequences in databases was searched. The results showed that it had a high homology of 78.8% with the gene of human TSC-22 analogous protein-2 (WO98/50425; SEQ ID NO:159). SEQ ID NO:160 shows the amino acid sequence of human TSC-22 analogous protein-2. Therefore it was deduced that TRDH-344 gene is TSC-22 analogous protein-2 gene in rat. SEQ ID NO:8 shows an amino acid sequence of rat TSC-22 analogous protein-2 of 150 amino acids encoded by the nucleotide sequence of SEQ ID NO:7.

The result of RT-PCR (anneal temperature of 60°C) using PCR primers having the nucleotide sequences shown by SEQ ID NOs: 143 and 144 showed that, over the entire period from day 2 to day 16 after administration of antibody, TRDH-344 gene showed a somewhat high expression level of 1.2 to 1.6 times more than the control group.

### (8) TRDH-122 gene (mac25)

The complete nucleotide sequence of cDNA clone of TRDH-122 gene was determined (shown by SEQ ID NO:157), and its homology with sequences in databases was searched. The results showed that it had a high homology of 80% or more with genes reported as mouse mac25 [GenBank accession number AB012886; Cell Growth & Differ., 4, 715 (1993)] or human prostacyclin-stimulation factor [GenBank accession number S75725; Biochem. J., 303, 591 (1994); SEQ ID NO:11], and it was therefore deduced that TRDH-122 gene was mac25 gene of the rat. SEQ ID NO:12 shows an amino acid sequence of human prostacyclin-stimulation factor, i.e., human mac25. mac25 has also been reported as IGFBP-7 belonging to the IGF binding protein family [J. Biol. Chem., 271, 30322 (1996)], and is considered to be involved in the regulation of cell proliferation. mac25 has an activin binding ability and is reported to inhibit the proliferation of cancer cell-derived cell strains HeLa, P19 and Saos-2 at a concentration of 10⁻⁷ mol/L [Mol. Med., 6, 126 (2000)]. The nucleotide sequence of cDNA of TRDH-122 gene shown by SEQ ID NO:157 encoded the amino acid sequence shown by SEQ ID NO:158.

The result of KT-PCR (anneal temperature of 58°C) using PCR primers having the nucleotide sequences shown by SEQ ID NOs: 153 and 154 showed that, over the entire period from day 2 to day 16 after administration of OX-7, TRDH-122 gene showed an increased expression level of approximately two times more than the control group.

### (9) TRDH-110 gene (α-2u globulin)

A nucleotide sequence of cDNA of TRDH-110 gene was determined and its homology with sequences in databases was searched. The results showed that it matched with rat α-2u globulin cDNA (GenBank accession number U31287). Rat α-2u globulin is a secreted polypeptide having 162 amino acids (precursor containing a signal peptide has 181 amino acids), and has been reported to male-specifically form a vitreous body with a toxic substance in proximal renal tubule by administration of a certain species of nephrotoxic substance and cause cell proliferation or tumorigenesis [Crit. Rev. Toxicol., 26, 309 (1996)]. However, there have been no reports concerning the expression level of this gene or polypeptide in Thy-1 nephritis rat. The nucleotide sequence of rat α-2u globulin cDNA is shown in SEQ ID NO:9, and the amino acid sequence is shown in SEQ ID NO:10.

The result of RT-PCR (anneal temperature of 62°C) using PCR primers having the nucleotide sequences shown by SEQ ID NOs: 155 and 156 showed that, while α -2u globulin gene showed an extremely high expression level of 44 times more than the control group on day 2 after administration of OX-7, the amount was 2.3 times on day 4 and the expression was hardly observed on day 6 and thereafter, thus showing a transient expression pattern. As the reaction solution for RT-PCR, solution obtained by adding 5% dimethylsulfoxide to the composition of (2) was used. Further, in the PCR, denaturation was carried out at 94°C and not 95°C.

The changes with time course in the relative expression level of the above 7 genes in Thy-1 nephritis rat kidney are shown in Table 1, with the level of expression in control group rat kidney taken as 1.

**Table 1**

| Changes with time course in the relative expression level of each gene in Thy-1 nephritis rat kidney, with the expression level in control group rat kidney is taken as 1 | | | | | | | | |
|---|---|---|---|---|---|---|---|---|
| Gene | Overall* | Day 2 | Day 4 | Day 6 | Day 8 | Day10 | Day13 | Day16 |
| TRDH-271 | 1.14 | 0.97 | 1.66 | 1.49 | 1.30 | 1.83 | 1.84 | 2.32 |
| TRDH-284 | 1.24 | 0.97 | 1.25 | 1.52 | 1.23 | 1.92 | 1.60 | 1.52 |
| TRDH-363 | 1.44 | 1.76 | 1.48 | 2.22 | 1.84 | 1.74 | 1.98 | 2.83 |
| TRDH-292 | 1.72 | 1.39 | 1.95 | 1.57 | 1.89 | 1.31 | 1.91 | 1.93 |
| TRDH-344 | 1.30 | 1.18 | 1.63 | 1.25 | 1.32 | 1.49 | 1.62 | 1.36 |
| TRDH-122 | 1.78 | 1.74 | 2.08 | 2.00 | 1.89 | 1.85 | 2.15 | 2.09 |
| TRDH-110 | 14.64 | 43.79 | 2.33 | 0.16 | 0.24 | 0.00 | 0.00 | -0.14 |

| | | | | | | | | |
|---|---|---|---|---|---|---|---|---|
| *: "Overall" represents the values for a mixture of equal amounts of samples from each of days 2, 4, 6, 8, 10, 13 and 16. | | | | | | | | |

### INDUSTRIAL APPLICABILITY

According to the present invention, a polypeptide useful in exploration and development of a therapeutic agent for actively repairing tissue which suffered from damage in renal disease, DNA which encodes the polypeptide, and an antibody which recognizes the polypeptide, as well as a method of utilizing the polypeptide, the DNA and the antibody, can be provided.

### Sequence Listing Free Text

SEQ ID NO:143 - Description of artificial sequence: Forward primer for amplification of TRDH-344 DNA.
SEQ ID NO:144 - Description of artificial sequence: Reverse primer for amplification of TRDH-344 DNA.
SEQ ID NO:145 - Description of artificial sequence: Forward primer for amplification of TRDH-271 DNA.
SEQ ID NO:146 - Description of artificial sequence: Reverse primer for amplification of TRDH-271 DNA.
SEQ ID NO: 147 - Description of artificial sequence: Forward primer for amplification of TRDH-284 DNA.
SEQ ID NO:148 - Description of artificial sequence: Reverse primer for amplification of TRDH-284 DNA.
SEQ ID NO:149 - Description of artificial sequence: Forward primer for amplification of TRDH-363 DNA.
SEQ ID NO:150 - Description of artificial sequence: Reverse primer for amplification of TRDH-363 DNA.
SEQ ID NO:151 - Description of artificial sequence: Forward primer for amplification of TRDH-292 DNA.
SEQ ID NO:152 - Description of artificial sequence: Reverse primer for amplification of TRDH-292 DNA.
SEQ ID NO:153 - Description of artificial sequence: Forward primer for amplification of TRDH-122 DNA.
SEQ ID NO:154 - Description of artificial sequence: Reverse primer for amplification of TRDH-122 DNA.
SEQ ID NO:155 - Description of artificial sequence: Forward primer for amplification of TRDH-110 DNA.
SEQ ID NO:156 - Description of artificial sequence: Reverse primer for amplification of TRDH-110 DNA.
SEQ ID NO:161 - Description of artificial sequence: Primer having T7 promoter and polythymidylic acid sequence.
SEQ ID NO:162 - Description of artificial sequence: Forward primer for amplification of G3PDH DNA.
SEQ ID NO:163 - Description of artificial sequence: Reverse primer for amplification of G3PDH DNA.

## Claims

1. DNA encoding a polypeptide having an amino acid sequence selected from the group consisting of the amino acid sequences shown by SEQ ID NOs: 2, 4 and 6.

2. DNA having a nucleotide sequence selected from the group consisting of the nucleotide sequences shown by SEQ ID NOs: 1, 3 and 5.

3. DNA which hybridizes under stringent conditions with DNA having a nucleotide sequence selected from the group consisting of the nucleotide sequences shown by SEQ ID NOs: 1, 3 and 5, and is capable of detecting a gene whose expression level increases in tissue affected by onset of proliferative glomerulonephritis

4. DNA having a sequence which is the same as consecutive 5 to 60 nucleotides within a nucleotide sequence selected from the group consisting of the nucleotide sequences shown by SEQ ID NOs: 1, 3 and 5.

5. DNA having a sequence complementary with the DNA having a nucleotide sequence selected from the group consisting of the nucleotide sequences shown by SEQ ID NOs: 1,3 and 5.

6. A method of detecting mRNA of a gene whose expression level increases in tissue affected by onset of proliferative glomerulonephritis by using the DNA according to any one of claims 1 to 5.

7. A diagnostic agent for renal disease, which comprises the DNA according to any one of claims 1 to 5.

8. A method of detecting a causative gene of renal disease by using the DNA according to any one of claims 1 to 5.

9. A method of screening a substance which suppresses or promotes transcription or translation of a gene whose expression level increases in tissue affected by onset of proliferative glomerulonephritis, by using the DNA according to any one of claims 1 to 5.

10. A method of screening a therapeutic agent for renal disease by using the DNA according to any one of claims 1 to 5.

11. A therapeutic agent for renal disease, which comprises the DNA according to any one of claims 1 to 5.

12. A recombinant vector which comprises the DNA according to any one of claims 1 to 5.

13. A recombinant vector which comprises RNA of a sequence which is homologous to a sense strand of the DNA according to any one of claims 1 to 5.

14. The vector according to claims 12 or 13, wherein the recombinant vector is a virus vector.

15. A therapeutic agent for renal disease, which comprises the recombinant vector according to any one of claims 12 to 14.

16. A method of detecting mRNA of a gene whose expression level increases in tissue affected by onset of proliferative glomerulonephritis by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

17. A diagnostic agent for renal disease, which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

18. A method of detecting a causative gene of renal disease by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

19. A method of screening a substance which suppresses or promotes transcription or translation of a gene whose expression level increases in tissue affected by onset of proliferative glomerulonephritis, by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

20. A method of screening a therapeutic agent for renal disease by using DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

21. A therapeutic agent for renal disease, which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

22. A recombinant vector which comprises DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

23. A recombinant vector which comprises RNA of a sequence which is homologous to a sense strand of DNA having a nucleotide sequence selected from the group consisting of the nucleotide sequences shown by SEQ ID NOs: 7, 9, 11, 13, 15, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 157 and 159.

24. The vector according to claim 22 or 23, wherein the recombinant vector is a virus vector.

25. A therapeutic agent for renal disease which comprises the recombinant vector according to any one of claims 22 to 24.

26. A polypeptide encoded by DNA according to claim 1 or 2.

27. A polypeptide having an amino acid sequence selected from the group consisting of the amino acid sequences shown by SEQ ID NOs: 2, 4, and 6.

28. A polypeptide having an amino acid sequence wherein one or more amino acids are deleted, substituted or added in the amino acid sequence of the polypeptide according to claims 26 or 27, and having activity involved in restoration of a kidney which suffered damage.

29. A recombinant DNA which is obtained by incorporating DNA encoding the polypeptide according to any one of claims 26 to 28 into a vector.

30. A transformant which is obtained by introducing the recombinant DNA according to claim 29 into a host cell.

31. A method of preparing a polypeptide wherein the transformant according to claim 30 is cultured in a medium, the polypeptides according to any one of claims 26 to 28 is produced and accumulated in the culture, and the polypeptide is collected from the culture product.

32. A method of screening a therapeutic agent for renal disease by using the culture which is obtained by culturing the transformant according to claim 30 in a medium.

33. A method of screening a therapeutic agent for renal disease by using the polypeptides according to any one of claims 26 to 28.

34. A therapeutic agent for renal disease, which comprises the polypeptide according to any one of claims 26 to 28.

35. An antibody which recognizes the polypeptide according to any one of claims 26 to 28

36. A method of immunologically detecting the polypeptide according to any one of claims 26 to 28 by using the antibody according to claim 35.

37. A method of screening a therapeutic agent for renal disease by using the antibody according to claim 35.

38. A method of screening a substance which suppresses or promotes transcription or translation of a gene whose expression level increases in tissue affected by onset of proliferative glomerulonephritis by using the antibody according to claim 35.

39. A diagnostic agent for renal disease, which comprises the antibody according to claim 35.

40. A therapeutic agent for renal disease, which comprises the antibody according to claim 35.

41. A method of drug delivery wherein a fusion antibody which is obtained by binding the antibody according to claim 35 and an agent selected from a radioisotope, a polypeptide or a low molecular weight compound is led to a site of kidney damage.

42. A recombinant DNA which is obtained by incorporating, into a vector, DNA encoding a polypeptide having an amino acid sequence selected from the group consisting of the amino acid sequences shown by SEQ ID NOs: 8, 10, 12, 14, 16, 158 and 160.

43. A method of screening a therapeutic agent for renal disease by using a culture which is obtained by culturing in a medium a transformant obtained by introducing the recombinant DNA according to claim 42 into a host cell.

44. A method of screening a therapeutic agent for renal disease by using a polypeptide having an amino acid sequence selected from the group consisting of the amino acid sequences shown by SEQ ID NOs: 8, 10, 12, 14, 16, 158 and 160.

45. A therapeutic agent for renal disease, which comprises a polypeptide having an amino acid sequence selected from the group consisting of the amino acid sequences shown by SEQ ID NOs: 8, 10, 12, 14, 16, 158 and 160.

46. A method of screening a therapeutic agent for renal disease by using an antibody which recognizes a polypeptide having an amino acid sequence selected from the group consisting of the amino acid sequences shown by SEQ ID NOs: 8, 10, 12, 14, 16, 158 and 160.

47. A method of screening a substance which suppresses or promotes transcription or translation of a gene whose expression level increases in tissue affected by onset of proliferative glomerulonephritis, by using an antibody which recognizes a polypeptide having an amino acid sequence selected from the group consisting of the amino acid sequences shown by SEQ ID NOs: 8, 10, 12, 14, 16, 158 and 160.

48. A diagnostic agent for renal disease, which comprises an antibody which recognizes a polypeptide having an amino acid sequence selected from the group consisting of the amino acid sequences shown by SEQ ID NOs: 8, 10, 12, 14, 16, 158 and 160.

49. A therapeutic agent for renal disease, which comprises an antibody which recognizes a polypeptide having an amino acid sequence selected from the group consisting of the amino acid sequences shown by SEQ ID NOs: 8, 10, 12, 14, 16, 158 and 160.

50. A method of drug delivery wherein a fusion antibody obtained by binding the antibody which recognizes a polypeptide having an amino acid sequence selected from the group consisting of the amino acid sequences shown by SEQ ID NOs: 8, 10, 12, 14, 16, 158 and 160 and an agent selected from a radioisotope, a polypeptide or a low molecular weight compound is led to a site of kidney damage.


SEQUENCE LISTING
<110> KYOWA HAKKO KOGYO CO., LTD

<120> Novel DNAs related to proliferative glomerulonephritis

<130> 11292

<140>
<141>

<150> JP 2000/90137
<151> 2000-03-29

<160> 163

<170> PatentIn Ver. 2.0

<210> 1
<211> 2470
<212> DNA
<213> Rattus norvegicus

<220>
<221> CDS
<222> (79)..(2160)

<400> 1
ggcggggatc tgcgcggcgg cggaggcggg acctctggca tcagtagcac cgtgagccca 60

gacattcctc tctgagtc atg acg gat gcc aag tat gtc ctc tgc cga tgg 111

Met Thr Asp Ala Lys Tyr Val Leu Cys Arg Trp
1               5                   10

gag aag cga ctg tgg cct gca aag gtt ttg gcc aga act gag act tca 159
Glu Lys Arg Leu Trp Pro Ala Lys Val Leu Ala Arg Thr Glu Thr Ser
            15                  20                  25

gca aaa aac aag aga aaa aag gaa ttc ttt cta gat gtt caa ata ctc 207
Ala Lys Asn Lys Arg Lys Lys Glu Phe Phe Leu Asp Val Gln Ile Leu
        30                  35                  40

tca cta aag gaa aag atc cag gtt aag agc tca gcc gtg gag gca ctg 255
Ser Leu Lys Glu Lys Ile Gln Val Lys Ser Ser Ala Val Glu Ala Leu
    45                  50                  55

cag aag tca cac att gag aac att gcc gcc ttc ttg gcc tct cag aat 303
Gln Lys Ser His Ile Glu Asn Ile Ala Ala Phe Leu Ala Ser Gln Asn
60                  65                  70                  75

gaa gtc cca gct act cct ctg gag gag ctg act tac cga cgg tcc ctg 351
Glu Val Pro Ala Thr Pro Leu Glu Glu Leu Thr Tyr Arg Arg Ser Leu
                80                  85                  90

cga gtg gcc ctg gat gtc ttg aac gag agg acc agt ttg agt cct gaa 399
Arg Val Ala Leu Asp Val Leu Asn Glu Arg Thr Ser Leu Ser Pro Glu
            95                  100                 105

agt cat cca gtc gaa aat ggg agc aca cca tct cag aag ggc aag cca 447
Ser His Pro Val Glu Asn Gly Ser Thr Pro Ser Gln Lys Gly Lys Pro
        110                 115                 120

gat gca gat gtg gcc tcg cgg gtc tct agt gct cct tct cca tct ttt 495
Asp Ala Asp Val Ala Ser Arg Val Ser Ser Ala Pro Ser Pro Ser Phe
    125                 130                 135

ctc agt gaa gat gat cag gct gtg gca gcc cag tgt gca tcc aag agg 543
Leu Ser Glu Asp Asp Gln Ala Val Ala Ala Gln Cys Ala Ser Lys Arg
140                 145                 150                 155

agg tgg gag tgc agt cca aaa agc ctg tcg ccg ttg tct gcc tcg gaa 591
Arg Trp Glu Cys Ser Pro Lys Ser Leu Ser Pro Leu Ser Ala Ser Glu
                160                 165                 170

gag gat ctc agg tgc aaa gtg gac ccc aag aca ggc ctc tca gag agt 639
Glu Asp Leu Arg Cys Lys Val Asp Pro Lys Thr Gly Leu Ser Glu Ser
            175                 180                 185

gga gcc ctg ggg act gaa gtg cct gcc ccc act ggg gat gag tct cag 687
Gly Ala Leu Gly Thr Glu Val Pro Ala Pro Thr Gly Asp Glu Ser Gln
        190                 195                 200

aat ggc tct ggg tca cag ctg gac cat gga cag gag agc aca acc aaa 735
Asn Gly Ser Gly Ser Gln Leu Asp His Gly Gln Glu Ser Thr Thr Lys
    205                 210                 215

aag aga cag agg aat tcg gga gag aaa cct gcc cgg cgt gga aaa gca 783
Lys Arg Gln Arg Asn Ser Gly Glu Lys Pro Ala Arg Arg Gly Lys Ala
220                 225                 230                 235

gag tct ggc ctt tcc aag gga gac agt gtc gca gag agc gga gga cag 831
Glu Ser Gly Leu Ser Lys Gly Asp Ser Val Ala Glu Ser Gly Gly Gln
                240                 245                 250

gca agc agc tgt gtg gcc ctg gct tca ccc agg ctg ccc tcc caa acc 879
Ala Ser Ser Cys Val Ala Leu Ala Ser Pro Arg Leu Pro Ser Gln Thr
            255                 260                 265

tgg gag ggg gat cca tgt gct gga gtc gaa ggc tgt gac cca gtt gag 927
Trp Glu Gly Asp Pro Cys Ala Gly Val Glu Gly Cys Asp Pro Val Glu
        270                 275                 280

tca tct ggc aac atc agg ccg ctt ctg gac tct gag aga agc aaa gga 975
Ser Ser Gly Asn Ile Arg Pro Leu Leu Asp Ser Glu Arg Ser Lys Gly
    285                 290                 295

cgc ctc aca aag agg cca cgc ttg gac gga ggc cgg aac cca ctg ccc 1023
Arg Leu Thr Lys Arg Pro Arg Leu Asp Gly Gly Arg Asn Pro Leu Pro
300                 305                 310                 315

aga cat cta gga acc aga act gtg ggg gca gtg ccc tcc cgt agg agc 1071
Arg His Leu Gly Thr Arg Thr Val Gly Ala Val Pro Ser Arg Arg Ser
                320                 325                 330

tgc tct ggg gag gtc acg acg ctg cgc agg gct gga gac agt gac aga 1119
Cys Ser Gly Glu Val Thr Thr Leu Arg Arg Ala Gly Asp Ser Asp Arg
            335                 340                 345

cca gag gaa gat cct atg tct tca gaa gaa tct aca ggg ttc aag tcc 1167
Pro Glu Glu Asp Pro Met Ser Ser Glu Glu Ser Thr Gly Phe Lys Ser
        350                 355                 360

gtc cac tcc ctg ctg gag gag gag gag gag gag gag gaa gag gag gag 1215
Val His Ser Leu Leu Glu Glu Glu Glu Glu Glu Glu Glu Glu Glu Glu

    365                 370                 375

gaa cca ccc cgg atc ctt ctg tat cac gaa cca cga tca ttt gaa gta 1263
Glu Pro Pro Arg Ile Leu Leu Tyr His Glu Pro Arg Ser Phe Glu Val
380                 385                 390                 395

gga atg ctg gtc tgg ctt aaa tac caa aaa tac cca ttc tgg cca gcc 1311
Gly Met Leu Val Trp Leu Lys Tyr Gln Lys Tyr Pro Phe Trp Pro Ala
                400                 405                 410

gtg gtc aag agt gtc cgg cgg agg gac aag aag gcc agt gtg ctc ttc 1359
Val Val Lys Ser Val Arg Arg Arg Asp Lys Lys Ala Ser Val Leu Phe
            415                 420                 425

att gag ggc aac atg aat ccc aag ggc cga gga atc acc gtg tcg ctg 1407
Ile Glu Gly Asn Met Asn Pro Lys Gly Arg Gly Ile Thr Val Ser Leu
        430                 435                 440

cga cgg ctc aag cac ttt gac tgc aag gaa aag cat gca cta ctg gac 1455
Arg Arg Leu Lys His Phe Asp Cys Lys Glu Lys His Ala Leu Leu Asp
    445                 450                 455

aga gcc aaa gag gac ttt gcc cag gct att ggc tgg tgt gtc tcg ctt 1503
Arg Ala Lys Glu Asp Phe Ala Gln Ala Ile Gly Trp Cys Val Ser Leu
460                 465                 470                 475

atc act gac tac cgc gtg cgg ctg ggc tgc ggc tcc ttc gcc ggg tcg 1551
Ile Thr Asp Tyr Arg Val Arg Leu Gly Cys Gly Ser Phe Ala Gly Ser
                480                 485                 490

ttc ttg gaa tat tac gct gct gat atc agc tat cct gtg cgc aag tct 1599

Phe Leu Glu Tyr Tyr Ala Ala Asp Ile Ser Tyr Pro Val Arg Lys Ser
            495                 500                 505

atc caa cag gac gtc ctg ggg acc agg ttt cct cag ctg ggc aag ggg 1647
Ile Gln Gln Asp Val Leu Gly Thr Arg Phe Pro Gln Leu Gly Lys Gly
        510                 515                 520

gac cct gag gag cct atg ggg gac agc cgg ctg gga cag tgg cgg cca 1695
Asp Pro Glu Glu Pro Met Gly Asp Ser Arg Leu Gly Gln Trp Arg Pro
    525                 530                 535

tgc agg aag gtg ctg cct gac cgc tcc agg gct gcc cgg gat aaa gcc 1743
Cys Arg Lys Val Leu Pro Asp Arg Ser Arg Ala Ala Arg Asp Lys Ala
540                 545                 550                 555

aac cag aag ctg gtg gag tac atc gtg aag gcc aag ggt gca gag agc 1791
Asn Gln Lys Leu Val Glu Tyr Ile Val Lys Ala Lys Gly Ala Glu Ser
                560                 565                 570

cac ctg cgg gct atc ctg cac agc cgc aag ccc tca cgc tgg ctg aag 1839
His Leu Arg Ala Ile Leu His Ser Arg Lys Pro Ser Arg Trp Leu Lys
            575                 580                 585

acg ttc ctg agc tcc aat cag tac gtg aca tgc atg gag acg tac ctg 1887
Thr Phe Leu Ser Ser Asn Gln Tyr Val Thr Cys Met Glu Thr Tyr Leu
        590                 595                 600

gag gat gag gcg cag ctg gat gag gtg gtg gag tac ctg cag ggc gtc 1935
Glu Asp Glu Ala Gln Leu Asp Glu Val Val Glu Tyr Leu Gln Gly Val
    605                 610                 615

tgc cga gac atg gat ggc gag atg cct gcg cgc ggc agc ggc gac cgc 1983
Cys Arg Asp Met Asp Gly Glu Met Pro Ala Arg Gly Ser Gly Asp Arg
620                 625                 630                 635

atc cgt ttc atc ctg gat gtg ctg ctg cct gag gcg atc atc tgc gcc 2031
Ile Arg Phe Ile Leu Asp Val Leu Leu Pro Glu Ala Ile Ile Cys Ala
                640                 645                 650

atc tcg gca gtg gag gca gtg gac tac aag aca gcc gag cag aag tac 2079
Ile Ser Ala Val Glu Ala Val Asp Tyr Lys Thr Ala Glu Gln Lys Tyr
            655                 660                 665

ctc cgt ggc ccc aca ctc agc tac cgg gaa aag gaa atc ttt gac aat 2127
Leu Arg Gly Pro Thr Leu Ser Tyr Arg Glu Lys Glu Ile Phe Asp Asn
        670                 675                 680

gaa ctc ctg gag gag agg aac cgt cgc cgt cgc tgatgccgta gtctccacct 2180
Glu Leu Leu Glu Glu Arg Asn Arg Arg Arg Arg
    685                 690

ggccagcacc gtgtctgtgg cctgccagag gcctgtgaga atgtgctaga agcaagaggc 2240

ctagtaatgt gctgactttg atctgtgcat gggttctgcg tcttcagccc tgagcctggg 2300

agatcagagg ccatcttcac actagaagac tgctgcatct atgaacagct gcttctggaa 2360

gtttctgtgt gtgtacgcgt gtatgtttgg ttttattttt ttaattatta ttttgtttat 2420

aaatgcgttt gaatgcaaaa aaaaaaaaaa aaaaaaaaaa aaaaaaaaaa 2470

<210> 2
<211> 694
<212> PRT
<213> Rattus norvegicus

<400> 2
Met Thr Asp Ala Lys Tyr Val Leu Cys Arg Trp Glu Lys Arg Leu Trp
1               5                   10                  15

Pro Ala Lys Val Leu Ala Arg Thr Glu Thr Ser Ala Lys Asn Lys Arg
            20                  25                  30

Lys Lys Glu Phe Phe Leu Asp Val Gln Ile Leu Ser Leu Lys Glu Lys
        35                  40                  45

Ile Gln Val Lys Ser Ser Ala Val Glu Ala Leu Gln Lys Ser His Ile
    50                  55                  60

Glu Asn Ile Ala Ala Phe Leu Ala Ser Gln Asn Glu Val Pro Ala Thr
65                  70                  75                  80

Pro Leu Glu Glu Leu Thr Tyr Arg Arg Ser Leu Arg Val Ala Leu Asp
                85                  90                  95

Val Leu Asn Glu Arg Thr Ser Leu Ser Pro Glu Ser His Pro Val Glu
            100                 105                 110

Asn Gly Ser Thr Pro Ser Gln Lys Gly Lys Pro Asp Ala Asp Val Ala
        115                 120                 125

Ser Arg Val Ser Ser Ala Pro Ser Pro Ser Phe Leu Ser Glu Asp Asp

    130                 135                 140

Gln Ala Val Ala Ala Gln Cys Ala Ser Lys Arg Arg Trp Glu Cys Ser
145                 150                 155                 160

Pro Lys Ser Leu Ser Pro Leu Ser Ala Ser Glu Glu Asp Leu Arg Cys
                165                 170                 175

Lys Val Asp Pro Lys Thr Gly Leu Ser Glu Ser Gly Ala Leu Gly Thr
            180                 185                 190

Glu Val Pro Ala Pro Thr Gly Asp Glu Ser Gln Asn Gly Ser Gly Ser
        195                 200                 205

Gln Leu Asp His Gly Gln Glu Ser Thr Thr Lys Lys Arg Gln Arg Asn
    210                 215                 220

Ser Gly Glu Lys Pro Ala Arg Arg Gly Lys Ala Glu Ser Gly Leu Ser
225                 230                 235                 240

Lys Gly Asp Ser Val Ala Glu Ser Gly Gly Gln Ala Ser Ser Cys Val
                245                 250                 255

Ala Leu Ala Ser Pro Arg Leu Pro Ser Gln Thr Trp Glu Gly Asp Pro
            260                 265                 270

Cys Ala Gly Val Glu Gly Cys Asp Pro Val Glu Ser Ser Gly Asn Ile
        275                 280                 285

Arg Pro Leu Leu Asp Ser Glu Arg Ser Lys Gly Arg Leu Thr Lys Arg
    290                 295                 300

Pro Arg Leu Asp Gly Gly Arg Asn Pro Leu Pro Arg His Leu Gly Thr
305                 310                 315                 320

Arg Thr Val Gly Ala Val Pro Ser Arg Arg Ser Cys Ser Gly Glu Val
                325                 330                 335

Thr Thr Leu Arg Arg Ala Gly Asp Ser Asp Arg Pro Glu Glu Asp Pro
            340                 345                 350

Met Ser Ser Glu Glu Ser Thr Gly Phe Lys Ser Val His Ser Leu Leu
        355                 360                 365

Glu Glu Glu Glu Glu Glu Glu Glu Glu Glu Glu Glu Pro Pro Arg Ile
    370                 375                 380

Leu Leu Tyr His Glu Pro Arg Ser Phe Glu Val Gly Met Leu Val Trp
385                 390                 395                 400

Leu Lys Tyr Gln Lys Tyr Pro Phe Trp Pro Ala Val Val Lys Ser Val
                405                 410                 415

Arg Arg Arg Asp Lys Lys Ala Ser Val Leu Phe Ile Glu Gly Asn Met
            420                 425                 430

Asn Pro Lys Gly Arg Gly Ile Thr Val Ser Leu Arg Arg Leu Lys His
        435                 440                 445

Phe Asp Cys Lys Glu Lys His Ala Leu Leu Asp Arg Ala Lys Glu Asp
    450                 455                 460

Phe Ala Gln Ala Ile Gly Trp Cys Val Ser Leu Ile Thr Asp Tyr Arg
465                 470                 475                 480

Val Arg Leu Gly Cys Gly Ser Phe Ala Gly Ser Phe Leu Glu Tyr Tyr
                485                 490                 495

Ala Ala Asp Ile Ser Tyr Pro Val Arg Lys Ser Ile Gln Gln Asp Val
            500                 505                 510

Leu Gly Thr Arg Phe Pro Gln Leu Gly Lys Gly Asp Pro Glu Glu Pro
        515                 520                 525

Met Gly Asp Ser Arg Leu Gly Gln Trp Arg Pro Cys Arg Lys Val Leu
    530                 535                 540

Pro Asp Arg Ser Arg Ala Ala Arg Asp Lys Ala Asn Gln Lys Leu Val
545                 550                 555                 560

Glu Tyr Ile Val Lys Ala Lys Gly Ala Glu Ser His Leu Arg Ala Ile
                565                 570                 575

Leu His Ser Arg Lys Pro Ser Arg Trp Leu Lys Thr Phe Leu Ser Ser
            580                 585                 590

Asn Gln Tyr Val Thr Cys Met Glu Thr Tyr Leu Glu Asp Glu Ala Gln
        595                 600                 605

Leu Asp Glu Val Val Glu Tyr Leu Gln Gly Val Cys Arg Asp Met Asp
    610                 615                 620

Gly Glu Met Pro Ala Arg Gly Ser Gly Asp Arg Ile Arg Phe Ile Leu

625                 630                 635                 640

Asp Val Leu Leu Pro Glu Ala Ile Ile Cys Ala Ile Ser Ala Val Glu
                645                 650                 655

Ala Val Asp Tyr Lys Thr Ala Glu Gln Lys Tyr Leu Arg Gly Pro Thr
            660                 665                 670

Leu Ser Tyr Arg Glu Lys Glu Ile Phe Asp Asn Glu Leu Leu Glu Glu
        675                 680                 685

Arg Asn Arg Arg Arg Arg
    690

<210> 3
<211> 1585
<212> DNA
<213> Rattus norvegicus

<220>
<221> CDS
<222> (48)..(1097)

<400> 3
   accggaagtt gtatcgaggc ttccgcacat ggatacttct ggagaac atg cca ctg   56
                                                       Met Pro Leu
                                                       1
   gtc gtg gtt tgc ggg ctg ccg tcc agc ggc aag agc cgg cgt acg gaa   104
   Val Val Val Cys Gly Leu Pro Ser Ser Gly Lys Ser Arg Arg Thr Glu
   5                    10                    15
   gag tta cgt cgg gcg ctg acc ggc gag gga cgt tcg gtg tat gtg gtg    152
   Glu Leu Arg Arg Ala Leu Thr Gly Glu Gly Arg Ser Val Tyr Val Val
   20                    25                    30                    35
   gac gat gct tcg gtg ctg ggc gcg cag gat tcc act gtg tac ggc gac    200
   Asp Asp Ala Ser Val Leu Gly Ala Gln Asp Ser Thr Val Tyr Gly Asp
                       40                    45                    50
   tct gcg ggt gag aag gcg cta cgt gct gcg ctg cgg gcc gcg gta gag    248
   Ser Ala Gly Glu Lys Ala Leu Arg Ala Ala Leu Arg Ala Ala Val Glu
                   55                    60                    65
   cgg cgc ctg agc cgg cag gac gtg gtc atc cta gac tcc atg aac tac    296
   Arg Arg Leu Ser Arg Gln Asp Val Val Ile Leu Asp Ser Met Asn Tyr
               70                    75                    80
   atc aag ggg ttc cgc tac gag ttg tac tgc ctt gcg cga gct gtg cgc    344
   Ile Lys Gly Phe Arg Tyr Glu Leu Tyr Cys Leu Ala Arg Ala Val Arg
           85                    90                    95
   acg ccg ctc tgc tta gtt tac tgc ata agg ccc ggc tgg cca agc cgc    392
   Thr Pro Leu Cys Leu Val Tyr Cys Ile Arg Pro Gly Trp Pro Ser Arg
   100                    105                    110                    115
   ggg ctt ccg gtg cct ggc gcc tgc gag agc tcg gac ccg gct gtc agt    440
   Gly Leu Pro Val Pro Gly Ala Cys Glu Ser Ser Asp Pro Ala Val Ser
                       120                    125                    130
   gtg agc tgg agg ccg cgc gcc gac tac ggc gag aag act cag gcg gtc    488
   Val Ser Trp Arg Pro Arg Ala Asp Tyr Gly Glu Lys Thr Gln Ala Val
   135 140 145
   ggc gct gta gag cag cgc gcc atc agc ccc tta gca aat ggg gga gtc 536
   Gly Ala Val Glu Gln Arg Ala Ile Ser Pro Leu Ala Asn Gly Gly Val
   150 155 160
   ccg acc gct gtc ccc aag gaa ctg gat cca aag gat atc ctg cca tca 584
   Pro Thr Ala Val Pro Lys Glu Leu Asp Pro Lys Asp Ile Leu Pro Ser
   165 170 175
   aat cct cca gct gta atg act ccg gaa tcc gag aaa tct gca gag cct 632
   Asn Pro Pro Ala Val Met Thr Pro Glu Ser Glu Lys Ser Ala Glu Pro
   180 185 190 195
   gcg cca tgt gcc ttt cct ccc gaa ctt ttg gag tcc tta gcg ctg cgc 680
   Ala Pro Cys Ala Phe Pro Pro Glu Leu Leu Glu Ser Leu Ala Leu Arg
   200 205 210
   ttt gaa gct ccc gac tct cgg aac cgc tgg gat cga ccc ttg ttc acc 728
   Phe Glu Ala Pro Asp Ser Arg Asn Arg Trp Asp Arg Pro Leu Phe Thr
   215 220 225
   gtg gtg ggt tta gaa gag cca ttg ccc ctg gct gag atc cgg tct gca 776
   Val Val Gly Leu Glu Glu Pro Leu Pro Leu Ala Glu Ile Arg Ser Ala
   230 235 240
   ctg ttc gag aat cgg gct ccc cca ccc cat cag tct aca cag tcc cag 824
   Leu Phe Glu Asn Arg Ala Pro Pro Pro His Gln Ser Thr Gln Ser Gln
   245 250 255
   ccc ctg gcc tct ggc agc ttt cta cac cag ttg gat cag gcc acg agc 872
   Pro Leu Ala Ser Gly Ser Phe Leu His Gln Leu Asp Gln Ala Thr Ser
   260 265 270 275
   cag gtg ttg act gct gtg atg gaa aca cag aag agc gct gta ccc gga 920
   Gln Val Leu Thr Ala Val Met Glu Thr Gln Lys Ser Ala Val Pro Gly
   280 285 290
   gac tta cta acg ctt cct ggc acc acg gag cac ctc cga ttt acc cgt 968
   Asp Leu Leu Thr Leu Pro Gly Thr Thr Glu His Leu Arg Phe Thr Arg
   295 300 305
   ccc ttg acc ttg gca gaa ttg agt cgc ctc cgt cgc cag ttt att tcc 1016
   Pro Leu Thr Leu Ala Glu Leu Ser Arg Leu Arg Arg Gln Phe Ile Ser
   310 315 320
   tac act aaa atg cat ccc aac aat gag aac ctg cct caa ttg gcc aac 1064
   Tyr Thr Lys Met His Pro Asn Asn Glu Asn Leu Pro Gln Leu Ala Asn
   325 330 335
   atg ttt ctt cag tat ctg aac cag agt ttg cac taatgggata gtggtctgca 1117
   Met Phe Leu Gln Tyr Leu Asn Gln Ser Leu His
   340 345 350
   gcggtggctc ttgtctgaat tcccctgtac tttggctagg aaaaatagtc cgaaggtctg 1177
   caaagcgcac tgtagtactg agatgctaaa tttgactcat tttcttaact gcctctgcca 1237
   taccctgagt gtgctgcata agctgaggca ttgagcacca gctccaaaaa taccaggtgg 1297
   cttcggttgg aatctacttg gggattcttc atacactgtt ttcctttcat cgggacggag 1357
   aattgttaag tcaactgtga gtagaaaccg aagataacag ttttgtattt atgatggccc 1417
   tttcatacta caaatacttt tgagcacagt gcctcttgct atctatcctg gaacttcgaa 1477
   cacagataaa tcttgttctg cccctgggaa actgatattt gtataagaca gccattagat 1537
   atttcctcta ataaaatctt ctaaaattaa aaaaaaaaaa aaaaaaaa 1585

<210> 4
<211> 350
<212> PRT
<213> Rattus norvegicus

<400> 4
Met Pro Leu Val Val Val Cys Gly Leu Pro Ser Ser Gly Lys Ser Arg
1               5                   10                  15

Arg Thr Glu Glu Leu Arg Arg Ala Leu Thr Gly Glu Gly Arg Ser Val
            20                  25                  30

Tyr Val Val Asp Asp Ala Ser Val Leu Gly Ala Gln Asp Ser Thr Val
        35                  40                  45

Tyr Gly Asp Ser Ala Gly Glu Lys Ala Leu Arg Ala Ala Leu Arg Ala
    50                  55                  60

Ala Val Glu Arg Arg Leu Ser Arg Gln Asp Val Val Ile Leu Asp Ser
65                  70                  75                  80

Met Asn Tyr Ile Lys Gly Phe Arg Tyr Glu Leu Tyr Cys Leu Ala Arg
                85                  90                  95

Ala Val Arg Thr Pro Leu Cys Leu Val Tyr Cys Ile Arg Pro Gly Trp
            100                 105                 110

Pro Ser Arg Gly Leu Pro Val Pro Gly Ala Cys Glu Ser Ser Asp Pro
        115                 120                 125

Ala Val Ser Val Ser Trp Arg Pro Arg Ala Asp Tyr Gly Glu Lys Thr
    130                 135                 140

Gln Ala Val Gly Ala Val Glu Gln Arg Ala Ile Ser Pro Leu Ala Asn
145                 150                 155                 160

Gly Gly Val Pro Thr Ala Val Pro Lys Glu Leu Asp Pro Lys Asp Ile
                165                 170                 175

Leu Pro Ser Asn Pro Pro Ala Val Met Thr Pro Glu Ser Glu Lys Ser
            180                 185                 190

Ala Glu Pro Ala Pro Cys Ala Phe Pro Pro Glu Leu Leu Glu Ser Leu
        195                 200                 205

Ala Leu Arg Phe Glu Ala Pro Asp Ser Arg Asn Arg Trp Asp Arg Pro
    210                 215                 220

Leu Phe Thr Val Val Gly Leu Glu Glu Pro Leu Pro Leu Ala Glu Ile
225                 230                 235                 240

Arg Ser Ala Leu Phe Glu Asn Arg Ala Pro Pro Pro His Gln Ser Thr

245                 250                 255

Gln Ser Gln Pro Leu Ala Ser Gly Ser Phe Leu His Gln Leu Asp Gln
        260                 265                 270

Ala Thr Ser Gln Val Leu Thr Ala Val Met Glu Thr Gln Lys Ser Ala
    275                 280                 285

Val Pro Gly Asp Leu Leu Thr Leu Pro Gly Thr Thr Glu His Leu Arg
    290                 295                 300

Phe Thr Arg Pro Leu Thr Leu Ala Glu Leu Ser Arg Leu Arg Arg Gln
305                 310                 315                 320

Phe Ile Ser Tyr Thr Lys Met His Pro Asn Asn Glu Asn Leu Pro Gln
                325                 330                 335

Leu Ala Asn Met Phe Leu Gln Tyr Leu Asn Gln Ser Leu His
        340                 345

<210> 5
<211> 1879
<212> DNA
<213> Rattus norvegicus

<220>
<221> CDS
<222> (343)..(1410)

<400> 5

ctagcccggg caggcccggc gggggggcg ttgaccttgc gggcggtcaa accggccacc 60

cgtttttccc tggcggtggc gctcgggagt ctgggtgggg gcctcggagc caggggccac 120

ggactgcatc acggtagaga gattcgcgag cctcaggcga gggacgcaac ctccagctcc 180

gcggagaccg agggtggcca cgtccaggga catctccggt tcattcattg ggttcctact 240

gtgtgctctt atacggcgct cagccagccc aactgatgtg gagcgctgtg cgcggccctg 300

ctaggcttct ttgtggatgg ccggggcgag gtcctcttca ct atg gcc cgg cgt 354
Met Ala Arg Arg
1

gca cgg agt agc agg gca tgg cac ttt gtc ctg agt gca gca cgc cga 402
Ala Arg Ser Ser Arg Ala Trp His Phe Val Leu Ser Ala Ala Arg Arg
5                   10                  15                  20

gat aca gat gct cga gct gtg gct ctg gca ggc aac tct aac tgg ggc 450
Asp Thr Asp Ala Arg Ala Val Ala Leu Ala Gly Asn Ser Asn Trp Gly
                25                  30                  35

tac gac tct gat ggg cag cac agc gac tcc gac tct gac cct gag tac 498
Tyr Asp Ser Asp Gly Gln His Ser Asp Ser Asp Ser Asp Pro Glu Tyr
            40                  45                  50

tct tcc ctg cca cca tcc atc ccc agt gct gtg cct gtg aca gga gag 546
Ser Ser Leu Pro Pro Ser Ile Pro Ser Ala Val Pro Val Thr Gly Glu
        55                  60                  65

tcc ttc tgt gac tgt gag ggc cag aat gag gct acc ttc tgc aac agt 594

Ser Phe Cys Asp Cys Glu Gly Gln Asn Glu Ala Thr Phe Cys Asn Ser
    70                  75                  80

tta cac aca gca cac cgt ggc aag gac tgc cgt tgt ggt gag gag gat 642
Leu His Thr Ala His Arg Gly Lys Asp Cys Arg Cys Gly Glu Glu Asp
85                  90                  95                  100

gag gat ttt gat tgg gta tgg gat gac ctg aac aag tcc tca gcc acc 690
Glu Asp Phe Asp Trp Val Trp Asp Asp Leu Asn Lys Ser Ser Ala Thr
                105                 110                 115

ttg ctg agc tgt gat aat cga aag gtt agc ttt cac atg gag tac agc 738
Leu Leu Ser Cys Asp Asn Arg Lys Val Ser Phe His Met Glu Tyr Ser
            120                 125                 130

tgt ggc aca gca gcc att cgg ggc acc aag gag cta ggg gat ggc caa 786
Cys Gly Thr Ala Ala Ile Arg Gly Thr Lys Glu Leu Gly Asp Gly Gln
        135                 140                 145

cac ttc tgg gaa atc aag atg acc tct ccg gtg tat ggc act gat atg 834
His Phe Trp Glu Ile Lys Met Thr Ser Pro Val Tyr Gly Thr Asp Met
    150                 155                 160

atg gtg ggc atc ggg aca tca gac gta gac ctg gac aag tac cac cac 882
Met Val Gly Ile Gly Thr Ser Asp Val Asp Leu Asp Lys Tyr His His
165                 170                 175                 180

acg ttc tgc agc ctg ctg ggc agg gat gaa gac agc tgg ggg ctc tcc 930
Thr Phe Cys Ser Leu Leu Gly Arg Asp Glu Asp Ser Trp Gly Leu Ser
                185                 190                 195

tac acg ggg ctc ctc cac cac aaa ggc gac aag acg agc ttc tct tca 978
Tyr Thr Gly Leu Leu His His Lys Gly Asp Lys Thr Ser Phe Ser Ser
            200                 205                 210

cgc ttc ggc cag ggc tct atc att ggc gta cac ttg gac acc tgg cat 1026
Arg Phe Gly Gln Gly Ser Ile Ile Gly Val His Leu Asp Thr Trp His
        215                 220                 225

ggg aca ctg act ttt ttc aag aat agg aag tgc ata gga gtg gct gcc 1074
Gly Thr Leu Thr Phe Phe Lys Asn Arg Lys Cys Ile Gly Val Ala Ala
    230                 235                 240

act cgg ctt cag aac aga agg ttc tac ccg atg gtc tgc tcg acc gcc 1122
Thr Arg Leu Gln Asn Arg Arg Phe Tyr Pro Met Val Cys Ser Thr Ala
245                 250                 255                 260

gcc aag agc agc atg aag gtc att cgc tcc tgt gcc agc tcc aca tcc 1170
Ala Lys Ser Ser Met Lys Val Ile Arg Ser Cys Ala Ser Ser Thr Ser
                265                 270                 275

ctg cag tac ctg tgc tgc tac cgc ctg cgc cag ttg cgg cca gac tca 1218
Leu Gln Tyr Leu Cys Cys Tyr Arg Leu Arg Gln Leu Arg Pro Asp Ser
            280                 285                 290

ggg gac acc ctc gag ggc ctg ccc ttg cca ccc ggc ctc aag cag gtg 1266
Gly Asp Thr Leu Glu Gly Leu Pro Leu Pro Pro Gly Leu Lys Gln Val
        295                 300                 305

ctg cat aac aag ctg ggc tgg gtc ctg agc atg aac tgc agc cac tgg 1314
Leu His Asn Lys Leu Gly Trp Val Leu Ser Met Asn Cys Ser His Trp
    310                 315                 320

aca tcc cct gca ccc cct ccg ggc aca gct gcc cca gcc gct gag aga 1362
Thr Ser Pro Ala Pro Pro Pro Gly Thr Ala Ala Pro Ala Ala Glu Arg
325                 330                 335                 340

gat tcc cgg gag acc agg ccc tgt cag agg aag cgc tgc cga aga agc 1410
Asp Ser Arg Glu Thr Arg Pro Cys Gln Arg Lys Arg Cys Arg Arg Ser
                345                 350                 355

tgacttctcc ccgggaatgc agacaccttt ctttcttgcc cttccagggc agcaggagag 1470

gggagaacgg aggtctaggc ttttccctgt ctccccgagg ccaggacagt cttctctgtt 1530

ggccatggag tgtgacagct gttctaccgc ctgtgctggt agggaaacag cactccttcc 1590

tgtttgtcct ttgagttgcc atgtatcctg ggagctgcag ccaggcgtct ggacctagat 1650

tccaagcctg ggaggctggc tgacgaagtg gagtgcattc atatcccagg gaagagatgg 1710

gctgtcccga cccacaggtc tgtggggttt tcctgacttg cattgcatgt tgtcagcgcc 1770

tgctcctgtc acagagatgt cagtgggtgc cctgggaagg gattctgtct cgtccccata 1830

ggttctatca ttaaaagcgt cctcacaaat gaaaaaaaaa aaaaaaaaa 1879

<210> 6
<211> 356
<212> PRT
<213> Rattus norvegicus

<400> 6
Met Ala Arg Arg Ala Arg Ser Ser Arg Ala Trp His Phe Val Leu Ser
1               5                   10                  15
Ala Ala Arg Arg Asp Thr Asp Ala Arg Ala Val Ala Leu Ala Gly Asn
            20                  25                  30
Ser Asn Trp Gly Tyr Asp Ser Asp Gly Gln His Ser Asp Ser Asp Ser
        35                  40                  45
Asp Pro Glu Tyr Ser Ser Leu Pro Pro Ser Ile Pro Ser Ala Val Pro
    50                  55                  60
Val Thr Gly Glu Ser Phe Cys Asp Cys Glu Gly Gln Asn Glu Ala Thr
65                  70                  75                  80
Phe Cys Asn Ser Leu His Thr Ala His Arg Gly Lys Asp Cys Arg Cys
                85                  90                  95
Gly Glu Glu Asp Glu Asp Phe Asp Trp Val Trp Asp Asp Leu Asn Lys
            100                 105                 110
Ser Ser Ala Thr Leu Leu Ser Cys Asp Asn Arg Lys Val Ser Phe His
        115                 120                 125
Met Glu Tyr Ser Cys Gly Thr Ala Ala Ile Arg Gly Thr Lys Glu Leu
    130                 135                 140
Gly Asp Gly Gln His Phe Trp Glu Ile Lys Met Thr Ser Pro Val Tyr
145                 150                 155                 160

Gly Thr Asp Met Met Val Gly Ile Gly Thr Ser Asp Val Asp Leu Asp
                165                 170                 175
Lys Tyr His His Thr Phe Cys Ser Leu Leu Gly Arg Asp Glu Asp Ser
            180                 185                 190
Trp Gly Leu Ser Tyr Thr Gly Leu Leu His His Lys Gly Asp Lys Thr
        195                 200                 205
Ser Phe Ser Ser Arg Phe Gly Gln Gly Ser Ile Ile Gly Val His Leu
    210                 215                 220
Asp Thr Trp His Gly Thr Leu Thr Phe Phe Lys Asn Arg Lys Cys Ile
225                 230                 235                 240
Gly Val Ala Ala Thr Arg Leu Gln Asn Arg Arg Phe Tyr Pro Met Val
                245                 250                 255
Cys Ser Thr Ala Ala Lys Ser Ser Met Lys Val Ile Arg Ser Cys Ala
            260                 265                 270
Ser Ser Thr Ser Leu Gln Tyr Leu Cys Cys Tyr Arg Leu Arg Gln Leu
        275                 280                 285
Arg Pro Asp Ser Gly Asp Thr Leu Glu Gly Leu Pro Leu Pro Pro Gly
    290                 295                 300
Leu Lys Gln Val Leu His Asn Lys Leu Gly Trp Val Leu Ser Met Asn
305                 310                 315                 320
Cys Ser His Trp Thr Ser Pro Ala Pro Pro Pro Gly Thr Ala Ala Pro

                325                 330                 335

Ala Ala Glu Arg Asp Ser Arg Glu Thr Arg Pro Cys Gln Arg Lys Arg
            340                 345                 350

Cys Arg Arg Ser
        355

<210> 7
<211> 1055
<212> DNA
<213> Rattus norvegicus

<220>
<221> CDS
<222> (102)..(551)

<400> 7
ctcaaaccct gccctccctg agggtagaag tggagtctgg gggtttagca gccggaaccc 60

ctcctctgtc acggagaaga gatggagcag ttcggctgag g atg gag tta gtt gct 116
Met Glu Leu Val Ala
1               5

cca gag gag aca ggg aag gta cct ccc atc gac tct cgc ccc aac tcc 164
Pro Glu Glu Thr Gly Lys Val Pro Pro Ile Asp Ser Arg Pro Asn Ser
                10                  15                  20

cca gcc ctc tac ttc gat gcc agc ctg gtt cac aag tct cca gac cca 212
Pro Ala Leu Tyr Phe Asp Ala Ser Leu Val His Lys Ser Pro Asp Pro

            25                  30                  35

ttc gga gct gca gca gcc cag agc ctc agc ctg gct cgg tcc atg ttg 260
Phe Gly Ala Ala Ala Ala Gln Ser Leu Ser Leu Ala Arg Ser Met Leu
        40                  45                  50

gcc atc agc ggt cac ctg gac agt gat gac gac agt ggt tcc gga agc 308
Ala Ile Ser Gly His Leu Asp Ser Asp Asp Asp Ser Gly Ser Gly Ser
    55                  60                  65

ctg gtt ggc att gac aac aag att gaa caa gcc atg gac ttg gtg aag 356
Leu Val Gly Ile Asp Asn Lys Ile Glu Gln Ala Met Asp Leu Val Lys
70                  75                  80                  85

tcc cac ctc atg ttt gcc gtg cga gag gag gtg gag gtg ctg aag gag 404
Ser His Leu Met Phe Ala Val Arg Glu Glu Val Glu Val Leu Lys Glu
                90                  95                  100

cag atc cgg gac ctg gca gag cgg aat gct gca ctg gag cag gaa aat 452
Gln Ile Arg Asp Leu Ala Glu Arg Asn Ala Ala Leu Glu Gln Glu Asn
            105                 110                 115

gga ttg ctg cgt gcc ctg gcc agc ccg gag cag ctg gcc cag ctg cca 500
Gly Leu Leu Arg Ala Leu Ala Ser Pro Glu Gln Leu Ala Gln Leu Pro
        120                 125                 130

tcc tcg ggg ctc cca agg ctc ggg ccc tct gca ccc aat ggg cct tcc 548
Ser Ser Gly Leu Pro Arg Leu Gly Pro Ser Ala Pro Asn Gly Pro Ser
    135                 140                 145

atc tgagccttct ttccctcaca atgtgccttt gggggctgcc actggccgcc 601

Ile
150150

gggccttgtg ccagcagcct gccccctctt cctatgtagc tttaatgccc acgcccgacc 661

ccaatgccca gggatgggag ttgaggctaa atattggcct gtcccttccc acctggtctc 721

cccagaagcc tcaggccttg ccggaagaga aagaacccag gaggggatgt ttatctgaag 781

cccctcatcc atgaaagaac ccagccccac ctccttccct gggtattagt gttctgggga 841

gcccctcagc agcagatggc tcagaaagat ttggaggttc cctggcaggc cccctcacca 901

tcccaccttg ttctcttcaa gtgccccctc tcctctgccc agggaggggg tatggacagt 961

atcttcaact tcttggattc aggttgttat taaaataata attataatta aaaaaaatct 1021

gaagaaactt gaaaaaaaaa aaaaaaaaaa aaaa 1055

<210> 8
<211> 150
<212> PRT
<213> Rattus norvegicus

<400> 8
Met Glu Leu Val Ala Pro Glu Glu Thr Gly Lys Val Pro Pro Ile Asp
1               5                   10                  15

Ser Arg Pro Asn Ser Pro Ala Leu Tyr Phe Asp Ala Ser Leu Val His
            20                  25                  30

Lys Ser Pro Asp Pro Phe Gly Ala Ala Ala Ala Gln Ser Leu Ser Leu
        35                  40                  45

Ala Arg Ser Met Leu Ala Ile Ser Gly His Leu Asp Ser Asp Asp Asp
    50                  55                  60

Ser Gly Ser Gly Ser Leu Val Gly Ile Asp Asn Lys Ile Glu Gln Ala
65                  70                  75                  80

Met Asp Leu Val Lys Ser His Leu Met Phe Ala Val Arg Glu Glu Val
                85                  90                  95

Glu Val Leu Lys Glu Gln Ile Arg Asp Leu Ala Glu Arg Asn Ala Ala
            100                 105                 110

Leu Glu Gln Glu Asn Gly Leu Leu Arg Ala Leu Ala Ser Pro Glu Gln
        115                 120                 125

Leu Ala Gln Leu Pro Ser Ser Gly Leu Pro Arg Leu Gly Pro Ser Ala
    130                 135                 140

Pro Asn Gly Pro Ser Ile
145                 150

<210> 9
<211> 878
<212> DNA
<213> Rattus norvegicus
<220>

<221> CDS
<222> (54)..(596)

<400> 9
   ggcacgagca gagagattgt cccaacagag aggcaattct attccctacc aac atg 56
   Met
   1
   aag ctg ttg ctg ctg ctg ctg tgt ctg ggc ctg aca ctg gtc tgt ggc 104
   Lys Leu Leu Leu Leu Leu Leu Cys Leu Gly Leu Thr Leu Val Cys Gly
   5 10 15
   cat gca gaa gaa gct agt tcc aca aga ggg aac ctc gat gtg gct aag 152
   His Ala Glu Glu Ala Ser Ser Thr Arg Gly Asn Leu Asp Val Ala Lys
   20 25 30
   ctc aat ggg gat tgg ttt tct att gtc gtg gcc tct aac aaa aga gaa 200
   Leu Asn Gly Asp Trp Phe Ser Ile Val Val Ala Ser Asn Lys Arg Glu
   35 40 45
   aag ata gaa gag aat ggc agc atg aga gtt ttt atg cag cac atc gat 248
   Lys Ile Glu Glu Asn Gly Ser Met Arg Val Phe Met Gln His Ile Asp
   50 55 60 65
   gtc ttg gag aat tcc tta ggc ttc aag ttc cgt att aag gaa aat gga 296
   Val Leu Glu Asn Ser Leu Gly Phe Lys Phe Arg Ile Lys Glu Asn Gly
   70 75 80
   gag tgc agg gaa cta tat ttg gtt gcc tac aaa acg cca gag gat ggc 344
   Glu Cys Arg Glu Leu Tyr Leu Val Ala Tyr Lys Thr Pro Glu Asp Gly
   85 90 95
   gaa tat ttt gtt gag tat gac gga ggg aat aca ttt act ata ctt aag 392
   Glu Tyr Phe Val Glu Tyr Asp Gly Gly Asn Thr Phe Thr Ile Leu Lys
   100 105 110
   aca gac tat gac aga tat gtc atg ttt cat ctc att aat ttc aag aac 440
   Thr Asp Tyr Asp Arg Tyr Val Met Phe His Leu Ile Asn Phe Lys Asn
   115 120 125
   ggg gaa acc ttc cag ctg atg gtg ctc tac ggc aga aca aag gat ctg 488
   Gly Glu Thr Phe Gln Leu Met Val Leu Tyr Gly Arg Thr Lys Asp Leu
   130 135 140 145
   agt tca gac atc aag gaa aag ttt gca aaa cta tgt gag gcg cat gga 536
   Ser Ser Asp Ile Lys Glu Lys Phe Ala Lys Leu Cys Glu Ala His Gly
   150 155 160
   atc act agg gac aat atc att gat cta acc aag act gat cgc tgt ctc 584
   Ile Thr Arg Asp Asn Ile Ile Asp Leu Thr Lys Thr Asp Arg Cys Leu
   165 170 175
   cag gcc cga gga tgaagaaagg cctgagcctc cagtgctgag tggagacttc 636
   Gln Ala Arg Gly
   180
   tcaccaggac tctagcatca ccatttcctg tccatggagc atcctgagac aaattctgcg 696
   atctgatttc catcctctgt cacagaaaag tgcaatcctg gtctctccag catcttccct 756
   aggttaccca ggacaacaca tcgagaatta aaagctttct taaatttctc ttggccccac 816
   ccatgatcat tccgcacaaa tatcttgctc ttgcagttca ataaatgatt acccttgcac 876
   tt 878

<210> 10
<211> 181
<212> PRT
<213> Rattus norvegicus

<400> 10
Met Lys Leu Leu Leu Leu Leu Leu Cys Leu Gly Leu Thr Leu Val Cys
1               5                   10                  15

Gly His Ala Glu Glu Ala Ser Ser Thr Arg Gly Asn Leu Asp Val Ala
            20                  25                  30

Lys Leu Asn Gly Asp Trp Phe Ser Ile Val Val Ala Ser Asn Lys Arg
        35                  40                  45

Glu Lys Ile Glu Glu Asn Gly Ser Met Arg Val Phe Met Gln His Ile
    50                  55                  60

Asp Val Leu Glu Asn Ser Leu Gly Phe Lys Phe Arg Ile Lys Glu Asn
65                  70                  75                  80

Gly Glu Cys Arg Glu Leu Tyr Leu Val Ala Tyr Lys Thr Pro Glu Asp
                85                  90                  95

Gly Glu Tyr Phe Val Glu Tyr Asp Gly Gly Asn Thr Phe Thr Ile Leu
            100                 105                 110

Lys Thr Asp Tyr Asp Arg Tyr Val Met Phe His Leu Ile Asn Phe Lys
        115                 120                 125

Asn Gly Glu Thr Phe Gln Leu Met Val Leu Tyr Gly Arg Thr Lys Asp
    130                 135                 140

Leu Ser Ser Asp Ile Lys Glu Lys Phe Ala Lys Leu Cys Glu Ala His
145                 150                 155                 160

Gly Ile Thr Arg Asp Asn Ile Ile Asp Leu Thr Lys Thr Asp Arg Cys
                165                 170                 175

Leu Gln Ala Arg Gly
            180

<210> 11
<211> 1124
<212> DNA
<213> Homo sapiens

<220>
<221> CDS
<222> (23)..(868)

<400> 11
gccgctgcca ccgcaccccg cc atg gag cgg ccg tcg ctg cgc gcc ctg ctc   52
                         Met Glu Arg Pro Ser Leu Arg Ala Leu Leu
                         1               5                   10

ctc ggc gcc gct ggg ctg ctg ctc ctg ctc ctg ccc ctc tcc tct tcc 100
Leu Gly Ala Ala Gly Leu Leu Leu Leu Leu Leu Pro Leu Ser Ser Ser
                15                  20                  25

tcc tct tcg gac acc tgc ggc ccc tgc gag ccg gcc tcc tgc ccg ccc 148
Ser Ser Ser Asp Thr Cys Gly Pro Cys Glu Pro Ala Ser Cys Pro Pro
            30                  35                  40

ctg ccc ccg ctg ggc tgc ctg ctg ggc gag acc cgc gac gcg tgc ggc 196
Leu Pro Pro Leu Gly Cys Leu Leu Gly Glu Thr Arg Asp Ala Cys Gly
        45                  50                  55

tgc tgc cct atg tgc gcc cgc ggc gag ggc gag ccg tgc ggg ggt ggc 244
Cys Cys Pro Met Cys Ala Arg Gly Glu Gly Glu Pro Cys Gly Gly Gly
    60                  65                  70

ggc gcc ggc agg ggg tac tgc gcg ccg ggc atg gag tgc gtg aag agc 292
Gly Ala Gly Arg Gly Tyr Cys Ala Pro Gly Met Glu Cys Val Lys Ser
75                  80                  85                  90

cgc aag agg cgg aag ggt aaa gcc ggg gca gca gcc ggc ggt ccg ggt 340
Arg Lys Arg Arg Lys Gly Lys Ala Gly Ala Ala Ala Gly Gly Pro Gly
                95                  100                 105

gta agc ggc gtg tgc gtg tgc aag agc cgc tac ccg gtg tgc ggc agc 388
Val Ser Gly Val Cys Val Cys Lys Ser Arg Tyr Pro Val Cys Gly Ser
            110                 115                 120

gac ggc acc acc tac ccg agc ggc tgc cag ctg cgc gcc gcc agc cag 436
Asp Gly Thr Thr Tyr Pro Ser Gly Cys Gln Leu Arg Ala Ala Ser Gln
        125                 130                 135

agg gcc gag agc cgc ggg gag aag gcc atc acc cag gtc agc aag ggc 484
Arg Ala Glu Ser Arg Gly Glu Lys Ala Ile Thr Gln Val Ser Lys Gly
    140                 145                 150

acc tgc gag caa ggt cct tcc ata gtg acg ccc ccc aag gac atc tgg 532
Thr Cys Glu Gln Gly Pro Ser Ile Val Thr Pro Pro Lys Asp Ile Trp
155                 160                 165                 170

aat gtc act ggt gcc cag gtg tac ttg agc tgt gag gtc atc gga atc 580
Asn Val Thr Gly Ala Gln Val Tyr Leu Ser Cys Glu Val Ile Gly Ile
                175                 180                 185

ccg aca cct gtc ctc atc tgg aac aag gta aaa agg ggt cac tat gga 628
Pro Thr Pro Val Leu Ile Trp Asn Lys Val Lys Arg Gly His Tyr Gly
            190                 195                 200

gtt caa agg aca gaa ctc ctg cct ggt gac cgg gac aac ctg gcc att 676
Val Gln Arg Thr Glu Leu Leu Pro Gly Asp Arg Asp Asn Leu Ala Ile
        205                 210                 215

cag acc cgg ggt ggc cca gaa aag cat gaa gta act ggc tgg gtg ctg 724
Gln Thr Arg Gly Gly Pro Glu Lys His Glu Val Thr Gly Trp Val Leu
    220                 225                 230

gta tct cct cta agt aag gaa gat gct gga gaa tat gag tgc cat gca 772
Val Ser Pro Leu Ser Lys Glu Asp Ala Gly Glu Tyr Glu Cys His Ala
235                 240                 245                 250

tcc aat tcc caa gga cag gct tca gca tca gca aaa att aca gtg gtt 820
Ser Asn Ser Gln Gly Gln Ala Ser Ala Ser Ala Lys Ile Thr Val Val

                255                 260                 265

gat gcc tta cat gaa ata cca gtg aaa aaa ggt gaa ggt gcc gag cta 868
Asp Ala Leu His Glu Ile Pro Val Lys Lys Gly Glu Gly Ala Glu Leu
            270                 275                 280

taaacctcca gaatattatt agtctgcatg gttaaaagta gtcatggata actacattac 928

ctgttcttgc ctaataagtt tcttttaatc caatccacta acactttagt tatattcact 988

ggttttacac agagaaatac aaaataaaga tcacacatca agactatcta caaaatttta 1048

ttatatattt acagaagaaa agcatgcata tcattaaaca aataaaatac tttttatcac 1108

aaaaaaaaaa aaaaaa 1124

<210> 12
<211> 282
<212> PRT
<213> Homo sapiens

<400> 12
Met Glu Arg Pro Ser Leu Arg Ala Leu Leu Leu Gly Ala Ala Gly Leu
1               5                   10                  15

Leu Leu Leu Leu Leu Pro Leu Ser Ser Ser Ser Ser Ser Asp Thr Cys
            20                  25                  30

Gly Pro Cys Glu Pro Ala Ser Cys Pro Pro Leu Pro Pro Leu Gly Cys
        35                  40                  45

Leu Leu Gly Glu Thr Arg Asp Ala Cys Gly Cys Cys Pro Met Cys Ala
    50                  55                  60
Arg Gly Glu Gly Glu Pro Cys Gly Gly Gly Gly Ala Gly Arg Gly Tyr
65                  70                  75                  80
Cys Ala Pro Gly Met Glu Cys Val Lys Ser Arg Lys Arg Arg Lys Gly
                85                  90                  95
Lys Ala Gly Ala Ala Ala Gly Gly Pro Gly Val Ser Gly Val Cys Val
            100                 105                 110
Cys Lys Ser Arg Tyr Pro Val Cys Gly Ser Asp Gly Thr Thr Tyr Pro
        115                 120                 125
Ser Gly Cys Gln Leu Arg Ala Ala Ser Gln Arg Ala Glu Ser Arg Gly
    130                 135                 140
Glu Lys Ala Ile Thr Gln Val Ser Lys Gly Thr Cys Glu Gln Gly Pro
145                 150                 155                 160
Ser Ile Val Thr Pro Pro Lys Asp Ile Trp Asn Val Thr Gly Ala Gln
                165                 170                 175
Val Tyr Leu Ser Cys Glu Val Ile Gly Ile Pro Thr Pro Val Leu Ile
            180                 185                 190
Trp Asn Lys Val Lys Arg Gly His Tyr Gly Val Gln Arg Thr Glu Leu
195, 200 205

Leu Pro Gly Asp Arg Asp Asn Leu Ala Ile Gln Thr Arg Gly Gly Pro
210 215 220

Glu Lys His Glu Val Thr Gly Trp Val Leu Val Ser Pro Leu Ser Lys
225 230 235 240

Glu Asp Ala Gly Glu Tyr Glu Cys His Ala Ser Asn Ser Gln Gly Gln
245 250 255

Ala Ser Ala Ser Ala Lys Ile Thr Val Val Asp Ala Leu His Glu Ile
260 265 270

Pro Val Lys Lys Gly Glu Gly Ala Glu Leu
275 280

<210> 13
<211> 1043
<212> DNA
<213> Rattus norvegicus

<220>
<221> CDS
<222> (15)..(674)

<400> 13
ggccggccgg cacg atg ttg ggc gcg agc cgc ggg tta gcg ggt ctg acg 50
Met Leu Gly Ala Ser Arg Gly Leu Ala Gly Leu Thr
1               5                   10

ctg ctg ggg ctg ctg ctg gcg ctc tcg gtg cgg agc ggt ggc gcg tcg 98

Leu Leu Gly Leu Leu Leu Ala Leu Ser Val Arg Ser Gly Gly Ala Ser
        15                  20                  25

aag gcc agc gcc ggg cta gtg acc tgc ggg tca gtg ctg aag cta ctc 146
Lys Ala Ser Ala Gly Leu Val Thr Cys Gly Ser Val Leu Lys Leu Leu
    30                  35                  40

aac acc cac cac aga gtg cgg ctg cac tca cat gac atc aaa tac gga 194
Asn Thr His His Arg Val Arg Leu His Ser His Asp Ile Lys Tyr Gly
45                  50                  55                  60

tcc ggc agc ggc caa cag tcg gta acc ggc gtg gag gcg tcc gac gat 242
Ser Gly Ser Gly Gln Gln Ser Val Thr Gly Val Glu Ala Ser Asp Asp
                65                  70                  75

gcc aat agt tac tgg cga att cgc ggc ggc tcc gag ggt ggg tgc ccg 290
Ala Asn Ser Tyr Trp Arg Ile Arg Gly Gly Ser Glu Gly Gly Cys Pro
            80                  85                  90

cgc ggg ctc cca gtg cgc tgt ggg cag gca gtg cgg ctc acg cac gtg 338
Arg Gly Leu Pro Val Arg Cys Gly Gln Ala Val Arg Leu Thr His Val
        95                  100                 105

ctc acc ggc aag aac ctg cac acg cac cac ttc ccg tca ccg cta tcc 386
Leu Thr Gly Lys Asn Leu His Thr His His Phe Pro Ser Pro Leu Ser
    110                 115                 120

aac aac cag gag gtg agt gct ttt ggg gaa gac ggt gag ggt gat gac 434
Asn Asn Gln Glu Val Ser Ala Phe Gly Glu Asp Gly Glu Gly Asp Asp
125                 130                 135                 140

ctg gac ctg tgg aca gta cga tgt tct ggg caa cac tgg gag cga gag 482
Leu Asp Leu Trp Thr Val Arg Cys Ser Gly Gln His Trp Glu Arg Glu
                145                 150                 155

gcc agt gtc cgt ttc cag cat gtt ggc acc tct gtg ttc ctg tca gtt 530
Ala Ser Val Arg Phe Gln His Val Gly Thr Ser Val Phe Leu Ser Val
            160                 165                 170

act ggt gaa cag tat ggt aac cca atc cgt ggg cag cat gag gtg cat 578
Thr Gly Glu Gln Tyr Gly Asn Pro Ile Arg Gly Gln His Glu Val His
        175                 180                 185

ggc atg cct agt gcc aat gca cac aac acg tgg aag gcc atg gaa gga 626
Gly Met Pro Ser Ala Asn Ala His Asn Thr Trp Lys Ala Met Glu Gly
    190                 195                 200

atc ttc atc aag ccc gga gca gat ccc tcc aca ggt cac gat gaa ctc 674
Ile Phe Ile Lys Pro Gly Ala Asp Pro Ser Thr Gly His Asp Glu Leu
205                 210                 215                 220

tgagccggat gggaagggag ggtggctgag tgggaatccg cagggctgct cttgtgtaag 734

actctgtagg ggccctcaag tgcctttctg attaaagaat gttggtttgt gattattttt 794

gctgtaccct ggggaggacc tgagggtgct agtcatatct gtccacatca tcatctcaca 854

tgtctcaagt acctgttcaa ataatttttg agaccgtccc actatgtatc cctggctggc 914

ctggaactcc cagagatcca cttgcctctg cctcctgagc gctggtatta aaggtgtata 974

cgaccacagc tggccccaac ctgttcaata aactaatttt tattacagtg tgaaaaaaaa 1034

aaaaaaaaa 1043

<210> 14
<211> 220
<212> PRT
<213> Rattus norvegicus

<400> 14
Met Leu Gly Ala Ser Arg Gly Leu Ala Gly Leu Thr Leu Leu Gly Leu
1               5                   10                  15

Leu Leu Ala Leu Ser Val Arg Ser Gly Gly Ala Ser Lys Ala Ser Ala
            20                  25                  30

Gly Leu Val Thr Cys Gly Ser Val Leu Lys Leu Leu Asn Thr His His
        35                  40                  45

Arg Val Arg Leu His Ser His Asp Ile Lys Tyr Gly Ser Gly Ser Gly
    50                  55                  60

Gln Gln Ser Val Thr Gly Val Glu Ala Ser Asp Asp Ala Asn Ser Tyr
65                  70                  75                  80

Trp Arg Ile Arg Gly Gly Ser Glu Gly Gly Cys Pro Arg Gly Leu Pro
                85                  90                  95

Val Arg Cys Gly Gln Ala Val Arg Leu Thr His Val Leu Thr Gly Lys
            100                 105                 110

Asn Leu His Thr His His Phe Pro Ser Pro Leu Ser Asn Asn Gln Glu
        115                 120                 125

Val Ser Ala Phe Gly Glu Asp Gly Glu Gly Asp Asp Leu Asp Leu Trp
    130                 135                 140

Thr Val Arg Cys Ser Gly Gln His Trp Glu Arg Glu Ala Ser Val Arg
145                 150                 155                 160

Phe Gln His Val Gly Thr Ser Val Phe Leu Ser Val Thr Gly Glu Gln
                165                 170                 175

Tyr Gly Asn Pro Ile Arg Gly Gln His Glu Val His Gly Met Pro Ser
            180                 185                 190

Ala Asn Ala His Asn Thr Trp Lys Ala Met Glu Gly Ile Phe Ile Lys
        195                 200                 205

Pro Gly Ala Asp Pro Ser Thr Gly His Asp Glu Leu
210 215

<210> 15
<211> 844
<212> DNA
<213> Homo sapiens

<220>
<221> CDS
<222> (39)..(701)

<400> 15
ggcccctggg cccgaggggc tggagccggg ccggggcg atg tgg agc gcg ggc cgc 56
Met Trp Ser Ala Gly Arg
1               5

ggc ggg gct gcc tgg ccg gtg ctg ttg ggg ctg ctg ctg gcg ctg tta 104
Gly Gly Ala Ala Trp Pro Val Leu Leu Gly Leu Leu Leu Ala Leu Leu
            10                  15                  20

gtg ccg ggc ggt ggt gcc gcc aag acc ggt gcg gag ctc gtg acc tgc 152
Val Pro Gly Gly Gly Ala Ala Lys Thr Gly Ala Glu Leu Val Thr Cys
        25                  30                  35

ggg tcg gtg ctg aag ctg ctc aat acg cac cac cgc gtg cgg ctg cac 200
Gly Ser Val Leu Lys Leu Leu Asn Thr His His Arg Val Arg Leu His
    40                  45                  50

tcg cac gac atc aaa tac gga tcc ggc agc ggc cag caa tcg gtg acc 248
Ser His Asp Ile Lys Tyr Gly Ser Gly Ser Gly Gln Gln Ser Val Thr
55                  60                  65                  70

ggc gta gag gcg tcg gac gac gcc aat agc tac tgg cgg atc cgc ggc 296
Gly Val Glu Ala Ser Asp Asp Ala Asn Ser Tyr Trp Arg Ile Arg Gly
                75                  80                  85

ggc tcg gag ggc ggg tgc cgc cgc ggg tcc ccg gtg cgc tgc ggg cag 344
Gly Ser Glu Gly Gly Cys Arg Arg Gly Ser Pro Val Arg Cys Gly Gln
            90                  95                  100

gcg gtg agg ctc acg cat gtg ctt acg ggc aag aac ctg cac acg cac 392
Ala Val Arg Leu Thr His Val Leu Thr Gly Lys Asn Leu His Thr His

        105                 110                 115

cac ttc ccg tcg ccg ctg tcc aac aac cag gag gtg agt gcc ttt ggg 440
His Phe Pro Ser Pro Leu Ser Asn Asn Gln Glu Val Ser Ala Phe Gly
    120                 125                 130

gaa gac ggc gag ggc gac gac ctg gac cta tgg aca gtg cgc tgc tct 488
Glu Asp Gly Glu Gly Asp Asp Leu Asp Leu Trp Thr Val Arg Cys Ser
135                 140                 145                 150

gga cag cac tgg gag cgt gag gct gct gtg cgc ttc cag cat gtg ggc 536
Gly Gln His Trp Glu Arg Glu Ala Ala Val Arg Phe Gln His Val Gly
                155                 160                 165

acc tct gtg ttc ctg tca gtc acg ggt gag cag tat gga agc ccc atc 584
Thr Ser Val Phe Leu Ser Val Thr Gly Glu Gln Tyr Gly Ser Pro Ile
            170                 175                 180

cgt ggg cag cat gag gtc cac ggc atg ccc agt gcc aac acg cac aat 632
Arg Gly Gln His Glu Val His Gly Met Pro Ser Ala Asn Thr His Asn
        185                 190                 195

acg tgg aag gcc atg gaa ggc atc ttc atc aag cct agt gtg gag ccc 680
Thr Trp Lys Ala Met Glu Gly Ile Phe Ile Lys Pro Ser Val Glu Pro
    200                 205                 210

tct gca ggt cac gat gaa ctc tgagtgtgtg gatggatggg tggatggagg 731
Ser Ala Gly His Asp Glu Leu
215                 220

gtggcaggtg gggcgtctgc agggccactc ttggcagaga ctttgggttt gtaggggtcc 791

tcaagtgcct ttgtgattaa agaatgttgg tctatgaaaa aaaaaaaaaa aaa 844

<210> 16
<211> 221
<212> PRT
<213> Homo sapiens

<400> 16
Met Trp Ser Ala Gly Arg Gly Gly Ala Ala Trp Pro Val Leu Leu Gly
1               5                   10                  15

Leu Leu Leu Ala Leu Leu Val Pro Gly Gly Gly Ala Ala Lys Thr Gly
            20                  25                  30

Ala Glu Leu Val Thr Cys Gly Ser Val Leu Lys Leu Leu Asn Thr His
        35                  40                  45

His Arg Val Arg Leu His Ser His Asp Ile Lys Tyr Gly Ser Gly Ser
    50                  55                  60

Gly Gln Gln Ser Val Thr Gly Val Glu Ala Ser Asp Asp Ala Asn Ser
65                  70                  75                  80

Tyr Trp Arg Ile Arg Gly Gly Ser Glu Gly Gly Cys Arg Arg Gly Ser
                85                  90                  95

Pro Val Arg Cys Gly Gln Ala Val Arg Leu Thr His Val Leu Thr Gly
            100                 105                 110

Lys Asn Leu His Thr His His Phe Pro Ser Pro Leu Ser Asn Asn Gln
        115                 120                 125

Glu Val Ser Ala Phe Gly Glu Asp Gly Glu Gly Asp Asp Leu Asp Leu
    130                 135                 140

Trp Thr Val Arg Cys Ser Gly Gln His Trp Glu Arg Glu Ala Ala Val
145                 150                 155                 160

Arg Phe Gln His Val Gly Thr Ser Val Phe Leu Ser Val Thr Gly Glu
                165                 170                 175

Gln Tyr Gly Ser Pro Ile Arg Gly Gln His Glu Val His Gly Met Pro
            180                 185                 190

Ser Ala Asn Thr His Asn Thr Trp Lys Ala Met Glu Gly Ile Phe Ile
        195                 200                 205

Lys Pro Ser Val Glu Pro Ser Ala Gly His Asp Glu Leu
    210                 215                 220

<210> 17
<211> 927
<212> DNA
<213> Rattus norvegicus

<400> 17
angctcgaaa ttaaccctca ctaaagggaa caaaagctgg agctccaccg cggtggcggc 60

cgctctagaa ctagtggatc ccccgggctg caggaattcg gcacgaggga cagagagcgc 120

atggagatgg gaagactgtc gccacccaga cagccacgca agtgccttta actttgagaa 180

ggccttttct ccttttctga tttggtgcta cggactcacg acagaactca gacaccagca 240

gacaagagtc tcggcctagg tggcggtggc cactctggcc agacgaaagc cagtttgttt 300

ctgatttttg ccttctttac aactaagcag ttttgtgtag cagggcaggc ctgttccggc 360

cagctttctt ttaagatccg ggttaatttt cctttccagc agccttctct ctggagtggc 420

ctctaccaca ctaacaggag gtgtcttcag agtatggaca gctagccacg aggcccctcc 480

gctcctggga gggctactcc gttcctagga caccagaggc cacaaactag ggttgggcca 540

caagcacaca atgctttctt ccacggcagg aattcatacc aaaaccacaa gcaaaaaaca 600

aaacaaaaaa aaaaaaaaaa aactcgaggg ggggcccggt acccaattcg ccctatagtg 660

gagtcgtatt acaattcant gggccgtcgt tttacaacgt cgtgactggg aaaaccctgg 720

ggttacccaa ctttaatcgc cttgnagcaa atcccccttt tggccagctg gggtaatagc 780

gaagaaggcc cgnaccggat tggcctttcc aaaagttgcg cagcttgaat ggggaatggg 840

aaattgtaag ggtaanaat ttggttaaaa atcggngtta aaattttggt aaaatcaggc 900

ccantttttt aacccaaaaa ggggggg 927

<210> 18

<211> 933
<212> DNA
<213> Rattus norvegicus

<400> 18
caaggtcgaa attaaccctc actaaaggga acaaaagctg gagctccacc gcggtggcgg 60

ccgctctaga actagtggat cccccgggct gcaggttttt tttttttttt ttcacnagct 120

tgatctatct ctccgttctc catggctctg atgtcaggcc gcagcctgca gtcagtcttg 180

ggaatcacgg tctccatctc cttgtctacc tcattcaaga ccatggcaaa gctggtaaaa 240

ttatacatct gagcagagtt gggaggccgc ggggctatcc gccacaggag cgcactgcca 300

ggaataacaa atacgctctc agagtccggc actgggcatt tcgtcagact cctcagatgg 360

cgcttgcctg tttggctgtt cttcttctct tctgtgtttt tcttatcgtt tttttgtaa 420

gcgtcaaaag tggcagggtc tacactgtac aagcactcgg tccacttccc gtagagagca 480

cagagtttct ttttgctttt ancttggatg tagccttcaa ctttgtgtaa ttccttgcca 540

aaaagaccac atggcttaaa attcaacaca cacttgtccc cagtcttgtg gtttangatt 600

tccacattgc catactgttc gatccagagt ttgcccacga tgatgttatg cacacagcag 660

tggggtttgt ccatgtgtat gggggggccc cggtacccaa tcgncctana gtgagtcgta 720

atacaattca cngggccggt cggtttacaa agtcgtggaa tgggaaaaac ctgggcggtt 780

acccaaactt naatcgcctt gcagcaaaaa ccccctttcg gcaaannggg ggnaaaaagc 840

gaanaaggcc cggaancgga attggcncct tccccaaaaa gnttgcggac nctngaaaag 900

ggggaaatgg gcaaaatgga aaancggtta aaa 933

<210> 19
<211> 933
<212> DNA
<213> Rattus norvegicus

<400> 19
caaggtcgaa attaaccctc actaaaggga acaaaagctg gagctccacc gcggtggcgg 60

ccgctctaga actagtggat cccccgggct gcaggttttt tttttttttt ttcacnagct 120

tgatctatct ctccgttctc catggctctg atgtcaggcc gcagcctgca gtcagtcttg 180

ggaatcacgg tctccatctc cttgtctacc tcattcaaga ccatggcaaa gctggtaaaa 240

ttatacatct gagcagagtt gggaggccgc ggggctatcc gccacaggag cgcactgcca 300

ggaataacaa atacgctctc agagtccggc actgggcatt tcgtcagact cctcagatgg 360

cgcttgcctg tttggctgtt cttcttctct tctgtgtttt tcttatcgtt tttttgtaa 420

gcgtcaaaag tggcagggtc tacactgtac aagcactcgg tccacttccc gtagagagca 480

cagagtttct ttttgctttt ancttggatg tagccttcaa ctttgtgtaa ttccttgcca 540

aaaagaccac atggcttaaa attcaacaca cacttgtccc cagtcttgtg gtttangatt 600

tccacattgc catactgttc gatccagagt ttgcccacga tgatgttatg cacacagcag 660

tggggtttgt ccatgtgtat gggggggcc cggtacccaa tcgncctana gtgagtcgta 720

atacaattca cngggccggt cggtttacaa agtcgtggaa tgggaaaaac ctgggcggtt 780

acccaaactt naatcgcctt gcagcaaaaa ccccctttcg gcaaannggg ggnaaaaagc 840

gaanaaggcc cggaancgga attggcncct tccccaaaaa gnttgcggac nctngaaaag 900

ggggaaatgg gcaaaatgga aaancggtta aaa 933

<210> 20
<211> 942
<212> DNA
<213> Rattus norvegicus

<400> 20
   caagngcgaa attaaccctc actaaaggga acaaaagctg gagctccacc gcggtggcgg 60
   ccgctctaga actagtggat cccccgggct gcaggctgac tttataggaa aactgattat 120
   atcaatgtgt atatgtgtta tatatacata tattcaatac tgccttctct ttttgtctac 180
   agtatcaaaa ttgactgacg gaatcatgaa aagaatgttc cccatcacca tttagagttt 240
   tatttttgtt ttctttgttt atcaatgaat ggtgtaagaa tcaagtctct tgtttttttg 300
   aagaaaaaaa gcaatattcc ttgaagagca aggaggattg aaggattttg tttgagtgag 360
   gaacagagtt cataactagt ttgttggata cttgtaaggt tggtatcttt gtgggcctat 420
   atactctaaa atgaaccttg gtggcttgtg ggccattact tgacctatga atctttaagg 480
   gcacaatcag ttatctttta catataaaga tcgcttggag tgatggccac cgctcctgcc 540
   cgncctccct ccctcccttc cttccgggaa aannngcggg ncnnnnnncc nccnnncnnn 600
   cccnncnnnn nnnnnnnnnn nnnnnnngcc gngggggggn ccggnnnccn nnngnccnnn 660
   nnnggggncg nnnnnnnnnn nnnnngggnn gnngnnnnnn nnngnnnnnn nnnggggnnn 720
   anccnggngn nnnnncnnnn nnnnnnngnn nngnnnnnnn nnnncccnnn nnnnnnnnng 780
   ggggnnnnnn nnnnnnggnn nngnnnnngn nnngnnnnnn nnnnnnnnnn nnnnnnnnnn 840
   nnnnnggnnn nnngggnnnn nnnnnnnnnn gnnnnnnnnn nnnngnnnnn nnnnnngngg 900
   nnnnnnnnnn nnnnnnnnnn nnnnnnnnnn nnnnnnnnnn nn 942

<210> 21
<211> 929
<212> DNA
<213> Rattus norvegicus

<400> 21
ncaagcgcng aaattaaccc tcactaaagg gaacaaaagc tggagctcca ccgcggtggc 60

ggccgctcta gaactagtgg atcccccggg ctgcagggtt gcgggctttg aaccgcctgg 120

ccgcgcggcc cgggggccag cccccaaccc tgctccttct gcccgtgcgc ggccgcaaga 180

cccgccacga tccgcctgcc aagtccaagg tcgggcgcgt gaaaatgcct cctgcagtgg 240

accctgcgga attgttcgtg ttgaccgagc gctaccgaca gtaccgggag acggtgcgcg 300

ctctcaggcg agagttcaca ttggaggtgc gagggaaatt gcacgaggcc cgagccgggg 360

ttctggctga gcgcaaggcg caagaggcca tcagagagca ccaggagctg atggcctgga 420

accgggagga gaaccggaga ctgcaggaac tacgggatag ctaggttgca gctcgaagca 480

caggcccagg agctgcggca ggctgaggtc caggcccaga gggcccagga ggagcaggct 540

tgggtgcaac tgaaagaaca agaagttctc aaactgcagg aggaggccaa aaacttcatc 600

actcggggag aacctggagg gcacggatag aagaggcctt ggactctccg aagagttata 660

actggggcgg ttcaccaaag aagggcaggt ggttcaggaa ctgagaacag aggctcttca 720

ggcccaaata aggacatgct tgcctaagga tggatattgg ggtagaaatt ggtgcatccc 780

aggagggtng caanancttg ttccagagcn agcccccatt tcatttctna ganttngcac 840

caaggtatag taccctgttc ttgacaccaa catnccaaac ttcgggacag canttaaaac 900

tcctgggnaa nttctatcaa accagaagg 929

<210> 22
<211> 925
<212> DNA
<213> Rattus norvegicus

<400> 22
ncaagcgncg aaattaaccc tcactaaagg gaacaaaagc tggagctcca ccgcggtggc 60

ggccgctcta gaactagtgg atcccccggg ctgcaggaat tcggcacgag atggcagacg 120

tcctcccagg tatgcagaag acaacttctg gagtcagttc tgtcaaccat gtgggtctca 180

gggatggaac tcagttggtc atgattggca gcaagcacct ttacctgctg acccacctca 240

gcactcctga tggagcagat gtagatgaac aatatcatct atgaaacatt ccaaacaaaa 300

ctaacttgaa tccatcaagc cttcccatca aacacgcaat ttttttaatt tgttttatta 360

ttttttgatg tctgtgtgtt atgtctgcat gtatgtctgt gtgccatgtt tgttcccggt 420

gccccctgga agtcagaaga aggaatcaga tcccctagaa ctggagtttc agaaagatga 480

gctgcaggtg ggggctggga attgaacctg tgtcctctgg aagagcagct ggtgctctta 540

acagctgaac acctctccag tgccaaacac accatttata agaaatacaa caggtggaaa 600

taacaaattc tgtggccatt ctggaggata actggtgtat aagcttcaac aatgtatcat 660

cctggaagaa acaatggctg tgtggggaaa aaaaaaatct aagggacatt acagcctgac 720

ccagatccna ttctggaaca gacaagctat aaaacacctt tcagcacaat tggaaggagg 780

aacgaaagcc atgggaatat ttggataaga tgaagttgtg ttgccatgca agccttggga 840

ggncattaag gaaacgggca agtccncaaa aagggggngn tgnnccanaa naaccccggg 900

gttaaaaann nnaaaagggg ggggg 925

<210> 23
<211> 1828
<212> DNA
<213> Rattus norvegicus

<400> 23
ncaagcgcga aattaacnnt cactaaaggg aacaaaagct ggagctccac cgcggtggcg 60

gccgctctag aactagtgga tcccccgggc tgcaggtttt tgccgagggg tcttgggctg 120

gggcggacag tgtacgggat ggaggcgact ttggagcagc atttggagga cacaatgaag 180

aatccatcca ttgttggagt cctatgcaca gattcacaag gacttaatct gggctgccgt 240

ggtaccctgt cggatgagca tgctggagtg atatctgttc tcgcccagca ggcagctaag 300

ctgacctctg accccaccga catccctgta gtgtgtttag agtcagataa cgggaatgtt 360

atgatccaga aacacgatgg catcacagtg gctgtgcaca aaatggcctc ttgacatctg 420

atgccagctc tccagtggtc tcccaccggg attcagtcat gcctgtctca gttaacttgt 480

aaaactatta aagttccaga aatcgggcca ttcacttaat gtccaatgtg gacttcttat 540

taatatgaca gtcagttacc aagacgtcag ttaggagtgt ggtggccttg tctgggcttt 600

tgtcactctg ctctttggtg acagccactg tagtccagga tcatatccct caggcctaga 660

actgtgtagc ccaggctgac ttcaaattta tggtcttcct gcttcaaact cctatatcct 720

ggggatttag cattgtcatg ggtctaggtc actttgtata tagaactttg ttgtgggtca 780

ataaaccggg ggggnccggg tacccaattc gnccnaaagt ggagtcggaa ttacaaattc 840

cactggccgt ccgtttttac aaaggtcgtg actgggaaaa aacctgggcg gttancccaa 900

cttnaaacgg cctgncaagc gcgaaattaa cnntcactaa agggaacaaa agctggagct 960

ccaccgcggt ggcggccgct ctagaactag tggatccccc gggctgcagg gttttgccga 1020

ggggtcttgg gctggggcgg acagtgtacg ggatggaggc gactttggag cagcatttgg 1080

aggacacaat gaagaatcca tccattgttg gagtcctatg cacagattca caaggactta 1140

atctgggctg ccgtggtacc ctgtcggatg agcatgctgg agtgatatct gttctcgccc 1200

agcaggcagc taagctgacc tctgacccca ccgacatccc tgtagtgtgt ttagagtcag 1260

ataacgggaa tgttatgatc cagaaacacg atggcatcac agtggctgtg cacaaaatgg 1320

cctcttgaca tctgatgcca gctctccagt ggtctcccac cgggattcag tcatgcctgt 1380

ctcagttaac ttgtaaaact attaaagttc cagaaatcgg gccattcact taatgtccaa 1440

tgtggacttc ttattaatat gacagtcagt taccaagacg tcagttagga gtgtggtggc 1500

cttgtctggg cttttgtcac tctgctcttt ggtgacagcc actgtagtcc aggatcatat 1560

ccctcaggcc tagaactgtg tagcccaggc tgacttcaaa tttatggtct tcctgcttca 1620

aactcctata tcctggggat ttagcattgt catgggtcta ggtcactttg tatatagaac 1680

tttgttgtgg gtcaataaac cgggggggnc cgggtaccca attcgnccna aagtggagtc 1740

ggaattacaa attccactgg ccgtccgttt ttacaaaggt cgtgactggg gaaaaacctg 1800

ggcggttanc ccaacttnaa acggcctg 1828

<210> 24
<211> 936
<212> DNA
<213> Rattus norvegicus

<400> 24
caagcgcgaa attaaccctc acgtaaaggg aacaaaagct ggagctccac cgcggtggcg 60

gccgctctag aactagtgga tcccccgggc tgcaggtcta cagcgatctc tcgttgatct 120

ccaactgccg cctccattcg ccatggaccc caactgctcc tgtgccacag atggatcctg 180

ctcctgcgct ggctcctgca aatgcaaaca atgcaaatgc acctcctgca agaaaagctg 240

ctgttcctgc tgccccgtgg gctgtgcgaa gtgctcccag ggctgcatct gcaaagaggc 300

ttcggacaag tgcagctgca gcgcctgaag tgggggcgtc ctcacaatgg tgtaaataaa 360

acaacgtagg gaacctagcc ttttttgta caaccctgac aggttctcca cactttttc 420

tataaagcat gtaactgnac aataaaataa aaaaacttgg acttggatta aaaaaaaaa 480

aaaaaaaaac tcgagggggg gcccggtacc caattcgccc tatagtgagt cgtattacaa 540

ttcactggcc gtcgttttac aacgtcgtga ctgggaaaac cctggcgtta cccaacttaa 600

tcggccttgc agcacatccc cctttcggcc agctggcgta aatagcgaag aggcccgcac 660

cggatcgccc ttcccaanag ttgcgcacct ggaatggcga atggcaaatt gtaagcgtta 720

atattttgtt aaaattcgcg ttaaattttt gntaaatcag ctcatttttt aaccaatagg 780

gccgaaatcg gggaaaatcc cttaataaat caaaagnata gnccggagat agggttgant 840

ggttgttccc agttttggaa ccaaggagtc caccnattta aagaaccgtg ggactccaan 900

ggccaaaagg gnggaaaaaa ccggnntaat caggggg 936

<210> 25
<211> 941
<212> DNA
<213> Rattus norvegicus

<400> 25
   ncaagcgcgg aaattaaccc gtcacgtaaa gggaacaaaa gctggagctc caccgcggtg 60
   gcggccgctc tagaactagt ggatcccccg ggctgcagga attcggcacg agcttccttg 120
   agactactgc gccatgagag cgaagtgggc ggaagaagag aatgcgcagg ctgaagcgca 180
   agagaagaaa gatgaggcag aggtccaagt aaaccatctt gtgcacccac gaagcctgcg 240
   ggagcagaag taagggatgc tgaagcccgg aacaagtggt tggactgtat gctgctgtcg 300
   gtaataagtc tcagtagacc cggaatgtca cctcgccgag atcagctggg aaaatgacta 360
   ccttcctcac aaccaaaaca gtcccgctgg ccctctgccc tgggaccttt gggcattctg 420
   ggactagttc tgttctcttg tggccaagtg taactcgtgt acaataaacc ctcttgctgt 480
   cagctggaag aatcaaaaaa aaaaaaaaaa aactcgaggg ggggcccggt acccaattcg 540
   ccctatagtg agtcgtatta caattcactg gccgtcgttt tacaacgtcg tgactgggaa 600
   aaccctggcg ttacccaact taatcgcctt gcagcacatc cccctttcgc cagctggggt 660
   naatagcgaa gaggcccgca ccgatcggcc cttcccaaca gttgcgcacc tggaatggcg 720
   aatgggcaaa ttgtaagcgt taataatttg ttaaaattcg cgttaaaatt tttgttaaat 780
   cagctcattt tttaaccaat agggcggaaa tcggcaaaaa tnccttataa atcaaaaagg 840
   ataggaccgg agataggggn tgaagtggtt ggtnccagnt ttnggnacaa agagtccccc 900
   taattaaaag gaangggggg gcctcccaaa nggtcnaaan g 941

<210> 26
<211> 929
<212> DNA
<213> Rattus norvegicus

<400> 26
   ncaagcgcgg aaattaaccc tcactaaagg gaacaaaagc tggagctcca ccgcggtggc 60
   ggccgctcta gaactagtgg atcccccggg ctgcaggctc aggatgagag agcacgtcat 120
   gaatgagatt gataacaaca aagaccgatt ggtgactctg gaggaattct tgagagccac 180
   agagaagaaa gaattcttgg agcccgatag ctgggagaca ctggaccagc agcagttatt 240
   caccgaggaa gagctcaaag agtatgaaag tatcattgct atccaagaga gtgaacttaa 300
   gaagaaggca gatgaactgc agaagcagaa ggaggagctg cagcgccagc acgaccacct 360
   tgagggccca gaagcaggag tatcagcagg gccgttacag cagctgggaa cagaagaaat 420
   tccaacaagg gattgctcca tcaggggccg gcaggagagc tgaagtttga gccaaacaca 480
   taaaagtcct gatgtctgcc agaacttggg aagaaaaccg ttgactcaac atctgtttca 540
   tctttcaaca tcccttcttt tctcttcact caataaatac tttaaaagca aaaaaaaaaa 600
   aaaaaaaaaa aactcgaggg ggggcccggt acccaattcg ccctatagtg agtcgtatta 660
   caattcactg gccgtcgttt tacaacgtcg tgactgggaa aaccctgggg ttacccaact 720
   taatcgcctt gnagcacatc cccctttcgc cagctggngt aaatagcgaa gaggcccgca 780
   ccggatnggc ccttcccnaa cagttgngca ccttgaaatg ggcggaatgg gcaaattgta 840
   agcgttaana ttttgttaaa attcgcgtta aatttttngn naaatcaggc ccantttttt 900
   aacccaatag ggccgaaatc ggnaaaatn 929

<210> 27
<211> 921
<212> DNA
<213> Rattus norvegicus

<400> 27
ncaagcgcga aattaaccct cactaaaggg aacaaaagct ggagctccac cgcggtggcg 60

gccgctctag aactagtgga tcccccgggc tgcaggagaa actatctcga gttttttttt 120

tttttttttt tttaattttg agactgggtc tctctatgtt gtccaggcta gtcttgaact 180

tctggattca agtcatctac ttgtgtcagc ctcttagctc ctaccaccac acttgacttt 240

gcttgtaact ttgaaaagtc cattcaaaat taagctctta agagactgaa tggaaaggca 300

attttgtctg aaggatattt cctatgtaag ggagaatagc atttgcagaa tataattctg 360

gtgctgctag ggaaaaaatc agtaggaagt tatagttccc agttggcttt aaccaactac 420

aaccttctct caatataaag tattcaagaa taaagagtat ggtatctact tatcagaaag 480

gcatgtttcc tattgggcaa agttagtgaa aaagtgactt tactcatttt gcatttacct 540

cggctgtata agcatttcct agcgcaggat gcttcttcca gaaatcaaga accaggtgaa 600

tacaggacta agaccttcct ggatgttctt cccacatcta gtatgttgac cccaacactg 660

aacttggcaa atcttaagtt gaccctggaa tactcaggct tccccnattt cccttcagct 720

gataacagaa tcntttggaa agctctcagc agatccgnan agttgcttac ccgataataa 780

atgcatatca aagcctttaa aggaaggaat ccnangccaa aggatccanc ccttnggnnt 840

tacnaaaggn tacctagggg ggattaangg aaaaaaggnt tggccccccc aaggtccttc 900

ccagntncng gggaggnaan a 921

<210> 28
<211> 925
<212> DNA
<213> Rattus norvegicus

<400> 28
ncaagcgcga aattaaccct cactaaaggg aacaaaagct ggagctccac cgcggtggcg 60

gccgctctag aactagtgga tcccccgggc tgcaggtggg aagatagtct taagaataac 120

cttttaatga aggagttggc aaatatttca agttgtgcct gctggttcca gggttcttaa 180

cctctctagt taagggctta gctttcttgg gacatcaact gtcttatttc tgaaaaagac 240

caaatgtaac tggtgtcacc agcagtgtgg gaatgaccaa gtatgacttt gtccctgtga 300

ttcaaaagat gtttgtcagg tagagttggg tgaatgccat tattgtgtgc atgggtatgt 360

atgggtggga tatggtctcc tggcagactg gaaataaatc agagcaattt aaaaaaaaaa 420

aaaaaaaact cgaggggggg cccggtaccc aattcgccct atagtgagtc gtattacaat 480

tcactggccg tcgttttaca acgtcgtgac tgggaaaacc ctggcgttac ccaacttaat 540

cgccttgcag cacatccccc tttcgccagc tggcgtaata gcgaagaggc ccgcaccgat 600

cgcccttccc aanagttgcg cacctggaat ggcgaatggc aaattgtaag cgttaaaatt 660

tgttaaaatc gngttaaatt ttgttaaatc agctcatttt taaccaatag gcgaaatcgg 720

gcaaaatccc ctataaatcc aaagnataga ccgngatagg ggtnagtgtt gttccagttt 780

gggacaagag tccccctatt taaagaaccg tgggctccca aaggtccaaa nggggggaaa 840

aaccggccta atccangggc gatgggccca otaacggggn acccatcaac ccnnaaanca 900

aggttttttt gggggcccaa ggtgc 925

<210> 29
<211> 918
<212> DNA
<213> Rattus norvegicus

<400> 29
ncaagcgngn aaattaaccc tcactaaagg gaacaaaagc tggagctcca ccgcggtggc 60

ggccgctcta gaactagtgg atcccccggg ctgcagggtt ctgatggtat aagcaaaaca 120

aataaaacat gtttctaaaa gttgtatctt gaaacactgg tgttcaacag ctagcagcta 180

aagtgattca caccatgcat tgttagtgtc acagactttg tggttatgtc taatagctgt 240

ttctgaagta ttttcgttta tcttttgtct aatttaaccc taagtgaatt ctctcctttt 300

tcttgaggac acacttatgc tcaaagtgtt gactctgccg tagtggcata aagagagtgt 360

accgtttgac agagatgcaa agttcagcag tggacctaac cagatgtcct gtggctggga 420

tctgtgctag cagtttggag cacgagctgt gtgcctgtga actggaatgc cacttgtccc 480

actccatcta cgccttgcag aatcagttcc acttgttaaa ggcaaaggct acttaccacc 540

ttaatgctat tttctgtaaa gaaattaaat tttactttta gcctttgca aacttttttt 600

ttccaagccg gtaatcagcc actccaaaac aactattctc agatattcat cattagacaa 660

ctgggagttt tttgcnggtt ttgtagccta ctaaaactgc ttaggctgtt gaacattcca 720

cattcaaagt tttgtagggt ggtgggataa tgggggaaac ttcaatgntt aatttaaaaa 780

taaataaaat aagttcctgg acttttaaaa aaaaaaaaaa aaaaccccga ggggggggcc 840

nggnacccaa ttcgncccaa aaggggggcc ggatnacaaa ttcccngggc cgccggtttt 900

aaaacggncg ggaccggg 918

<210> 30
<211> 918
<212> DNA
<213> Rattus norvegicus

<400> 30
ncaagntcga aattaaccct cactaaaggg aacaaaagct ggagctccac cgcggtggcg 60

gccgctctag aactagtgga tcccccgggc tgcaggtcga gttttttttt tttttttttt 120

ttttaaaagg tgagtcaaga tacacagctt taatacatat tagaaatatc caatgtgcca 180

ccaatacgat tcccctaaaa cacagcaagt gccagcgctt ggggccacac tcatctgtct 240

ttgtatcact agacatctga atgaccaacc atccattttt cccacatcct gccattcatt 300

aaggtatttt cagccagatt ttttagcaat atgctttttt tctttctttc aaatacaaca 360

agccacacag ggagttctac tatggaatgt ccaacaacaa cagggctgta tgggggccaa 420

gccttttctg gaaaaacatg gcggatctct aaaagattct ctgtcttccc tttatggagt 480

cagcagtgct ccacgttaat taagccactt caatttactg tatcagtttg gatattcgtt 540

ttaattgtgg gactagacac agaaactcac atttctggcc ttttcctctg catttctcaa 600

tatactatgg gttttttttt cccacaccgt aaatacagca tggattgaca ggtagaaact 660

cgtgtcaata gtctgtgggn tttatgccaa ctcagtggag tgatactata tattantncc 720

agntccctcn caaggcctan antaagatgn ngnaatagtt gcnatggtgg gtaaccttcc 780

tggcggttaa gagaagtgac ggcancctgn ccttagatca gaaggtaaaa acccccaatt 840

ggccaaggaa aaggccggcc caggngggac cggncaggnt naaaggaaan gccttaanna 900

aatgggaacc cccggnng 918

<210> 31
<211> 925
<212> DNA
<213> Rattus norvegicus

<400> 31
   gcaagcgtcg aaattaaccc tcactaaagg gaacaaaagc tggagctcca ccgcggtggc 60
   ggccgctcta gaactagtgg atcccccggg ctgcaggctt ccctgaccca cagttggacc 120
   gggcattgta gccagggtcc gtcgcacttt tcggtggtct gcacggactg agccaactcg 180
   gtgtggacga tctcctggct gtggctgtgt ggctagtcca cctttgcagt tccagtagtc 240
   agactggagt ctctttggaa gcagctctaa ggaatacacc aggatctcag ggtttatctg 300
   tgtagccctg gctatcctgg agctgtctct gtagaccagg ctgggctgag actgaggccc 360
   agttgcctct gcctcttgag tgctgggatt aaagagtctc aatgtcttcg ctgacgctcc 420
   tttggatgta cccccaaatc tctggaccac atcaccctgg gacccccgat gcggctgcct 480
   gagcaacctg ggagatggaa agcctgagaa tgagacaaag ggggaccaag aaacccccga 540
   aaggggagag gagccacgga gaagcccagc ccctgacttc cccacctggg aaaagatgcc 600
   gttccaccat gtaactgctg ggcttgttgt acaagggaat tacctcaacc gntcttctgt 660
   ctgcaggcag cgacagtgag cagttgggct aatatctctg tggaagaatc gatgataaga 720
   gtcaaaatcg ttcaaaggaa agctgggctt ggtggctgtc cactggaanc ccagtatcca 780
   ggggactaaa gaccaggagc tgatgccggn ttccantacc cagggggnaa ttgtcctttg 840
   gaaaaccaac gggtgaanaa tgtaagcccc gtggnaaaaa ntgcnggggc cttgtgctgc 900
   aaaaaagcnn gtttaatnaa anncc 925

<210> 32
<211> 921
<212> DNA
<213> Rattus norvegicus

<400> 32
tntgcaagcg cgaaattaaa cctcactaaa gggaacaaaa gctggagctc caccgcggtg 60

gcggccgctc tagaactagt ggatcccccg ggctgcaggt gggcctcgtg cgtttgggtg 120

tgtggtataa ctccttccgg gcctggaagg gaggcttctc tggaaacttt gaaggcgaag 180

gcttcatcct cggaggggtt tttgtgatag gatctggaaa gcagggcgtt cttcttgagc 240

accgagaaaa agagtttgga gacagagtga acctgctctc tgttctggaa gccgtaaaga 300

agatcaagcc acagacccca gcctccaggc aaagctgatc acctgctggc tggggggggg 360

ggaacggggg cctgtgcagt gttcaccaga tgagctgtgc tttcactgtg accccaagag 420

ctaggaggcc attgcaccat atttactggg aattggtgat gtattttaaa attgtctgtt 480

taggtcccag aatgtttaac attccgttta gacccaatag ggcaaatagg tcccagacag 540

aacagagtaa aatctaacaa atcagtgaga gttatttgag gaaagatcta gaaaatttaa 600

ggcctaaagt tgactgttaa gcctcccgtt cacaggaata tgtcctaagt gccagggatg 660

tgaagtagag gaagntttca tgcctaatta aaaagaaaac atctgaaatc tgagaaaagt 720

ggggactaag aaacaactac aactccagtg gtagagcatt tacctaacgt gcacatggnc 780

ctgggtagga taccccagac cagaccagac cattcacacc acctaagaga agctgatggg 840

ttgacttgat aattagggga atatcctaaa gccaattgtg ccggngttcc tnggacagtt 900

tggccaangg naaaattcca a 921

<210> 33
<211> 933
<212> DNA
<213> Rattus norvegicus

<400> 33

ncaagcgcga aattaaccct cactaaaggg aacaaaagct ggagctccac cgcggtggcg 60

gccgctctag aactagtgga tcccccgggc tgcaggcgtg gtcgcgctcg cgtgctccgt 120

tccctgcggc tgcccggacc cttggccatg tcctgaatgg gaaacagcac atcctcgttt 180

tgggggaagt cagccactac tcctgtgaac cagatccaag aaacaatttc taataattgt 240

gtggtgattt tctcaaaatc atcctgctca tactgttcaa tggccaagaa gattttccat 300

gacatgaatg tcaactataa agtcgtggag ttggatatgg tggaatatgg tagccagttt 360

caagaggctc tttacaagat ggactggaga aagaactgtt cccagggata tttgtgaatg 420

gaatatttat cggaggtggc gggccgacac tcacaggctt cacaaagaag ggaaattgct 480

ggcctctggt tcaccagtgg ctatttaaac aaaagcaaga ggaaagacgt cgaatgacat 540

ggctagtcgc cgtaccagta aacgttagtg cagtcataac ctttcacttg aggatgtttt 600

cagtgtgtgg gatgccctca taaagatgaa aataatgaac aataaattgc catggacccc 660

tcaaaaaaaa aaaaanaann nnnnnnnnnn nnnnnannnn nnnnnnnngg gnnnnnnnnn 720

nnnnnnnnnn nnnnnnnnnn nnnnnnnnnn nnnnnnnnnn nnnnnnnnnn nnnnnnnnnn 780

cncggggggg ggnnnnnnnn nnnnnnnccc nnnnnnnngg gnnnnnnnnn nnnnnnnnng 840

nnnngnnnnn nnnnnnnnnn nnnnnnnggg gnnccccnnn ggnnnnnnnn cnnnnnnnnn 900

nnnnnnnnnn nnnnnnnnnc nnnnnngggg ggg 933

<210> 34
<211> 945
<212> DNA
<213> Rattus norvegicus

<400> 34
agttatgcaa ggnngaaatt aacccgtcac taaagggaac aaaagctgga gctccaccgc 60

ggtggcggcc gctctagaac tagtggatcc cccgggctgc aggaattcgg cacgagggcg 120

gccagaagaa ggagagactt cggagcacaa tgccagcatg gactttgcag accttccagc 180

tctatttggg gccactctga gcgatgaggg actccagggg ttccttgtgg aggcccaccc 240

agaaaatgcc tgcagtccta ttgccccacc accctcagcc ccagtcaatg ggtcagtctt 300

tattgcactg cttcgaagat tcgactgcaa ctttgacctc aaggtcctaa atgctcagaa 360

agctgggtat ggtgcagctg tggtacacaa cgtgaattcc aatgaacttc taaacatggt 420

gtggaatagt gaggaaatcc aacaacagat ctgggatccc atctgtattt atcggagaga 480

gaagtgcaga gtacttacga gctctttttg tctacgagaa gggggctcgg gtgcttctgg 540

tcccagacaa tagcttcccc ttgggctatt acctcattcc tttcactggg gattgtagga 600

ctgctggttt tgggccatgg ggaacagtat tgatagttcg ttgcatccag caccggaaac 660

ggcttcaacg gaacagactt accaaagagc aactgaaaca gattcctact catgattatc 720

aaaaagggag atgagtatga tgtctgtgcc atctgtctgg atgagtatgg aggacgggga 780

caagctttcg ggatacttcc ctggtggcnc caaggcntta ccaacagtcg ctgtgtggga 840

nccccctgggg tcaattcaga acccggcaag aacctggccc caancnggna aaanaagcct 900

ggtccaaccg ggggggcct tgggggatn aagggaaaaa ggnan 945

<210> 35
<211> 975
<212> DNA
<213> Rattus norvegicus

<400> 35
gtcgttgttn ntngnanngg tnnnaatnaa cccncacgta aagggaacaa aagctggagc 60

tccaccgcgg tggcggccgc tctagaacta gtggatcccc cgggctgcag gctcctcttc 120

ttcctcctct tcctcctcct cttcctcctc ttcctcctct tcttcctcct cttcctcctc 180

ctcttcctct ctctgtgtgt gcgtgtgtgt gtgtgtgtgt gtgtgtgtgt gtgtgtgtgt 240

tattatctaa gtgaatgtgt atttaccatt tcttttatga aaacgaaccc cacactgttt 300

gtctccctgg taagtagtat ctccagttaa atgaggcccc tccctgcctc tgcttctgag 360

ttcagtctta gaggaccagg gatttaaaca ggttgcagtg gacacagtct tccctaccat 420

gttctccacc tcatccagtg tacacgcaca tagccgtcct ttctaaaaca ccaagaacct 480

tggaattgcg tgagtctccc ctagcttctc aataaacact gatttttttt tctccagaat 540

ctgaaaacta actacacaag gaaattattt tcaaatggct gctcagtttt ggtggcttgg 600

gctatataca ctgtcccaaa acctggctgg actttnaaaa ganacatata ctttaaatct 660

aaagcacttn cacacaagan atccagagat tcacaatcaa aaggggacac tgatgtggga 720

attctccaaa atactcaaaa aggcatgaat ttgtnttgaa tttggtttct gggagaattc 780

tttctttcct tcataataaa aatagctccn atngaagggc tggaataagn aaacggaaca 840

atggcaaagg cctaagtnca aagggggggg ggnccggggn acccnaaant tcggccccta 900

anaaggtgga agccgggaan nnancaattc caactggggc cgggccgggt tntaaanaan 960

ggccggtgga acntg 975

<210> 36
<210> 1036
<211> <212> DNA
<213> Rattus norvegicus

<400> 36
cgcacattaa ccctcactaa agggaacaaa agctggagct ccaccgcggt ggcggccgct 60

ctagaactag tggatccccc gggctgcagg ctggagagat ggatcagcag ttaagaacac 120

taactgttct tccagaggtc ttgagttcaa ttcccagcaa ccacatggtg gctcacaacc 180

atctgcagtg ggatccgatg ccctcttctg gtacacacaa ctacagtgta ctcatacata 240

atanataaac ctttaaaaaa agatacataa ctgcaagtaa ttaanaaaaa aaaaaaaaaa 300

ctcgaggggg ggcccggtac ccaattcgcc ctatagtgag tcgtattaca attcactggg 360

ccgtcgtttt anaangtcgt gactgggaaa accctggcgg ttacccaact taatcgcctt 420

ggcaggcana tccccctttc gccagctggg gtaatagcga agagggcccg gcaccggatc 480

ggcccttccc aanagttgcg gcagcctgga atggcgaatg ggaaattgta agngttaata 540

ttttgttaaa attcgggnta aanttttgtt aaatcagcnn atttttnaac cnntaggngg 600

naaangggca aaannncnta aaaatnaang gntttgcanc gagtcanggt tnngccatnt 660

nncagattgg gcnaaaaagn ccnccaccga tagnccntgg caacaantgc gnaccgggaa 720

tggcgaatgg caaatngtaa gcgntaanaa tttggttaaa aattcgcgtn aaaattttgt 780

taaaatccag ccccantttt taaaccaata gggccgggga atccggnaaa anggccccnn 840

nngnaattca aaaagantaa anccggnnaa aaggggttta aatngnnggt ncccantttt 900

gggaacaaan agnncccccn natttaaagn aacnnggggg cccccaacgg nccaaaaggg 960

gggaaaaaac ccggncnaan taaggggngn annggccccc ctaaanggng aacccatnnn 1020

cccccnaanc aaaggg 1036

<210> 37
<211> 1023
<212> DNA
<213> Rattus norvegicus

<400> 37
ncaagcggga aattaaccct cacgtaaagg gaacaaaagc tggagctcca ccgcggtggc 60

ggccgctcta gaactagtgg atcccccggg ctgcaggttc tatacattgc ctacagtgat 120

gaaagcgtct acggtctgtg aagttgctgt cccggaggtg ggggttccat tctacaaaga 180

gaggtggcgc tccttccttg gcatccagtt cctccttcag gctcaaacac catctccttt 240

cttcaggacc tgcacttaat gtttgaggct gtctctccag tccctctgag caggaggggt 300

aatggtagat gtacagcggg gggggcccgg tacccaattc gccctatagt gagtcgtatt 360

acaattcact gggccgtcgt tttacaacgt cgtgactggg gaaaaccctg gcgttaccca 420

acttaatcgc cttgcagcac atcccccttt cgccagctgg cgttaatagc gaagagggcc 480

cgcaccgatc gcccttccca acagttgcgc acctggaatg gcgaatgggc aaatgtaagc 540

gttaatattt tgttaaaatc gcgttaaatt ttgttaaatc agctcatttt ttaaccaata 600

ggccgaaatc ggcaaaatcc cttataaaat caaaagnata gaccgagata gggttgagtg 660

ttgttccagt ttggaacaag agtccactat taaagaacgt ggatccaacg tcaaagggc 720

gaaaaaccgt cnannagggg ggatggccca cnaacgtgaa accaattntc cctggnggga 780

agangttttg gggnaaggaa gtaaactggg ggaanccctt aaaagggggg gaccccgaan 840

tttggaggcc ttcnaggggg ggaaaagccg ggngnaaagg tgggggnnga aaagggaagg 900

gggaggaaaa gggaaaaggg aangggggg gtaaggggg tttgggaaaa tttaangggg 960

taaanttggg ggggaaaann aanaaaaaac nggggggggg tttaaatngg ggggtaaaag 1020

ggg 1023

<210> 38
<211> 979
<212> DNA
<213> Rattus norvegicus

<400> 38
aagcgcgaaa ttaaccctca ctaaagggaa caaaagctgg agctccaccg cggtggcggc 60

cgctctagaa ctagtggatc ccccgggctg cagggagtaa tggtggagca gaatggatgc 120

cacttgtcac tgtgctcgat gacaagttgc agcccataaa aaggttgact tgcttgcaaa 180

cacattgtgt tcgttggcat tttctcagct tctcctcact acctctgggt ggagatgggc 240

accttctgtg ggcctgggct gggggccacc cctgctatgc aatggagagg caaaggcaga 300

ggtccaggaa taaggaggct tctaccaatg atttgtttta atggtgcttg acagagatat 360

tgtatggttc tctggagagc tcccctggaa aaccttacct ccaaccacac aagggcttcc 420

tcccagagag cgctcgctgg gcagcaaggg acacactccc atacttgcca agcatatcaa 480

gtacccaaag attggcagaa aagatcctgg cctgaccacc cagccacatc cttcagggct 540

ccaccggatt gactgtgtgt ctgagatgga gagggctttg tgacatttaa gtgcctttca 600

gaaatgcctt atacggtgag aagccaaagg tttatgtcag catggcagag ctcctgagac 660

cgaagccttc ctggagcctt tcgttactgg cagcgttctt tccgaagcca ccggggtnca 720

ttccacagat cgtattaagg aggagctcna caaaanctcg tggggcnagt tttcagcaag 780

ggcgatagnn gntgcttgca accatgantc cnagcaactg gccnnnngaa nnagtnggaa 840

anaaannanc ccggnagcan tcnagggggt ntaagnanag gggncaancc anggnnnngn 900

antgggaant tgggatgcga tngnaaantn ccggnnaaan ccgggttgaa ancgganagt 960

tgaaaaangg gtcgggatt 979

<210> 39
<211> 1112
<212> DNA
<213> Rattus norvegicus

<400> 39
aaacggcant anccctnact aaagggnacn aaagctggag ctccaccgcg gtggcggccg 60

ctctagaact agtggatccc ccgggctgca ggttgaatat taactcgtgc cagcaggtga 120

aacaaaaaga aaccttctgt cgtcgtagaa gaatatttcg cccaggctgt gcgacgacat 180

tcacagcatt tcaaaccaga ccatctctgt aaatagctga gtgcctaata aaccattatt 240

ttggtaaaaa aaaaaaaaaa aaaaaannaa aaaaaacncg nggggggccc cggtacccaa 300

ttngccctat agtgagtcgt attanaattc nctggccgtc gttttanaac gtacgtgnac 360

tgggnaaacc ctggggttac ccannttaat gcgccttngg gtnanntccc cctttgcgcc 420

agctggngtg aataagcgaa gaggccngga ncattggccn ttncccanaa aattgngcnn 480

nnnnatnggn aaanggnaaa ttnngngggg taanaatttg ngtnaanagn ngcgcgtnaa 540

annttnaggn gaaangcggn gcanttntna gcnaaaaggg ccaaaaaggg gannaaancg 600

ccngangatg agaanaggat aggacgngnn gaanngnnag ggatgaggga ganaatnnng 660

naanaanggg nacngnnagg aagaaaggnn aggngnaagt gganaganng acaaagtnga 720

gagaagnana gngggagang agggacggag agggaanaan ngagaganng nggagntann 780

cgggaggtnn angnggntnn ggagagnaga gngngnanag gnngaggaga ngagagggng 840

ganggaagag acgaaagngg gaggnnnann nnggggatgg ggagngnnng gancagngna 900

ggggangaca ggtnntggan tgggggnaga atngagantg tgnagngagg gntnnatata 960

gagaggtgna gagaantggg gganagntgn gacnnngaga taaggagaag ganacngacg 1020

agangggngaa gnaggnagag tantgangaa agaaanacga gagaagagag tnannancnt 1080

agatanacga ggngaagnnn agnnacgngg tc 1112

<210> 40
<211> 1026
<212> DNA
<213> Rattus norvegicus

<400> 40
aagcgggaaa ttaaccctca ctaaagggaa caaaagctgg agctccaccg cggtggcggc 60

cgctctagaa ctagtggatc ccccgggctg caggggacga gatgctcagc atggtgagtg 120

aaggggaggg aaaacccatg agagagtgag atggtcagag aatgggagct gattgtgaca 180

tggaactgca gagagaagca cagacttgaa acatcgctaa gatgtgtgca tacaaaaatg 240

aagcaagtta tgctaagtac acacagtgtc cagcacattt tattttcact tttggttttg 300

aagacaaagt ctcattatgc agaccaggtt gactttgaat tcagatctgc ctgtctctgc 360

ctctggagta ttaggatgaa aggtgttatg tcaccatgcc cagcctctta gtatatttca 420

gaacagtaaa tactgcatga aaggtcattg taaattcccc tcttaattat tgcttcaatc 480

aagttggaaa tgctttcatg tattaaagac aatgttttta atggcaagaa aaaagtaatg 540

ttttattttt atagtttata agccatgcat tacnattttt atgtaaaaaa aagnactaat 600

gtagaatttn ggccgaatat aaaagtggng ttgtgatana attaaaaaat tagggggcng 660

gggttnagcc caatgggana gcgcntgncc gaaggaagnc acaaggncnc ggggtanggg 720

nnccccagnc nccggaaaaa aaagacccnn ganaanaaga nangaaaaaa cnccnagggg 780

gggggncccg ggtnanccna aantcggccc cnaanngggg aagnccnaaa gnannaantt 840

cncnngggnc ggnngggttt aacaaanggc ggngggcnng ggggaaaaac cccgggggga 900

nnnncccagc gnganttngg cnnggngggg ggnaancccc ccnnnnnnng ccnggngggg 960

nggnnnatng gngnnnggaa nccnngcgnn nngaaagnng ggannnncna anngaanngg 1020

gggncg 1026

<210> 41
<211> 1044
<212> DNA
<213> Rattus norvegicus

<400> 41
aagcnngaaa ttaaccctca ctaaagggaa caaaagctgg agctccaccg cggtggcggc 60

cgctctagaa ctagtggatc ccccgggctg caggcgagtt tttttttttt tttttttttt 120

tttttttttt tttttgattt ttatggaaat tttaattggc aaatttaaaa aaataagttt 180

gtaaccatta ttttatatag aaatattcaa ctttcccaag atttctcaca aacagnggta 240

caaaagttgg ctctaaattc atccaaggta ttttaagaac taaatggnct tgcacttgat 300

tgactccagt ctcagtgatg ctgggaagga agcctaggac cttgcacatg cncagtaaga 360

gctttaatgc caagccacag gcccattccn cagttgacnc cttatcaata atcttcatct 420

tgggagtttt cnccaagaat caattcacag ggntgttcag tctttctcta cctcaaccct 480

acccagtgng nctaaatcan cagtttagtc catttcggga aacaaaccac ttgtcaaacg 540

nggaaatgaa atgaagagat cttagtagtc aggnattntg gtaccanccc aactgggggg 600

gncaatagta gaaatggctg taaacaaaag ngaatctaan cnaagggggg ggcncggtnn 660

ccaaanncgn cccaaaangn ggagngcgga aacaaaaaat cngccgggng gnccgttata 720

ananangttg ggggannggng gnaaaaaccc tgnggtgttn gcngaaantn attcggccgg 780

tgggggggan aaaaccacnn ccttgggganc ngggggggaa aaaaagagaa aaaagncccn 840

ganggggggg gcccggttan cccaaattcg gccncnaatn ggagnaggnc ggaaatgnga 900

aattcccntg ggccggncgg ttttnanaan ggnccgggga nctgggggga aaaancccng 960

gggggntaac cccaaccctt aaancggccc ttngnggnga naatnccccc cttttggnca 1020

aggcggggng gnaaaaaagc ggag 1044

<210> 42

<211> 997
<212> DNA
<213> Rattus norvegicus

<400> 42
aagcgngaaa ttaaccctca cgtaaaggga acaaaagctg gagctccacc gcggtggcgg 60

ccgctctaga actagtggat cccccgggct gcaggcttct tttagtgcca gctcagtggc 120

tttatcgctc aagagagaca gccggtaaca gataggctgc ccctctgctc acttttctgt 180

ttcacagaca caaggtgttt ttgtcccaag aaagcctcct ggcttagctg tgtgactaaa 240

tgctatttgc cctcttcagt ggacctctat tctcgagggg gggcccggta cccaattcgc 300

cctatagtga gtcgtattac aattcactgg ccgtcgtttt acaacgtcgt gactgggaaa 360

accctggcgt tacccaactt aatcgccttg cagcacatcc ccctttcgcc agctggcgta 420

atagcgaaga ggcccgcacc gatcgccctt cccaacagtt gcgcacctga atggcgaatg 480

gcaaattgta agcgttaata ttttgttaaa attcgcgtta aatttttgtt aaatcagctc 540

atttttaac caacaggccg aaatcggcaa aatcccttat aaatcaaaag aatagaccga 600

gatagggttg agtgttgttc cagtttggga acaagagtcc actattaaag aacgtgggac 660

tccaacgtca aaggggcgaa aaaccgtcta tcagggcgga tgggcccact acgtgaanca 720

tcaccctaat ccaagttttt ttggggncga ggtggccgtn aaaagcnact aaaatcggga 780

acccctaaaa gggagccccc cggtttagaa gctttnaagg ggggaaaanc cngggggaac 840

gtgggccnag aaaaggnagg ggnggnaaan cggaaagggg ncggncgctn aggggannag 900

gccaagggnn aannggntng ngntgngggg nannccnnnn nnnannccnn nngggggnga 960

aaanncgggg gnaaaaacgg gngnnnnaag gnnnggg 997

<210> 43
<211> 1019
<212> DNA
<213> Rattus norvegicus

<400> 43
aagcnggaaa ttaaccctca ctaaagggaa caaaagctgg agctccaccg cggtggcggc 60

cgctctagaa ctagtggatc ccccgggctg caggcgaact ccagcacttc tgctcttgtt 120

tttgttttgt tttctgcgta aacctctggc ccactctcaa aaggcaagat gtccagtcat 180

gtcccggcgg atatgattaa tttgcgcctc atcttggtga gtggaaagac gaaagagttc 240

ctcttctccc caaacgactc tgcctctgac atcgcaaagc acgtgtatga caactggccc 300

atggactggg aagaagagca ggtcagcagc ccgaacattc ttcgactcat ttatcaaggc 360

agatttctac acgggaaacg tgcaccctag ggagcattaa aacttccttt tggcaaaaca 420

acagtgatgg catttggtgg ccagagagac cctggccaga gcccaattca caaggtcaga 480

gaaaccggga gaaaactggt gagagcaact gctgtgtgat cctgtaacat cgtcgccagc 540

gcagtgtggc agtctgttac cactgcgggg acagaggaga ctcggcagct tccgganacc 600

tgtgggacag tcgcccgcac atcnaggact gaaccactnc atgagctctg tgatctctcc 660

tcacaaagtt aaaaggaacc aaggaacatt tcncagttct ggtcctttan tccngtnnct 720

cttgtctggt gtttgagcca ntctgnaaat ggcacagggg gtcttcnaag ggggnaaatt 780

agcgaagtct tctnaagggg gggtttctgn aagggggggg ggcccgggta anccaaattt 840

nggccctaan aaggnggngn nggnaattna caannttcaa ctggggccgg nggtttttaa 900

aaanggtcgn tggnnnnggg gaaaaacctt gggggggnan cncaaanttn naanngngnt 960

ttnnggggna anncnccntt ntggaaagng ggggggaaat ttgggnaana angggggggg 1019

<210> 44
<211> 952
<212> DNA
<213> Rattus norvegicus

<400> 44
agctngaaat taaccctcac taaagggaac aaaagctgga gctccaccgc ggtggcggcc 60

gctctagaac tagtggatcc cccgggctgc aggcgagttt tttttgtggt ttggtattta 120

tttgaaccac gtgatctcgt gtagtttggg ctagcctcaa tttaaactta aactcctaac 180

cttccttcct ccactctgag tgaggggctt gggggatata ccaggctcta attcttttta 240

ctttactttt ttagatgtac ttacgtcact ttatgtgtat gaacgttttg cctacatgca 300

tgtatgttca ctgtgtctgt aggctcctcc taggattaca gacagttgtg agccaccatg 360

tggtgtctgg ggaatggagt ctgggttctc ttcaagagca acagtgttct cggcccctgg 420

aagtcaggtt ctaatacctg ttaggtaagc agtgttgggc tgatcagatg caaagtgatt 480

tagcccctat cataacagac tgtcagtctc ggcctccagg cactccacca cctgctactc 540

cagttgaagt gtcctgccag gtgaccttgg ctgggctatc ggatcatgtg aaatacagac 600

cctgctcaaa ggaacaagct tgcgggntgg agagaggctc ancggttaag agcacctgac 660

tgctctccag aggtccgagt caatcccagn aaccacaggt ggctcacaan canctntaaa 720

gagatccgaa gccnncttct gggggaacng aaganancta cagngnacta nannnaanaa 780

aaggngaann aaacntnann aaaaanaann nnnnnnnnnn nnnnnnnnnn nnggnnnnnn 840

nnnnnnngnn nnnnngnnnn nnannnnnnn nnnnnggggg gggggggnng nnnnnnnnnn 900

nnnnnnnnnn nngnnnnnnn nngnaaaann nnnnggnnnn nngggnaaaa tg 952

<210> 45
<211> 993
<212> DNA
<213> Rattus norvegicus

<400> 45

aagcgcgaaa ttanccctca cgtaaaggga acaaaagctg gagctccacc gcggtggcgg 60

ccgctctaga actagtggat cccccgggct gcaggcaaac tggatgaaac tttgttctaa 120

ggggaatttc atttaaaagt ttacctttac cagagcagga ggcgtagagt cagctctggg 180

gaaggagtgg gtaacttcac gacactctca ttctccgcac ttactgctcc acctgagtag 240

ctgtaaagga acttgggctg ggatggggtg gcaggcagtg tctctccttc atgggcctat 300

ggctgaatca aacaatcctt ccatagcaca tgcttaaccc tggactcact cttaagtccc 360

ttctttccca ttctgctaca aagtcaggct ccctaataac atgtaactgg agctgccttg 420

tcaacagaga aagaagaaag ctaacgaata cccatgatcc tattcttcac cgtccatgtc 480

tcgatgctcc atctccttcc tggatcctct tgttgctttc tagaattttc accaactatc 540

actcgantta ntagtccaat ctgtcttgaa agaaaaataa agttgaacaa agcaacaaaa 600

nannaanaan naaaaaaaaa ctcggagggg gggcccggna ccnaattggg nctannagng 660

ngggggnaat aacaatgang gggggtngtt tnnanaangn nggggntggg gaaaacnctg 720

gggtngancn naattaatgg gnctngnagg naaagggccn ntttnggggg agggggggga 780

nagaagggna gggggnccgg nannggnggg ggcnnnngnn agnnntgggg gaggnnggan 840

tgggngaagg ggngnaagng nganngnggn naagnattnn gngaaaaaan ngggggnnaa 900

aatnnnnggn aaannggggg ggnantgggn taangnanng gggngnanan nggggagaaa 960

angggngnnn ggaagnnnaa annggnggnc ggg 993

<210> 46
<211> 1033
<212> DNA
<213> Rattus norvegicus

<400> 46
aagcgngaaa ttaaccctca cgtaaaggga acaaaagctg gagctccacc gcggtggcgg 60

ccgctctaga actagtggat cccccgggct gcaggcaagt ttagtcctag tgcccacatn 120

aagtggttca cagctgccct gtaaccccag ctccagaaga tccaagaccc cctctagcct 180

ctgagcacat agccccatgc atacacacat cacatacatg atttaaaatt aagtaagctt 240

tttaggcctt atatttaatt cacctattaa atgcttagac accttcaaga aatttggcaa 300

gtttgaagta ataagggaag gaaatgagta ttggttgagt aaaacagcct caagacagac 360

acctgggtca aatgtatgtg gcagcagcat gccaaggccc tagctccagn ttactggtga 420

gaaactggag cttgagagag accacataac ctgggagtga gtcataatga aaaccaagtg 480

gcagacctgt ttcaaaagta taacctcagg ggttggggat ttagctcagt ggtagagcan 540

ttgcctagca agcacaaggc cctgggttcg gtccccagct ccgaaaaaaa anaaaaaaaa 600

aactcgaggg ggggccnggt nacccaattn ggncctanng tgngncgtat tanattnant 660

gggccgggcg ttttaanaan gtcgngacng gggaaaaccn ngggggttng ccnnanntan 720

angggngtgg gaagcgagat ncggccntgt gggggagntg gggnnngata ggggnngngg 780

gnncnngnnn nnggantcgg gccnntgnnn nanggagnng gggnnggnng gaantggggn 840

ananantggg ananttngga nngngngtna ggnnnnngng gnggaangag ttgnggggtt 900

gaagntntgn ggggaggaan nnggngnggg anttntggaa ancggaggaa ggnggngaaa 960

nggggngagn anngcnggng aagangngaa naaggnnngg gncgggggan ngaggggnnn 1020

ggnngttttn ttg 1033

<210> 47
<211> 1005
<212> DNA
<213> Rattus norvegicus

<400> 47
aagcgnggaa attanccctc actaaaggga acaaaagctg gagctccacc gcggtggcgg 60

ccgctctaga actagtggat cccccgggct gcaggaggct gccgtttntg agtttnagtc 120

cttgagaggc tgggaaggca cagagctctg gctctgcact gttcttactg agtgactagg 180

tgtgagccct ttacattaga gggaacctgg tttgagctca cttgtacttt gtgtggcgtt 240

agtgttccat tactggcccc tctaagtaat ggtcttcaca gtgcacagca agttcccagt 300

gtgtagaaag ccatacacca ggatgtgggt caaccatgaa gatgtggcat tgcagacagg 360

ggaacatgtg gatgcatggn tatcaccttg agcagcccct gcagttgctt gtgttaacac 420

aaaagtgttt agcattctgc cgnttttata tttatgtaat aactctttaa agccattcag 480

atggataact atttaatttc ttaaagacag ttgtaaaggt ctctctctga ggacaatgac 540

ttggtaaaac tgggggcaca gccagtccca gacactggtc gtggntacag tgggnttttt 600

gggctcaggn tcaacacgca tcagagtagg actggggnca acangtggtg ggngtgtgca 660

aacaggnngg cnctnganca gcccaggncc tttggagagc acgtnctctg gcaccaaggn 720

ccctcngntn tgggaagggg gaaaactttc acaagggaaa tgggngncaa gcttttannc 780

cncngaaggn cntgggnggg gggcangggc aagngggggc gggnggggga cnnntgnttt 840

ggggggnann ttntgggggn cngaggggnn naaanccgcn ccctgnaggn nggcggaggn 900

gggtgnnann naccngttgg agnaagagcg ngggggntna agggnggtgg naaggatgtg 960

ggncggaacg ttgngggaag tnggaagagn nagggnganc cgcgg 1005

<210> 48
<211> 975
<212> DNA

<213> Rattus norvegicus

<400> 48

aagngcgaaa ttaaccctca ctaaagggaa caaaagctgg nagctccacc gcggtggcgg 60

ccgctctaga actagtggat cccccgggct gcaggtcat acttaggaat ttctcctact 120

ctacactctc tgtacaaaaa taaagcaaaa caacaacaac aacaacaaca acaacaacaa 180

ccataccaga acaagaacaa gaacaacaat ggtttacatg aacacagctg ctgaagaggc 240

gagagacaga atgataatcc agtaagcaca cgtttattca cgggtgtcag ctttgctttc 300

cctgaaggct cttggtgaca gtgtgtgtgt gtgtgtgtgt gtgtgtgtgt gtgtgtgtgt 360

ggtgtgtact tgtttggaga agtacatgtg tacacatgtg aggacctggg ggcacctgga 420

ccagaacgaa caagggcgaa cccctttcaa atgggcagca tttccatgga agacacactt 480

aaaacctaca acttcaaaat gttcatattc tatacaaaag aaaaatagat aaatataaac 540

attttgaagt tgtagcattt ccatgaagac acacttaaaa cctacagggg gggcccggta 600

cccaattcgc cctatagtga gtcgtattac aatcactggg ccgtcgtttt acaacgtcgt 660

gactggggaa aaccctgggc gttacccaac ttaatcggcc ttgcagcaca tccccctttt 720

ggccantggn gnaatagcgg agangcccgc accgattggc ccttcccaac anttggcggc 780

nnctgaaatg ggcggaatgg gccaaatttg ttaaggcggn naaaaatttt ggttaaaaaa 840

ttngcgggtn aaaatttttg ggaaaaacca gcccnatttt ttnaanccaa taggggggga 900

aattngggaa aaaacccccc tnataaannc naaanggaat naggccccgg ngaaangggg 960

ttgnaattgt tgttc 975

<210> 49
<211> 949
<212> DNA
<213> Rattus norvegicus

<400> 49
aagctcgaaa ttaaccctca ctaaagggna acaaaagctg gagctccacc gcggtggcgg 60

ccgctctaga actagtggat cccccgggct gcaggattag acttaccgct accaaacaat 120

tctttactta gattataggt gccctcctcc cattgttagc atgggggata ttaggataat 180

atcactttaa gataacacat gaggggttgg ggatttggct cagtggtgga gcgcttgcct 240

ggggagcgca aggccctggg ttcgatcccc agctccgaaa aaaaaagaa ccaaaaaaaa 300

aaaaaaaaa ctcgaggggg ggcccggtac ccaattcgcc ctatagtgag tcgtattaca 360

attcactggc cgtcgtttta caacgtcgtg actggggaaa accctggcgt tacccaactt 420

aatcgccttg gcagcacatc ccccttttcgc cagctggcgt taatagcgaa gaggcccgca 480

ccgatcgccc ttcccaanag ttgcgcacct gnaatggcga atggcaaatt gtaagcgtta 540

atatttgtt aaaattcgcg ttaaattttt gttaaatcag ctcatttttt aaccaatagg 600

ccgaaatcgg caaaatccct tataaatcaa aagaatagac cgagataggg ttgagtgttg 660

ttccatttgg nacaagagtc cactattaaa gaangtggac tccaacgtca aagggcgaaa 720

aaccgtctat cagggcgat ggcccactac gtgaaccatc accctaatca agttttttgg 780

gggtcgaggt ggccgtaaag cnctaaatcg ggaaccctaa agggggnccc ccgatttaga 840

gccttnangg gggnaanccc gggggaaacg tgggcggaga aaaggaaggg gaagaaaacc 900

gnaaaggnan cnggggcgct aaaggggnct gggaaaattg tancgggnn 949

<210> 50
<211> 958
<212> DNA
<213> Rattus norvegicus

<400> 50
aagntcgaaa ttaaccctca ctaaagggaa caaaagctgg agctccaccg cggtggcggc 60

cgctctagaa ctagtggatc ccccgggctg caggaattcg gcacgagatc atggctgcag 120

tcagatctcc gttgtctctg tggaggttcc agttgagcac tcggcgagca cggcgggtct 180

gtactcgggt cgcagcccag cgccactccg atgctctgct cgcgacgtgg tcccagccct 240

ttgaagtggg gcagcctcgc cgccctttca gctccgaggc agaatctggt agctcaaaag 300

tcaagaaacc tacttttatg gatgaggagg tccagagcat cctcaccaag atgacaggcc 360

tggacttggc agaagacttt caagcctggc tgtacaacca ctggaagcca ccaacctaca 420

agttaatgac ccaggcacag ctggagggag gctacgagac tgggcagttg aggcagctaa 480

agtacgatta aagatgccac cagttctggg aagaacgaaa gccaataaat gatgtgttag 540

ccgaggataa gatcttggaa ggaacagaaa caaacaaata tgtgtttact gacatatcgt 600

ataacatacc acaccgggaa cgttttattg ttgttagaga accaagtggg cacactacgc 660

aaagctttca tgggaaagaa cggggacang gtgatacaaa tttatttccc gaaagaaggt 720

cgtagagttt tgccaccagt aatttttcaa agntgagaac cttaagacca tgtacagcca 780

agaccgggca tgctgatgtn cctcnaatct ctgtgttgcc cagtttttga gccagattcc 840

antggggtat anccaagggg tnntcaccca gacccnnngg aggntttnng nccggncntg 900

ggnaaanang gggtnttac ggggccaana anggcanctt ttgggggga atggggtg 958

<210> 51
<211> 979
<212> DNA
<213> Rattus norvegicus

<400> 51
   gcaagntcga aatnaaccct cactaaaggg aacaaaagct ggagctccac cgaggtggcg 60
   gccgctctag aactagtgga tcccccgggc tgcaggagat caaacactcc tggttttgat 120
   ctgtgagctc attatcacat gttagggaag aancaaactg tgataatgag ctcacagatc 180
   aaaaccagga gtgtttgatg tttgcactag gagctcctga acaaataaag tttagcaatt 240
   gcagcataaa aaaaaaaaaa aaaaactcga gggggggccc ggtacccaat tcgccctata 300
   gtgagtcgta ttacaattca ctggccgtcg ttttacaacg tcgtgactgg gaaaaccctg 360
   gcgttaccca acttaatcgc cttgcagcac atcccccttt cgccagctgg cgttaatagc 420
   gaagaggccc gcaccgatcg cccttcccaa cagttgcgca nctgaaatgg cggaatggca 480
   aattgtaagn gttaatattt tgttaaaatt cgcgttaaat ttttgttaaa tcagctcatt 540
   ttttanccaa taggccgaaa tcggcaaaat cccttataaa tnaaaagnnt agaccgngat 600
   agggttgatg ttgtttccag tttggaacaa agagtccact attaaagaac gtgggactcc 660
   aacgtcaaag gggcnnaaaa accgtntnat caggcgatgg ccccactacg tgaaaccgtc 720
   accctaancc aagttttttg ggggtcgaag ggtgnccggn aaaagcactt aaatcgggga 780
   aaccctaaaa gggggaggcc cccggatttt tagagcttgg acggggggga aagnccgggn 840
   ggaacgttgg gnggaaaaaa gggnaagggn anaaanccng nnaaaggnag gggggnctnn 900
   aggggcgngg gaanagnagg gggggnnngg gggggggnga gnagcgagna aagacncggg 960
   gggnanngan agggggggg 979

<210> 52
<211> 951
<212> DNA
<213> Rattus norvegicus

<400> 52
aaggtcgaaa ttaaccctca ctaaagggaa caaaagctgg agctccaccg cggtggcggc 60

cgctctagaa ctagtggatc ccccgggctg caggcacata tctaagttgc ccaaagcacc 120

ttagaagcag aggctacaca gcttttctct gctatccatt ttccttaccc ttcctacacc 180

acctctacag ccaaagaagg gggaggtggg tgcttgtagc cccagcccca cttagcactg 240

atgtcctacc cctccccagc actgagcagg caagtgctcc aagacctctt cctagggaca 300

gccagcctgg ctggcacatt tccccaacaa atgctccctg gccacacggg gcagctctca 360

ccacctccgg gctggccaaa cagcagtctg cgagtcagta agtagtccga ggctagcagt 420

ctcccagcca gctctcccgg gatgctcctg ccagcacagg gttcagcagg gcatgcatgc 480

cccaggcaga gagaatgagc catgctgccc tttcctgctc agggnccctt gtcctttggg 540

ttaagtgtaa gacgggggtg gtgaaggctc cacattgtca gtgctcagga atgtgaactg 600

ggagaacgct gaagccataa tccccaacta tttcccttgg ctggatgccc aagtaatcag 660

ctgggccaat ctacagccag actccagccc tgctgcttca aatgtgggaa gtttagagaa 720

gaggccatga agaatctgaa tggattgcac agttactcct gtgggttcat cttaactggg 780

aaanantttg ttctgtagat ataataaata ttaacctagn attgggaaaa aaaaaaaana 840

aaaaaaaccc nngggggggg gccnggnanc cnaatttggc ccnaaaaagg ggggnnggnn 900

ttaaaattcn atngggnggg ggttttaaaa aggnngggaa tnggggaaaa c 951

<210> 53
<211> 962
<212> DNA
<213> Rattus norvegicus

<400> 53
gcaagntcga aattaaccct cactaaaggg aacaaaagct ggagctccac cgcggtggcg 60

gccgctctag aactagtgga tcccccgggc tgcaggaatt cggcacgaga ttatactata 120

aaggttttca gtggatcaaa atgttctact atggaatata tggggagcca gccttaatca 180

taaataggag ttaactgagg gtggacaaga gcaggctatt cttacaaatg ttctgcataa 240

aatgatgcat tatataatta agaaaagggt atttcatttt tcttatgtgt atgggtgttt 300

tgtatgcatg tgtgtctctg tactgtgtgt gtgcagcacc ctctgagtta agagaaggga 360

atttggaact agggatgcag atggttgtgn agatgctatg tgggtgtagg gaatagaagt 420

caggccctct agaagagcaa ccagtgttct taactgctga gccatcgccc tatcccaata 480

tttataattt taattttttt ggaaacaccg cctcagttat cctaggctgg ccttggaata 540

tgttctgtag ctgagcatgg ccttggaact tctcatcctc ctaactccag cctccctgcc 600

ggattacagg tgagtgctat catgctcagt ttatggcatg ttgacattta aggccctggt 660

tcactaattt tatgcaaaga tctacatctc tagccccata catatattat ttaagggggt 720

tanttttata atgggatata nggtanatgg gccttagcat tccnatcaaa aaataaaatg 780

ggatttanga aaaataggaa tataggcagg aaacntncnt ttttggnttg gccngaaggg 840

atggaaatnc atgggncctg gnaaccanac ntaagggcaa accattaagg ncacctgagg 900

ntaanggagc ccccaggtnc ccaaaggaan ttttggggga ccnggaggcc ctaaccggga 960

ag 962

<210> 54
<211> 991
<212> DNA
<213> Rattus norvegicus

<400> 54
   ncaaggtcga aatanccctc actaaaggga acaaaagctg gagctccacc gcggtggcgg 60
   ccgctctaga actagtggat cccccgggct gcaggcaaag tggaattcaa gttatgtcta 120
   ttatagatgc taaactgaag cacctactga tctctaccta catcagatta atgctctgtg 180
   tcccaaatgt tcgtcagtgt tgtgtgacgg tgtttagaaa ttggcctatc atatcagtac 240
   cttcaggcat gtgatataaa accgctatgt gatgttactt atggtattta atgaactgct 300
   cactctacct tttatacgtg aaactagttc atcagcgtgg tacaaaattt aatattttat 360
   caaaactatc attctggcca aatattgtta aactaatttt aaagggcgga atgcattagc 420
   atttactgca ggtgagcaaa aaaaatttat tttggctttt ctgggaaatc aaaaggtcat 480
   gctgtcttgc cagccgtgag taccccaaat gtcaatataa ttaatagata attganataa 540
   aaatttcgtc aactgggcat ctgtaattca gctccatata caacttcgtc ctttccaacc 600
   ctggtgtaca gggttgtgcc ccttcanant tggghtgtac gttccaccat atagttaggt 660
   ttgtattnac ctaaaacaaa cttngttana gctgggtgga aancagccca tcggaactag 720
   ccccatccaa tggtcacggt attttagatt ccttaatcna acgnncaaaa cnagnggtca 780
   gttccacaaa ncttangngg aanaaaatng ggccaaggga aaagacnatt gaagnaaaaa 840
   gccgctttan aggccaaggg gggtgggcgn cccgtaaccc gggggnccct gggggngccg 900
   gagncnccga aaccaccgag gtcaccnggc cgnattnaaa ccnaacggnt tcccagggag 960
   ccaggggctg gcttccaagg cggtgnacnt c 991

<210> 55

<211> 956

<212> DNA

<213> Rattus norvegicus

<400> 55

aaggccgaaa ttaaccctca ctaaagggaa caaaagctgg agctccaccg cggtggcggc 60

cgctctagaa ctagtggatc ccccgggctg caggcacagc caagtggcta tgacaacctg 120

accttcctcc cagacaacaa ggccaagtgg tcacccacct ccaaccggaa gccagagccg 180

ggccctgagc ctgtccagcc gcccctccgg cctcctagtc ccatgtcttc cagtcccacg 240

ccccccagct ccatgcctcc tagccctcag cccaaagctt ccgggtctcc caagacagtc 300

caggcagggg acagtccttc agccgtgagt ctatcctgga ctaaggagcg gcggccggna 360

ggggagggcg gctacaaggg ctgtgtggtt cgggcaagga catcgggggc agaggctgat 420

gtggtggttc tcaacgaacc caccgccgac gtgggacagc gccagtgcct cgggaagtga 480

ggggagcgat gatgatgatg accctgacca gaagaagagt ctccgccttg gcgcagtcgc 540

agacaacact tacgtctagc tcagcgcccg gactctccgc cccaagccac tcccctttcc 600

tcctctaatt aaatagcact tttcgaaaaa aaaaaaaaaa aaacttcgaa ggggggcccg 660

gtacccaatt cgccctatag tgagtcgtat taaaatttca atggccgtcg ttttaaaaag 720

tcgtgactgg gaaaaaccctg gggttancc aacttaatcg gcttgnagca aatcccctt 780

ttggnanntg gggtaatagn gaagaaggcc cgnaacggat tggnncttcc caaaaatttg 840

gggcagnttg aaattgggga atgggaaatt gtnaagcggt taaaaatttt gggtaaaaat 900

tcgggggtta aaatttttgg tnaaaatcaa ggncaatttt tttaaaccna aagang 956

<210> 56
<211> 969
<212> DNA
<213> Rattus norvegicus

<400> 56
aagctcgaaa ttaaccctca ctaaagggaa caaaagctgg agctccaccg cggtggcggc 60

cgctctagaa ctagtggatc ccccgggctg caggcccctg gagaggtgaa tagatatgaa 120

ctcagggaac tgggaaggcc tggtgtccta gggtattgga ggcaggtact agatgtgatt 180

gctgaaagtc cccggggcag agtgtccttt cagcgtaagg ataaacacac acacacacac 240

acacacacac acacacacac atgtgcaccc cctgattatt tatgaatcga aatatttgtg 300

acttaaaatt tttaatgcaa aaaaaaaaaa aaaaaaaaaa aaaaaaaaaa aaaaaaaaaa 360

aaaaaaacnc gggggggggc ccggtaccca attcgcccta tagtgggtcg ttttanaatt 420

cactggcngt cgttttanaa cgtcgtgact gggaaaaacc ctggggttac ccaanttaat 480

cgccttgnag naaatccccc tttngccagc tggggtaata gcgaaaaggc ccgcaccgtt 540

ggcccttccc aaaagttggg cncctgnaat ggngaatggc aaattntaag ngtnaatatt 600

ttgttaaaat tcgngttaaa ttttngtnaa ancagcccca tttttaacc aatagggcng 660

gaaatngggn aaaatccctt ntaaatccaa aagantagcc cgngnaangg gttgantgtt 720

gttcccgttt tgggaacaag nggnccncta ttnaangaac ggngggactc ccaacggtca 780

aaaggggggg aaaaaaccgt ctattcaggg gggagggccc nncnnggggn anccattnac 840

ancannatca aagnttttt ngggggncga gggtnccgga aaaggnnctt aaatttggga 900

accccnaaag ggggnccccc ggattttaga gnnttngacn gggggaaacc cggggaaacn 960

ttggngatg 969

<210> 57
<211> 888
<212> DNA
<213> Rattus norvegicus

<400> 57
aagcncgaaa ttaaccctca ctaaagggaa caaaagctgg agcnccaccg cggtggcggc 60

cgctctagaa cnagtggatc ccccgggctg caggggggcg acgaggtgtg gctggccgtc 120

aacgactaca acggcatggt gggcactgag ggctctgaca gcgtcttctc tggtttccta 180

ctgtttcctg actagaatgg caggctgggt ccagcacccg gacgcccgcc tcgctccctc 240

tgctttcccc atcctcactc agacctcttc cttcaggaag tccaccctgg ttcctgaccc 300

atcagccctc tgtctcctca gagtttctct gggaatcact gactggttcc attccagtgg 360

ncagtttatc gagaccttta tgagactatt tttttttcag gtgggaagag agaaaaataa 420

atagatcact aaataaaaaa aaaaaaaaaa aaaacncgag ggggggcccg gtacccaatt 480

cgccctatag tgagtcgtat tacaattcac nggccgtcgt tttacaacgt cgtgactggg 540

aaaaccctgg cgttacccaa cttaatcgcc ttgcagcana tncccctttc gccagctggc 600

gtaaatagcg caagaggccc gnaccgatcg accttcccaa cagttgcgca gctgnaatgn 660

cgaatggcaa attgtaagcg ttaatatttt gtnaaaattc gcgttaaaat ttttgttaaa 720

tccagcccaa tttttaacc caatagggcg gaaaatcggc aaaaatnccn taataaaatc 780

caaaaggaat agaccggnga ataaggggtt tnaagtggtn gntnccaagt ttgggaaana 840

agaaggccca ncgaatttaa aggaacggtg gganctccca anggtcaa 888

<210> 58
<211> 931
<212> DNA
<213> Rattus norvegicus

<400> 58
   tagcgcaagc ncgaaattaa ccctcactaa agggaacaaa agctggagct ccaccgcggt 60
   ggcggccgct ctagaactag tggatccccc gggctgcagg cttcaatccc aacctttaca 120
   atgatggcaa ggtttgttta agcatcctga atacgtggca tggaagacca gaagagaagt 180
   ggaatcctca gacatcaagt tttttgcaag tgttggtttc tgtccagtcc cttatattag 240
   tagctgagcc ttacttcaat gaaccaggat atgaacggtc tagaggcact cccagtggca 300
   cacagagctc tcgagggggg gcccggtacc caattcgccc tatagtgagt cgtattacaa 360
   ttcactgggc cgtcgtttta caacgtcgtg gactgggcaa aaccctggcg ttacccaact 420
   taatcgcctt gcagcacatc cccctttcgc cagctggcgt aatagcgaag agggcccgca 480
   ccgatcggcc cttcccaaca gttgcgcanc tgaatggcga atggcaaatt gtaagcgtta 540
   atattttgtt aaaattcgcg ttaaattttt gttaaatcag ctcatttttt aaccaatagg 600
   gccgaaatcg gcaaaatccc ttnataaatc aaaagaatag accggagata gggttggagt 660
   gttgtttcca gtttggaaca agattccact antaaaagaa cgtgggantt ccaaacgtcc 720
   aaaggggcgn aaaaaaccgt cctatcaggg gcgnatgggc cccactaacg gtggaaacca 780
   tcaaccctta aatccaagnt tttttttggg gggtaaaggg tgcccggtaa aaagccaccc 840
   naaaatcggg ggaaccccct aaaaggggaa gcccccccgg gattttaaga acccttggaa 900
   cgggggggaa aagcccgggc gaaacggtgg g 931

<210> 59
<211> 964
<212> DNA
<213> Rattus norvegicus

<400> 59
   nnaagcncga aattaaccct cactaaaggg aacaaaagct ggagctccac cgcggtggcg 60
   gccgctctag aactagtgga tcccccgggc tgcaggtgag ctcttgctgc agcttctctc 120
   ctctgccctg gtttctgcct gacattagaa agcagcccag gagaaaatcg actccccgga 180
   cgctgatttc ctgtgtcacc ttttgatgag tgttcctggg ctctgccatt ggttttcgcc 240
   tccctgcgac acacacagga atggccatct ccagggtgtg gcggaaccgc ctgtccttca 300
   tggccatcat gatcctcgtg gccatggtcc tgtccctgca tgtcctacgc tctcctctgg 360
   aaggctggac aacctccact gacgtaccta acctgcagaa tcggcttctc acaacttnct 420
   gtctgtggca aggcaggcac attggcttct ctnagagtgt tacaactttc ctggagctgg 480
   gggtgctggg gatacctcaa gttggccttg ccctggctag ggcttggtgt gtatggagcc 540
   ctggtcctcg catcttcgtc cctctgnctc tcctccttgc ccagtgcaac agtggatgca 600
   gggcacaatg gcgggctagc cgtgggctnt nctgggggca atccnctgat gctggttgng 660
   acggngggaa ctaaagncct cttgcnctnt tccttgggng gtggaaangg gctccangnt 720
   cctcnctttt cctggggggn cctgngcctt tnctaancnn ctngtggacn ctnggcccca 780
   aggaccntta actccaaanc aatcntnngg cctnaatggg cctaaggggt naaanggntc 840
   cccaancnaa ccgaaggana aaagaaaggg gancaangga aaaccaactg ggggnaacaa 900
   actggcctna agncccaagg ncccntnaac aaaaaagggc cctaanngng gaaangggga 960
   aatg 964

<210> 60
<211> 868
<212> DNA
<213> Rattus norvegicus

<400> 60
ggcaagcncg caaattaacc ctcactaaag ggaacaaaag ctggagctcc accgcggtgg 60

cggccgctct agaactagtg gatcccccgg gctgcagggc ctggtgctga ccatctttgc 120

taacctcttc ccctcagcct acagcggcgt gaacgagcgc acgttcttgg cagtgaagcc 180

cgacggcgtg cagcggcggc tggtgggcga gatcgtgcgt cgctttgaaa ggaagggctt 240

caagctggtg gcactgaagc tagtgcaggc ctccgaagag ctactgcggn gagcattatg 300

tcgagctgcg ggagagacct ttctacagcc gattagttaa atacatgggc tctggtcccg 360

tggtggccat ggtgtggcaa gggctggatg tcgtgcgcgc ttcgnggncc ctcatagggg 420

ccactgaccc aggggacgcc acgcccggta cgatccgtgg tgatttctgt gtggaggttg 480

gcaatgctca gagagagatc gctctttggt tccgtgagga tgagcttctg tgctgggagg 540

acagcgcggg acactggcta tatgatagac gctaaatcaa cattaccaat ctggaggttg 600

ttggtcttct gtgatcttca catgaacatg ctatgtgggt gcaagtccac ccaacccagt 660

ctgtccaggg gcaaccactt ccacatccca ccctctattt cctttcataa taaaccgcag 720

aaaacccttt tgcgctggtg cagtttcaag acaaaaaaaa aanangnnna nnnaannnnn 780

nnnagnngaa nngnnnnnnn nnnannnnna aaaaaaacct cgaggggggg ggcccggnaa 840

ccccaaattc cgccccaaan aggggntg 868

<210> 61
<211> 887
<212> DNA
<213> Rattus norvegicus

<400> 61
tnggcaagcn cgaaattaac cctcactaaa gggaacaaaa gctggagctc caccgcggtg 60

gcggccgctc tagaactagt ggatcccccg ggctgcaggg agaactagtc tcgagtttta 120

ttttattttt ttattttcta tttttttgcg atgctctaac tgtaaagtag actgaagaca 180

aaaggaaaaa cacaacaaga cacagttctt cgagcagcaa ccaacagaga gtcagagtca 240

caggagaacg cctcacgcag ccgcgggtta ccagggttgt gcaagcatct cccagcatcc 300

ttgtgctgct gctttaggct caaccagtct cgccccgggc gttcacgttc tacactgtaa 360

gaattggacg ctccgtgcat ccgtataaac gtgcaaggtt tgctttgctt gggtggacag 420

cagcccctgt accatttgaa ctcattttgt aacagcaatt tcgcttgcaa aaaaaaaaaa 480

aaaaaaaaaa aaaaaaaaaa actcgagggg gggcccggta cccaattcgc cctatagtga 540

gtcgtattac aattcactgg ccgtcgtttt acaacgtcgt gactgggaaa accctggngt 600

tacccaactt aatcgccttg cagcanatcc ccctttcgcc agctgggtta atagcgaaga 660

ggcccgcacc ggatcggccc ttcccaaaag ttgngcagcc tggaatggcg naatggcaaa 720

ttgtaagngg ttaatatttt gttaaaattc gcgttaaatt tttggttaaa tcagccncat 780

tttttaacca atagggccga aatcgggcaa aaatccctta ataantncaa aaaggattga 840

nccggnggat taggggttga antggtggtt nccagntttn ggaaana 887

<210> 62
<211> 864
<212> DNA
<213> Rattus norvegicus

<400> 62
   tnccgcaagc ncngaaatta accctcacta aagggaacaa aagctggagc tccaccgcgg 60
   tggcggccgc tctagaacta gtggatcccc cgggctgcag gaattcggca cgagccaaga 120
   cgatcttttc ctttaggttg aatatttgaa tcttatgtgt atcaaaaaag aaatgggttt 180
   tagtactttc tgtgccctga tattttgtat actcctgact tccccagtgt gctggctctg 240
   agggcgtgtg gagagctctg taatgcctgg ttgggcactg ctgaggggcc tgccgagctt 300
   gtttctattt catacttttt atactttgtg gaaaaagtca acggaaaact atagtattgg 360
   agggaaccag tgtgaccaag gnaaaagatg atttcaacaa gcagcctcca tgggnacttg 420
   gcgtgcactc tgggttccag ttatctcgag ctgctccacc cctccccagc ccaacggttc 480
   tctctgcaaa cgcttggatc taagaagcta gtctcctggg ttagctgatg cctgccctgc 540
   tttctggtta cttacattct gtttcttgct ttaaaagaaa gacaagactg ttggaccagt 600
   attgcaattc tgtagagtcg tttcttatta aaacaataat gtgattacca aaattggcat 660
   atttaaggcc taatgccatt ctaataaagg caaaaatttc tttttacnac taaaaaaaga 720
   aaananaaaa nanaannaaa aaaaaaaccc gagggggggc ccggtaccca attngcccna 780
   tagggagncg tattacaaat tcactgggcc ggncgtttta aaaaangtcg gtnactgggg 840
   gaaaaccctn gggggttacc caaa 864

<210> 63

<211> 864

<212> DNA
<213> Rattus norvegicus

<400> 63
cncaagcncg aaattaaccc tcactaaagg gaacaaaagc tggagctcca ccgcggtggc 60

ggccgctcta gaactagtgg atcccccggg ctgcaggggg ggggtgttat gtgtacagtg 120

gaatgaagac cagaagaggg cattggttac agggagttga gatccaccat gcaggggctg 180

caaatccttt tgtaagagtt cttagagcat atttttgttg ttgttggttt tttgtttgtt 240

gttttttttt ttgtggaaac agggttttcc tctgtatatc tggctatttg aactcagatc 300

tatctgcctc tacctcatga gtgctggggt taaagacctg tgccaccata ctgagctctg 360

tagtaacagc tcgtaacctt ggaaccattg gcttaagtct gggnaaacnc ctaatagtgg 420

ttatttctaa gacctggaac ttggaatcat tagttttggt gggtattttt cagttgagtg 480

gaatgaatca ctcaaattac tgaagttata atcttccaat taaaaaaaaa aacatctgcg 540

ggttggggat ttagctcagt ggtagagcgc ttgcctagga agcgcaaggc cctgggttcg 600

gtccccagct ccgaaaaaaa gaacccaaaa aaaaaaaaaa gggggggccc ggtacccaat 660

tcgccctaaa agtgagncgt attacaattc acnggccgtc gttttancaa cgtcgtgact 720

ggggaaaacc ctggcgttac ccaacttaat cgccttgcag caacatcccc ccctttcgcc 780

aactggcgta atagcgaaga ggcccgcnac cgantggccc tttccccaaa ccaagttggc 840

gcaagcctgg aaatggcgga aang 864

<210> 64
<211> 899
<212> DNA
<213> Rattus norvegicus

<400> 64
caagctcgaa attaaccctc actaaaggga acaaaagctg gagctccacc gcggtggcgg 60

ccgctctaga actagtggat cccccgggct gcaggaattc ggcacgagag tgtaaccgct 120

gtctgcctgg ttgaacttct gggatcaaga aggtgtgttg aaatcggttt cctttgggag 180

cggtgggcac agctaacgca actgtgaaca gacacgtctc acacaatcac ctgctgctgg 240

cactcggcct gggtctgcct ttgcccgccc tgccctccgc catagctgtg tggtggccct 300

tagaatagat ggggaggctt caggtagcag ccgtgggact gaccaccgct gggcttgggg 360

cgctttggct gcacccctgc tttcttaagt cttaagtgat tgccccatcc aagccatggt 420

ccccactcct ccactcccac ccttgggcca aagcttagat tgtaatctcc cttccctctg 480

gaaattggcc gtgggtgagg aattcagggc ttcccgtctc cccaccttta tcaaggggtg 540

ctgctttccc ctcctcaagt cccttgttgc ccgtcaccac ccaacacttg ctgtggccag 600

aagccaccag atgaggttgg aagagcctgg cctccctcaa ttagctccgg accacaatcg 660

ttcacctgcc aacagcctgg gaagggagcg ccgggtcctc gggccctgcc aacaaccatc 720

agcccttgag ctttgagctc aggtctagag gtgaacagag cagtcaacgg gggcgaatca 780

agaaggggcc aancgntcaa ggggtccctt gggaatataa ntgccttaga agaaaagggc 840

caatgcngga gaagntcctt cgggtggnan aatggggtnc tgnagtttgg gttcctttg 899

<210> 65
<211> 941
<212> DNA
<213> Rattus norvegicus

<400> 65
   gcaagcgcga aattaaccct cactaaaggg aacaaaagct ggagctccac cgcggtggcg 60
   gccgctctag aactagtgga tcccccgggc tgcaggcact ttcctaaata gaaaanggta 120
   gctcacaggc ggcagagcac agaaacactg gtgggtgtgc ccagccagat gccagagttt 180
   ctgtgctctg ccgcctgtga gctaccactt tcctaaatag aaaatggcat tatttttatt 240
   tactttttgt aaagtgattt ccagtcttct gttggcgttc agggtggccc tgtttctgca 300
   ctgtgtacag taatagatgc acacggttga cctgtcctgg ggcctaggtg ggttgtacac 360
   tgagcatcag ctcacgtaat ggcattgcct gtaacgatgc taataaaacg tctccttctt 420
   aaaaaaaaaa aaaaaaaaaa ctcgaggggg ggcccggtac ccaattcgcc ctatagtgag 480
   tcgtattaca attcactggc cgtcgtttta caacgtcgtg actgggaaaa ccctggcgtt 540
   acccaactta atcgccttgn agcacatccc cctttcgcca gctggggtaa tagcgaagag 600
   gcccgcaacg atcgcccttt cccaaacagt ttgcggcanc tggaatgggc gnaatgggcn 660
   aattgtaagc ggtttaaata aatttggtta aaaattcgcg ttaaaaattt tggtaaaatc 720
   cagccncaat ttttttaaac ccaananggc cggaaaatcg ggcaaaaatn ccccttataa 780
   aatccaaaaa ggnattagac ccggngaata aaggggttna aatggttggn tncccagttt 840
   tgggaacaaa gaggtcccac ctaattaaaa gaaancggng ggaccnccca aanggtcaaa 900
   aaggggggga aaaaanccgg gctatcaang gggggnangg c 941

<210> 66
<211> 877
<212> DNA
<213> Rattus norvegicus

<400> 66
aagcgcgaaa ttaaccctca ctaaagggaa caaaagctgg agcnccaccg cggtggcggc 60

cgctctagaa cnagtggatc ccccgggctg cagggtctga gctccctacc ctgnaagagg 120

ctgtgggctt gctcgtagcc taacccctca ccgacagttt gatggcgaag agacggtcct 180

tgtgcgtaag gaaggacagt tctcacnccg cagaataaca ggagacagaa atgttcaatt 240

aaaaagagtt tactttagac cacagcctgc tgtgtgccca ctcactgccc tgtctgctgg 300

gaggcgggat caggggagat aggcggatgg ctgtctcatt aatgtgctat gcctagttag 360

tgtggaggtg ggagaaagga ggtgtgtgtg tggggggggg cgggggatct tgttatgtaa 420

ccctgtgctt tctgtccttg aaccnctggg gtgggaggaa ccctattatc tgcctctcgg 480

ataacaaagg acggattgat tattctgggg acncctaagt ggggagaggg gtgaggcatt 540

tgcaagtgac ccctgggacc tggaaccctc aagaggagcc taatgtctct gcccacagga 600

acatctgtgg nttcantact tcttgttttg tccctgtatg cttttctctg cactcaaatg 660

gggtgatgag ggaaggcggg gggctctcaa atcctgtctg tgaacttttc ccttcttgct 720

gatgcnactt cttcgncaag ccaaggtctg aaagaaggag tcctcccagg ntgntgncng 780

nttgccggag atcataaagc ananggtcag gggctggncn ccctgggnnn caagtncggg 840

ctttggccng tgggatgccn ggggcaaaag gacctng 877

<210> 67
<211> 895
<212> DNA
<213> Rattus norvegicus

<400> 67
caagcgcgaa atnaaccctc actaaaggga acaaaagctg gagctccacc gcggtggcgg 60

nccgctctag aactagtgga tcccccgggc tgcaggtggg acgccggatt cgcaagcccg 120

atttggtcag tcggtgaagg ggcttctcac ggaaaaggtg aacacctgcg gcaccgacgt 180

aatcgcgctc accaagcagg tgctgaaagg ctcgcgaact tctgagctgc tggggcaggc 240

agctcgaaat atggtgctac aggaagatgc catcttgcac tcagaagata gtttaaggaa 300

gatggcgata ataacaacac accttcagta ccagcaagaa gctattcaga agaacgttga 360

acagtcgcct ggacctgcaa gaccagctgg agtcatttac tggaagtagc tctcgccaga 420

acagcagttg gacttctttg gcctgatgct gagaaggacg cgggccttag tttccatttt 480

catctccaaa aattcatcta gaaaagactt gtgaaaagaa gaagccaagt gaccaaacgt 540

gaaagcactt cttaagttgg gagtaactca tcttcaagtg gttttatatt aaaattaata 600

ggttgaatca tttagcatca gatccctctc tctctctccc tcctgtgtag cccgctgctt 660

ttgaactcat aatccccgtg ccgtagcctc tcaagtagtg agattaaaaa ccaccatacc 720

tggccactgg agtaacacat tagtcgcaga tacttgggag gctgagggca gggaggatcc 780

tgtggagccc cnaacggttg agtccatcca gggnnacaca ggaagaacct atctccaaga 840

aggagaaggg gcaaggggaa gggggcttct aactggggat cacgtgagaa gtgnt 895

<210> 68

<211> 947
<212> DNA
<213> Rattus norvegicus

<400> 68
tntgcaagcn cgaaattaac cctcactaaa gggaacaaaa gctggagctc caccgcggtg 60

gcggccgctc tagaactagt ggatcccccg ggctgcagga attcggcacg agctactttc 120

cagtgtaacc atagcctgct tagcttgggg taagacttag tagaaaatgg tgcttcagta 180

aaccgcttac ttccagtcac aatcaccttg ttgctgtggg acccgaccct gtttgagccg 240

gctgccctca ttcccactta aatcaaagca gctggctagt ccccctgttt ccttcccaaa 300

tcctgttttc atgtacaaga caaaataaag gactcaattc tccctacaaa aaaaaaaaaa 360

aaaaaactcg aggggggggcc cggtacccaa ttcgccctat agtgagtcgt attacaattc 420

actggccgtc gttttacaac gtcgtgactg ggaaaaccc tggcgttacc caacttaatc 480

gccttggcag cacatccccc tttcgccagc tggcgtaata gcgaagaagg cccgcaccgg 540

atngcccttc ccaacagttg cgcanctgna atgggcgaat gggcaaattg taagcgttaa 600

natttgtta aaattcgcgt taaatttttg ntaaatcagc tcatttttta ancaatnggc 660

cgaaatcggc aaaatccctt tataaaatcn aaaagantag gaccgagata gggtttgagt 720

gntgttccna gttttgggaa caaagangtn acacctattt naaaagaaac gtgggagctc 780

ncaacggtna aaaggggcgg aaaaaanccg gtctaatnca ngggcggatn gggcccaacg 840

gaanggtgaa acccaatgga anngtaaatc naaggatlnt tnngggggn cncgaaggtn 900

gccgggaaaa ggcacctaaa aatncgggga ncccntaaaa gggggng 947

<210> 69
<211> 895
<212> DNA
<213> Rattus norvegicus

<400> 69
caagcgcgaa attaaccctc actaaaggga acaaaagctg gagctccacc gcggtggcgg 60

ccgctctaga actagtggat cccccgggct gcaggttttt ttttaaggat ggaaactgtt 120

tatttatggg taaagaatag ctgagagaac acttgaattt gatgaagctg tgccactttg 180

caggtcgggt tggtttatca tcaaaaggtc caaaataaaa gttacttcac agaaaggagg 240

aggaagcaat aagttaatgc ataataagcg cttttacaag catactttat aggaaggaga 300

ttcataatta tagccaatat attctagaca gtaactttga ctatttcaca agaacataaa 360

attactgagt atggaatggg tggcagacac gaccatggac gaagaagggc atatgttgtg 420

tacctggcca tggatcacag ctcctaagct ttggaactac attttggctg tgggacacaa 480

gaacataaga ttttctctag gagttaaggg agtggccaat gggctgatag tgggcagtgg 540

agaaagaaca ctgtacattc ttaaaagtct gccatagttg aagagatgag tgaggtttgt 600

agttaacaaa aacatggact ttttcctttt taatacaggt ttacctgcta atgcaaattt 660

agaaggaatt taaccaagtc agtaaaaatg ttgaaggctt tcaccggaac caatgactgt 720

tttggcctct ttattcaaag tacaagatgg atgtcaccaa aactgggatt tgagantgga 780

aaatttccaa aagggggaga aaaatccngg ggttatttan aggttaaaaa accggggaag 840

gatttggtta aaggccanca ggataggtnc aggacccaat tgggaaccca tatnc 895

<210> 70
<211> 896
<212> DNA
<213> Rattus norvegicus

<400> 70
   ncaagctcga aattaaccct cactaaaggg aacaaaagct ggagctccac cgcggtggcg 60
   gccgctctag aactagtgga tcccccgggc tgcaggaatt cggcacgaga agaactcaag 120
   agagtagcca ccatcttaaa gcaaagtagc aggtggggaa aaggtgggta gaggagatgc 180
   tacttagggg gtgggatttt ccagtcaggg ccatttagac acgggaatcg ctgaggcttt 240
   cagtcgatgg ggctttcttt tttccctgct tcatctctcg gcctcaggag aggtattaac 300
   agtattatca ccatttatat cctagctgtc ctgagccaaa tctgccattg gaggtgtctt 360
   ccctgtgttg gttctccaag ggacgttgca tggggatttg tgggggcagg gcagcaaggc 420
   cttgttctct aaatgtccag aaacactcta attaccattt ccacctgggt gcctcacaca 480
   ctccccagag gggaggttaa catctctgcc ccatttccct catgttcctt ggcttggtca 540
   ttccctacct ttctattttg tgttaaactt ggcttttttt tttttttcat attgaaaaga 600
   tgacattgcc ccgagagcca aaaataaatg gggaatggaa aaaaaaaaaa aannnanaan 660
   nnananannn annannaaaa aaaancccgn gggggggccc ggtacccaat tngcccnaaa 720
   agggggnggn atnaaaattc cngnggccgg ncggttttaa aanggncggn angggnaaaa 780
   ncccnggggg gttnacccan nttaaancgc cttnggagga aaaatccccc cttttnngca 840
   aaannggggg gaaanaangg aaaaagggcc cggaacnggn atggcncttt tccnag 896

<210> 71
<211> 929
<212> DNA
<213> Rattus norvegicus

<400> 71
cncaagcgcg aaatnaaccc tcactaaagg gaacaaaagc tggagctcca ccgcggtggc 60

ggccgctcta gaactagtgg atcccccggg ctgcaggaat tcggcacgag aatagagctt 120

ttgtgcggcg gcagcggcgg cggcgtctct ctgatttgaa cgccgaacag cggtagcttc 180

tcatctgtgg cctgacctcg aagcctaaga acagagcggc gagatgacgg accggtacac 240

catccacagc cagctcgagc atctgcagtc caagtacatc ggcacgggcc acgccgacac 300

caccaagtgg gaatggcttg tgaaccagca tcgggattcc tactgctcct acatgggtca 360

cttcgacctc ctcaactnac ttcgccattg ctgagaatgn agagcaaagc gcgcgtgncg 420

ttttcaacct ggatggagaa aatgctgcag cccagcgggc cgaccggcgg gacaagccgg 480

aggagaactg aggcgagcgc ttcccagcct tccccatctg ccatctgtgg accgatcctc 540

ctgactcctg cttctcgacc attctccgtt gggtgtatcg cctgacctgg cttacctgtg 600

ggacggttcc gaacaagtca tcgagagact gtcgggtctc ctggggaagc tgtgcgggaa 660

ggagtgatcc cagaatcggg caaagcgang ggagaagact gcctggggaa tggatgacgc 720

attccgagtt cagcttttcg aataagttga tgtcgttctc gccttttttn tttttttaaa 780

tanntannat acataaaagt tagggatttt gntaaaaaaa aaaaananaa aaaaaaaact 840

tcgnaggggg ggggnccggg taccccaaat ttcggcccct aaanaaggng gaagtncggn 900

atttaaaaaa tttcaacttg ggccngntg 929

<210> 72
<211> 944
<212> DNA
<213> Rattus norvegicus

<400> 72

ntcaagcgtc gaaattaacc ctcacntaaa gggaacaaaa gctggagctc caccgcggtg 60

gcggccgctc tagaactagt ggatcccccg ggctgcaggc ggccttggaa ccgctcgatt 120

tctatagaga agctggcggc tggtgccagt ggcttctgag ttctcgattc ttggacnccg 180

agaaggctgc aaggccatgc tggcttggcg cgtggcacgc ggcgcgtgga gggtcccttc 240

gcgtggctgt ccggcctccg ggggcgcggc tcggcagggg cggctcccgc aggccctgct 300

accacccgcg gcctgctgcc tgggctgcct ggccgagcgc tggcggctgc gtccggccgc 360

gttcgccttg cggctgccag gcaccagccc gcggacccac tgnctccggc gccgggaagg 420

cagccccgga gcccgcagcc ggaggagatn ccgccggcgc agncccctan gnncccggtg 480

ggtccgggcg agcgcaacca nctcgtatga aaatccatgg acaatcccaa atttgttgtc 540

aatgacaaga attancctgg ccccantgtt gggctatctg attcttgaag aanattttaa 600

tgttgcacna gggtgttttt gctttaacta ggactaacgg gatttgttgg natgggnatt 660

taatnncnnn aaaactnngg cncaatnaaa aatnaagctt tngggaaant gctcttggat 720

cccaactngc ttgnanaaan gtttnttaan caaggaatct tgaanaagaa tnangccngg 780

nanctaatgn nnagaatcct taaattcgaa antncccaag nnaacttaac aggnntaaat 840

tttcaangaa gnatggaaan gggtggaaac ggcaggcnga gnnntnttaa nnnnnaanat 900

aaacgagaaa nnnntggnga aaaaaccggg gaaacagant anng 944

<210> 73
<211> 886
<212> DNA
<213> Rattus norvegicus

<400> 73
caagcgtcga aattaaccct cactaaaggg aacaaaagct ggagctccac cgcggtggcg 60

ggccgctcta gaactagtgg atcccccggg ctgcaggcca tcgccccact ccaccctcct 120

tcctcttggg cttggtgtgg atggacctgt ccatcagctt ggtgtggtgt tcacaaataa 180

ctgacaggcc agggaaggtc ctgctgtggc cgcctgagag atggctcaag tactctgtga 240

ttccttgtgg cccagagccc tgtgtgctgg ctgctatcag acaccttgcc cctgtgctgg 300

tcttaaagaa tcattatcca tcgtggtcac tacgtcctgc ttcctctggg catctggagt 360

tcccagattc ctctgtccct ttcctggata tgcttttgta tacacatttt tagcacgaga 420

ctttccccct ttacaaaggt gtcaacttga aaaatgtttt aaaccacagg atagcacttt 480

caatctaact tttgtggtat cttccatcag aatcatcttt tcctatctgt tttttccctt 540

cacgggttaa ggttcacatc cccatggaaa ccagtttaaa tctgcctcag aacaatttgt 600

atctttggga aggaattgtg tccttggagg cccatgtaag tggattctaa gtgggggcca 660

gctgctcctg tgtgcatgct gggactctgg ggaggagagc cccctggcat acacctcaat 720

ttgccctcag tcaaggtgag gcaaggggtg ctggagtttc ctcancccag caaggctttg 780

ctgtcaataa ggaaggaggg aagaaaagtc ncccggtggc naatgaggac catagcatac 840

ctaananggc ccaataagaa nggaaacaag tggnctacna aagccg 886

<210> 74
<211> 888
<212> DNA
<213> Rattus norvegicus

<400> 74
gcaagcncga aattaaccct cactaaaggg aacaaaagct ggagctccac cgcggtggcg 60

gccgctctag aactagtgga tcccccgggc tgcaggcatg ggcagagttt tcaccaccaa 120

aaacatgtgc ctcaagccag tacccggatc cctgaggcca cagaaggaaa acctccagac 180

acaagcacgg ctgtacagtt tcagagcacc cagcagtcca cttttccatc tggagcacca 240

tccttgaaca aagagctcac ccgccactgg gaaacaacca ttctcccttc aggctatggt 300

ctggaggcta ggcctgtggc tgaggcaaat gagaaacagc acaaacagca aaaagaacca 360

ggagctggtg ctgggcacac aagccttggt gccggtgcta tccctcctgg gccatcgtct 420

tcttcctcgt gggcagccat ggtctgtgtg ctgtgcaaca ggaggagtga ctcgggcaga 480

gctcttcagg tttgcagctc tgttatacac ctgttgaacc aagaccccag ggcctgtagc 540

aagaatggaa gctcgtgaga gatgacgtgg gagaggtggc agacagactg gcaggctagg 600

ctccatcaac gaactgaatc tgagctcatt ttttctggtg atgttttgaa tcaaagtagc 660

cattatgtaa tctagggaca gctttgaact acagagcctg tgccccgacc tcctagattc 720

tgggattata gatatatcct actgcatctg ccctcgtcta atttcataaa taaggtntaa 780

attttcangg ttttgttttg gtttagcgga agaatcttat ttagnccaag ccaacctcaa 840

aatnccccaa gggaancnct aanggncccc aggccttcct taaaaaag 888

<210> 75
<211> 893
<212> DNA
<213> Rattus norvegicus

<400> 75
   caagcgcgaa attaaccctc actaaaggga acaaaagctg gagctccacc gcggtggcgg 60
   ccgctctaga acnagtggat cccccgggct gcagggagaa gagatcctgg atcacagtgc 120
   tgtccgccat gacagaggag gcagctgttg caatcaaggc catggcaaaa taactggctt 180
   ccagggtggc ggtggtggca ncagtgatcc atgagcctac agaggcccct cccccagctc 240
   tggctgggcc cttggctgga ctcctatcca atttatttga cgttttattt tggttttcct 300
   cacnccttca aactgtcggg gagaccctga cccttcaccn agctcccttg ggccaggcat 360
   gaaggggagc catggccttg gtgcaagcta cctgnccttc ttctctcgca gccctgaatg 420
   ggggaaaggg agtgggtact gcctgtggtt taggttcccc tctccctttt tctttttaat 480
   tcaatttgga atcagaaagc tgtggattcn ggcaaatggt cttgtgtccn ttatnccact 540
   caaacccatc tggncccctg tnctccatag tccttcannc ccaagcacca ntgtacagac 600
   tgggggacca gcccccttcc cngcctgtgt ctcttcccaa acncctctat aggggtgaca 660
   agaagagggg gggganggga cacgatccct cctcaaggca tctggggaan gccttgcccc 720
   catggggctt taaacctttc ctgtggggtt tctccctgaa aaaatttggn aaaaatcaaa 780
   acctgnataa aacganaagn ttaaatatgg aaaaaaaann nnnnnnnnna ngnnnnnnng 840
   gnnnnantnn annnngnana annnnnnnnn nnanggggnn gggggggggn nnn 893

<210> 76
<211> 940
<212> DNA
<213> Rattus norvegicus

<400> 76
nancgcaagc gcngaaatta accctcacta aagggaacaa aagctggagc tccaccgcgg 60

tggcggccgc tctagaacta gtggatcccc cgggctgcag gaattcggca cgagccgcat 120

ccaagaagac aggtggcagt tctaagaacc ttggtggcaa atcacgaggc aaacactatg 180

gcatcaagaa aatggaaggt cactacgttc atgccggcaa catccttggc actcagcggc 240

agttcagatg gcacccaggc gcccatgtgg gactggggaa gaacaagtgc ctgtatgccc 300

tggaggaggg gatagttcgc tacacgaaag aagtctacgt gcccaatccc aaaaactcgg 360

aggctgtgaa tctggtcact agtctgccca agggtgctgt gctctacaag acttttgtcc 420

acgtggttcc tgccaaaccg gaggnaacct tcaaactggt agacatggct ttgaagtcct 480

gttgagacca tcggatgacg ggcgaccgga acccaggtca caggagcaag tgatgatgga 540

agtcaagggt cagggtgagg acaaggtctc cacagaagag gcctattgga tggggactct 600

gcaggggcct ttgtgctgtg gttgctggaa anctcttggn agctctggca tgantgtcaa 660

taaagctgna ggaattcctg gaaaaaaaan aaaaannaaa naaaaaacct cgaggggggg 720

ggcccgggtt accccaaatt cgnccctaat annngaaanc ggnaanttaa caaattcaac 780

ngggccggtc ngntttaaac aaaggtccgt nganctgggg naaaaacccc ngggnnggtt 840

taccccaaac ttaaatccgg nncttggaga gggaaaattc cccccctttt gggccaaggc 900

ctgggggnaa gataagcgga naaaagggcc ccgcaacccg 940

<210> 77

<211> 896
<212> DNA
<213> Rattus norvegicus

<400> 77
cgcaagcncg aaattaaccc tcactaaagg gaacaaaagc tggagctcca ccgcggtggc 60

ggccgctcta gaactagtgg atcccccggg ctgcaggtgt tagcctccgg taccggctct 120

cttctttttc aatgtagcgc ttgaagccct agctagagca ataaggtaag gaaactaaag 180

gagcataaat agaaaaagtc aaattatccc tattagccaa tgatattatg catatgagat 240

tctaaacact ttcaggagag tggaaggata caaaaacaaa ttacaaaacc cagtagctct 300

tctctatgcc aataacagca tcctgcgaaa gaaatcctgg aaaaccatcc cattcacaac 360

aacctcaaac acgtatacat tcgtactcat ccccataaaa agcctaacca aggggttgcg 420

agacctctac aatgatgatt ttacatctct gagggaagac aatagaggat gggaagacct 480

tccacgctca tggactggta gaattcagag tgtggcaatg gtcacgccta aaaaccatgt 540

gcggattcat gacagtgcaa ccacctggta atcaaccacc gaaaaatgca aacgagagga 600

cagaaagtat tatttacaaa cggggctgga aaacattgga tgtccacagg tagaagaatg 660

agattagaac ctttaccgct tcctctggca caaaaccaac tcccaccagg tccaagatgc 720

cagtgngaaa ctttcagtct ctgggaaccc tgcagactct tggagagcaa tggttacaca 780

gggagaatcc tcatttccgg ctttatgggg ggagactacc tggagggaaa agtgcccngt 840

ccttccccng gggcttctaa ggaaaccctt cttagaggag gggtaaaaat taaacc 896

<210> 78
<211> 892
<212> DNA
<213> Rattus norvegicus

<400> 78
caagctcgaa attaaccctc actaaaggga acaaaagctg gagctccacc gcggtggcgg 60

ccgctctaga actagtggat cccccgggct gcaggcccaa ccagcccaca tttgttctct 120

aaacccaaac agctgtcccc tgtctgtctg tggctttttg ttcattttat ttagtgatgt 180

tttttcagtt aaaccaccgt ggacaaatgt ctcactaaga aatccgtgtg aagctgtata 240

gcttacacct gtaattgtag aacgtgggag gctggactga ggggatagca ttgagttcaa 300

ggccagccag agctgtcagc tatatagagt tccaggctga tctcagtcac agagtaagac 360

cctttctcag aaagacaaag aatctaaatg aggtaggatt ctgtgggctc agtggtaatt 420

ggcctaactt ggctgttcac acttgtaagg cccagaattt gatccccaca ccctccaaaa 480

aagaagtttg gggatgtgaa ctgaattagc atcagtgcct ctgatcctct ctcagccgta 540

gactagaatg acgaggagcc ctggtttaac cttggcactg ctgcctaccc tctctaagct 600

cgctttcctc atctgtgagg agcctcggga tggagcctca gggagtgcgg gtggatattt 660

ttatattgtc tattaaaatg taggcattaa gctccaacat ttntgcttgt tacaatttta 720

nggcctatat tttattgatt aaaaaatgcc cctggcgggg ttgggggatt tagccccagt 780

ggtagagcgg cttgnctagc aagcgcaaag gccctngggg nttgggttcc cagcncccgn 840

aaaaaaaaaa annannnnna nnanaaaaaa aaccnncgag gggggggccc cg 892

<210> 79
<211> 979
<212> DNA
<213> Rattus norvegicus

<400> 79
aagcgcgaaa ttaaccctca ctaaagggaa caaaagctgg agctccaccg cggtggcggc 60

cgctctagaa ctagtggatc ccccgggctg caggctgaca tgtggcactg gtggtttttc 120

attaccttgg atgtcagagg actttcattg aaacaaaatt ttatgttgga ctggaaaatg 180

ggggctagaa gtatcatgta tggtcaaacg gaagactggg taacctccaa acagagcatt 240

catgaaattg tcaacagtat tcgtcccaag tattttcata tgctgtcaca caagaggacc 300

agatgctgga ggatctccag tctgtcccct ctcagcagag aggaaagaca gtatggcaga 360

aaacctgtgt agctttagct tcaggtcctg ttaaagcatt actgtttgca cagcaggaaa 420

ttccccctgn aactgtcagc ttttccctgt gttactggca ctgttggaat gaggtggaaa 480

gtacacgaat ggatgccatg gtcttgttgg tggtcagggt cctactgccg tgtaatgaag 540

gcctgcagtg cagacactca cttgtttctc tctattcaca gtattctccg gaaacgcatt 600

cgagaggata gaaaggctac aaccgctcag aaggtgcagc agatgagaca gaggctaaat 660

gaaactgaac ggaaaaggaa aaggccaaga ttgacagaca cctaaatgtt catgacttga 720

gactattctg cagctataaa ttttgaacct ttgatgtgca aagcaagacc tgaagcccac 780

tccggaaact aaagtgaggc ttgctaancc tgtagattgc ctcacaagnt gtctgtttac 840

aaagtaagct ttacatccag gggatgaaga aacgccacca gcagagactt gcaaacccct 900

taaantngan ggaattggnn ttttaaccan ggnggtatga attggaggaa agatgtaaag 960

naaaatnaat ttaggggggg 979

<210> 80
<211> 973
<212> DNA
<213> Rattus norvegicus

<400> 80
agncaagcgc gaaattaacc ctcactaaag ggaacaaaag ctggagctcc accgcggtgg 60

cggccgctct agaactagtg gatcccccgg gctgcaggtt tttttttttt tttcacaatg 120

aatatgtctc atttattagg tagaaaacac ttaactgcat aaaacttaca gggaaaaatg 180

gccgtatttg aaaacagcta aaaggatcag agtagaacac agaaccgtaa tgagcagtgt 240

cacggagcac actaaggagg tgtgtatagt cagtcccact ggcacctgca ctgtcaaatt 300

cttaagtatt gatttgtact gcatgttttt ccactgggca gatctcctca ctcttcaaag 360

aacaagggag ctgctacttt ctgactgagc ccagcatttc aaaattgggg aactcttggt 420

cacagtgcat cagtaagtca gggttgttga ccacaatgga gggtgtctcc atccttctta 480

tgtggacgca atttggggc tccttcgggc acttcggca cctctacagc caccgcggca 540

ccggcgggcg ggtttggcgg atttggaaca acaactacaa ctgcaggctc tgcattcagc 600

ttttctgccc caacaaacac agggcagtac aggccttctc ggcggnactc agaacaaagg 660

ttttggcttt ggcactggtt ttggcacatc gacgggtact ggcactggtt taggcactgg 720

cttgggaanc ggacttggat tcggaggatt taacacccag cagcagcagc agcagcagca 780

gacttcttta ggcggtctct tcagtcagcc tgcacanggc cctgcgcagt ccaaaccaac 840

tcatcaaact ggcagggtct ttctggncca aagcnaantg gggggtgaaa aaangccanc 900

ttggcaaagt gggaccagtt gcagggcttc tgggggaaag ggaaagggga tttccataan 960

aaaaaatccc ccc 973

<210> 81
<211> 1004
<212> DNA
<213> Rattus norvegicus

<400> 81
   cgcaagcgnn gaaattaacc ctcacgtaaa gggaacaaaa gctggagctc caccgcggtg 60
   gcggccgctc tagaactagt ggatcccccg ggctgcaggt ttgatttcaa atggatctac 120
   actgttaact gaatgatgag actccactgt gattcactcg tttacttaat caaaaaattt 180
   cagggatgtc tgtaaatttc agtgttgtgc acaacaagaa gtgctgttgg ttgatttaag 240
   gagggaccag aaataatttc tactattcca gtactgaagg aaaaaaaata ctgatttata 300
   ctgtttttaa aaactaaata ttaataaagc cccctgtcag aaatttaacc ttaaaaatta 360
   ttttaaatat catcctatat tattagaagg gaactacaag tgactggata aataccaaaa 420
   agattcacaa gcagcttcat ttaaaaagca caaagaggtt ctggtgtgaa atgcccaaat 480
   ctcaaatgtt ttctgtagtt ctgagtttac agatgtaaaa gctgtccctg gaaggctgca 540
   gtacctgtat ctgctgtccc tgtgaactac actcgcacca ccccagaaac cctggtactg 600
   gatagggtag cgtgggggta cagtctcaga gggggtgagc cacagtcacc caacggggtc 660
   agcttgcaga aagaccaaaa cagggaaagg cgagtgggat aattacttaa cagcacattc 720
   actctctacg gagtaatcac atatgggttg aaatttgaag agcagattgt ggatcatttt 780
   gacccctggn caaaatccct gtacttggag aantttggag gcggacgtgg gcatccacgt 840
   gggcggttgc ctttaccaac aggnccgtgg gactggttgc caaaaaggnc ggggtncnct 900
   ggaaaaaaag ccaggncccc ccncggtggc ctggcaaagg accaggttan gggaaaggaa 960
   aancccnnnn tatnttngnn gaacccaaan ttttaatncc ccgg 1004

<210> 82
<211> 1003
<212> DNA
<213> Rattus norvegicus

<400> 82
aggcaagcgc ngaaattaac cctcactaaa gggaacaaaa gctggagctc caccgcggtg 60

gcggccgctc tagaactagt ggatcccccg ggctgcagga attcggcacg aggctatcca 120

gacgggcaga gttacccagg agctgcagga caggtacctg gaccacaccc cggtggctac 180

tatcctggac ctccccatgg tgggggccag tatggcagtg gattcccccc tggtggttac 240

ggagctcctg cccctggagg accctatggc taccccagtg ctggaggaac cccctctgga 300

actccaggcg gaccatatgg cggtggacct ccaggaggcc cctatggtgg tggacctcca 360

ggaggcccct atggtcaggc acatccaagt ccctatggta cccagccgcc tggaccttat 420

ggacagggtg gtgtcccccc caatgtcgat cctgagggcc tactcctggt tccagtcagt 480

ggatgccgac cacagtggct atatctcact tcaaggagct gaagcaggcc ctggtcaact 540

ccaactggtc ctcattcaat gatgagacgt gcctcatgat gataaacatg tttgacaaga 600

ccaagactgg ccgaattgat gtcgtcggct tctcagcctt atggaattcc tccagcagtg 660

gaagaacctc ttttcagcag tatgaccggg accactcggg atccatcagc tccacagaag 720

ctgcagcaag cgctgtccca gatgggctac aaacctgnag cctcagttca agcaagcttc 780

ctggttttcc cgaatactgt aaaaggntct ggncaattcc cggccaatgc agctgggaat 840

ggtttcaatc aagggtggtg tnacccaagc tttcaanggt gttgaactga aggccttccg 900

gggaagaaag ggtanggggt tgtaaaaggg gaaaaaattc cgggntcnag gcttttgaag 960

gganttttgn caacnatgna ggggtttaan ggatngcaat nnn 1003

<210> 83
<211> 1004
<212> DNA
<213> Rattus norvegicus

<400> 83
cgcaagcgcg gaaattaacc ctcactaaag ggaacaaaag ctggagctcc accgcggtgg 60

cggccgctct agaactagtg gatcccccgg gctgcagggt gggcagaggg attggccctg 120

ctttgtgacc cctacctgat gcctccttcc acaacatgat tgcagccctt agaacctagc 180

tcagagcccc tcagcacaag ccccgccccc agcagccagc caaagccact gggtgagcgg 240

gtacatctgc ggacccatcc ctcagcctcg atcaaccagg atccctcctt cccagagcct 300

gtccctggga gagcttttgc caccaaggtt ctagccctgg actttctaac cacttcttct 360

catgggagac accctggctg gctactncca agggaccagt ttggcttaac ttcacagccc 420

acatccatgg tcatctttaa ccttctttcc tggcagattg ccaggttgct aatctgctgt 480

cccctctggc tgtaagcaca gtgtcaggac ctagtgagga ggtacaagga gcaggccgtg 540

cttggagtgc ccattccccc taaccctctg ggntggcgcc tcctcctcac tggagacccg 600

gaactctgna gagagccaaa gcacacaggg acccagcagt gtgggttaga caaagctgca 660

gctaagatcg gggagtcctg gnactgcagg ccaggccagg gtccccacct aagccacata 720

atctgcctgc cggagnctgg cccccgagcc ccctcctggg gaaagtgctg cccatttgcc 780

agtgtctgcc caggaggaag gggatctgct tcagagggnt tcctgagaac cctgggtccc 840

aagnctnagc tggtaaaggc ctctggggtg ggaaaaggnt tgctggtggg ggaagnccaa 900

aaacggggaa agttttgnaa naagggngan aaggttttta agnaggngga gcggaggcaa 960

aaaggggttt ttagagccaa gggncaanaa attttntttt aatg 1004

<210> 84

<211> 982
<212> DNA
<213> Rattus norvegicus

<400> 84
aagcgcgaaa ttaaccctca cgtaaaggga acaaaagctg gagctccacc gcggtggcgg 60

ccgctctaga actagtggat cccccgggct gcaggaatca tcgctgaggc cctaacaagg 120

gtcatctaca acctgacaga gaaggggacg cccccagaca tgccagtgtt cacggagcag 180

atgcaggtcc agcaggagca gatagactca gtcatggact ggctcaccaa ccagccccgg 240

gccgcccaac tgctggacaa ggacgggacg ttcctgagta cactggagca cttcctgagc 300

cgctacctga aggacgtgcg gcagcaccac gtgaaggccg acaagcggga ccctggagtt 360

tgtcttctat ggaccagctg aagcaagtga tgaacgctta cagggtcaag ccagccatct 420

ttgacctgct gctggccttg tgcattgggg cgtacctggg gcatggcata cacagccgtc 480

cagcacttcc atgtgctgta caagacggtg cagagactgc tgctcaaggc caaggcacag 540

tgacagtggc catgcacagg tggcccagga ggtactagcc cacgcaccca cagcagccag 600

actgaaacac agagggtttg gatgggtcac taggatgcag ggacacctct ccctgtccat 660

ttctttgaat gtccctggag gagagccccg cccgcctgca gacgagccca ctgggatgga 720

atgatgaccc gggccaaatg cactgaaagg ccgcacaatg ctgttggcct ccccagtggc 780

tgggttccag aagcctgtct ctgcagtttc ccaagaggna gccaacgttc agcctggctt 840

ggcccagcaa cgggcaagan ccaanaatgt tntccctgat ggtcctcctc aaaacccctg 900

ttccggcctt gtttgttaac ttttgnaaca atttgaacca accttgggtn ccccaagctt 960

gggattttga gccaccctga gg 982

<210> 85
<211> 983
<212> DNA
<213> Rattus norvegicus

<400> 85
caagcgngga aattaaccct cactaaaggg aacaaaagct ggagctccac cgcggtggcg 60

gccgctctag aactagtgga tcccccgggc tgcagggttt tttttttttt tttttaaaa 120

cttaaaaaca tttttatttt ttttggtttc gagacaggat ctaagtagtg cagattgatc 180

ttaaactcag cctgcctctg actttcaaga gctgggatta aagatacatg acactatatc 240

aaccaattca ccccaattta tttttcttat atatttaatt gtttccttcc cacttaaaaa 300

atcataacaa aataatagat ttcatgacac ttccaaagga atcattgtac tttgctaata 360

tttgctattt gttccagtaa tatggagact aaatttagcc actcactcct gnaccagtcc 420

taactttaaa tgtgtttgga ttaaacttgt aatcccaggt atttgggaat taggattgaa 480

aagggaggct aggggttggg gatttagctc agtggtagag cgcttgccta ggaaacacaa 540

ggccctgggt tcggtcccca gctccgaaaa aaagaaccaa aaaaaaaaaa aaagaaagan 600

aaaaaagaaa atgggaggtt acatagtaac ttcaaggcca acctagacgg gggggcccgg 660

tacccaattc gccctatagt gagtcgtatt acaattcact ggccgtcgtt ttacaacgtc 720

gtgactggga aaancctggc gttaccccaa cttaaatcgc cntggcagca natcccctt 780

tcgccagctg gcgtaaatag cgaagnaggg cccgcaccga tcggcccttc ccaaacagtt 840

gcgcancctg aaatggcgga atgggcaaat tggaagcggt aaanaatttn ggnaaaaatt 900

cgnggnaaaa ttttgggnaa aanncagccc aantttttta acccaanagg ggccggaaan 960

ccggggaaaa annccccta aaa 983

<210> 86
<211> 943
<212> DNA
<213> Rattus norvegicus

<400> 86
tcaangcgaa ataaccctca ctaaagggaa caaaagctgg agctccaccg cggtggcggc 60

cgctctagaa cnagtggatc ccccgggctg caggtttgca tgcccggcag gtgcagacac 120

acgaaggtct tcagctcgcc aatgagctgg gtagtctctt ccttgaaatt tctactagtg 180

aaaactacga agacgtctgc gatgtgtttc aacatctctg caaagaagtg agcaagctgc 240

acagccttag cggggagcgg aggagagcat ccatcatccc ccggccccga tcccccaaca 300

tgcaggacct gaagaggcgc ttcaggcaag ccctgtcctc caaagcaaaa gcagcctcca 360

ccctgggctg atccatcgca gacagactga catagtatta tcaataagca tttgtgctgc 420

cacaaagact ggtcctttcc tcctttaaaa catatccagg ggttggggat ttagctcagt 480

ggtagagcgc ttgcctagca agtgcaaggc cctgggttca gtccccagct ccaaaaaaaa 540

agaaccaaaa aaaaaanaaa aanaacnaga acaaaaaaac aaaaaaccat atccagagtt 600

tatttttata atggacttta ttgggctttc aagtgtatgt atatttctga aaaattcaaa 660

cagtggnttt ttttaatggg tttttntttt nattttatnt tantttacng naaaccgtta 720

gccactcttc cattaaaggc aaaaatggca anaccaaaaa angaaaagan ganannnnan 780

anannngngan nnaaaanana ananananaang aanaagaaaa aaancnccna gggggggggn 840

ccnagaaacc caattggccn nnaaaagggg gggaggattn aaanatncna ggngcngnag 900

gnttttaaaa aggcgnnagc caggggggaaa ncccaggggg gtc 943

<210> 87
<211> 939
<212> DNA
<213> Rattus norvegicus

<400> 87
cgtcaagcgn ngaaatnaac cctcacttaa agggaacaaa agctggagcn ccaccgcggt 60
ggcggccgct ctagaacnag tggatccccc gggctgcagg tgacgcaact ttgtcangaa 120
aacgaatgca gtcgctctcc ctgaataagt aancnggcct gtgggaggan atgccggggg 180
aactgggccg tgccgccagg anctctgcca tgtctcaccc actctgtgcc ctggcgcngc 240
tgcagcagcc cctacggcca ngagccccta cggcctgggg cctcctcttc atcttggcac 300
agaaattgtt caggggaagn ggaaggggct gggggagggg gcagctgcta tctttgagac 360
agaaagatgc aggcacagca tttcatacgt aaccatttga atgtttttga ctgtttttag 420
aattcgggcc ctggtggggt gggtggggtg cctgggaatg gcgtaaggag attccatttg 480
tccagtagat tgcacgttag tgtggggagg ggggtgtggt gccagcaggc agctgctgtg 540
ggagttgatg acaaccagcc cagatcatct gggtgctcac tcagaggggc tctccgggan 600
cctgtgcctc gnaagtccgt tccgatgaag cctctcctct ccactctgcc cccttcccac 660
ctacctggtc agggctagtg cccattttta accctaccca ttgancattt caagaaaacc 720
tctggttact gtgctcaccc agancaagac gtgctcctca aatncaactt gnatagntgg 780
gcagattaaa acaacattna tncanaaaag aaaannnana aagggggggg cccggaaacc 840
caaattnggc cctaataagn gaancnggaa taccaaattc aatnggccgg acgntnttaa 900

aaaacgnccg nganagggna aaaccctggn cgnaaaccn 939

<210> 88
<211> 1014
<212> DNA
<213> Rattus norvegicus

<400> 88
   nnctcaagca ngnaaatnaa ccctcactaa agggaacaaa agctggagct ccaccgcggt 60
   ggcggccgct ctagaactag tggatccccc gggctgcagg tcgagttttt tttttttttt 120
   tttttctttt ttttaagata atggttttta attgaattat tgagatgaag agacagtgaa 180
   gccctgtttg ctacttacat gaaaagattt taaaaacaat cacngcacaa aatacaaagg 240
   ggcagggtat gctgtggcat tgaatttcnc ctcacttttt ttcttgacgt ctcaagaaca 300
   aattaaagtt tccacagcaa atttgttctc aaaangccga atggtgaaac agttacgggc 360
   ttcacgcttc tgnaataccn ctaatggttt ccctgacgcn gcatttgtag gtttccttgt 420
   cgtgacacag tcggnaaatg aagaagccca gggggtccac gttttngang cggtcggtga 480
   tcaccatgtg ctcatggatg aggtatgacn gaggcaagta ggtcccggcc ttgatgtcaa 540
   taagaagctc caacagtttc ngggnggcat aacaanggca ggngtncana ggnatcaagn 600
   tnncacntga nccaanattn aagggcncaa ataagnaaan gaannntgca ngtnnaaann 660
   tcatncacaa tgnttggnca ggaaacgctn nnccgcaaan ctccagggna acaggntana 720
   cngnatgcaa ttacnacggg ncgnccatcc cacnaaagaa gcnaaagaaa nnctcnnnca 780
   aaatagttca ggganancga annancnngg ngagcanccg agaanntaag ngcaactnna 840
   nacanatatt gancgnnnca accnantgaa tgaaaaactg anannccnaa naannaggan 900
   nnnacaanca ancacanggn nnnaatgngn ngaantaana ncaatgaaga aggtgagang 960
   nnccgacncc angagaagga acgnaganac ggngntnnan aggggncaag attc 1014

<210> 89
<211> 955
<212> DNA
<213> Rattus norvegicus

<400> 89
accgcngaaa ctnaaccctc acntaaaggg aacaaaagct ggagcnccac cgcggtggcg 60

gccgctctag aacnagtgga tcccccgggc tgcagggctg tgcagcggcg gaagttatct 120

ctgcagggaa gatgcttccc ttgtcgctgc tgaagacagc ccagaatcac cccatgctgg 180

tggagctgaa gaatggggag acctacaacg ggcacctggt gagctgcgac aactggatga 240

acatcaacct tcgagaagtg atctgcacat cgagggacgg tgacaagttc tggaggatgc 300

ccgagtgcta catccgaggc agcaccatca agtacctgac gtatcccggc atgcagatca 360

ttgcacatgg tgagggcaag aggaccgcca agggccgagg gcgaggagga ccgncagcag 420

cagaagcagc cagaaaggcc gaggccatgg gtggcgctgg cagaggtgtg tttggtggcc 480

ggggccgggg tggcatccct ggtgcaggcc gaggccagcc ggnacaagaa gccagggcgg 540

ccaggcaggc aagcagtgca gcagtcccag cctgaactga gtccaggaag gtgggtgagg 600

agacctccgg gcgcctttgc gtgaagcccc acttggcgtc tgatccagtg aaatccctga 660

ctggccactt actcagtttc tggaagttcc cagtctgatt nactgttaag ccttggatgt 720

cctttgaaag gctggcttct tccaggcttg tttgagtttn atgttggagc tgccagctcc 780

gcacaatggg tggtttanct gtcctttccc aagcccccac cccctaagtt tttctggttg 840

gaaaaaaaat taaaggcaaa ccaaccaaca ggaaaaaana anaaanggng gnnntngcag 900

nnanaaanng nnnnnannnn anannnnnnn nnngangaan nnnnnnnngn annng 955

<210> 90
<211> 964
<212> DNA
<213> Rattus norvegicus

<400> 90
cgttaagcgn tgaaatnanc cctcacgtaa agggaacaaa atctggagct cctccgcggt 60

ggcggccgct ctagaactag tggatccccc gggctgcagg acgcgctcag ccacgtttgg 120

acacgggact gacgcaacac acgtgtaact gtcagccggg ccctgagtaa tcacttaaag 180

atgttcctgc ggggttgttg ctgttgatgt ncntgttttt gtttttgtt ttttgttttt 240

tttttggtct tattattttt ttgtattata taaaaaagtt ctatttctat gagaaaagag 300

gcgtatgtat atttgagaa cctttttccgt ttcgagcatt aaagtgaaga cattttaata 360

aacttttttg gagaatgttt aaaaaaaaaa aaaaaaaaaa aaactcgagg gggggcccgg 420

tacccaattc gccctatagt gagtcgtatt acaattcacn ggccgtcgtt ttacaacgtc 480

gtgacnggga aaaccctggc gttacccaac ttaatcgcct tgcagcacat ccccctttcg 540

ccagctggcg taatagcgaa gaggcccgca ccgnatngcc cttcccaaca gttgcgcanc 600

tgnaatggcg aatggncaaa ttgtaagcgt taaatatttt gttaaaaatt cggcgntaaa 660

ttttngtnaa atcagctcca gtttttaacc caanaggncc gaaattcggc aaaatccctt 720

ataaatccaa aagaaataga ccgagatagg gttgantgnt gntccagttt ggaacaagag 780

tccacctaat taaagaacgt ggactccaac gtccaaaggg cgaaaaaacc ggnctnaatc 840

caggggcgaa gggcccacta ngggaaacca tcancctaaa ncaaggtttt tnggggggnc 900

naaggngccg ntaaaggcnc ctaaaatccg ggaancccna aannggggan ncccccnaat 960

ttta 964

<210> 91
<211> 945
<212> DNA
<213> Rattus norvegicus

<400> 91
   aagntcgaaa tnaaccctca ctaaagggaa caaaagctgg agctccaccg cggtggcggc 60
   cgctctagaa cnagtggatc ccccgggctg caggaattcg gcacgaggtt tacgcggccg 120
   ccttgcgcgg tttgcgaacc cggggaaacc tatcctgaaa cccaacaagc ctcttatctt 180
   agctaatcgc gttgggaacc gacgccgaga gaagggcgtt cttcccctcc agaggcaact 240
   tgtatcacgg agatgtcaat gatgatggct tgctggaagc agaatgaatt ccgcgacgag 300
   gcgtgcagga aagagatcca ggacttcttc gattgttctt ccaaggctca ggaagctggg 360
   aagatgagat caatccagga gactctggga catctggaag tttaccnccc cacaaaatgn 420
   actaagttgt tacagagatt tcccaataaa tctcatctga gctgaaaatg gagaaacatt 480
   ttcaacgaac tctcatttct gaaagctaca cagaggcgta ttagggatgt ttgcatgnca 540
   ttgccatgcg tttttgaagg gtaaaatgag gcaaaacact caattttgct cttctgaatg 600
   aatcgtgttc tggatacgtg tcttgaaata aaaccctcta aaaaaaaaan gaaaaaaaac 660
   ncgagggggg gcccggtnac ccaattcggc cctatagtga gtcgtattac aattcacngg 720
   ccgtcgtttt anaacggtcg tgactgggga aaaccctggc gttacccaac ttaatcgcct 780
   tgcangcana tccccctttc ggccagcggg ggtaatagcg gaanaggccc gcaccggatt 840
   ggcccttccc caaaagttgn cgcagctgaa atgggcgaat gggaaatttg gaaggggtna 900
   anaattnggt naaaaattcc ggggttaaaa tttttnggnn aaant 945

<210> 92
<211> 968
<212> DNA
<213> Rattus norvegicus

<400> 92
aagcgcgcaa attaaccctc actaaaggga acaaaagctg gagctccacc gcggtggcgg 60

ccgctctaga acnagtggat cccccgggct gcaggattcg gcacgagctg gatcccaagt 120

tcctggtgaa cttggaccct tctcactgca gcaacaacgg tactgtccac ctgatctgca 180

agctggatga caaggacctc cctagtgtgc caccactgga gctcagtgta cctgctgact 240

accctgccca gagcccgatg tgggtcgacc gtcagtggca atatgatgcc aaccccttcc 300

tgcagtcagt gcaccggtgc atgacctcca ggctgctgca gctccctgac aagcactcag 360

tcacagccct ggctcaacac ctgggcccag agcatccacc aggcctgcct ctcagctgcc 420

tagcaaactt ggaacttcag ggacggccag cagcccttct ggctgagggt ctcataccac 480

ctaccaaacg tcactaggtg ttggcttctt agagggccgg ggctaggtta cctttcctgc 540

ttttaccttc tgccttggag acctgcccgc tctccccatc ttgtgcagta ttgaccaggc 600

agctgtggag ctggctgcat gaggctgggg gtgttcccac aaggttttcc attgtcgttt 660

tcccccagag tcagtcccca cacttctaca gcctttctgg gcttccatgt ccactcagca 720

gcatgagaac tcagggtccc atcaaagcat ctctgtgtta aaaccccatt gtgctcataa 780

tctggagaat gtgggaggac acagggaaan ccttcaccat acatacgggn tctccagtca 840

aaangggggt tcaggctggt gcggcctaaa gggaatgcgg aaaanggtgc angnattcag 900

nctggaaatt aagggggaaa ggattttaag gcntgggaag aaaggggcaa gtaagggaat 960

tcaggagg 968

<210> 93
<211> 958
<212> DNA
<213> Rattus norvegicus

<400> 93
aagcgcagaa attaaccctc acgtaaaggg aacaaaagct ggagctccac cgcggtggcg 60

gccgctctag aactagtgga tcccccgggc tgcaggaatt cggcacgagg cccttcaaat 120

ttttactaag actgtgcgtt ccaaccatga aatgtaggga gtcaagagct atctcactga 180

ggacagggtt tgtttggatg ctgggttcct cacaagatgg gtgatatgtt taacagtgga 240

gttctgtaaa gtcaccagat gtaactgtaa accacactgt gtcacaaaag gctcacagca 300

cagcatgtgt gggcactcag ggtcagtcgg ggtgagaaag ggccagctcc tgtgtggtgt 360

ggctgttaga gcaacctgtt gacctggggg cagaagtgac cagggcagaa tgaaagcgta 420

cagactggga ggataagggc tagtgctgtc ttgagggacc aggacccaag ctctccctca 480

gctgtagact agtttggtga agctggtgtc agcgattaca tccatgtcat gattctcgat 540

ccagagacaa tggccccgat gggatggagc cggaagcgtn catcgagagt aactggaatg 600

agattgtgga tagcttcgat gacatgaatc tctcagaatc cctcctccgt ggtatttatg 660

cctatggttt tgagaagccc tctgccatcc agcagcggag ctattcttcc ttgtatcaag 720

ggttatgatg tgattggctc aagcccagtc tggggactgg gaaaacagct acatttgccc 780

atatccattc tgcagcagat tgaaattaga tctaaaaggc cantcaggct ttggttctgg 840

ganccacncg tggaattggg ccagcagatt caaaangggg gtaatggcac tggggagact 900

aaatgggngn cccctggcca tgncnggaat tgggggggac caaacggtgc gtgnctnt 958

<210> 94
<211> 989
<212> DNA
<213> Rattus norvegicus

<400> 94
   aagcgcgaaa ttaaccctca ctaaagggaa caaaagctgg agctccaccg cggtggcggc 60
   cgctctagaa ctagtggatc ccccgggctg caggtgatga ggggtgagca atgttactaa 120
   aggaaattgg taaaatggca gctacattgg ctgggtgtcc tctgatagtg tcctggaagg 180
   ggtgttttgg attcgcatat actcttcacc cactattttg aaaatttgta ataacaccca 240
   aaaacgtaaa agttcacgcg attctcttct gctagaaaag attgcagatt ggctatgcac 300
   atagagtgtg tgactagaag tggaaatgct tggaaaggaa aaagagccag ggggtgaaca 360
   aggcttggtg aatgagactg gtaatgtacc ccatggaagg gaaagggaaa gaaatagact 420
   ggaagggaaa gtgcagccct gccctgctcc ctgtgctttc actaccaggt gcccaaatgc 480
   ctcagggaga caaggggctg ggaagagcag gaggcaacac aggcacaaaa ggatgatctg 540
   tgagtgagtg cactcccaca agattttctt aggcctgcag aaacgcatgc atccttccca 600
   gtgtctctat agcatgtgcc tgctactgat gctattcctg acagtgagct cacctggtga 660
   ctggaggaaa tgccagattt tgaagcattt gacgaatctg ttgccttgta tcattacatt 720
   tncccatant aatgnaacac taaataacta aggccacaga atgaggtgat nccacaagat 780
   tagatgggac cgggttgatg gtccanctgg gncaaccaat ggccaatggg gttttccang 840
   gtgggaggcc ttnaagnnta cagganccag gccccngtaa attaaccaag tggggaataa 900
   cccggntacc ggaagaccga gcctgcnttg ggngaanggg gganccangg cnggnccntt 960
   anggggggaa taaaancccg aannccngg 989

<210> 95
<211> 998
<212> DNA
<213> Rattus norvegicus

<400> 95
caagcgcgaa attaaccctc actaaaggga acaaaagctg gagctccacc gcggtggcgg 60

ccgctctaga actagtggat cccccgggct gcaggaattc ggcacgagat atgctgctgc 120

caaggaagct gcaagtcgaa gcaaggactc tgtaacccac gagatccaaa tgctgcttgc 180

atttcacagt agttaaccat gttaaagaga gaatgcttta aaaatagact gttttaaagc 240

ccgccgtgcg cactcatctt gatgttacta aagactgtgt tccaaacgtc tgcgtgcggt 300

aaacccggcg tgctatccta gcctatacgt catcacagga cttttaagtt cattccagat 360

catcgtatct ttaatagaat aatagtattt aatttcagta cagaaaattc tctgggctgt 420

acactttcag aaaaattctc ctcagtgtac acttcagaaa aattctcctc agtgtcttaa 480

ccttatttag tattatttga ctctaaactt ccgtttacct cttgccattc caacacgtta 540

cagagaaagt catctctgcc actgtttatc ctgggggtca tctgggttcc tcctcaagac 600

gcagctcctg catcaagttg tgtttggntc ataaagtcct ttgcctataa tcataagggn 660

acaccagagt gaacagggaa tgttgcaagg gtgtaatctc atgcaaagag tagcancggt 720

ggcattccct tcctccctgc gggaaaatan ggcangaacc antttccntc tggtagacct 780

ctggccccac ggacaggcta gggattggcc caagcggcaa aaggccaggg natatggagg 840

ctggtaactc ccnggctggt aancccnaag ggggggnacc ncccccggtg gganggtnca 900

nctggnggcc cagnggggta aagntgggnn ccnggttnaa accaacccac caggaaaggc 960

ccctggatga aagcccaaaa gggnaaccca aggngggg 998

<210> 96
<211> 986
<212> DNA
<213> Rattus norvegicus

<400> 96
aagcgcggaa attaaccctc acgtaaaggg aacaaaagct ggagctccac cgcggtggcg 60

gccgctctag aactagtgga tcccccgggc tgcaggcagc ctcagtcgca gccgggcctc 120

gctcctcaac ttggcaaaaa tgcctacaga gactgagaga tgcatcgagt ccctgattgc 180

tgttttccag aagtacagtg ggaaggatgg aaatagctgt catctctcca aaactgagtt 240

cctttccttc atgaacacgg agctggccgc cttcacgaag aaccagaagg accccggtgt 300

cctcgaccgc atgatgaaga agctggacct caacagtgat gggcagctag atttccaaga 360

gtttctcaac cttattggtg gcttagctat agcatggcca tgagtccttc ctccagactt 420

cccagaagcg tatctaaccc tctccattcc cttccagcca ccaagtcatc gcctcctcca 480

ctccttcccc catccacacc tggcactgga gcccaccaca cctaccacac atgcagccca 540

ngcctgacag ggaaaataaa acaatgtcat ttttttaaat gtaaaaaaaa aaaaaaaaaa 600

ctcgaggggg ggcccggtac ccaattcgcc ctatagtgag tcgtattaca attcactggc 660

cgtcgtttta caangtcgtg antgggaaaa ccctggggtt acccaactta atcggccttg 720

caggaaatcc ccctttcggc agatggggta atagcgaaga ggcccggaac ggttggcctt 780

tccaaaagtt gggagctgga atgggggaat ggnaaattgg aaggnggnaa anaattttgt 840

taaaattcgg nggtaaaant ttggnaaaat caggcccatt ttttaaacca atnagggngg 900

gaaaatnggg gaaaaanccc cttanaaaan ncnaaangga ntaggcccgn ggaananggg 960

ttnaagnggt tggttcccaa gttttg 986

<210> 97
<211> 1006
<212> DNA
<213> Rattus norvegicus

<400> 97

aagcgcngaa attaaccctc acgtaaaggg aacaaaagct ggagctccac cgcggtggcg 60
gccgctctag aactagtgga tcccccgggc tgcagggcat acattaatat tttattccaa 120
agagaaatct ttggttagaa agacatacaa aaatggaagc taacactgtg tctgtctgtc 180
cccccacact tccagcgtat gcagtgttaa gagatatcct ggcacttgta tctttgggat 240
tccattttgg ttctcgaaca ttgggaaaaa caaatggctt ggttctgtgt gattaggcca 300
aggttgggga agctagacac ctgccattcc acagataact tgctgaacgt ctacactctg 360
ttttccttgc agtaatactg tttcctgcca tctccccggc ttctggccac cctggcaata 420
gcacccttgg cctctagagc cattggtcca gagtatgcat ggcacacctg atggcacaga 480
ggtgcccaga tatgtcctcc caccttccat atctaccacg gaggatgcca tcacgccata 540
aaatctggaa cccagtgatg aatacattta catgttaaaa aaacagccac ttggtaaaaa 600
tcagatctta cttaggataa aggaattctg ggctttcata gaagcttcgg tagttcaggg 660
aagaaaacgc cngggaggaa gatcattcag ncaagctgtt tgtagggtta ggaaaaggga 720
agtaaaaaac actatctnaa gtctgtctgg gtcactttca ttgaaacacg tttctggcan 780
atttccctca aagggactct aactggaggc ctctgggcag actctggcat cggaccctca 840
aggtgggagg acctgaccna agaatctgtg gtantttagg ggaggttttg gtncaaaaaa 900
caattttta aactggcang naatttaaaa aaaanatcna tnanttnncg ngggnctaaa 960

aatgggcnaa ttccaaaang gatgggaatt nggccnaatg gttngg 1006

<210> 98
<211> 978
<212> DNA
<213> Rattus norvegicus

<400> 98
cgcaagcgcn gaaattaacc ctcactaaag ggaacaaaag ctggagctcc accgcggtgg 60

cggccgctct agaactagtg gatcccccgg gctgcaggaa ttcggcacga gattactttg 120

ggctcataat agccaacaaa ataaatgtag aggccccatt taaaaaagaa agggaggggt 180

ggagtgggag agagggtaat ggaaacaaaa caaagaatca agtcttatga ctaaggaca 240

caagaaaaag attgaatgag cagatggaaa tattaccacc taggtgtaaa cttatgtgtc 300

tatggatgta ggttcttcac acacttaaag tacgatgcat ctctaatgat actaaatatg 360

tatttatagg actttagtgt ggagggagga cacagcaggt gattcatctt gaataataaa 420

acaaaaatag ccctaaatat ttacctggaa agtcatgcaa tgaaaaggaa ttagttttac 480

tgaatgttaa agttttttac ttatctgtga aacagtagga atattaaaca ccaatacatg 540

attttnttct caaagacatt tttttaagtc atgcctggtg gtgcatgcct gtaatcccag 600

cccttaggag gctggggcag gtttgctgtg aatttaatgt cagcttgtgc tatagaacaa 660

gttccaggct aaatgtagac nacagagtta gaaaacctat ctnaaaaaaa aaaantcaca 720

caaacataca cccaaaaaaa aaattgttta gnatagtaca cgttcacttc acgtgtgtgg 780

ttagggcccg aacaacctcc agcngntggt tncccaggga gttggtanct tttaagggta 840

aggaacccca ccagccngga accnctaagg aagggcngna gcngggtcaa cnagccaagn 900

cccntanggg gctgnccgga ccccaaggnn cngggggatg naaaaaggta anaaggnggn 960

ggaccaannc cncanccc 978

<210> 99
<211> 988
<212> DNA
<213> Rattus norvegicus

<400> 99
aagcgcgaaa tnaaccctca ctaaagggaa caaaagctgg agcnccaccg cggtggcggc 60

cgctctagaa cnagtggatc ccccgggctg caggccctgt ttgtggtgac nggtcatgaa 120

gtaagtcacc aggatgaccc tgtctttcca attgcccacc acctgtggct cttttnnncc 180

ccccnnnncc cccatatttc ccttccctga cacctcctgt cccaacctct ctaacccccc 240

tggggcattt tctggcttgc ctggatagtt ttagagaccn gcttgttggc tataatgtct 300

tttcattcat tcattcttct ttttcttttt ttaaaaaaac aaaatgaaac aacaaaacca 360

aaaagtatcc agaaaaaaaa aaaaaaaaac nncgaggggg ggcccggtac ccaattcgcc 420

ctatagtgag tcgtattaca attcacnggc cgtcgtttta caacgtcgtg actgggaaaa 480

ccctggcgtt anccaactta atcgccttgc agcacatccc cctttcgcna gctggcgtaa 540

tagcggaaga ggcccgcacc gatcgccctt cccaanagtt gcgcagnctg naatggcgaa 600

tggcaaattg taagcgttaa tattttgtta aaattcgngt taaatttgt taaatcagct 660

catttttaa ccaataggng aaatgggnaa aatcccttat gaaatcaaaa gatagaccgn 720

aganagggtn gagtgttggn tcccantttg ggaaaagaa gtccacnatt aaagaacgtg 780

ggacnccaaa gtcaaagggg ggaaaaaaac ggtntaatca ggggngatgg gccactaagg 840

ggaaccaatc aacccnaaat caagtttttt nggggggcga agggngccng naaaangcac 900

taaaancggg gaaaccccta aaangggnng ccccccgnat tttaagaaga ntganggggg 960

aaaanccggg ggaaangngg gngaagaa 988

<210> 100
<211> 971
<212> DNA
<213> Rattus norvegicus

<400> 100
aagcgcggaa attaaccctc acgtaaaggg aacaaaagct ggagctccac cgcggtggcg 60

gccgctctag aactagtgga tccaccgggc tgcaggttcc ccccctgttt gggtgaaagt 120

ggttctagaa cctgcactga atagtagtaa agcaataagg cccgattcat cccacagcac 180

tgatcatctt tcaatgcccc accccaagcg aacggtaaga aggcctctct taagaagggg 240

agacagatgg ccctaactac tcagtgacag aggcagttac tgtgagagac ttgtaggaat 300

ccttttcttt cctagcgaag tcaaagctct ctctgaatgt actgtgtgac aatgcatcat 360

ggcatgaacc ttcggtcagg gacgtcattg gggaagtgac ttcaaaagta ttcaaaattt 420

gacatgctgt ttgtttagtc actacagtgc cctcaaaggg cagacattgc agccttttta 480

tattgcctgc caaaatttga agtattagaa caaagtgtgc catgagagaa aaacttaaca 540

aggagttttg aaaagtaatg caaagaacaa aactacaaca ctatttttaa aaagttgagt 600

atctgagtta aaattttcaa atctttattt tacaccactt aaaattatac gagaacaagg 660

tacatgcatt atgtgtcaca ttactgggca aactgttcaa atattttttt taaaccnccc 720

tgtatagaaa atatcattaa gggatgtaaa agccatgctt gcctatttgc ngtatacatg 780

taatgaaatt ggtagataaa aggggtagtg cattggaaac caaatggaac aaaaaagtag 840

ntacttttac tataccaagg gtgccnggtg caggaaaaaa atataanana anttngtggg 900

naatggnagc antttaaaac cnttccaagg gggtataaaa aaaaaaaggg ggggggcccg 960

ggaccccaaa n 971

<210> 101
<211> 1006
<212> DNA
<213> Rattus norvegicus

<400> 101
agcgcgaaat naaccctcac gtaaagggaa caaaagctgg agctccaccg ccgtggcggn 60

cgctctagaa cnagtggatc ccccgggctg caggcttaaa tagaattcaa ttctaaaatc 120

ctaaaaccca tctccgggtt accaaagaat gatcttacag gtcctgtcag tgttctctgt 180

gtgatggttc tgagaattaa aacaactata gtcctctgtg attcatggct tctctccatg 240

ctatcaagac tatctgaaga ggttatagaa atccttggcc ggtctgcacc tcacctgccc 300

tccaggacac atgtgatgtg tgcatccaga tgaccaaaaa cagaccctta gggcttcgat 360

aagggtttac aatagtttga agtgctttcg ctcacagcta gggaaccttt acaaaccaag 420

ctagcaattt aacnccttgc tcttcatggg gacacatctt gggcttgaag tcagcagtag 480

agtcaggaga aagttcccaa agatgtttga gcaattcaat ttcatcccng ctcccattga 540

tttagacttc aggtttcgag gggagcctac taaagtatac attaaggata atttgcttag 600

ggcatatcna atngtggttg tccgagtaga tgaacgcaga gtctctcaga agtccacacc 660

catcacagca acaagtaaga agctcataac agaggaacat ctactattng ctttgtntta 720

aaaaaccccc cagatggcna aancatcctt tctgtttcca nncngntncg cctncaagcn 780

ctggngtggt gnggtgnntg tggtgngngn gngatgagng agtgtngtgg tggtgnggng 840

nngngngtnt gnnggggggg gannggnaag caggtccagg gnagaatncc acaaaggggg 900

caagaggggg anggggtcca gnanaanccc cggaaanggg ggagannacn cctggggagg 960

ggnnaaccaa caanttcccn aanggnnntt nnacccagna aanana 1006

<210> 102
<211> 968
<212> DNA
<213> Rattus norvegicus

<400> 102
agcgcggaaa ttaaccctca cgtaaaggga acaaaagctg gagctccacc gcggtggcgg 60

ccgctctaga acnagtggat cccccgggct gcaggaattc ggcacgaggt cccctggagc 120

tgagtccctc agagtggggt cttcagtagg gtctcctaca gtcagagagg ggcctgagga 180

tggaccagac agcacaataa gtgaggctgc cactttacct tggggaactg acccccatcc 240

cagcgcccca cttccggatc ccccgggctg gcgagatatt gggccagagc tcctagagtc 300

agaagcacct attaagtcgg aggaaccact caaagaggat gccaacctgc tccctgagaa 360

gacagttagg gnccttcgtg ncccattgac ctacagtggc atcgagcggn aagcctncag 420

gaggagcgca ttcaagcatc gtgagggccg gaccaggcga gctcaggaat ttcttgccag 480

cccgactcag ccaccctgag cccccagagc gcaatggggc tgaggcagtt gtgagacccc 540

ctggccggtc ctgcgggggc tgtggaagct gtggaggccg tgagcactga gagctgtagc 600

ctcggtggtg gctgccctca tcttcttccc ctgcctgctg tatggagcat acgccttcct 660

gcctttcgat gntccaaggc tgcccaccat gagctcccgc ttggtctata ccctccgctg 720

tggggtcttt gcnaacttcc ccatcgtgct ggggctcctc gtgtangggc tgaagctatt 780

gtgcttttnn gccctttggc cctttggaag agccgagaag ggaagtagaa gattccacgg 840

gcagtangtg ggcccaagtt ctgtgcaggt tnttnaannc tctantttct ttaaacctgg 900

gctgtggctt ttcaacctaa nntggnccca aggaanaang gtnaaaggnt ggancccccc 960

ttggnnaa 968

<210> 103
<211> 1033
<212> DNA
<213> Rattus norvegicus

<400> 103
aagcgcgaaa ttaaccctca ctaaagggaa caaaagctgg agctccaccg cggtggcggc 60

cgctctagaa cnagtggatc ccccgggctg cagggctggg cttgccttct gtggtaatac 120

atgacagagg cattagactt ttttagctgc cctaataagt atatgatgaa taaataaatg 180

ccaataccca taacaatttt tctagtgggg acaaaggtca tttgattaat ctaggttttc 240

ataccgaccc gattattaaa tgcacttaaa aaaaaacngc atttaaagca ttggccttag 300

cagaattaac tgacttagtt ccttacnggt gagtgaattc agttccctac caactaatgc 360

gcagaatcta agaatcccat cctgcacaca ttggttggga gccctgagtg gagtatcagc 420

agcaccaact ggataagagg cacaagagaa gtggggaaca tctggagtcc tgttggacgt 480

ggacagtgtt tcttggatat gtatgccaca gtgccttggt ggccagcttg gagtcctacc 540

tactcagcct ttgtccccac ncctccngtg ttagcttnca tcttgcatat tcnatttttt 600

ncnactttgt taaccnactt tatgncntta nctcaataaa aangcntacc aagggnaaaa 660

aaaaanaaan aaaaaaaaag gggcccggna cccaatnncg ccctanagtg gaggcngnaa 720

tnancaantc cacggggncg gtcgttttaa ccaacggtcg tnaaggnggg aaaancnctg 780

gncggntanc caaanntaaa nncggccttg gcaagcanca aaacccncct atnacggaca 840

agaangggcg ngtaataaga cgnaaaaggg ncccgacaac cgnattaagc cccgtncncc 900

aaaaaanttt ggggagccct ggaaannngg cgaaanggga caaatnnnta ananggntaa 960

anaaanttgg ganaaaaagt cgcggtgnaa aaanatnnng gaaanaactg gcaccgnttt 1020

ganaccaaaa agg 1033

<210> 104
<211> 1011
<212> DNA
<213> Rattus norvegicus

<400> 104
agcgcggaaa ttaaccctca ctaaagggaa caaaagctgg agctccaccg cggtggcggc 60

cgctctagaa ctagtggatc ccccgggctg caggtcagaa gcaaagccac tgtggcagag 120

aggaagcccc tctgcccctc cgcccccctg cccctccatc cctccgctgg tgtttctggg 180

gattattcac tctccttttc ccttcacaag ggccttctct gcaggagcga tagagaatgc 240

atgtctgccc cattgggcct tttggtctgg gatncctcca accacatgac ctatacccca 300

agcccgcctc tccatgcgct tcgccccctg gatgcactaa gagttgctct cgtttgttcc 360

tggtctggat ggcaaaacaa ggagatggtt atttaaagag aattcctatt tatttggaca 420

caaaaagtcc agttaatata ttaatgtgaa ataaaccctg tttggcacct tgaaaaaaaa 480

aaaaaaaaaa cncgaggggg ggcccggtac ccaattcgcc ctatagtgag tcgtattaca 540

attcactggc cgtcgtttta caacgtcgtg actgggaaaa ccctggcgtt acccaactta 600

atcgccttga agcanatccc cctttcgcca gctggcgtaa atagcgaana ggcccggnac 660

cgattcgnaa ttcccaanag ttgcgcanct ggaatggcga atgggcaaat tggtaagcgg 720

tnaaanaatt tgttaaaaaa ttcggggttt aaattttttg gttaaatcca gnnccatttt 780

tttaaaccaa tangggcgga aaatcgggna aaaatcccct taataaaatc aaaaaggaat 840

tangnnccgg agnatnaggg ggttgagtgg tngttcccan tttgggaaca angagnccca 900

cctaattaaa ngnanggngg gnnnnccaaa agggcaaaan ggggggaaaa accgggctna 960

tnaagggggg natgggccca ataanggngg aanccaatna ancccnnaat g 1011

<210> 105
<211> 1013
<212> DNA
<213> Rattus norvegicus

<400> 105
ctcaagcgnt gaaatnaacc ctcactaaag ggaacaaaag ctggagctcc accgcggtgg 60

cggccgctct agaacnagtg gatcccccgg gctgcaggcg gcagcgagga aaagcctggc 120

tgggcccagg tttcatgtgg ngggggaggg ggcaagcctt gcgatcagct ctgtaataag 180

cgtagcaccg gggaattcaa gaggccctga gaagcctgga gtcccaggac gtcttacttc 240

ttttcctaca catgtctctg agccatgttt ttgcttaaat tctctctcaa gaaagacaca 300

cactaaaagt gaaatctaac agccgcaaaa ggttactttt ttttgtttag atggttttgt 360

ctgatttcat tttgncctgg aaagttctga aataagaatc aaagtattta aagtctcgcg 420

acacgacagt gttnacagga ctggctgtaa ccgtgttatg taatcagagc gctccaacaa 480

gggaaatctg gtaggattcc attggaacgg gtattggaga gctgaaccag gcaggtggtg 540

gtaaggggtt gccctaagag tctgttacac aatgcggtgt catgggaatt tctcagagcc 600

atgggaactc tcaggaaggc aaagagatat tctaacctgg agagcacagg gtccccaggt 660

ccgggtctaa ggctggactt gtgatgcaca ggtggntatt tccagaacct tagggaatta 720

acagtttcca cctggcaanc agcntgcctg nacnaaatct atnggattnn cnaagcngaa 780

ttaatcccag tccaagggaa gcaagcccaa tcnggnnnnc ttnccggggg cagnaancta 840

atggggcgga ngaaaagaag gnngaaaanc acaaaggccc caaaaaggaa tgggncttgg 900

ggtaaaggnn ggaacccagg ccaangaatc gnncantgaa aaatggggaa aaaangggng 960

gngntggtga acgaggatat ggaaatncnn acatgaaagt ttaaaganca atc 1013

<210> 106
<211> 989
<212> DNA
<213> Rattus norvegicus

<400> 106
aagcgcggaa attaaccctc acgtaaaggg aacaaaagct ggagctccac cgcggtggcg 60

gccgctctag aactagtgga tcccccgggc tgcaggccac tgcaaggcgt cagacagaac 120

tagtatcttc acacttgaca aggagncaca ctgtcactaa cagtgttgtg tggccgccct 180

gtgggtcagt gctccggcac acagggctcc ctggaaagcg atggagatga aggagctgga 240

gaacatctct gtcccggtga gcggctgggc tgtcacctct gctgagcacg gtgctttgct 300

cacacggctt accgagatgt tacattcaca cccagtgaac ctgggcttta tgagtatttt 360

tatgaatgct gattgcgatt atgnattttt gttttgacct ttatgtgtgt gtccctgtga 420

gttgtgggcg cctgcaagga tcagaaaatg gtatacgatt cactggatag agttccgggt 480

ggtcgagggc ctcctggaag tgggaaccaa actcaggcag aagaaccatc ctcaagagcg 540

ctcagcctcc gagccaactc cccagccccc ttggttgtta tgtttccaac acacagctca 600

atggattaga atccagggga aaataggaaa caatctctgg actctggggg actgaaaagt 660

ctattaagga tcttgtgatt cttttggacc atcagaaatc gtctttccta aaatagacat 720

ggatgttctg gttaaaataa aaacaacata tcagaaaaaa aaaaaaannn nnnngngana 780

aaaaaaancg cnnggggggg ccccggnacc caaattcgcc ctaaagngga ancgnattaa 840

caattcantg ggccgccggt ttnanaacgn cgtgacctgg ggaaaaancc ctgggggta 900

cccccaactt taaancgccn tggaagnaaa attcccccct tttcggccaa gnntggggn 960

aaaaggcgaa aaaggccccg caccggntc 989

<210> 107
<211> 1020
<212> DNA
<213> Rattus norvegicus

<400> 107
ctcaaggcga aataaaccct cacnaaaggg aacaaaagct ggagctccac cgcggtggcg 60

gacgctctag aacnagtgga tcccccgggc tgcaggggct gcggagaagg cagcgccgga 120

cccagcggtg cggtacggca accacggccg cacgggacct cgggccggag anccccggcg 180

gcggccgccc ctcgcgccgg ggccgttagt cagcagaacg cgaacgcgcg tgaaggggcc 240

acacgaancg cgcccgggca ccgcagctct cattcgcgtt ctcgcctggc cgggccgcgc 300

gcggagtcgg cgggcggcgc cccgcggagg aacagcgcgt tggacggccg cggcccccag 360

gctgcagaag atgaataatc tttcatttag tggagctatg ttgcctcttc tgctgtgcca 420

ccttgaccca aggaaaatcg ttcaaaacta agcattctng ncacctgatc caacttacac 480

actgatgtgt gatgagagtg gaagccgctg nacctttana cctgtcagaa ngancggant 540

ggnagtantn gtncaganag aaagatggta ttggagtgtt ntcatgnacg agaaancagt 600

aanagnnaac agaattggct tgtangtttg ttgcggtgnt naccaaatgn tnataatatg 660

accttnacng ntcttcagag aggaatgacn gntgaatctn gggttcagaa tgannggagt 720

tttttaacat anggnactgg ggnggtcccg ggaatgaaat ntgtgaagta aatatnncng 780

cnatgannga atnnactnnn gggnatgagg gagggctaag ttcgtgngta aagnncanac 840

naagaganag tnaanntggn nagtgncaag ancaagtaga aancgngacn gngngggana 900

gcgtnnanng tggacanaag nantacaggg gaannnngga cnatngnaaa anggcgaaat 960

ngaanatatg aggacaatga agnagngang gggganc ggn aacangggng ntgngtnnnc 1020

<210> 108
<211> 917
<212> DNA
<213> Rattus norvegicus

<400> 108
gcaagctcga aattaaccct cactaaaggg aaaaaagctg gagctccacc gcggtggcgg 60

ccgctctaga actagtggat cccccgggct gcaggtcaaa actcagttct agaaggacct 120

tgacaaacag acatacttac ctcctggtta gtggaactct gggacaatgt cctttgtgat 180

ggtgttttca ggagtcctta ggttatgttg agaaaactca gtgtaggcca aaccaaaact 240

gatggttctg tttttgtaat tgtctctttc atgtcctatt atactctgta ttaaaaaaca 300

gggttaaaag gccatagtcc atttcctaaa atgtaatgcg caccttgtat ctgtgaggtg 360

gtctgttggt gtttttatcc gaataggttg tcaggtgaat atgacccctt cccgttaccc 420

agagggtata ttctccaggt ggtcagagtg caggttattt cttacacaga gaacctacct 480

tccctccata tgattcttga cctctgccct catctcccat ttaataattt aaacaaatct 540

gaggccaggt gttgtggcac agtggcatac acctgcaaat ctgcacatgg gaagttaaag 600

caggaggatc aggaatcggg gatagccttg gctacagaga gagttcaaat ccagtctggt 660

atacatggag tcccagcctc aaaaccaaac acaggggcgt ctggaaggaa gaactctccg 720

gctggtgctg ttcagggggc actggcagag tgtctgtgcc cagtcaaagg tggttaaccc 780

agtgaacaga taanctggaa atggacagtg ttggataggg aaataagggt ctggtgccac 840

catgggagtc aagtcanttg accaattcct ggacccaaag cgaaggcttg antaanacca 900

ccggtggagg cctacgg 917

<210> 109
<211> 895
<212> DNA
<213> Rattus norvegicus

<400> 109
gcaagcgcgg aaattaaccc tcactaaagg gaacaaaagc tggagctcca ccgcggtggc 60

ggccgctcta gaactagtgg atcccccggg ctgcaggaag gtgggccact ctctgtccac 120

attgccttta ttttcttgga ttgaacgcat aacgtggccc ctcacattct ccaggacctg 180

accagctgtg ggccactgac tgcttgcaaa cccggactgt gctaagttac tagcgtgtag 240

cccttgggga cccacctggc ccatctggac acatctcaag gctccagcga ggatggatgt 300

aaaaatattt ccttgcttgc atccagattg ctcatggata cggggctgaa ggcagaagca 360

gctgtctggg tacgacatgg agggggagct gggtcctgct ggccgggata gctcagctgt 420

ggactttggt ctctggagtg gatgtcctgg tcatgttagc aaacattcac tgccctttct 480

cagtgccctc gctctctcgc ctccacgtta ctcccgcgct actcttgccg tttctcgccc 540

gcgtttctga gcacaccagg tcctgcctgg agtcttggtg tcgcggatga ctgactgaag 600

gggcctttga gagctgatgg gttctgccat ggactcctcc cggtgattag caatgactgg 660

ggccttaccc acccacctac cctcgtaatg aagttctgtg gagtggctgg acaggtttga 720

gggaaggtgg aggtggttta aactggtttg gggagtgcta gggctgggga cccagaagca 780

agcccagggt gtccccaacc ctttcccgca nggtcttgct aaatgttctg atctctgtaa 840

aaccccntcc ctctttcaga aggancctgg ggtggggccc ctctgaaatt cccna 895

<210> 110
<211> 901
<212> DNA
<213> Rattus norvegicus

<400> 110
aagcgcgaaa ttaaccctca ctaaagggaa caaaagctgg agctccaccg cggtggcggc 60
cgctctagaa ctagtggatc ccccgggctg caggtttata tttaaaaaca attgtccctt 120
aattcatggg tacaccttgc agttgtaaat tgtttttct ctagtacaca tcctaatgat 180
ggtagcatct gtatttctca catataaaga aaatgtacta aaattcgtct aactccatta 240
ctagagcata atagaggtaa cttcaggttc ctccttttct ctgagctctt tatcctgggc 300
gtggctggct cctgcctcgt ctgctctttg catctctgta tatagagtta cacaggcctg 360
gaatgtggtc ctgctcacta acaggcaggt gttcactgtg tacctgcacc gcctgtctgc 420
agagtattac gtaatcggcg tctgaccagg agctggattt gagtcactca gtctggttca 480
gggacactgg gaaacatggc accagttctt cagcccatcc agatataccc cgaaacaagt 540
attcttttg ttctacctac agccagcaag ctttctgta aacggccagg tagtgtttta 600
aggtctgnca agtcatgtag ctctcttaat actcctttct gctgttacag ctataaataa 660
tatgcaaatg tacgagtgga gctgtgatgc aataaacctt tactaaaaga cagacggacg 720
gaggcctgca tttgtccctt acctcacagc tgctggcccc aggccctaat tcaatttaag 780
acctctaacc tttaanctgc atctttcccc ctttttaatt gcataattca cttnaaagca 840
gaaatgtgtg tggtacacnc ggngggggaa tncccggggn cnggaancac ctaaaaggct 900

g 901

<210> 111
<211> 924
<212> DNA
<213> Rattus norvegicus

<400> 111
tnncgcaagc gcggaaatta accctcacta aagggaacaa aagctggagc tccaccgcgg 60

tggcggccgc tctagaacta gtggatcccc cgggctgcag gtttggcatt aggccaaagg 120

ccatagcaac agaaggttag gatatagaca ctcaaactat gatctttaaa gtgggttgta 180

tcacttaaag gacttggtta tatccataaa ctgggaatgg tggctgtgtg atacttcatc 240

aagtaacaga tgtgggtgta tcctacttgg ttactctcct gggatgttaa cttccttttc 300

taggtgacag atttcttcac ataataggaa taataatgta tgtgagaact tactctgtgc 360

caggcagtgt gtaggcacat acacagacca ctgcatcccc ttagcagccc tgtgagaaca 420

catcggtctg cataaagcat tgnaaacagg aatatgcatc tcgtgatgag gaatgatcga 480

ttatacctat caattaagat aaatcaataa atcatatacc atagtttata tgcctttgaa 540

aagctacata taatcactaa gggtttttc ttaataagtt aaaatgttag ttacagcctg 600

ccagtgtcta acatttggag gctgcagctt tcagaacact tggagtaact actgaatggg 660

ccattgagct ttcttttgct gcctatagga aaacagtacc ttantaagtt ttacccttcc 720

tacagtcact tgttaaatgg cagttactga caaaacttta acaagtactt gctgctgcct 780

gtcnangctc tccctgccna aagggcctgt nccatgcacn aaatccgnca ttaaacctag 840

ggtaggcctc aggaggtggg gttncggtng gacctnaaac tngcccattc caaggaaatg 900

ggggaacnac nattaccggt gntc 924

<210> 112
<211> 893
<212> DNA
<213> Rattus norvegicus

<400> 112
   aagcgcgaaa ttaaccctca ctaaagggaa caaaagctgg agctccaccg cggtggcggc 60
   cgctctagaa ctagtggatc ccccgggctg caggttccct ccctctcctt actatggaat 120
   tttcttgttg tttaatcaca aaaacaaaca ggttctgttt ctctcctcag ggtcggtttt 180
   cagtttttgg gttgggtttg ggttttgggt tgggtttggg gttggggttg ggtttggggt 240
   ttggtttggt tgtaagtttg gggtttggtt tggttttttg ttttcctttt atgctctaga 300
   gaacaaggat agaggtggca gctgagatct ttggaaccaa agcagagcat gctgagtcca 360
   tgatctaggg cactggccac caaaggcagg gccctcacat atgncccata agccagagcc 420
   cccatgctgt ggccacatca ccctgtcttc tccgtactgc atcctcttct acgtgcttta 480
   ttagctagtc caaagggggc agcagtagta atgagagctg tgtgagcaca tggaactcca 540
   tgcactgaaa cccacatacc actcatttag aaaaagacct cgtaggcact gcacaaccag 600
   ccagctctgc tgcctgtgtc tgcccggctc cctcctcctt agattgcttg cctgctaaac 660
   ccatggctag aactcactgt ctggcagaag gatacccaag cctgttctgg gattttgtct 720
   gtcttcagcc agatgcttcc tgcccttcct tccttcctcc tcatattgct gtttctcnct 780
   ggtgtgcaag ttctgngcca tttccatagc actgatctta acacccagat aaagcnaagg 840
   gcttgggcca aagatcanct ttcnncncct tacaganggn gaaaccccaa cct 893

<210> 113
<211> 1012
<212> DNA
<213> Rattus norvegicus

<400> 113
ngcgnaagcg nngaaattaa ccctcactaa agggaacaaa agctggagct ccaccgcggt 60

ggcggccgct ctagaactag tggatccccc gggctgcagg aattcggcac gaggccagtt 120

ccagattcag tcttctctgt tagaaagaat tttctacttg gtcaactaga catacctgcc 180

agccatatgc tgacagcgtt taaattcagg gcttactcca tatccttata agacctagca 240

tatgtgggct tcgaatattc aattacttgt tgttgccagt tttactaagc ttagtcaaat 300

aactcactct ttttcaatag gattttattt cattcaaggg ttctttgacc aatgatctta 360

aagggtggtg gtgttgggga tggagctgtt aggagcactg gacatctctt ccagaggtcc 420

tgagttcaat tcccagcaac acatggtggc tcctctgtaa tgggatctga tgacctcttc 480

tggatctgag acagctacag tgtacttata tacataaaan aaatctttaa aaganaaaaa 540

gggagtatta tatttgcttg ctaataagaa aaatcttatt ttgttgttgt ttctaaaaac 600

gtaagatcag tttcttagtt tggcctacat tttaaaaacc gtagtgattc cgagtgtggg 660

tggaatctcc catggaagcg tactgttgag aagaggctga gttttggtgt aggcacatcc 720

cagccagcac cgcatggacc anttggtcac gcttcatgtg cctgccccac cntccanana 780

tgggnttccn ggtngccntg gnntccgggt cngggaaant ngaaancccc agaccccctg 840

gggtttggcg agaaaaaana ntggcantaa ctggagccgg nggaancccc caagncccct 900

ggggnagaac ctncctngga ggnagagang gccaatttcg gaanagccna acnaattaca 960

attggaaggt nntaggnncc ngggggngna cnaagaangn ngaaaagggg gc 1012

<210> 114
<211> 993
<212> DNA

<213> Rattus norvegicus

<400> 114
cgttaancgn ngaaattaac cctcacgtaa agggaacaaa agctggagct ccaccgcggt 60

ggcggccgct ctagaactag tggatccccc gggctgcagg ttagtgaatt cagggctagc 120

cttggctata tgagaccatc tctcattcaa acattaaaga tttttcatcc tttagtatgt 180

gactgtcctg ttttctctag actactttaa atatctctac tctgatgaaa ttaagggaat 240

tgtaagtttc aagttccaaa ttacagcatt gagtctagaa atgtagccaa actccatatt 300

ttcatagtcc ctgtttttat ttcctacata aacngataaa ttttccctgt acttaagggc 360

tttggcagta attctgtcta tatttaatct atnggcaccc tctaaattgt gttttctttc 420

aacacagtca tccnggccaa atcctttaaa atgtttttgc ttcagtaaag aggttgaaga 480

gctcctattt aaaagggcag ttgaatgtca ccagcaacag atgcaaagcc taggttcaag 540

ggttgtaaag aggccagcac tgtgtggtgc agatttcaag gcaccctgtt gatcacnggg 600

gagagactgg ctgtcacaca gtcactggga cagatggact tgggactcat accaaaggct 660

tctaaatcag aaataaatag tataagggca tctgtntccc cgaaccttcc taggtccatg 720

aaaggtctcc agctcactgt tactgttttc canttcancn gacgcttaga tgagtagaga 780

tagaagttgg ncattgggaa gctaagtaga gccttagtcn gggctcncta gaaggggcaa 840

aaggccagcc ccaanggaat taanctagga ggctcaacct nancngtccc cattngggac 900

naantccata agggaattgg gcacccaggg cttttccntt ngaaaagcnn ccagccttgg 960

ntgggggggg anaagaaggt tgaatncccn tnc 993

<210> 115
<211> 997
<212> DNA
<213> Rattus norvegicus

<400> 115
aagcgcngaa attaaccctc actaaaggga acaaaagctg gagctccacc gcggtggcgg 60

ccgctctaga acnagtggat cccccgggct gcaggctcga gagtcatcat ggatcttatc 120

tttgtaacac acttgtcaac ctacagtgac agagcatgtg tgtcattcaa acagcaggac 180

ttccaaaaac atcatcatct acattgtcaa gttctccatc atggagatat gaggccctga 240

aaagtataga tcaggggacc tgtggagact tgaggttcca gagtgaggag agcactggct 300

gcctggtctc atgcctgtct catgggaccc taggaaggac gatggaacaa cactgttcta 360

cttgagggct atgncataag ctttctcaaa acactaccac acactggaca tcccctcact 420

ctggcagtcg tttacactac tggcccaaat tcctcagctg tatcaagagg ctcgacncca 480

aggcctttat gacagagggg aagactccag gtgaaggact cagaaggagc tgctcagtgc 540

tggggactca tgtgtagcct caaatctaat tgtgggtgac ctgctgctct ctggggtcag 600

tcacagagca gctgcttcca gacaagcagg aagaaggaac agactctgtg cactttagca 660

caaaggactg gagccaagtg acagagacat gggacagtcc caagtgtttc cccagataac 720

ccancctctt cctttacctt tcagaaacan ttttacnctg caaaggaggt cttggttaat 780

gtctgaaact ggtggcanag agggattagn ntgtttaatt tccaggagaa gcataagttt 840

ggtnaancac cttttangct ggaaatggaa atttagggga ggttaattan naacntcaac 900

aaaactccca anccnttttt anancctttt nggttttnan agagnnnnca ccataaaccc 960

cgggnnggac cnggananan ggaaatggaa accnagg 997

<210> 116
<211> 979
<212> DNA
<213> Rattus norvegicus

<400> 116
aagcgcgaaa ttaaccctca ctaaagggaa caaaagctgg agctccaccg cggtggcggc 60

cgctctagaa ctagtggatc ccccgggctg caggcaggag tactcctcag cctagcttgg 120

gtgcccctga tctgctttga tccttcccac caagcaaatg aattgtttag caaatgaatt 180

ttgcctggga gctgcctccc ttccccctag gagtggttgg ttcattgagt ctgctaacat 240

tcttcaaaaa attactataa ccaagaaaga ggaggctacc tgtctttgag gatgccaacc 300

atggccccca tcagcacagg tcccatggca tggtgagcaa tagcagaggg gtttaacaag 360

ggaggaaaag acagggtctg ccctgcagga tcctttctgg ctggggaggt gttggtcaag 420

ggagaagtgg tactcctgga gtgacaccca tggcacccag gagtatggtg accagcctgt 480

tagggcccca gcaggcaccc tttgctcaac cctccaagga gttgcctagg ttccagaatt 540

ctgctgtggt tggcaggttc aatctggtct ccttcacttc catattagaa gtgtcttttc 600

ctcattgcgg aactgtgcct cctcggggga cagatgcctt tagatgcaac tgcttcaagg 660

accccaaagg ccattcccac aatataagca atgacttctt tttccttttt agtcatgcta 720

agtagttaga caatccttga acttgggatc tctggttctg gtgtaagagc caattatgtt 780

ttaactactc attgggaaga gctgagggtt tccagtggtg tgtcctaaag agaaggctga 840

aggcttgcca atggtgttcc ataaagagaa ccccagttgg naaaattggg ggatgccaag 900

ccggtttttg gnaaaggttc caaaaccaag ggggantngn caattgggaa anacccagcc 960

accaggntna aatggggggn 979

<210> 117
<211> 979
<212> DNA
<213> Rattus norvegicus

<400> 117

aagcgcgaaa ttaaccctca ctaaagggaa caaaagctgg agctccaccg cggtggcggc 60

cgctctagaa ctagtggatc ccccgggctg cagggtccag gccggggacg tgatcaccat 120

cgacaaggcc actggcaaga tttccaagct gggccgctct ttcacacgtg cccgagacta 180

tgatgccatg ggctcccaga ccaagtttgt gcagtgccca gacggagagc tgcaaaaacg 240

caaggaggtg gtgcacaccg tgtccctcca tgagattgac gtcatcaact cccgcactca 300

gggcttcttg gctctcttct caggagacac aggggagatc aagtcagaag tccgagaaca 360

gatcaatgcc aaggtggcag agtggaggga ggagggcaaa gcggagatca tccctggggt 420

gctgttcatc gatgaggtcc acatgctgga cattgagagt ttctctttcc taaaccgggc 480

cctggagagt gacatggcgc ctgttcttat catggccacc aaccgaggca tcacccggat 540

ccgaggcacc agctaccaaa gtccccacgg catccccatt gacctgctag accggctgct 600

cattgtgttc aacatcgccc tacagtgaga aggacaccaa acagatccta cgtatccgct 660

gtgaggagga agatgtggag atgagtgaga cgcctacaca gtgctgaccc ggcattgggc 720

tcgagggggg gccggtaccc aattcgncct atagtgagtc gtattacaat ttcaatggcc 780

gtcgttttac aaagtcgtga ctgggaaaan cctggcggtt acccaaactt aatcgccttg 840

nagcaanatc ccccttttcg gcaagctggg gtaaataagc gaagaangcc cggaaccgga 900

ttnggccntt cccaaaagnt tgcggaacct tgaaaatggg cgaaattggc aaaatttgta 960

aggcggtaaa tantttttng 979

<210> 118
<211> 989
<212> DNA
<213> Rattus norvegicus

<400> 118
aagcgcgaaa ttaaccctca ctaaagggaa caaaagctgg agctccaccg cggtggcggc 60

cgctctagaa ctagtggatc ccccgggctg caggctggcc acttgctcat aggccagggc 120

tctcaaatga gcaccttttt aagggtcctt cacccactct gtgctctcca cagaggcttc 180

cacgttgcta cataatggac acactccgat atagccaatg ggcaggaaat ccggggcact 240

tgtgcagggc cggggatggg actggggata agggaaagat tagaggacaa gggtaagatt 300

tttatttttg ggtgggttgg gtaagacaac gtatttcagt aataaaatac agaatggaaa 360

aaaaaaaaaa aaaactcgag ggggggcccg gtacccaatt cgccctatag tgagtcgtat 420

tacaattcac tggccgtcgt tttacaacgt cgtgactggg aaaaccctgg cgttacccaa 480

cttaatcgcc ttgcagcaca tccccctttc gccagctggc gtaaatagcg aagaggcccg 540

caccgatcgc ccttcccaac agttgcgcan ctgaatggcg aatggcaaat tgtaagcgtt 600

aatattttgt taaaattcgc gttaaatttt tgttaaatca gctcattttt aaccaatagg 660

ccgaaatcgg caaaatccct tataaatcaa aagaatagga ccgagatagg gttgagtgtt 720

gttccagttt gggaacaaga gtccactatt aaagaacgtg ggactccaac gtccaaaggg 780

gcgaaaaacc gtctatcang ggggatgggc ccctangtga aaccaatcac cccaatcaag 840

nttttttggg ggcccaaggt gnccgnaaaa ggacctaaat cgggaacccc taaaagggga 900

gccccccggn tttaagaggc ttgancgggg gaaaacccgg ggnaangtgg gcganaaaag 960

ggaaggggan aaaaaccgaa aggggccgg 989

<210> 119
<211> 978
<212> DNA
<213> Rattus norvegicus

<400> 119
aagcgcngaa attaaccctc actaaaggga acaaaagctg gagctccacc gcggtggcgg 60

ccgctctaga actagtggat cccccgggct gcaggcagca taactccttg aggacagaga 120

gttttgttac tgcttcttcc ataggacttg ggccagtgct gagcgtgtga aaagcactct 180

ttgactgggc agacaggagg gcccacatgg gccatgcacc attggtggca ttgggaccaa 240

gcgtgctgcg gccagaactt agtctgaggg tatattcctc tccgccacag gaacagctct 300

cactttctta cggttattct tagtttgtta cacatgactc ctctgtggag ctctctgaca 360

ggctgaggtc ctatgaagta gggtggaaga gaatagctac agaattgggc ctcagcgttc 420

ctatcgcttg agcatccagt cagggcaatt ccggcaggct gcatcatcct tgattgttac 480

aaacactaat gaagaaaggc agcattcctg tgattttaaa ggaaacacag aattttagct 540

tcaagtatgg gcattccttt gtgaaacttc tcaggaaaat gttgtttcta agtaagttta 600

tctgagaata tagggctgtt acagaatggg atgctgttct gcagaaagtc ttttcattcc 660

ataagaagga atagtgatat tatacaaaga ccgggaaggg ttccctgtta aagtatcttt 720

tatnctcctg ttgtaatgta gtcttagagg ttcactgcct ctgtctccta acctagggtc 780

ctggaagctt ctgggcctct gtacagtcta atctagggcc tagaaaggtt tccaggctcg 840

gagaattcaa tggcngaaat aagctcaacc cttcccaagt ccnttccnga aangaaacnc 900

cttggcccag gactcccncc tccaagggct ggacnnggan cnaaanccng gggntccccc 960

cnccnnccnc cnggaaag 978

<210> 120
<211> 992
<212> DNA
<213> Rattus norvegicus

<400> 120
aagcgcgaaa ttaaccctca ctaaagggaa caaaagctgg agctccaccg cggtggcggc 60
cgctctagaa cnagtggatc ccccgggctg caggaatcat tgacatagac cacaatgtag 120
cttcacgatg tgagaatncc ttctctacca taaacacaca tgactaggga tttgccttct 180
gaagtctggg actttgggaa aaggaatccc acactgtggt ttcatttttg cattcactat 240
acacagtaag taaactaaga acatgttgta accatgtatt tactctgcca agtgcctata 300
tgccaataaa acattcatcg ctgaagggct gtccagatac ctttatttag aatggggctg 360
gcttcatctt taagcaaatg tcacactgga gctgcaggaa cagcccttag aaatgaaagg 420
ggcaagtcac tacctggctg gactctggga aatttcacgn cctncttgtt gttggggaga 480
aatccactgg ggcacgactt gatgtccaat gaaattctgc tttgataaca cacatggctt 540
atttttcaag ggaatggatc aattccacaa accagcaatg gggcaattac tcttgatata 600
attgaacggc tgttcaaact taatanattt tcagcgggcg agactggagt aaaacgttcc 660
ntccanctgt aattataaat gagacagtgt ttacttacta aaaaaaagaa aaaaaaaaac 720
tcgagggggg gcccggtacc caattcgccc taaagtgagt cgaaattaca attcangggn 780
cggncgtttt aaaangtcgn gacngggnaa ancccnggcg ttanccaacc ttaatcggcc 840
ntgngggnan aatcncccct ttncgccanc ngggngnaaa tancgaaaga ggccngnnac 900

cgaatnggnc cataccgana nggtggnnca ncnatggaat ggngaaaggg gaaaattgga 960

aanggggtaa aaaatttggn nnaaaaaanc gg 992

<210> 121
<211> 983
<212> DNA
<213> Rattus norvegicus

<400> 121
aagcgcgaaa ttaaccctca cgtaaaggga acaaaagctg gagctccacc gcggtggcgg 60

ccgctctaga actagtggat cccccgggct gcaggatgaa gatgtcattt aattatttgc 120

caaaactagg gttttaatga agtggcttcc ccagtcccct ttttatagtt attttctggt 180

gtagaccctt gaggtggctc agggtataaa ggtgcctctc ccaagcctga caacctacgc 240

ttgatccctg gggttcacac tgtagaagga gaggactacc ctggtacatt gttctcagac 300

ttctgtatgt gtgcgcgctc gcgcttcaca cacacacaca cacacacaca cacgcgcgcg 360

cgnnncaaag ngcaaattaa aaggtatata aaagttaaat ttctgtttta taaaacggtg 420

tttaataaga aaatatttat aaaatttaag tacaaaatta tctattaaaa attcctattc 480

ctgccagtca atggtggagt ttgcctttaa tccaagctct ctggaagcaa ggcaggttct 540

ctgaattgga ggccaacctg gcctgtggag tgagttccaa gacagctggg gcgacacaga 600

gaaactgtct caaaaacaaa accaaatcaa aacaaaaacn gctattccag ttgatactga 660

taaccatgta aaagagagca gaaatatcat caatacaata tcgtcccttt gggantccct 720

gggggggaag cagattgtat ttgtgtcatt ggtatctgcc ctgtttcttt ataaataana 780

ctctagaatt cttgccttgg ggtttgcaga gttgttgaga aggaaactgg tcgccgtgtt 840

ttttgggaag gtggaagtgg tacctaagga ttaattaatg aagganaacg ggngnanctc 900

cnaaggnaaa gnggcggtgg aagggaaanc gcctagnggg nnttccccgg ctttntnnnn 960

nnngggggntn ggnannggcc cnc 983

<210> 122
<211> 973
<212> DNA
<213> Rattus norvegicus

<400> 122
   ncaagcncng aaattaaccc tcactaaagg gaacaaaagc tggagctcca ccgcggtggc 60
   ggccgctcta gaactagtgg atcccccggg ctgcaggcgc agtggcggcc gactcctttc 120
   ggggcctaaa gcagctggat atgttggatc tgtccaataa ctctctgtcc agcactcccc 180
   cgggtctgtg ggcgttcctg ggagaccgga cccgcgatat gcaggatggt ttcgatgtct 240
   cccacaaccc ctgggtctgt gacaaggacc tcgtggacct gtgccgctgg ctggccgcca 300
   accgacataa gatgttctcg cagaacgaca cactctgtgc ggggcccgag gccgtgaggg 360
   gacagcgact gctggacgtg gcagagctgg ggaccttgtg aggatggcaa ctggggtgcg 420
   agccaagggt accccgcttg ccactgaagc aatttggtcc catgtcagaa tgcagattcc 480
   cagcatctgc cattccccat tccctcagcc aggaatgcta ttccctgact ctccctcagg 540
   ctcctctcca tttgccccaa ctcttccacc tctcactgtt cctgtgctgg cccccaggct 600
   accatgtgtt tatctagctt tgcctcatat gtttcagggt caccaaagca gttaataaaa 660
   cagctcccgg ctggctgagc cgctcaaaaa aaaaaaaaaa naaaaaaaaa nnannannnn 720
   nnnnnnnnnn nnnnnnnnnn nnnnnnnana aaaaaaactc nngggggggc ccggnaccca 780
   attcgcccta aaggggggng tattaaaatt nannggnngn ngttttaaaa ggnngggnan 840
   tggggaaaaa cccngggggt tacccaantt taannggctt tgnngnaaan tccccctttt 900
   tggcaannnn ggggnaanaa nggaaaangg cccgnnanng gntnggccnt ttccaaaaan 960
   nttggggggn ttg 973

<210> 123
<211> 976
<212> DNA
<213> Rattus norvegicus

<400> 123

aagcgcgaaa ttaaccctca ctaaagggaa caaaagctgg agctccaccg cggtggcggc 60

cgctctagaa ctagtggatc ccccgggcng caggtttctg ggtagaaaca tgcattttgc 120

ttactgagta gagaacaaag tttacattgg acttgtgggg caggacgaca ggggaagctt 180

gggaagccag caacctgcag gaagagtaag ccggtctacc aacaggtttt gacctttggt 240

ttcaaaaata aactcaaggt gctagattca gtatcaccaa ggttttacca tgtgaccttg 300

ggcaagtcag ttcctctcct tgggctcagt tcaccctgac ctggggaaat cagacaccag 360

gattaggtca tctttcatga ccccttccag ccctggacat tccataggga ccattctaat 420

cagttggcat gtgccaggca cccagcagga agctgcctgt ggcatttcca tttcactgaa 480

ttctccttgt aaccctttgg ctttctaagg tgggcaccaa gcatggttga atgactcgcc 540

caaggccaca tggctaataa ggaacagagc cggtctgcga gactctaagt gactgaagaa 600

cttcttatgt gtggtttctg ttcttggcag ggagtcttag tgggctccag ggcaggggga 660

aaagagagag ctacatgaga atgggcatgc ccgtcttcca acagccatct ccactgtcca 720

accgttgcta ggcaacatga agcccatgac tgcagctctt caggaggccg aggcagggag 780

atcacaagtt caagcctacc tggggctgca aagtgagttc aaagctgggg cagcttagtg 840

agaccttgtn tcagagtggt aaagtaaaag gatgggtaag gatgtaanag cncagtggaa 900

agggactcgg ccaanggggt gaagnactaa gggcacgagn attcaanccc caagnaactg 960

canaagaagn ggggng 976

<210> 124
<211> 987
<212> DNA
<213> Rattus norvegicus

<400> 124
gcaagcgcga aattaaccct cactaaaggg aacaaaagct ggagctccac cgcggtggcg 60

gccgctctag aactagtgga tcccccgggc tgcaggaatt cggcacgagg cgtgcggggc 120

gggggcgccc gacggcgtcc gagggcgcgg cggacgaggc ctgagggagg ggacgcgatg 180

ctggagaccc tgcgcgagcg gctgctgagc gtgcagcagg atttcacctc cgggctaaag 240

acgttaagcg acaagtcaaa agaagcaaaa gtaaaaagca gacccaggac tgctccctac 300

ttaccaaagt actctgctgg gctggactta cttagcaggt atgaggatac gtgggctgca 360

cttcacagaa gagccaagga atgtgcagac gctggcgagc tggtggacag cgaggtggtc 420

atgctgtctg cccactgggg agaagaagag gaccagcctg gccgagctgc aggagcagct 480

gcagcagctg ccagctctcc tccaggacgt ggagtccttg atggcaagcc tggctcattt 540

agagacgagt tttgaagagg tagaaaacca cctgttgcat ctggaggact tgtgtgggca 600

gtgtgagtta gaaagacata aacaggccca tgcccgacac ctggaggatt acaagaaaag 660

taagaggaag gagcntgaag ccttcaaagc tgaactcgat acagaacacg cacagaagat 720

cctggaaatg gagcacaccc agcagctgaa gctgaaagga gcggcagaag tttctttcga 780

ggangctttc cagcaggaca tggagcaata cctgtccaca ngtcacctgc agattgcaga 840

naaggcgaga gccccattgg ggcagcatgt ccttccatgg gaagtgaatg ttggacgtnc 900

ttggagcaga atggacctng attggaccct ctttngaccc aagaangggg ctcggatggc 960

cttcctttaa acnnctgggg ggngang 987

<210> 125
<211> 998
<212> DNA
<213> Rattus norvegicus

<400> 125
caagcgcnga aattaaccct cacgtaaagg gaacaaaagc tggagctcca ccgcggtggc 60

ggccgctcta gaactagtgg atcccccggg ctgcaggtgg aggctgagat tgtacagcaa 120

caggcacccc cttcctatgg acagcttatt gcccagggtg ctatcccacc tgtagaagac 180

ttccccacgg agaaccccaa tgacaactct gtgctgggga acctacgttc tctgcttcag 240

atcttgcgcc aggatatgac tccaggaggt acttccgggg gccggcgtcg ccagcgtggc 300

cgctcagttc gtcggctggt tcgcaggctc cgtcgttggg gcctgcttcc tcgaacaaat 360

actccggctc gggctcctga gaccagatcc caggccacac cttctgttcc ctctgaggcc 420

ctggatgaca gcacaggtca agcctgtgag ggtggggcag taggagggca ggatggggag 480

caggctcctc cactacccat caagtccccc ataccaaccc caagcacact tccagccctt 540

gctactgcct ctgaacctcc agggccacta ccctcagtgc ctgtagaatc atcactgttg 600

tctggagttg tccaggttcc taaggaggcc gcctcctacc cagcctgtgg cccccaggnc 660

ccaattggac cccaatggaa ctcacacagc agtcctgcct tctagaggat gaggatgatg 720

tatgttgatg ccattggctg agccaagaag tctgggtggt ttgaggcaga ggatgaanca 780

atggctttgc ctgagagaac tctagacaca atgtaatgaa tctggtggna agtgggttca 840

gaaaagttaa gtgtcccctc gaaggggggg ggcccgggta accccaaatt gggccctaan 900

aagtggagtn cggaattaaa aattcaantg ggccngtnng gttttaaaaa aggnnggtga 960

antnggggaa aaacccttgg gggttaacca aactttta 998

<210> 126
<211> 978
<212> DNA
<213> Rattus norvegicus

<400> 126
   aagcgcgaaa tnaaccctca ctaaagggaa caaaagctgg agctccaccg cggtggcggc 60
   cgctctagaa cnagtggatc ccccgggctg caggctaaac tttattgaca aacttgctgc 120
   agacattttg acataggtta agctacctat ttaggcagac ttacacatgt agcatttaat 180
   cttccaaaaa caaaatggga ggacggtaca aatccattag gactttaact tatgtacaaa 240
   gtggactttg attctcttct cattcagctg cagtgtccct ttttatgtca tgctagtgtt 300
   gagacatact taactaccgt ggcaacagtg cgaaactgac aatggtcaac ttaatgaaca 360
   gacgtcactt ttcggncccc agtgtccaag tgnagttttt catggagtgc agaatctcag 420
   atggacaaaa tacncttgga cattttaaat actgaaaatt tggattatnc agtactatta 480
   ttgaaaagac tgtggctaaa aagaactgtc agacnccatt aggcggccag cttnccnccc 540
   cagcaaccta ttcaaccccc ccccccacta agtatctctc aacacngtat gtctggggct 600
   agatttcaaa acccacagaa tgaaaaaggc attttacaaa cctaaatttt gttgttgttt 660
   taagncaatt taacgntnaa aaatngcatc caacnattta antcatgaga tctttcntat 720
   naaanattna aaccntaagn attcaacccg gccangnggc ttttaaaagg ggaaatgntt 780
   tttagnagac aatccngngg cncccctttt tacnaagggg gggcaccnaa aggggccggt 840
   naanaantgg tgaattntta caggcntaaa gccagncccn ggaaattnga aanggaggcc 900
   ccagtttngg ggaatccngg caaccnangg ncntnnancg gggggggccc acaaaancna 960
   aaggccnaaa gnnaaaan 978

<210> 127
<211> 936
<212> DNA
<213> Rattus norvegicus

<400> 127
agcgcgaaat taaccctcac taaagggaac aaaagctgga gctccaccgc ggtggcggcc 60

gctctagaac nagtggatcc cccgggctgc agggtgaacc tcagttctcc atcaaacatg 120

cttcctgtcc ggccacaaac taaccctctg atgggggac ccatgcctat gaacatgccc 180

ggtgtaatga cgggcaccat gggaatggcc cctctgggga acaccgcagg atgagccagg 240

gcatagtggg catgaacatg aacatgggga tgtcggcctc ggggatgggc ttgacaggca 300

ccatgggaat ggggatgccc agcatggcca tgccgttctg gaactgtgca gcccaagcaa 360

gatgaccttt gcaaactttg gccaacttta gtaaataaaa ggttgtaacg gagcgagtgg 420

aagaagcctc tgtagctgca ataggtgatg ttgggctgga agatgctaag cagttccctt 480

ttctttcatc agttaattaa ataaccacat aaagaaccaa aaaggctgct gtttcagaag 540

cgatgcaaga gcacttcaga cgaggcagtc aggatcggtt tccccagtga agatacatac 600

gctcctaaat ggggcgaggg ggcacgagag cctctctgtc agagagcatg tgtcccagcg 660

tagtctgtgg gaggactggc atggatgggg gctgagtaag tgtgcttcac tctctaactt 720

tatactttct ctccctgagg aanttgattt tctgtccctc agncgccttg tcatgantgg 780

gnctgttcct ttantaccaa tctccaagtc caaggtaatg aaancattaa aagttngggn 840

gnatcagntt tttttatnaa aaatataaaa nggngggggcc aaaaaaaaaa gggatancca 900

anggggaatt atgggcngag tccaaaggga accnng 936

<210> 128
<211> 931
<212> DNA
<213> Rattus norvegicus

<400> 128
gcaagcgcga aattaaccct cactaaaggg aacaaaagct ggagctccac cgcggtggcg 60

gccgctctag aactagtgga tcccccgggc tgcaggaatt cggcacgaga ttaggagtac 120

cagcctgctc taacggtttc agggaagatt ggctgtgggt ttccgcagag tgtgggggag 180

ttcctgctta tccaactggc tcgccatggc ttccctgtgg gcaagggctt ggcaagagcc 240

ctggacaaaa aacgggacat cattgagaag acacctgctt tgtgcgaggt gttctgccgg 300

caagggaggg ggggcccggt acccaattcg ccctatagtg agtcgtatta caattcactg 360

gccgtcgttt tacaacgtcg tgactgggaa aaccctggcg ttacccaact taatcgcctt 420

gcagcacatc cccctttcgc cagctggcgt aatagcgaag aggcccgcac cgatcgccct 480

tcccaacagt tgcgcacctg naatggcgaa tggcaaattg taagcgttaa tattttgtta 540

aaattcgcgt taaatttttg ttaaatcagc tcatttttta accaataggc cgaaatcggc 600

aaaatccctt ataaatcaaa agaatagacc ggagataggg ttgagtgttg ttccagtttg 660

gaacaagagt ccactattaa agaacgtgga ctccaacgtc aaagggcgaa aaaccgtcta 720

tcagggcgga tggcccacta cgtgaaccat caccctaatc aagttttttg gggtcgaggt 780

gccgtaaagc actaaatcgg aaccctaaan ggagcccccc ggatttagag cttgangggg 840

gaaagccggg cgaacgtggg cgagaaaagg gaagggaaga aaagcggaaa ggagcggggc 900

gctaagggcc ctgggcaaat ggtaaccggg t 931

<210> 129
<211> 936
<212> DNA
<213> Rattus norvegicus

<400> 129
gcaagcgngg aaattaaccc tcactaaagg gaacaaaagc tggagctcca ccgcggtggc 60

ggccgctcta gaactagtgg atcccccggg ctgcaggaat tgacggttct gagggtgaag 120

ggaagctggg tctgtaacct agtatctctg acaagacagt gtttacatga gctaatcctc 180

ttcaagactc agagctgaga caaagtcatt tttctttcag tttttgttgt cacacctttt 240

tttattttat tgtattaaaa cctagccata atgaagatag aatttctgtt tacattttgg 300

gcatgatgtg gctgcatgca gaggcttcat gcttttgaac cctgtatttg attgtgctgc 360

atgggaggct tttattcttg gacagcttag tacagaagca ggagaaggtt tgagttcttg 420

gggatgcaaa ggacggatgg cctatattct aaagacagtg tcatcgccct tcctgtgtgc 480

tgacccaggg ctgtgtgtgt gctaggcgag tgctgttgaa aggtagacac gctggtggag 540

agaaacaagc tgctctcatc accacacact tctgcagagc ctttgtgctg cagctccccg 600

tgaggctgtc ctccagttct ctccagccag aattgctgtg gcaacaatat ttttataaag 660

cagtgggctt catcttagga ccagtcaatt aataagcgtg gccgtagctg agaagagcca 720

ctttccaaga gcgcaccaga cacagtgagt ggtgatcagc cccttctgg cctgcctgta 780

tgattgagaa tcccaaaaac tctggtaaat ccataagtgg gggaacagaa ggcaccggat 840

ccttccaata agccagagaa gggaantngg ggctttaagg accatttggt gccaaaaagg 900

gtttttggg ggggnttggg ggggaggtcc cgtttt 936

<210> 130
<211> 955
<212> DNA
<213> Rattus norvegicus

<400> 130
caagcgcgaa attaaccctc actaaaggga acaaaagctg gagctccacc gcggtggcgg 60
ccgctctaga actagtggat cccccgggct gcaggccagc ctctgtctct gaggagagct 120
gttagagact tgactgagga aagccagcac ggagtcccag aatttaaacc gtgactatac 180
tttaaacatt tactagcact ccctagccag ccctgtcaga ggataacagc agcactagct 240
gacattactg aacagtgctg gtcgagactg ctttgtgtaa gtgtcttgac caatcttcaa 300
gagaactctg tgaggttact atgattatcc aaatgctact gaggaaaaca gaagattaga 360
gatactggac ttattcagat tctggcaact attaaatggg caggacgtaa tcgttactgt 420
ggtcagaaaa tccccctttt agatgagatt ccagcccctc tcctaatgcc tcaggttcac 480
aaggaaggca agagagggca gaacccagag ggatgtggtc atgagtgtgg gtagggaaaa 540
gtgcaggaag ctgagaatag gattgctact ggagtattga tgggattgca gagcggtccc 600
aggtaatgcc cctaagtatg gcaccattcc catgaaaaaa cactcagggc aagcagggtg 660
aactctcaac tccaaatatt tacgtgctaa aattcctaga aagtgacact ggactctacc 720
tggtcgtgaa gttccctatt tgggtctcta acaattatct tctgttcaca cangggatcc 780
ctgtatctca agtctcccat ggagattcca ggcctttcaa nggggctggg gggagttgaa 840
agggggagcc actggggcct tgaaaggggg ggccactngg gcccttggtt tngtcccngn 900

gggctaaaag ggcacggggg gtaaatccaa ggttccccct gggnnaacaa gggaa 955

<210> 131
<211> 929
<212> DNA
<213> Rattus norvegicus

<400> 131
   aagcgcgaaa ttaaccctca ctaaagggaa caaaagctgg agctccaccg cggtggcggc 60
   cgctctagaa ctagtggatc ccccgggctg cagggctgag gcagccatct tgctcttgcc 120
   gcgtgctggt gttagaggac cctccccgct gcagatttac caacagcatg aatcaagaaa 180
   agttagccaa gcttcaagct caggtccgga tagggggcaa ggggacagct cgcaggaaga 240
   agaaggtggt gcataggaca gctactgctg atgacaaaaa gcttcagagt tcactaaaga 300
   aactggctgt gaacaatata gctggtattg aagaggtgaa catgattaaa gacgatggaa 360
   cagttattca tttcaacaat cccaaagtcc aggcttccct ctccgctaac acctttggca 420
   attactggtc atgcagaagc caaaccaatc acagaaatgc ttcctggaat actaagtcag 480
   cttggtgctg acagcttaac gagccttaga aagttagctg aacagttccc acgacaagta 540
   ttggatagta aagcacccaa accagaagac attgatgaag aggatgatga cgttccagat 600
   cttgtagaaa attttgatga agcatcaaaa aatgaagcta actaaaatct tctgggtttt 660
   ggaagctggc atggactaga tttaacaatc agctctgttg ttccaaagtt ttacagacat 720
   ggagaacatc acctgttatt agttccgtaa tataaatgtn nngtatatta atgatgctgt 780
   tttatcagca tttcctggtc attgggattt tgcattttgc acttcttccc agggatcgga 840
   ttcctttggg ncaaaatatg gaggaattgg gtaccagggt gaaggggtgg ttttggnttt 900
   tttggggggg gncctttggg gnggtggaa 929

<210> 132
<211> 730
<212> DNA
<213> Rattus norvegicus

<400> 132
gaggagctgt gccggcagat ccagcaggag gaggacgaaa agcagcggct acagaatgag 60

gtgaggcagc tcacggagaa attggcccgc gtcaacgaga acctggcgcg caagatcgcc 120

tctcgcaacg agtttgaccg gaccattgcg gagacagagg ccgcctacct caagatcctg 180

gagagctctc agactctgct tagtgtaatc aaaagagaag ctgggaacct gactaaagcc 240

acggcttcag accagaagag tagtggaggc aaagacagct gacaagccct gtctcagccg 300

tggcctatgg ctgctcccca acatgtctgt cctaaagcat ctttgttctc catggcctca 360

gatgtctttt atctctggtg ccctgagtgt caatttctga cctccacctg ccttgagtga 420

caagacaagc cccaggacag tccaatggaa gatgtgttcc cagctccgca ctcacatgtg 480

ttattggaac cttgagctcc tggtcagctc catgaggagc ctctttctga agctcctgta 540

tctccttggg ctgccaggaa tgtctcggtc ctagtccagg tactgtggga gcccctcact 600

ttgtctcctc agccactagg gccccaggcc aggcatattg aagaaagaat cttggctcca 660

gaaccaaaac cttagggccc atccccatga cctctggtgt ttcaataaag gtgtttgcaa 720

aaaaaaaaaa 730

<210> 133
<211> 709
<212> DNA
<213> Rattus norvegicus

<400> 133
tttttttttt ttttttttag gactcatgcc tctctttatt cagatttccc cacccactta 60

cagcaaattg tgaacccagc ctcaggtctg gggtcagggg ccctgaacct ccctaggatt 120

ccccagctct gtccagcaac ttttctttcc agttcacaga cagagagatg gagacccagt 180

gggcacagcc agtctggcaa tctttaataa gaagagaggc cttggccagg gctctgtatg 240

tcctcgctgg gctgtggttg tctctggctg gggttcaggg agctgttgat ctggtgtgtg 300

agggtttcca gtcttggggt ctggtttggg atcccagatc ctcctcttag cacttgtggc 360

atgtttgagg gccctgcagg caggggtggt gcagagggta ccacttcctc tcccatcagc 420

atctctgcca ggactcgtgt ctgtgtccag tcactatggt tcctctcttg tgggggcttg 480

gcagagcctg ggcttgttgg aggtgcccag ggtgggggcc gcacagatgg agatatgtgg 540

cctggggcct caggctccag cacctgctgt ggtgcatggc attctctcca tgggcaggcc 600

cagtgggtca aggacactcg gcacagagga cagaggtgat agcagtagca gaggggtggc 660

agtacatggc agaggcagct ggagtcatcc tgcagggcac acggtggca 709

<210> 134
<211> 376
<212> DNA
<213> Rattus norvegicus

<400> 134
gattctatgg cttcagtgtg aagaactgtg gagcccagcc tgccctgcac acctggaccc 60

tgtccctgca ctgcctgtgt tcccttccac agccaacctt gctggtccag cctgggggta 120

gggggtgggg acatctgcat cctgtcactc cttgctgccc tgagcttcag cccctcactc 180

cacactgaga ataagaatct gagtgtgaac ttgattgttc acatccttga cacaagtgtg 240

gatggctttt taaattactg gatggaatac ttagaggttt ttgttttggg gttttgcttt 300

gttttgtttt gtttttgaaa aaaataaatg gcagatgaag aagcttccaa aaaaaaaaaa 360

aaaaaaaaaa aaaaaa 376

<210> 135
<211> 723
<212> DNA
<213> Rattus norvegicus

<400> 135
tttttttttt tttttttttt agctttcaaa ttgctttaat acttgcactt acaagcaaca 60

ggtaaacaac gaacagttat tactgggcag tgtcacaggg gtgggggctg gagaggggag 120

tagaggatca agcaagcttc acacaaaggc gtttatgtga aggacaggaa acacgggcat 180

gctaaattct gcagagaata caacacatac acactacagg ggctggagac gtggatgaca 240

ttaagaaaca tgtctgaaat atgaagcttg gtatacagtt tatatgtgga gtgtccccgg 300

tgatggccat tcttggttgt caacttgact ccatcttgaa tgaactaaaa tctaatgaca 360

gagggcacac ctgggaggga gttttgctta atttgaagtc agtagatctt ctttaatcct 420

gatcttgagg tgggaagaca cacctctagt ctgggccacg ccttctgcta gaagtctact 480

tgaggacagg acgaagggag cttttgctct ttgcctgctt ggcctcacct tgttggcaag 540

tccattcctt cactggcgtt agagcctacg tcattgggat tccagagtct actgaagacc 600

agccgagaca cccagcctca tggcccgaac tgctggattt ttggactttc tgctcactgt 660

tggattagct ggacagaagc ctgtaagtca ttcttcttct gggaattcct tctgtaagtg 720

ctg 723

<210> 136
<211> 594
<212> DNA
<213> Rattus norvegicus

<400> 136
ctccccttca tgaacttcct gcctatcact tattgccttt cctaattaat gttgagaatt 60

ttacaagccg tattgattga cccacctgtt catccatggc agaatgacgc gtctttgtct 120

actgtcttct ttgtttacta tatgcccaag gttattccag acatgggaaa tacagacaca 180

catgtttctg ttcctctctg tgatgggcac acaatccaag gagcacatcc tgcacttttc 240

aaaactgaac ataactagat cagctggtga ctttgtcact tggctgaaat ccccagaata 300

agccaattcc atcctaacct ttgcatatcc agtacgacta cccaacattg aagaacaatg 360

attgttctta gagtctaatg aattttcaag tatttcttac ccgaaaaata ccaagaacta 420

tgcaaaaatg gaaaacaaag gtttagttac ttacaagctt taaacttatg gatgtaagga 480

atgtgtacct gatttaagat ggtgatgagc ccacagtagg cttatttact tgaaaacatt 540

ttagtcatca tgtatttccg gttgctaaat gtgcttgtgt ttataaaatt caaa 594

<210> 137
<211> 433
<212> DNA
<213> Rattus norvegicus

<400> 137
gagtgtgggg ctggagagat ggctcagtgg ttaagagcac ccgactgctc ttctagaggt 60

catgaattca attcccagca accgcatggt ggctcacaac catctataaa gagatccgat 120

gccctcttct ggtgtatctg aggaaagcta cagtgtactt atatataata agtatagaaa 180

tctttaaaaa aaaaaaaaaa gaatctgagt gtgaacttga ctgttcacat ccttgacaca 240

agtgtggatg gctttttaaa ttactggatt gagaatactt agaggttgtt ttttggtttt 300

gttttgtttt ctttgttttt aaataaatgg caggtggaga agctcccaaa aaaaaaaaaa 360

aaaaaaaaaa aaaaaaaaaa aaaaaaaaaa aaaaaaaaaa aaaaaaaaaa aaaaaaaaaa 420

aaaaaaaaaa aaa 433

<210> 138
<211> 619
<212> DNA
<213> Rattus norvegicus

<400> 138

tgaatttagt gctgaacatg aagagtaaac tatttaccaa aaagaagttc ctggagtttg 60

gagaagtaac gaatgtatcc atctgtacat gatttacatg ttgtggatgc tttgtaaaca 120

tttccccatg ttttaattgt gtttcagcag gttgtaattg cctctgtgtg tagctgaaca 180

tgagtcatta tctggtcctg tatgaaatgg aatgtatggt attttctgta tcattttcct 240

gaggctgtgt ttggggagcc acacattcga atacagtttt cctgatcact tgatttcttg 300

tgcacctgat ttttgctctc aaaggaatta ctgccacaat atattttatt tattctttag 360

attttagcct tgtcagttga agtgcttcac atgatggtgt taaaaactac ttgtcccttt 420

actgggggtt tgggggttgt taaaagatgg ggaggaagaa tgcaaatggg tcattgttaa 480

cctgtcccca ctgatcccac ctgtactcat agtcccttcc aggatggtat tctgatgttt 540

cctacaatac ggtgaccata ggcaacttgt tacctgaata aaggatcgat tttaaacagc 600

caaaaaaaaa aaaaaaaaa 619

<210> 139
<211> 1018
<212> DNA
<213> Rattus norvegicus

<400> 139
actngaaatt aaccctcact aaagggaaca aaagctggag ctccaccgcg gtggcggccg 60

ctctagaact agtggatccc ccgggctgca ggattgacca ggcccatagg cagaatgtct 120

cctctctttt tctgccagtg attgagtctg tgaatccttg cttaattctg gttgttcgca 180

gagaaaatat tgtaggagat gcaatggaag tcctcaggaa aaccaagaat atagattata 240

aaaagccact caaagttata tttgttggag aagatgctgt tgatgctgga ggtgtacgca 300

aagaattttt cttgctcatc atgagggaat tattggatcc taaatatggc atgtttcgat 360

attatgaaga ttccagggct atttggtttt cagataagac atttgaagac agtgatttgt 420

tccacttaat tggtgttatc tgtggattag caatttataa ttttactatg tggacctcca 480

cttccctttg gctttatata agaagctact gaaaaggaag ccatccctgg atgatctgaa 540

agagctgatg ccagatgtag gggagaagca tgcaacaatt gctggactac ccggaggacg 600

atatagaggg aaacatttgt ctaaacttta cgatcacagt tgaaaatttg gnggcaacag 660

gaagtgaaag agcggntctg aaagggtggc agacancgct ggttaacaaa cagnaatcgg 720

gcagggagtn tgtgaagccn aaggngggnt accanatncg anaaaatnca gngggnnnct 780

ttaaaaaagg ggctttccca aggccgggnt ttccataagg gtncngnggg ngggaaaaaa 840

gtncttccgg gnnncntccn agnccccaaa nggaaattta nnaaggggna nggggnnaat 900

nggggaanna ccgaanntaa ngnnntgggg gaaggnaacn tgggnnngan gaaagannen 960

gggnngnncn nnangnaaag ggnannnggg ggggggaaaa acccccnngg ngnnaaag 1018

<210> 140
<211> 371
<212> DNA
<213> Rattus norvegicus

<400> 140
tcctagcaca ggggctccaa tgccctgggc agcagaggat tatggtctaa gccgtttgag 60

taatcatcgg cttcttccca gcacattggt gaggaaacag gccacgactt gtcactcagc 120

actaaccccc agttgttgaa cagccttctc cagccctgct ttaggatgac aaatgaataa 180

cacctaggca tagaaaccag tctctctggt ttgtttgtat tatgttcttc aacattaaag 240

atttaaacaa caaaggatat actacagtct tgaatctaaa gtaatttgct aactattttg 300

attcttcaga gaactactaa taaaaatcta aaaggtaaaa aaaaaaaaaa aaaaaaaaaa 360

aaaaaaaaaa a 371

<210> 141
<211> 1024
<212> DNA
<213> Rattus norvegicus

<400> 141
aagcgngaaa ttaaccctca ctaaagggaa caaaagcagg agctccaccg cggtggcggc 60

cgctctagaa ctagtggatc ccccgggctg caggcttttt cgcacatccc gacctgttcg 120

tgagcattag tgatcagaag gaccccaggg atcggatggt tcaggttgtg aaatggtacc 180

tctcggcctt ccatgcagga aggagaggat cagtggccaa aaagccgtac aatcctattt 240

tgggtgagat ctttcagtgc cactggacgt tgccgaatga tactgaagag aatgcagagc 300

tcgtttcaga agggccggtt ccctgggtgt ctaagaacag tgtaacattt gtggctgagc 360

aagtttccca tcatccgccc atttcagcct tttatgctga gtgttttaac aagaagatac 420

aattcaatgc tcaattgaag gggaatggaa tggcatcatg tatgcaaaat atgcaaccgg 480

ggaaaaaact gtctttgtag acaccaagaa gttgcctata atcaagaaaa aagtgaggaa 540

gttggaagat cagaacgagt atgagtcccg aagcctttgg aagatgtcac tttcaattta 600

aaaatcagag acattgatgc agcaacggga agcaaagcac agacttgaag gagagacaaa 660

gagcagaagc gcgagaacgg gaaaggagaa gggaaattcc agtggggaga cgagggctct 720

ttccacgaag anggaagaat gccggggttt acgnatggaa ncctttantg gaaagcggnc 780

ttggggtacc tggggaagcc attaaagccn gaaanccggg gttccaccgg gggtgnaccc 840

agggggcant nnggcgnaac nnaaggnnaa caaatcngnt tcttccaggg ggnaancttg 900

nccacttncc cttncnttaa aagggggggg ggtncccaaa ncnccngggg ganancgggg 960

anttngannn cnggnnggac cnaattttaa aagggggaaa ggnggnttnc cccnttttta 1020

aang 1024

<210> 142
<211> 790
<212> DNA
<213> Rattus norvegicus

<400> 142
gcggctgtga ccgagagcac agagaatgaa ccagccttgc aggagaaaac actacaaatt 60

agctgacagc tggtagagaa gatgctctat ttctgtgctc agtgcctgtg ggatctgtga 120

tggaaatgat ggccggctgt ggtgaaattg atcactcact aaatatgctt cctaccaata 180

agaaggcgag tgagacctgt tctaacactg caccttctct aacagttccc gagtgtgcca 240

tttgtctaca aacatgtgtt catccagtca gtctgccctg taagcatgtt ttctgttatc 300

tgtgtgtaaa gggcgcttca tggctcggga agcgatgtgc tctttgtcgg caagagattc 360

ctgaggattt tcttgacaag ccaaccttgt tgtcaccaga agaacttaag gctgcaagca 420

gaggaaatgg tgaatatgtg tggtattatg aaggaagaaa tggatggtgg cagtatgatg 480

agcgcaccag tcgggagcta gaagatgctt tttccaaagg taaaaagaac acggaaatgt 540

taattgctgg atttctgtac gttgctgatc ttgaaaacat ggttcaatat aggagaaatg 600

aacatggacg tcgcagaaag attaaaagag atataataga tataccaaag aagggagtgg 660

ctggacttcg gctggactgt gacagcaaca ctgtaaatct agccagagag agttctgccg 720

atggtgcgga cagtgggtca gcacacactg gagcttctgt gcagcttcca gtgccatctt 780

ctacaggcct 790

<210> 143
<211> 19
<212> DNA
<213> Artificial Sequence

<220>
<223> forward primer for amplification of TRDH-344 DNA

<400> 143
agggtagaag tggagtctg 19

<210> 144
<211> 20
<212> DNA
<213> Artificial Sequence

<220>
<223> reverse primer for amplification of TRDH-344 DNA

<400> 144
caaaggcaca ttgtgaggga 20

<210> 145
<211> 20
<212> DNA
<213> Artificial Sequence

<220>
<223> forward primer for amplification of TRDH-271 DNA

<400> 145
tgaagtagga atgctggtct                                                    20


<210> 146
<211> 20
<212> DNA
<213> Artificial Sequence

<220>
<223> reverse primer for amplification of TRDH-271 DNA

<400> 146
acgatgtact ccaccagctt                                                    20


<210> 147
<211> 19
<212> DNA
<213> Artificial Sequence

<220>

<223> forward primer for amplification of TRDH-284 DNA

<400> 147
agaacatgcc actggtcgt                                                    19

<210> 148
<211> 19
<212> DNA
<213> Artificial Sequence

<220>
<223> reverse primer for amplification of TRDH-284 DNA

<400> 148
acagtgcaga ccgatctca                                                    19

<210> 149
<211> 21
<212> DNA
<213> Artificial Sequence

<220>
<223> forward primer for amplification of TRDH-363 DNA

<400> 149
gggtatggga tgacctgaac a                                                 21

<210> 150

<211> 21
<212> DNA
<213> Artificial Sequence

<220>
<223> reverse primer for amplification of TRDH-363 DNA

<400> 150
agcacaggta ctgcagggat g                                                21

<210> 151
<211> 18
<212> DNA
<213> Artificial Sequence

<220>
<223> forward primer for amplification of TRDH-292 DNA

<400> 151
ggcgtccgac gatgccaa                                                    18

<210> 152
<211> 20
<212> DNA
<213> Artificial Sequence

<220>
<223> reverse primer for amplification of TRDH-292 DNA

<400> 152
gccctacaga gtcttacaca                                                    20

<210> 153
<211> 20
<212> DNA
<213> Artificial Sequence

<220>
<223> forward primer for amplification of TRDH-122 DNA

<400> 153
gagcaaggtc cttccatagt                                                    20

<210> 154
<211> 20
<212> DNA
<213> Artificial Sequence

<220>
<223> reverse primer for amplification of TRDH-122 DNA

<400> 154
atgtcagcag gagtgggtta                                                    20

<210> 155
<211> 20
<212> DNA

<213> Artificial Sequence

<220>
<223> reverse primer for amplification of TRDH-110 DNA

<400> 155
agattgtccc aacagagagg 20

<210> 156
<211> 20
<212> DNA
<213> Artificial Sequence

<220>
<223> reverse primer for amplification of TRDH-110 DNA

<400> 156
gacaggaaat ggtgatgcta 20

<210> 157
<211> 883
<212> DNA
<213> Rattus norvegicus

<400> 157
gccggcaggg ggcactgcgc gccgggcatg gagtgcgtga agagccgcaa gaggcggaag 60

ggtaaagccg gggcagcagc cggcggtccc gcgaccctcg ccgtgtgcgt gtgcaagagc 120

cgctacccgg tgtgcggcag cgacggcgtc acctacccca gcggctgcca gctgcgcgcc 180

gccagcctgc gcgctgagag ccgcggagag aaggccatca cccaggtcag caaaggcacc 240

tgcgagcaag gtccttccat agtgacgccc cccaaggaca tctggaacat cactggcgcc 300

aaggtgtact tgagctgcga agtcatcgga atcccaaccc ccgtcctcat ctggaacaag 360

gtaaaaaggg atcactctgg agttcaaagg acagaactct tgcctggtga ccgggaaaac 420

ctggccattc agacccgggg tggtccagaa aagcatgaag taactggctg ggtgctggta 480

tctcctctaa gtaaggaaga cactggagaa tacgagtgcc acgcgtccaa ttcccaagga 540

caggcttcag cgtcggccaa aattacagtg gttgatgcca tacacgaaat accagtgaaa 600

aaaggtgaag gtgctcagct ataaacctgc gaatacatta gcctctgtag ctgacgcgct 660

ctcagacagc tgacagctgt aaccccactc ctgcctgaca tattcctttg aacctaacac 720

actaacactt tattacagcc agctgatttt acagagaaat caaagataac acataagact 780

atctacaaaa gtttattgtt tatttacaga aaaagcatgc agagctttaa acaaaacaaa 840

taaaattctt attacaacag gaaaaaaaaa aaaaaaaaaa aaa 883

<210> 158
<211> 207
<212> PRT
<213> Rattus norvegicus

<400> 158
Ala Gly Arg Gly His Cys Ala Pro Gly Met Glu Cys Val Lys Ser Arg
1               5                   10                  15

Lys Arg Arg Lys Gly Lys Ala Gly Ala Ala Ala Gly Gly Pro Ala Thr
            20                  25                  30

Leu Ala Val Cys Val Cys Lys Ser Arg Tyr Pro Val Cys Gly Ser Asp
        35                  40                  45

Gly Val Thr Tyr Pro Ser Gly Cys Gln Leu Arg Ala Ala Ser Leu Arg
    50                  55                  60

Ala Glu Ser Arg Gly Glu Lys Ala Ile Thr Gln Val Ser Lys Gly Thr
65                  70                  75                  80

Cys Glu Gln Gly Pro Ser Ile Val Thr Pro Pro Lys Asp Ile Trp Asn
                85                  90                  95

Ile Thr Gly Ala Lys Val Tyr Leu Ser Cys Glu Val Ile Gly Ile Pro
            100                 105                 110

Thr Pro Val Leu Ile Trp Asn Lys Val Lys Arg Asp His Ser Gly Val
        115                 120                 125

Gln Arg Thr Glu Leu Leu Pro Gly Asp Arg Glu Asn Leu Ala Ile Gln
    130                 135                 140

Thr Arg Gly Gly Pro Glu Lys His Glu Val Thr Gly Trp Val Leu Val
145                 150                 155                 160

Ser Pro Leu Ser Lys Glu Asp Thr Gly Glu Tyr Glu Cys His Ala Ser
                165                 170                 175

Asn Ser Gln Gly Gln Ala Ser Ala Ser Ala Lys Ile Thr Val Val Asp
            180                 185                 190

Ala Ile His Glu Ile Pro Val Lys Lys Gly Glu Gly Ala Gln Leu
        195                 200                 205

<210> 159
<211> 1120
<212> DNA
<213> Homo sapiens

<220>
<221> CDS
<222> (211)..(1086)

<400> 159
ctgggccagc tggtggtgcc cagcaaagcc aaggcagaga aacccccact gtcggcctcc 60

tcaccccagc agcgcccccc agagcctgag accggtgaga gtgcgggcac atcccgggct 120

gccacgcccc tgccctctct gagggtggaa gcggaggctg ggggctcagg ggccaggacc 180

cctccactgt cccggaggaa agctgtagac atg cgg ctg cgg atg gag ttg ggt 234
Met Arg Leu Arg Met Glu Leu Gly
1               5

gct cca gaa gag atg ggg cag gtg ccc cca ctt gac tct cgc ccc agc 282

Ala Pro Glu Glu Met Gly Gln Val Pro Pro Leu Asp Ser Arg Pro Ser
    10                  15                  20

tcc cca gcc ctc tac ttc acc cac gat gcc agc ctg gtt cac aaa tct 330
Ser Pro Ala Leu Tyr Phe Thr His Asp Ala Ser Leu Val His Lys Ser
25                  30                  35                  40

cca gac ccc ttc gga gca gta gca gct cag aag ttc agc ctg gcc cac 378
Pro Asp Pro Phe Gly Ala Val Ala Ala Gln Lys Phe Ser Leu Ala His
                45                  50                  55

tcc atg ttg gcc atc agt ggt cac cta gac agc gac gat gat agt ggc 426
Ser Met Leu Ala Ile Ser Gly His Leu Asp Ser Asp Asp Asp Ser Gly
            60                  65                  70

tcc gga agc ctg gtt ggc att gac aac aaa atc gag caa gcc atg gac 474
Ser Gly Ser Leu Val Gly Ile Asp Asn Lys Ile Glu Gln Ala Met Asp
        75                  80                  85

ttg gtg aag tcc cac ctc atg ttt gcg gtc cgg gag gag gtg gag gtg 522
Leu Val Lys Ser His Leu Met Phe Ala Val Arg Glu Glu Val Glu Val
    90                  95                  100

ctg aag gag cag atc cgg gaa ctg gcg gag cgg aac gct gcg ctg gag 570
Leu Lys Glu Gln Ile Arg Glu Leu Ala Glu Arg Asn Ala Ala Leu Glu
105                 110                 115                 120

cag gag aat ggg ctg ctg cgc gcc ctg gcc agc ccg gag cag ctg gct 618
Gln Glu Asn Gly Leu Leu Arg Ala Leu Ala Ser Pro Glu Gln Leu Ala
                125                 130                 135

cag ctg gcc ctc ctc ggg ggt ccc acg gct tgg gcc ccc tgc gcc caa 666
Gln Leu Ala Leu Leu Gly Gly Pro Thr Ala Trp Ala Pro Cys Ala Gln
            140                 145                 150

tgg gcc ctc cgt ctg agc ctc cct tcc ctt aca atg tgc ctt tgg ggc 714
Trp Ala Leu Arg Leu Ser Leu Pro Ser Leu Thr Met Cys Leu Trp Gly
        155                 160                 165

tgc ccg gcc ttg cgt cag ccg cct gcc ccc tct tcc tat gca gct tta 762
Cys Pro Ala Leu Arg Gln Pro Pro Ala Pro Ser Ser Tyr Ala Ala Leu
    170                 175                 180

atg tcc ccg tgt ccc cgg ggt ggg agt tca agg ctc agt aat ggc ctg 810
Met Ser Pro Cys Pro Arg Gly Gly Ser Ser Arg Leu Ser Asn Gly Leu
185                 190                 195                 200

gtc ccc cgg ccc ctg ccc cat ctc ctc atc atc ccc agc ctt gat gga 858
Val Pro Arg Pro Leu Pro His Leu Leu Ile Ile Pro Ser Leu Asp Gly
                205                 210                 215

gga ggg agg gct tca gga cgg ggc gtc aga ggg agc ccc ctc tgg gag 906
Gly Gly Arg Ala Ser Gly Arg Gly Val Arg Gly Ser Pro Leu Trp Glu
            220                 225                 230

gga acc aac ccc cac cct ccc tcc tgg gac ccc cca gca gta gac ggc 954
Gly Thr Asn Pro His Pro Pro Ser Trp Asp Pro Pro Ala Val Asp Gly
        235                 240                 245

ttg ggg gag tcg gag gct ccc cgg cag aca ccc cac ccc cat ctt gtt 1002
Leu Gly Glu Ser Glu Ala Pro Arg Gln Thr Pro His Pro His Leu Val
    250                 255                 260

ccc ttg agg tgc ctc ctc tcc tct gcc cag ggg agg gag tgt gga cag 1050
Pro Leu Arg Cys Leu Leu Ser Ser Ala Gln Gly Arg Glu Cys Gly Gln
265                 270                 275                 280

tat ctg gaa gtt ctg gga ttc agg ttg tta tta aaa taataataat 1096
Tyr Leu Glu Val Leu Gly Phe Arg Leu Leu Leu Lys
                285                 290

aattaaaaac tctgaagaaa cttg 1120

<210> 160
<211> 292
<212> PRT
<213> Homo sapiens

<400> 160
Met Arg Leu Arg Met Glu Leu Gly Ala Pro Glu Glu Met Gly Gln Val
1               5                   10                  15

Pro Pro Leu Asp Ser Arg Pro Ser Ser Pro Ala Leu Tyr Phe Thr His
            20                  25                  30

Asp Ala Ser Leu Val His Lys Ser Pro Asp Pro Phe Gly Ala Val Ala
        35                  40                  45

Ala Gln Lys Phe Ser Leu Ala His Ser Met Leu Ala Ile Ser Gly His
    50                  55                  60

Leu Asp Ser Asp Asp Asp Ser Gly Ser Gly Ser Leu Val Gly Ile Asp

65                  70                  75                  80

Asn Lys Ile Glu Gln Ala Met Asp Leu Val Lys Ser His Leu Met Phe
                85                  90                  95

Ala Val Arg Glu Glu Val Glu Val Leu Lys Glu Gln Ile Arg Glu Leu
            100                 105                 110

Ala Glu Arg Asn Ala Ala Leu Glu Gln Glu Asn Gly Leu Leu Arg Ala
        115                 120                 125

Leu Ala Ser Pro Glu Gln Leu Ala Gln Leu Ala Leu Leu Gly Gly Pro
    130                 135                 140

Thr Ala Trp Ala Pro Cys Ala Gln Trp Ala Leu Arg Leu Ser Leu Pro
145                 150                 155                 160

Ser Leu Thr Met Cys Leu Trp Gly Cys Pro Ala Leu Arg Gln Pro Pro
                165                 170                 175

Ala Pro Ser Ser Tyr Ala Ala Leu Met Ser Pro Cys Pro Arg Gly Gly
            180                 185                 190

Ser Ser Arg Leu Ser Asn Gly Leu Val Pro Arg Pro Leu Pro His Leu
        195                 200                 205

Leu Ile Ile Pro Ser Leu Asp Gly Gly Gly Arg Ala Ser Gly Arg Gly
    210                 215                 220

Val Arg Gly Ser Pro Leu Trp Glu Gly Thr Asn Pro His Pro Pro Ser
225                 230                 235                 240

Trp Asp Pro Pro Ala Val Asp Gly Leu Gly Glu Ser Glu Ala Pro Arg
                245                 250                 255

Gln Thr Pro His Pro His Leu Val Pro Leu Arg Cys Leu Leu Ser Ser
            260                 265                 270

Ala Gln Gly Arg Glu Cys Gly Gln Tyr Leu Glu Val Leu Gly Phe Arg
        275                 280                 285

Leu Leu Leu Lys
    290

<210> 161
<211> 66
<212> DNA
<213> Artificial Sequence

<220>
<223> primer with T7 promoter and poly thymidylate sequence

<400> 161
aaacgacggc cagtgaattg taatacgact cactataggg cgtttttttt tttttttttt 60
tttttt 66

<210> 162
<211> 19
<212> DNA
<213> Artificial Sequence

<220>
<223> forward primer for amplification of G3PDH DNA

<400> 162
atcaccatct tccaggagc                                                    19

<210> 163
<211> 21
<212> DNA
<213> Artificial Sequence

<220>
<223> reverse primer for amplification of G3PDH DNA

<400> 163
caccttcttg atgtcatcat a                                                 21